# EUROPEAN PATENT SPECIFICATION

(11) **EP 2 776 398 B1**
(45) Date of publication and mention of the grant of the patent: **02.09.2020**
(21) Application number: 12781340.0
(22) Date of filing: 08.11.2012
(51) Int. Cl.: A61P 1/00, A61P 3/04, A61P 7/00, A61P 9/00, A61P 11/00, A61P 13/00, A61P 15/00, A61P 17/00, A61P 17/04, A61P 19/02, A61P 19/10, A61P 23/02, A61P 25/00

(54) **SUBSTITUTED PYRAZOLYL-BASED UREA DERIVATIVES AS VANILLOID RECEPTOR LIGANDS**
SUBSTITUIERTE PYRAZOLYL HARNSTOFFDERIVATE ALS VANILLOID-REZEPTORLIGANDEN
DÉRIVÉS D'URÉE À BASE DE PYRAZOLYL TELS QUE LES LIGANDS DU RÉCEPTEUR VANILLOIDE

(30) Priority: 09.11.2011 EP 11008914
(43) Date of publication of application: 17.09.2014
(73) Proprietor: Medifron DBT Inc., Gangseo-gu Seoul, 157-779 (KR)
(72) Inventor: FRANK-FOLTYN, Robert, 52070 Aachen (DE); CHRISTOPH, Thomas, 52080 Aachen (DE); SCHIENE, Klaus, 41363 Jüchen (DE); DE VRY, Jean, B-2200 Herentals (BE); DAMANN, Nils, 50939 Köln (DE); LESCH, Bernhard, 52076 Aachen (DE); BAHRENBERG, Gregor, 52156 Monschau-Konzen (DE); SAUNDERS, Derek John, 52072 Aachen (DE); STOCKHAUSEN, Hannelore, 52393 Hürtgenwald (DE); KIM, Yong-Soo, Yangsan Gyengnam (KR); KIM, Myeong-Seop, Yeongwol-gun Gangw-do (KR); LEE, Jeewoo, Seoul 151-742 (KR)
(74) Representative: Brosch, Oliver
(86) International application number: PCT/EP2012/072140
(87) International publication number: WO 2013/068461

(56) References cited:
- WO-A1-2010/127856

## Description

### FIELD OF THE INVENTION

The invention relates to substituted pyrazolyl-based urea derivatives bearing a phenyl moiety substituted with an O-containing group as vanilloid receptor ligands, to pharmaceutical compositions containing these compounds and also to these compounds for use in the treatment and/or prophylaxis of pain and further diseases and/or disorders.

### BACKGROUND OF THE INVENTION

The treatment of pain, in particular of neuropathic pain, is very important in medicine. There is a worldwide demand for effective pain therapies. The urgent need for action for a patient-focused and target-oriented treatment of chronic and non-chronic states of pain, this being understood to mean the successful and satisfactory treatment of pain for the patient, is also documented in the large number of scientific studies which have recently appeared in the field of applied analgesics or basic research on nociception.

The subtype 1 vanilloid receptor (VR1/TRPV1), which is often also referred to as the capsaicin receptor, is a suitable starting point for the treatment of pain, in particular of pain selected from the group consisting of acute pain, chronic pain, neuropathic pain and visceral pain. This receptor is stimulated inter alia by vanilloids such as capsaicin, heat and protons and plays a central role in the formation of pain. In addition, it is important for a large number of further physiological and pathophysiological processes and is a suitable target for the therapy of a large number of further disorders such as, for example, migraine, depression, neurodegenerative diseases, cognitive disorders, states of anxiety, epilepsy, coughs, diarrhoea, pruritus, inflammations, disorders of the cardiovascular system, eating disorders, medication dependency, misuse of medication and urinary incontinence.

Compounds which have an affinity for the subtype 1 vanilloid receptor (VR1/TRPV1) are e.g. known from WO 2010/127855-A2 and WO 2010/127856-A2.

There is a demand for further compounds having comparable or better properties, not only with regard to affinity to vanilloid receptors 1 (VR1/TRPV1 receptors) per se (*potency, efficacy*)*.* Thus, it may be advantageous to improve the metabolic stability, the solubility in aqueous media or the permeability of the compounds. These factors can have a beneficial effect on oral bioavailability or can alter the PK/PD (pharmacokinetic/pharmacodynamic) profile; this can lead to a more beneficial period of effectiveness, for example.

A weak or non-existent interaction with transporter molecules, which are involved in the ingestion and the excretion of pharmaceutical compositions, is also to be regarded as an indication of improved bioavailability and at most low interactions of pharmaceutical compositions. Furthermore, the interactions with the enzymes involved in the decomposition and the excretion of pharmaceutical compositions should also be as low as possible, as such test results also suggest that at most low interactions or no interactions at all, of pharmaceutical compositions are to be expected.

It was therefore an object of the invention to provide novel compounds, preferably having advantages over the prior-art compounds. The compounds should be suitable in particular as pharmacological active ingredients in pharmaceutical compositions, preferably in pharmaceutical compositions for the treatment and/or prophylaxis of disorders or diseases which are at least partially mediated by vanilloid receptors 1 (VR1/TRPV1 receptors).

This object is achieved by the subject matter described herein.

It has surprisingly been found that the compounds as defined in the claims display outstanding affinity to the subtype 1 vanilloid receptor (VR1/TRPV1 receptor) and are therefore particularly suitable for the prophylaxis and/or treatment of disorders or diseases which are at least partially mediated by vanilloid receptors 1 (VR1/TRPV1).

Particularly suitable are substituted compounds of general formula (Q), as given below, that in addition to their activity with regard to the VR1-receptor show one or more additional advantageous properties, for example, suitable potency, suitable efficacy, no increase in body temperature and/or heat pain threshold; appropriate solubility in biologically relevant media such as aqueous media, in particular in aqueous media at a physiologically acceptable pH value, such as in buffer systems, for instance in phosphate buffer systems; suitable metabolic stability and diversity (e.g. sufficient stability towards the oxidative capabilities of hepatic enzymes such as cytochrome P450 (CYP) enzymes and sufficient diversity with regard to the metabolic elimination via these enzymes); and the like.

In the following, the present invention is defined by the appended claims. The embodiments not falling under the scope of the claims are labelled as part of the disclosure.

The present disclosure therefore relates to a substituted compound of general formula (Q), 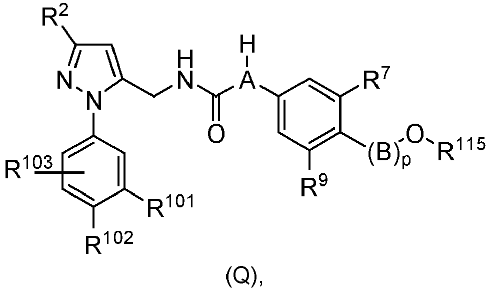 in which
R¹⁰¹, R¹⁰² and R¹⁰³ are independently of one another selected from the group consisting of H, F, Cl, Br, CFH₂, CF₂H, CF₃, CN, CH₂-OH, CH₂CH₂-OH, CH₂-OCH₃, CH₂CH₂-OCH₃, OCFH₂, OCF₂H, OCF₃, OH, NH₂, a C₁₋₄ alkyl, an O-C₁₋₄ alkyl, a NH-C₁₋₄ alkyl, and a N(C₁₋₄ alkyl)₂, wherein the C₁₋₄ alkyl is in each case unsubstituted,
R² represents CF₃, an unsubstituted C₁₋₄ alkyl or an unsubstituted C₃₋₆ cycloalkyl,
R⁷ and R⁹ are independently of one another selected from the group consisting of H, F, Cl, Br, CFH₂, CF₂H, CF₃, CN, OH, OCF₃, a C₁₋₄ alkyl, and an O-C₁₋₄ alkyl, wherein the C₁₋₄ alkyl is in each case unsubstituted,
A denotes N, CH or C(CH₃),
p denotes 0 or 1,
R¹¹⁵ represents H or a C₁₋₆ alkyl, which is unsubstituted or mono-, di- or trisubstituted with 1, 2 or 3 substituents independently of one another selected from the group consisting of OH and OCH₃,
B denotes C(R^{116a})(R^{116b}), wherein
   R^{116a} is selected from the group consisting of H, CH₃, CH₂OH and OH, and R^{116b} is selected from the group consisting of H, CH₃ and CH₂OH,
   with the proviso that R^{116a} cannot denote OH, when R¹¹⁵ represents H,
   or R^{116a} and R^{116b} together with the carbon atom connecting them form an unsubstituted C₃₋₆ cycloalkyl or an unsubstituted 3 to 6 membered heterocyclyl, wherein at least one ring member of the heterocyclyl is selected from the group consisting of O, S, N, NH and N(C₁₋₄ alkyl),
or
B denotes C(R^{116c})(R^{116d})-C(R^{117a})(R^{117b}), wherein
   R^{116c} is selected from the group consisting of H, CH₃, CH₂OH and OH, and R^{116d} is selected from the group consisting of H, CH₃ and CH₂OH,
   or R^{116c} and R^{116d} together with the carbon atom connecting them form an unsubstituted C₃₋₆ cycloalkyl or an unsubstituted 3 to 6 membered heterocyclyl, wherein at least one ring member of the heterocyclyl is selected from the group consisting of O, S, N, NH and N(C₁₋₄ alkyl),
   R^{117a} is selected from the group consisting of H, CH₃, CH₂OH and OH, and R^{117b} is selected from the group consisting of H, CH₃ and CH₂OH,
   with the proviso that R^{117a} cannot denote OH, when R¹¹⁵ represents H,
   or R^{117a} and R^{117b} together with the carbon atom connecting them form an unsubstituted C₃₋₆ cycloalkyl or an unsubstituted 3 to 6 membered heterocyclyl, wherein at least one ring member of the heterocyclyl is selected from the group consisting of O, S, N, NH and N(C₁₋₄ alkyl),
optionally in the form of a single stereoisomer or a mixture of stereoisomers, in the form of the free compound and/or a physiologically acceptable salt and/or a physiologically acceptable solvate thereof.

### DETAILED DESCRIPTION

The term "single stereoisomer" preferably means in the sense of the present invention an individual enantiomer or diastereomer. The term "mixture of stereoisomers" means in the sense of this invention the racemate and mixtures of enantiomers and/or diastereomers in any mixing ratio.

The term "physiologically acceptable salt" preferably comprises in the sense of this invention a salt of at least one compound according to the present invention and at least one physiologically acceptable acid or base.

A physiologically acceptable salt of at least one compound according to the present invention and at least one physiologically acceptable acid preferably refers in the sense of this invention to a salt of at least one compound according to the present invention with at least one inorganic or organic acid which is physiologically acceptable - in particular when used in human beings and/or other mammals. Examples of physiologically acceptable acids are: hydrochloric acid, hydrobromic acid, sulphuric acid, methanesulphonic acid, p-toluenesulphonic acid, carbonic acid, formic acid, acetic acid, oxalic acid, succinic acid, tartaric acid, mandelic acid, fumaric acid, maleic acid, lactic acid, citric acid, glutamic acid, saccharic acid, monomethylsebacic acid, 5-oxoproline, hexane-1-sulphonic acid, nicotinic acid, 2, 3 or 4-aminobenzoic acid, 2,4,6-trimethylbenzoic acid, α-lipoic acid, acetyl glycine, hippuric acid, phosphoric acid, aspartic acid. Citric acid and hydrochloric acid are particularly preferred. Hydrochloride salts and citrate salts are therefore particularly preferred salts.

A physiologically acceptable salt of at least one compound according to the present invention and at least one physiologically acceptable base preferably refers in the sense of this invention to a salt of at least one compound according to the present invention as an anion with at least one preferably inorganic cation, which is physiologically acceptable - in particular when used in human beings and/or other mammals. Particularly preferred are the salts of the alkali and alkaline earth metals but also ammonium salts [NHₓR₄₋ₓ]⁺, in which x = 0, 1, 2, 3 or 4 and R represents a branched or unbranched C₁₋₄ alkyl residue, in particular (mono-) or (di)sodium, (mono-) or (di)potassium, magnesium or calcium salts.

The terms "alkyl", "C₁₋₆ alkyl", and "C₁₋₄ alkyl" preferably comprise in the sense of this invention acyclic saturated aliphatic hydrocarbon residues, which can be respectively branched or unbranched and can be unsubstituted or can be mono- or polysubstituted, e.g. mono-, di- or trisubstituted, and which contain 1 to 6 carbon atoms, i.e. 1, 2, 3, 4, 5 or 6 carbon atoms, or 1 to 4, i.e. 1, 2, 3 or 4 carbon atoms, respectively, i.e. C₁₋₆ aliphatic residues and C₁₋₄ aliphatic residues, i.e. C₁₋₆ alkanyls as well as C₁₋₄ alkanyls. Preferred C₁₋₆ alkanyl residues are selected from the group consisting of methyl, ethyl, n-propyl, 2-propyl, n-butyl, isobutyl, sec.-butyl, tert.-butyl, n-pentyl, isopentyl, neopentyl, and n-hexyl. Preferred C₁₋₄ alkanyl residues are selected from the group consisting of methyl, ethyl, n-propyl, 2-propyl, n-butyl, isobutyl, sec.-butyl, and tert.-butyl.

In relation to the terms "alkyl", "C₁₋₆ alkyl" and "C₁₋₄ alkyl", the term "monosubstituted" or "polysubstituted" such as di- or tri-substituted refers in the sense of this invention, with respect to the corresponding residues or groups, to the single substitution or multiple substitution, e.g. disubstitution or trisubstitution, of one or more hydrogen atoms each independently of one another by at least one substituent. The term "polysubstituted" such as di- or tri-substituted with respect to polysubstituted residues and groups such as di- or tri-substituted residues and groups includes the polysubstitution of these residues and groups either on different or on the same atoms, for example trisubstituted on the same carbon atom, as in the case of CF₃ or CH₂CF₃ or at various points, as in the case of CH(OH)-CH₂CH₂-CHCl₂. The multiple substitution can be carried out using the same or using different substituents.

The terms "cycloalkyl" and "C₃₋₆ cycloalkyl" preferably mean for the purposes of this invention cyclic aliphatic (cycloaliphatic) hydrocarbons containing 3, 4, 5, or 6 carbon atoms, i.e. C₃₋₆₋ cycloaliphatic residues, wherein the hydrocarbons are saturated, which are unsubstituted. The cycloalkyl can be bound to the respective superordinate general structure via any desired and possible ring member of the cycloalkyl residue. Preferably, cycloalkyl is selected from the group consisting of cyclopropyl, cyclobutyl, cyclopentyl and cyclohexyl, more preferably from the group consisting of cyclopropyl and cyclobutyl. A particularly preferred cycloalkyl is cyclopropyl.

The terms "heterocyclyl" and "3 to 6 membered heterocyclyl" preferably comprise in the sense of this invention aliphatic saturated (but not aromatic) heterocycloalkyls having 3 to 6, i.e. 3, 4, 5, or 6, ring members, i.e. a heterocyclyl according to the invention is a 3 to 6 membered heterocyclyl, in which at least one, if appropriate also two or three carbon atoms are replaced by a heteroatom or a heteroatom group each selected independently of one another from the group consisting of O, S, N, NH and N(C₁₋₈ alkyl), preferably O, wherein the ring members are unsubstituted. Heterocyclyls are thus heterocycloaliphatic residues. The heterocyclyl can be bound to the superordinate general structure via any desired and possible ring member of the heterocyclyl residue. Heterocyclyl residues from the group comprising azetidinyl, aziridinyl, dithiolanyl, dihydropyrrolyl, dioxanyl, dioxolanyl, dihydropyridinyl, dihydrofuranyl, imidazolidinyl, isoxazolidinyl, morpholinyl, oxiranyl, oxetanyl, pyrrolidinyl, piperazinyl, 4-methylpiperazinyl, piperidinyl, pyrazolidinyl, pyranyl, tetrahydropyrrolyl, tetrahydropyranyl, tetrahydrofuranyl, tetrahydropyridinyl, tetrahydrothiophenyl, thiazolidinyl and thiomorpholinyl are preferred. Particularly preferred are oxiranyl and oxetanyl, most preferred is oxetanyl.

Within the scope of the present invention, the symbol 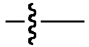 used in the formulae denotes a link of a corresponding residue to the respective superordinate general structure.

In a preferred embodiment of the compound according to the present disclosure
R¹⁰¹, R¹⁰² and R¹⁰³ are independently of one another selected from the group consisting of H, F, Cl, Br, CFH₂, CF₂H, CF₃, CN, CH₂-OH, CH₂-OCH₃, OCF₃, OH, CH₃, CH₂CH₃, CH(CH₃)₂, O-CH₃, O-CH₂CH₃, NH₂, NH(CH₃), and N(CH₃)₂.

Preferably,
R¹⁰¹, R¹⁰² and R¹⁰³ are independently of one another selected from the group consisting of H, F, Cl, Br, CFH₂, CF₂H, CF₃, CN, CH₂-OH, CH₂-OCH₃, OCF₃, OH, CH₃, O-CH₃, O-CH₂CH₃, NH₂, NH(CH₃), and N(CH₃)₂.

More preferably,
R¹⁰¹, R¹⁰² and R¹⁰³ are independently of one another selected from the group consisting of H, F, Cl, CFH₂, CF₂H, CF₃, CN, CH₂-OCH₃, OCF₃, CH₃, O-CH₃, O-CH₂CH₃ and N(CH₃)₂.

Even more preferably,
R¹⁰¹, R¹⁰² and R¹⁰³ are independently of one another selected from the group consisting of H, F, Cl, CFH₂, CF₂H, CF₃, OCF₃, CH₃, O-CH₃, and O-CH₂CH₃.

Still more preferably,
R¹⁰¹, R¹⁰² and R¹⁰³ are independently of one another selected from the group consisting of H, F, Cl, CF₃, OCF₃, CH₃ and O-CH₃.

Particularly,
R¹⁰¹, R¹⁰² and R¹⁰³ are independently of one another selected from the group consisting of H, F, Cl, CF₃ and O-CH₃.

Even more particularly preferred
R¹⁰¹, R¹⁰² and R¹⁰³ are independently of one another selected from the group consisting of H, F, Cl and O-CH₃.

In a preferred embodiment of the compound according to the present disclosure at least one of R¹⁰¹, R¹⁰² and R¹⁰³ is ≠ H.

In another preferred embodiment of the compound according to the present disclosure one or two of R¹⁰¹, R¹⁰² and R¹⁰³, preferably R¹⁰² and/or R¹⁰³, denote(s) H.

In another preferred embodiment of the compound according to the present disclosure one of R¹⁰¹, R¹⁰² and R¹⁰³ represents H, preferably R¹⁰³ represents H.

In another preferred embodiment of the compound according to the present disclosure
R¹⁰¹ and R¹⁰² are independently of one another selected from the group consisting of H, F, Cl, Br, CFH₂, CF₂H, CF₃, CN, CH₂-OH, CH₂-OCH₃, OCF₃, OH, CH₃, CH₂CH₃, CH(CH₃)₂, O-CH₃, O-CH₂CH₃, NH₂, NH(CH₃), and N(CH₃)₂,
and R¹⁰³ represents H.

Preferably,
R¹⁰¹ and R¹⁰² are independently of one another selected from the group consisting of H, F, Cl, Br, CFH₂, CF₂H, CF₃, CN, CH₂-OH, CH₂-OCH₃, OCF₃, OH, CH₃, O-CH₃, O-CH₂CH₃, NH₂, NH(CH₃), and N(CH₃)₂, more preferably are independently of one another selected from the group consisting of H, F, Cl, CFH₂, CF₂H, CF₃, CN, CH₂-OCH₃, OCF₃, CH₃, O-CH₃, O-CH₂CH₃ and N(CH₃)₂, even more preferably are independently of one another selected from the group consisting of H, F, Cl, CFH₂, CF₂H, CF₃, OCF₃, CH₃, O-CH₃, and O-CH₂CH₃, still more preferably are independently of one another selected from the group consisting of H, F, Cl, CF₃, OCF₃, CH₃ and O-CH₃, in particular are independently of one another selected from the group consisting of H, F, Cl, CF₃ and O-CH₃, even more particularly preferred are independently of one another selected from the group consisting of H, F, Cl, and O-CH₃,
and R¹⁰³ represents H.

In yet another preferred embodiment of the compound according to the present disclosure
R¹⁰¹ is selected from the group consisting of F, Cl, Br, CFH₂, CF₂H, CF₃, CN, CH₂-OH, CH₂-OCH₃, OCF₃, OH, CH₃, CH₂CH₃, CH(CH₃)₂, O-CH₃, O-CH₂CH₃, NH₂, NH(CH₃), and N(CH₃)₂,
and both R¹⁰² and R¹⁰³ represents H.

Preferably,
R¹⁰¹ is selected from the group consisting of F, Cl, Br, CFH₂, CF₂H, CF₃, CN, CH₂-OH, CH₂-OCH₃, OCF₃, OH, CH₃, O-CH₃, O-CH₂CH₃, NH₂, NH(CH₃), and N(CH₃)₂, more preferably is selected from the group consisting of F, Cl, CFH₂, CF₂H, CF₃, CN, CH₂-OCH₃, OCF₃, CH₃, O-CH₃, O-CH₂CH₃ and N(CH₃)₂, even more preferably is selected from the group consisting of F, Cl, CFH₂, CF₂H, CF₃, OCF₃, CH₃, O-CH₃, and O-CH₂CH₃, still more preferably is selected from the group consisting of F, Cl, CF₃, OCF₃, CH₃ and O-CH₃, in particular is selected from the group consisting of F, Cl, CF₃ and O-CH₃, even more particularly preferred is selected from the group consisting of F, Cl, and O-CH₃,
and both R¹⁰² and R¹⁰³ represents H.

In still another preferred embodiment of the compound according to the present disclosure
R¹⁰² is selected from the group consisting of F, Cl, Br, CFH₂, CF₂H, CF₃, CN, CH₂-OH, CH₂-OCH₃, OCF₃, OH, CH₃, CH₂CH₃, CH(CH₃)₂, O-CH₃, O-CH₂CH₃, NH₂, NH(CH₃), and N(CH₃)₂,
and both R¹⁰¹ and R¹⁰³ represents H.

Preferably,
R¹⁰² is selected from the group consisting of F, Cl, Br, CFH₂, CF₂H, CF₃, CN, CH₂-OH, CH₂-OCH₃, OCF₃, OH, CH₃, O-CH₃, O-CH₂CH₃, NH₂, NH(CH₃), and N(CH₃)₂, more preferably is selected from the group consisting of F, Cl, CFH₂, CF₂H, CF₃, CN, CH₂-OCH₃, OCF₃, CH₃, O-CH₃, O-CH₂CH₃ and N(CH₃)₂, even more preferably is selected from the group consisting of F, Cl, CFH₂, CF₂H, CF₃, OCF₃, CH₃, O-CH₃, and O-CH₂CH₃, still more preferably is selected from the group consisting of F, Cl, CF₃, OCF₃, CH₃ and O-CH₃, in particular is selected from the group consisting of F, Cl, CF₃ and O-CH₃, even more particularly preferred is selected from the group consisting of F, Cl, and O-CH₃,
and both R¹⁰¹ and R¹⁰³ represents H.

In yet a further preferred embodiment of the compound according to the present disclosure
R¹⁰¹ is selected from the group consisting of F, Cl, Br, CFH₂, CF₂H, CF₃, CN, CH₂-OH, CH₂-OCH₃, OCF₃, OH, CH₃, CH₂CH₃, CH(CH₃)₂, O-CH₃, O-CH₂CH₃, NH₂, NH(CH₃), and N(CH₃)₂,
R¹⁰² is selected from the group consisting of H, F, Cl, Br, CFH₂, CF₂H, CF₃, CN, CH₂-OH, CH₂-OCH₃, OCF₃, OH, CH₃, CH₂CH₃, CH(CH₃)₂, O-CH₃, O-CH₂CH₃, NH₂, NH(CH₃), and N(CH₃)₂,
and R¹⁰³ represents H.

Preferably,
R¹⁰¹ is selected from the group consisting of F, Cl, Br, CFH₂, CF₂H, CF₃, CN, CH₂-OH, CH₂-OCH₃, OCF₃, OH, CH₃, O-CH₃, O-CH₂CH₃, NH₂, NH(CH₃), and N(CH₃)₂, more preferably is selected from the group consisting of F, Cl, CFH₂, CF₂H, CF₃, CN, CH₂-OCH₃, OCF₃, CH₃, O-CH₃, O-CH₂CH₃ and N(CH₃)₂, even more preferably is selected from the group consisting of F, Cl, CFH₂, CF₂H, CF₃, OCF₃, CH₃, O-CH₃, and O-CH₂CH₃, still more preferably is selected from the group consisting of F, Cl, CF₃, OCF₃, CH₃ and O-CH₃, in particular is selected from the group consisting of F, Cl, CF₃ and O-CH₃, even more particularly preferred is selected from the group consisting of F, Cl, and O-CH₃,
R¹⁰² is selected from the group consisting of H, F, Cl, Br, CFH₂, CF₂H, CF₃, CN, CH₂-OH, CH₂-OCH₃, OCF₃, OH, CH₃, O-CH₃, O-CH₂CH₃, NH₂, NH(CH₃), and N(CH₃)₂, more preferably is selected from the group consisting of H, F, Cl, CFH₂, CF₂H, CF₃, CN, CH₂-OCH₃, OCF₃, CH₃, O-CH₃, O-CH₂CH₃ and N(CH₃)₂, even more preferably is selected from the group consisting of H, F, Cl, CFH₂, CF₂H, CF₃, OCF₃, CH₃, O-CH₃, and O-CH₂CH₃, still more preferably is selected from the group consisting of H, F, Cl, CF₃, OCF₃, CH₃ and O-CH₃, in particular is selected from the group consisting of H, F, Cl, CF₃ and O-CH₃, even more particularly preferred is selected from the group consisting of H, F, Cl, and O-CH₃,
and R¹⁰³ represents H.

In another particularly preferred embodiment according to the present disclosure the part structure (QS2) 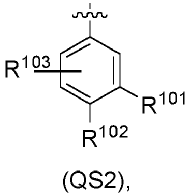 is selected from the group consisting of

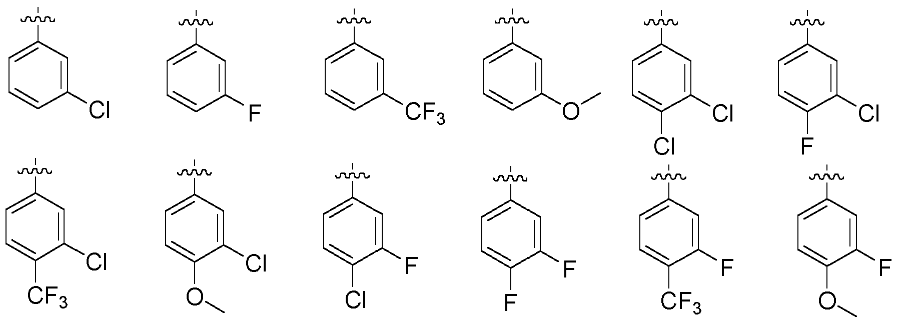

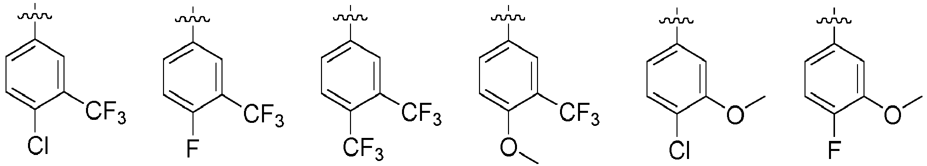

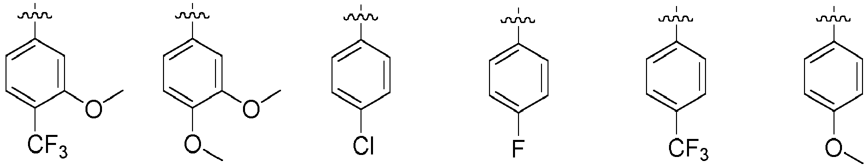

Even more particularly preferred, the part structure (QS2) 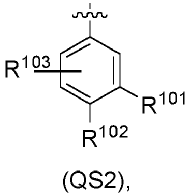 is selected from the group consisting of

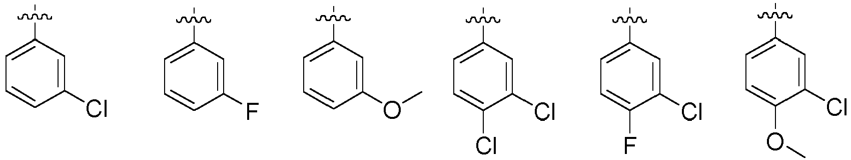

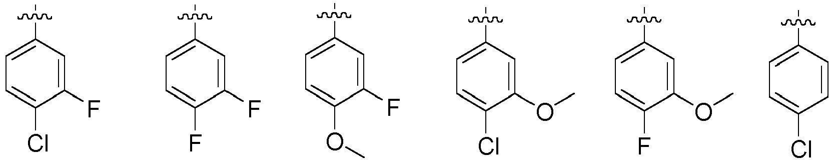

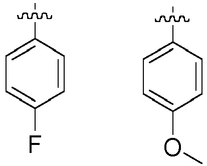

Most preferred, the part structure (QS2) 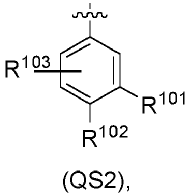 is selected from the group consisting of 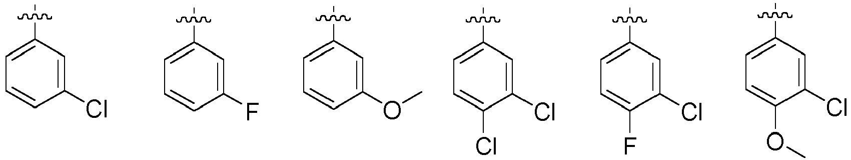 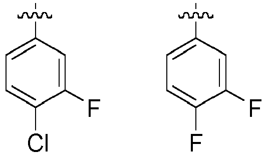 preferably selected from the group consisting of

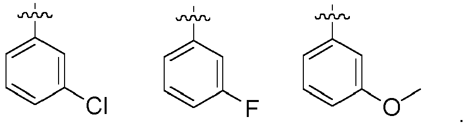

In another preferred embodiment of the compound according to the present disclosure
R² represents CF₃, methyl, ethyl, n-propyl, 2-propyl, n-butyl, iso-butyl, sec.-butyl, tert.-butyl, cyclopropyl, cyclobutyl, cyclopentyl or cyclohexyl.

Preferably,
R² represents CF₃, 2-propyl, n-butyl, iso-butyl, sec.-butyl, tert.-butyl, cyclopropyl, or cyclobutyl.

More preferably,
R² represents CF₃, tert.-butyl or cyclopropyl.

In a particularly preferred embodiment of the compound according to the present disclosure R² represents CF₃.

In another particularly preferred embodiment of the compound according to the present disclosure R² represents tert.-butyl.

In another particularly preferred embodiment of the compound according to the present disclosure R² represents cyclopropyl.

In a further preferred embodiment of the compound according to the present disclosure
R⁷ and R⁹ are independently of one another selected from the group consisting of H, F, Cl, Br, CF₃, CN, OH, OCF₃, CH₃, CH₂CH₃, CH(CH₃)₂, O-CH₃, and O-CH₂CH₃.

Preferably,
R⁷ and R⁹ are independently of one another selected from the group consisting of H, F, Cl, CF₃, CN, OH, OCF₃, CH₃, O-CH₃, and O-CH₂CH₃.

More preferably,
R⁷ and R⁹ are independently of one another selected from the group consisting of H, F, Cl, CF₃, OH, O-CH₃, and O-CH₂CH₃.

Even more preferably,
R⁷ and R⁹ are independently of one another selected from the group consisting of H, F, Cl, and O-CH₃, still more preferably are independently of one another selected from the group consisting of H, F and Cl.

In yet a further preferred embodiment of the compound according to the present disclosure at least one of R⁷ and R⁹ is ≠ H.

In a further preferred embodiment of the compound according to the present disclosure R⁹ denotes H.

In yet another preferred embodiment of the compound according to the present disclosure
R⁷ is selected from the group consisting of F, Cl, Br, CF₃, CN, OH, OCF₃, CH₃, CH₂CH₃, CH(CH₃)₂, O-CH₃, and O-CH₂CH₃, preferably is selected from the group consisting of F, Cl, CF₃, CN, OH, OCF₃, CH₃, O-CH₃, and O-CH₂CH₃, more preferably is selected from the group consisting of F, Cl, CF₃, O-CH₃, and O-CH₂CH₃, even more preferably is selected from the group consisting of F, Cl, and O-CH₃, still more preferably is selected from the group consisting of F and Cl,
and R⁹ represents H.

In another preferred embodiment of the compound according to the present disclosure
A denotes N or C(CH₃).

In a particularly preferred embodiment of the compound according to the present disclosure A denotes N.

In another particularly preferred embodiment of the compound according to the present disclosure A denotes C(CH₃).

In another preferred embodiment of the compound according to the present disclosure the part structure (QS1) 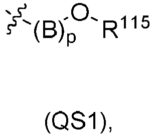 represents
(a) part structure (PQ1) 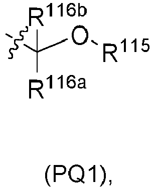 wherein
   R¹¹⁵ represents H, CH₃, CH₂OH, CH₂OCH₃, CH₂CH₃, CH(OH)CH₃, CH₂CH₂OH, CH(OH)CH₂OH, CH(OCH₃)CH₂OH, CH(OH)CH₂OCH₃, or CH₂CH₂OCH₃,
   R^{116a} is selected from the group consisting of H, CH₃, CH₂OH and OH, and R^{116b} is selected from the group consisting of H, CH₃ and CH₂OH,
      with the proviso that R^{116a} cannot denote OH, when R¹¹⁵ represents H,
   or R^{116a} and R^{116b} together with the carbon atom connecting them form an unsubstituted C₃₋₆ cycloalkyl or an unsubstituted 3 to 6 membered heterocyclyl, wherein at least one ring member of the heterocyclyl is selected from the group consisting of O, S, N, NH and N(C₁₋₄ alkyl), preferably one ring member of the heterocyclyl is O,
   or
(b) part structure (PQ2) 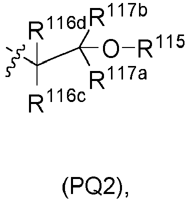 wherein
   R¹¹⁵ represents H, CH₃, CH₂OH, CH₂OCH₃, CH₂CH₃, CH(OH)CH₃, CH₂CH₂OH, CH(OH)CH₂OH, CH(OCH₃)CH₂OH, CH(OH)CH₂OCH₃, or CH₂CH₂OCH₃,
   R^{116c} is selected from the group consisting of H, CH₃, CH₂OH and OH, and R^{116d} is selected from the group consisting of H, CH₃ and CH₂OH,
   or R^{116c} and R^{116d} together with the carbon atom connecting them form an unsubstituted C₃₋₆ cycloalkyl or an unsubstituted 3 to 6 membered heterocyclyl, wherein at least one ring member of the heterocyclyl is selected from the group consisting of O, S, N, NH and N(C₁₋₄ alkyl), preferably one ring member of the heterocyclyl is O,
   R^{117a} is selected from the group consisting of H, CH₃, CH₂OH and OH, and R^{117b} is selected from the group consisting of H, CH₃ and CH₂OH,
      with the proviso that R^{117a} cannot denote OH, when R¹¹⁵ represents H,
   or R^{117a} and R^{117b} together with the carbon atom connecting them form an unsubstituted C₃₋₆ cycloalkyl or an unsubstituted 3 to 6 membered heterocyclyl, wherein at least one ring member of the heterocyclyl is selected from the group consisting of O, S, N, NH and N(C₁₋₄ alkyl), preferably one ring member of the heterocyclyl is O,
   or, when p is 0,
(c) OH, OCH₃, OCH₂CH₃, or part structure (PQ3), preferably part structure (PQ3), 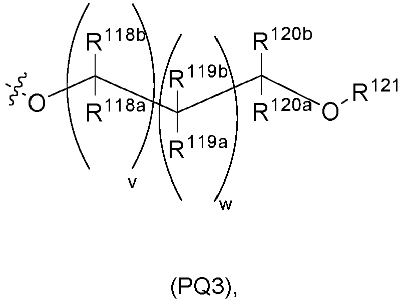 wherein
   - v: denotes 0 or 1,
   - w: denotes 0 or 1,
   - R^{118a}: is selected from the group consisting of H and OH, and
   - R^{118b}: is selected from the group consisting of H and CH₃,
   - R^{119a}: is selected from the group consisting of H and OH, and
   - R^{119b}: is selected from the group consisting of H and CH₃,
   - R^{120a}: is selected from the group consisting of H and OH, and
   - R^{120b}: is selected from the group consisting of H and CH₃,
   - R¹²¹: represents H or CH₃, with the proviso that R^{120a} cannot denote OH, when R¹²¹ represents H.

Preferably,
the part structure (QS1) 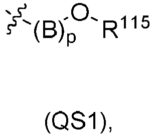 represents
(a) part structure (PQ1) 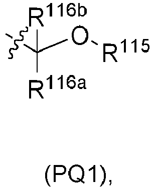 wherein
   R¹¹⁵ represents H, CH₂OH, CH₂CH₂OH, CH(OH)CH₂OH, CH(OCH₃)CH₂OH,
   R^{116a} is selected from the group consisting of H, CH₃, and OH, and R^{116b} is selected from the group consisting of H, and CH₃,
      with the proviso that R^{116a} cannot denote OH, when R¹¹⁵ represents H, and
   or R^{116a} and R^{116b} together with the carbon atom connecting them form an unsubstituted moiety selected from the group consisting of cyclopropyl, cyclobutyl, oxiranyl and oxetanyl,
   or
(b) part structure (PQ2) 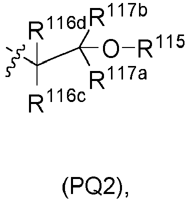 wherein
   R¹¹⁵ represents H, CH₃, CH₂OH, CH₂OCH₃, CH₂CH₃, CH(OH)CH₃, CH₂CH₂OH, CH(OH)CH₂OH, CH(OCH₃)CH₂OH, CH(OH)CH₂OCH₃, or CH₂CH₂OCH₃,
   R^{116c} is selected from the group consisting of H, CH₃, and CH₂OH, and R^{116d} is selected from the group consisting of H, CH₃ and CH₂OH,
   R^{117a} is selected from the group consisting of H, CH₃, CH₂OH and OH, and R^{117b} is selected from the group consisting of H, CH₃ and CH₂OH,
      with the proviso that R^{117a} cannot denote OH, when R¹¹⁵ represents H,
   or, when p is 0,
(c) part structure (PQ3) 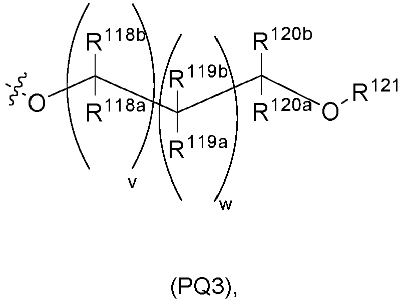 wherein
   - v: denotes 0 or 1,
   - w: denotes 0 or 1,
   - R^{118a}: is selected from the group consisting of H and OH, and
   - R^{118b}: is selected from the group consisting of H and CH₃,
   - R^{119a}: is selected from the group consisting of H and OH, and
   - R^{119b}: is selected from the group consisting of H and CH₃,
   - R^{120a}: is selected from the group consisting of H and OH, and
   - R^{120b}: is selected from the group consisting of H and CH₃,
   - R¹²¹: represents H or CH₃, with the proviso that R^{120a} cannot denote OH, when R¹²¹ represents H.

More preferably, the
part structure (QS1) 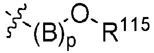 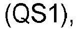 represents
(a) part structure (PQ1) 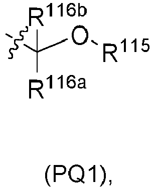 wherein
   R¹¹⁵ represents H, CH₂OH, or CH₂CH₂OH,
   R^{116a} is selected from the group consisting of H, CH₃, and OH, and R^{116b} is selected from the group consisting of H, and CH₃,
      with the proviso that R^{116a} cannot denote OH, when R¹¹⁵ represents H,
   or R^{116a} and R^{116b} together with the carbon atom connecting them form an unsubstituted moiety selected from the group consisting of cyclopropyl, cyclobutyl, oxiranyl and oxetanyl,
   or
(b) part structure (PQ2) 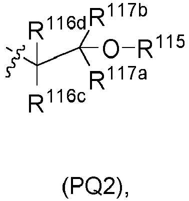 wherein
   R¹¹⁵ represents H, CH₂OH, CH₂CH₂OH, or CH(OH)CH₂OH,
   R^{116c} is selected from the group consisting of H, CH₃, and CH₂OH, and R^{116d} is selected from the group consisting of H, CH₃ and CH₂OH,
   R^{117a} is selected from the group consisting of H, CH₃, CH₂OH and OH, and R^{117b} is selected from the group consisting of H, CH₃ and CH₂OH,
      with the proviso that R^{117a} cannot denote OH, when R¹¹⁵ represents H,
   or, when p is 0,
(c) part structure (PQ3) 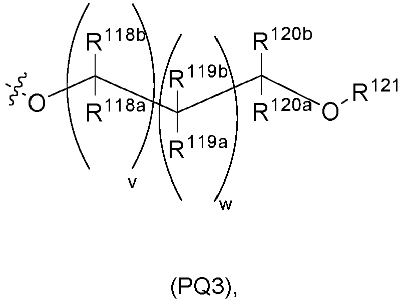 wherein
   - v: denotes 0 or 1,
   - w: denotes 0 or 1,
   - R^{118a}: is selected from the group consisting of H and OH, and
   - R^{118b}: is selected from the group consisting of H and CH₃,
   - R^{119a}: is selected from the group consisting of H and OH, and
   - R^{119b}: is selected from the group consisting of H and CH₃,
   - R^{120a}: is selected from the group consisting of H and OH, and
   - R^{120b}: is selected from the group consisting of H and CH₃,
   - R¹²¹: represents H or CH₃, with the proviso that R^{120a} cannot denote OH, when R¹²¹ represents H.

Even more preferably, part structure (QS1) 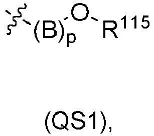 represents
(a) part structure (PQ1) 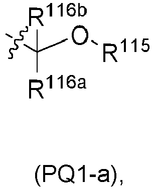 wherein
   R¹¹⁵ represents H, CH₂OH, or CH₂CH₂OH, preferably represents H or CH₂CH₂OH,
   R^{116a} is selected from the group consisting of H, CH₃ and OH, and R^{116b} is selected from the group consisting of H and CH₃,
      with the proviso that R^{116a} cannot denote OH, when R¹¹⁵ represents H,
   or R^{116a} and R^{116b} together with the carbon atom connecting them form an unsubstituted cyclopropyl or an unsubstituted oxetanyl,
   or
(b) part structure (PQ2) 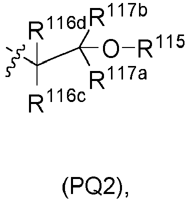 wherein
   R¹¹⁵ represents H, CH₂OH, or CH₂CH₂OH, preferably H,
   R^{116c} is selected from the group consisting of H, CH₃ and CH₂OH, and R^{116d} is selected from the group consisting of H, CH₃ and CH₂OH,
   R^{117a} is selected from the group consisting of H, CH₃, CH₂OH and OH, and R^{117b} is selected from the group consisting of H, CH₃ and CH₂OH,
      with the proviso that R^{117a} cannot denote OH, when R¹¹⁵ represents H,
   or, when p is 0
(c) part structure (PQ3) 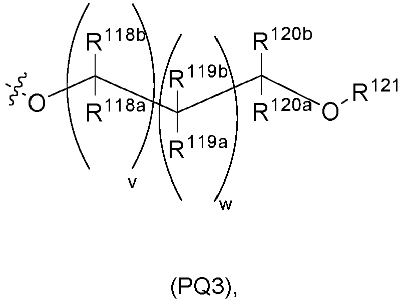 wherein
   - v: denotes 0 or 1,
   - w: denotes 0 or 1,
   - R^{118a}: is selected from the group consisting of H and OH, and
   - R^{118b}: is selected from the group consisting of H and CH₃,
   - R^{119a}: is selected from the group consisting of H and OH, and
   - R^{119b}: is selected from the group consisting of H and CH₃,
   - R^{120a}: is selected from the group consisting of H and OH, and
   - R^{120b}: is selected from the group consisting of H and CH₃,
   - R¹²¹: represents H,
   with the proviso that R^{120a} cannot denote OH, when R¹²¹ represents H.

Still more preferably, the
part structure (QS1) 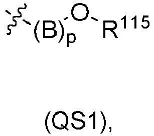 represents
(a) part structure (PQ1-1) 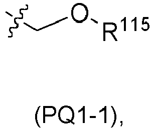 wherein
   R¹¹⁵ represents H, CH₂OH, or CH₂CH₂OH, preferably H or CH₂CH₂OH,
   or
(b) part structure (PQ2-1) 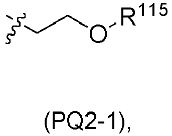 wherein
   R¹¹⁵ represents H, CH₂OH, or CH₂CH₂OH, preferably H,
   or, when p is 0,
(c) part structure (PQ3-1) 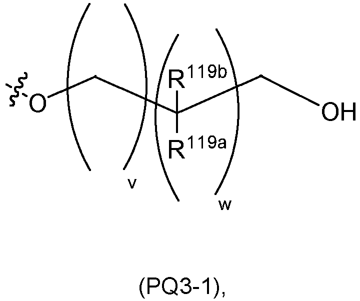 wherein
   - v: denotes 0 or 1,
   - w: denotes 0 or 1,
   - R^{119a}: is selected from the group consisting of H and OH, and
   - R^{119b}: is selected from the group consisting of H and CH₃.

In particular, the
part structure (QS1) 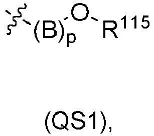 represents
(a) part structure (PQ1-2) or part structure (PQ1-3) 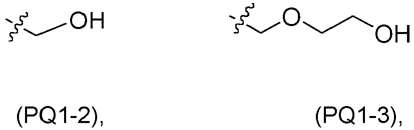
(b) part structure (PQ2-1) 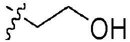 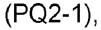 or, when p is 0,
(c) part structure (PQ3-2) 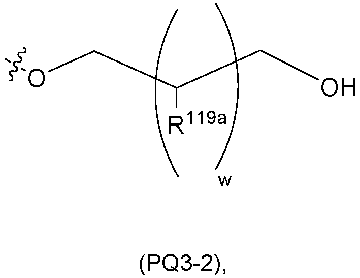 wherein
   w denotes 1, and R^{119a} represents OH,
   or
   w denotes 0.

In a particularly preferred embodiment of the present disclosure,
R¹⁰¹, R¹⁰² and R¹⁰³ are independently of one another selected from the group consisting of H, F, Cl, Br, CFH₂, CF₂H, CF₃, CN, CH₂-OH, CH₂-OCH₃, OCF₃, OH, CH₃, CH₂CH₃, CH(CH₃)₂, O-CH₃, O-CH₂CH₃, NH₂, NH(CH₃), and N(CH₃)₂,
preferably, wherein at least one of R¹⁰¹, R¹⁰² and R¹⁰³ is ≠ H,
R² represents CF₃, tert.-butyl or cyclopropyl,
R⁷ and R⁹ are independently of one another selected from the group consisting of H, F, Cl, Br, CF₃, CN, OCF₃, CH₃, CH₂CH₃, CH(CH₃)₂, O-CH₃, and O-CH₂CH₃,
preferably, wherein at least one of R⁷ and R⁹ is ≠ H,
A denotes N, CH or C(CH₃), and the part structure (QS1) 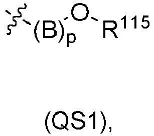 represents
(a) CH₂OH or CH₂O-CH₂CH₂OH,
(b) CH₂CH₂OH, or
(c) O-CH₂CH₂OH or O-CH-CH(OH)-CH₂OH.

Preferred embodiments of the compound according to the disclosure of general formula (Q) have general formulae (Q0-a) and/or (Q0-b): 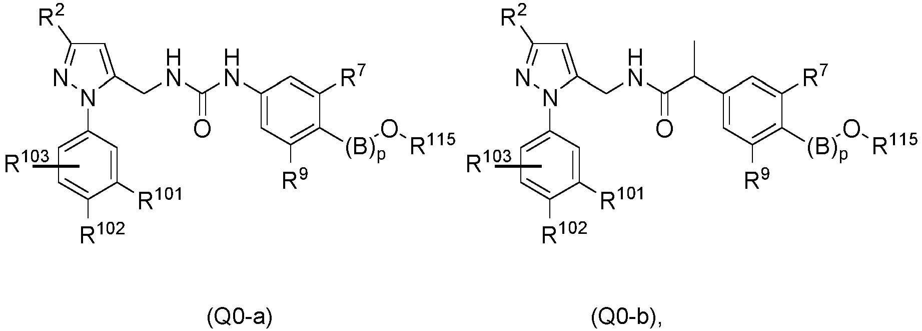 wherein the particular radicals, variables and indices have the meanings described herein in connection with the compounds according to the disclosure and preferred embodiments thereof.

Further preferred embodiments of the compound according to the disclosure of general formula (Q) have general formulae (Q1-a), (Q1-a-1) and/or (Q1-a-2): 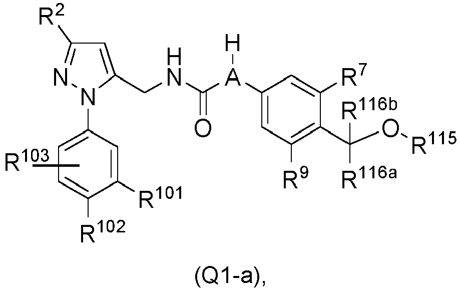 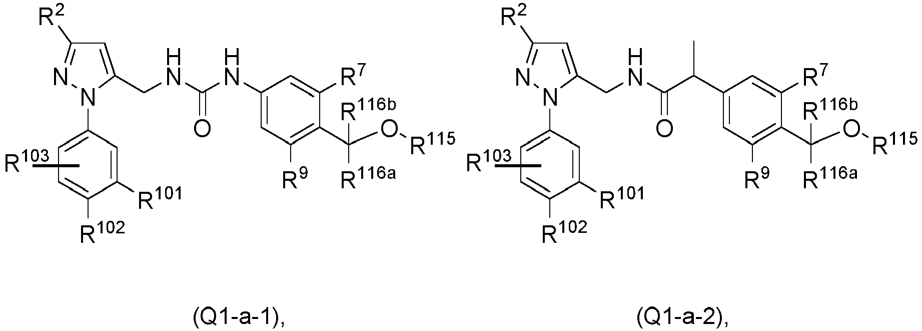 wherein the particular radicals, variables and indices have the meanings described herein in connection with the compounds according to the disclosure and preferred embodiments thereof.

Moreover, preferred embodiments of the compound according to the disclosure of general formula (Q) have general formulae (Q1-b), (Q1-b-1) and/or (Q1-b-2): 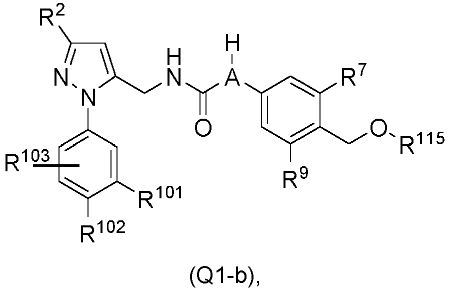 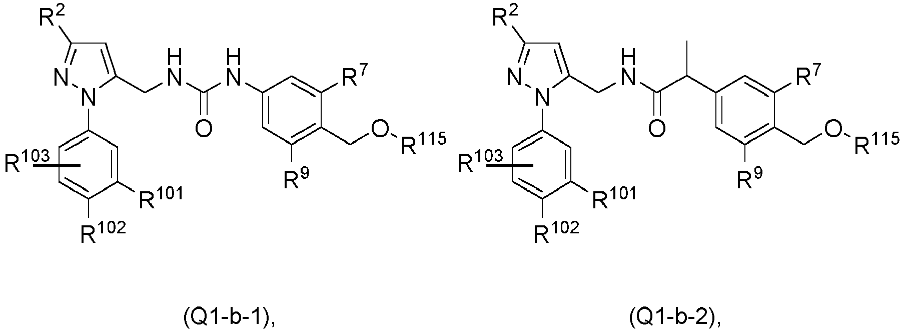 wherein the particular radicals, variables and indices have the meanings described herein in connection with the compounds according to the disclosure and preferred embodiments thereof.

In addition, preferred embodiments of the compound according to the disclosure of general formula (Q) have general formulae (Q1-c), (Q1-c-1) and/or (Q1-c-2): 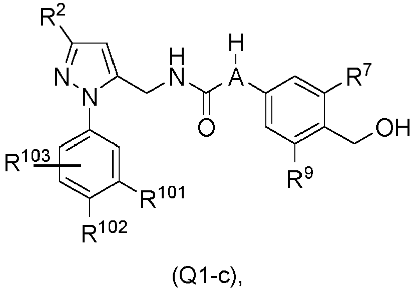 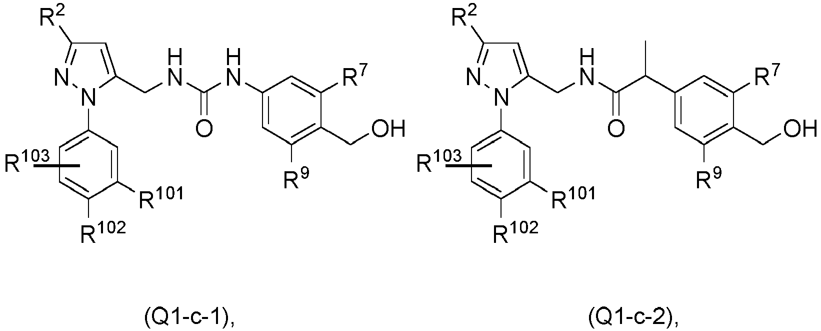 wherein the particular radicals, variables and indices have the meanings described herein in connection with the compounds according to the disclosure and preferred embodiments thereof.

Yet further preferred embodiments of the compound according to the disclosure of general formula (Q) have general formulae (Q1-d), (Q1-d-1) and/or (Q1-d-2): 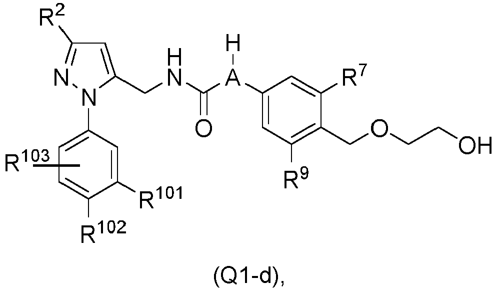 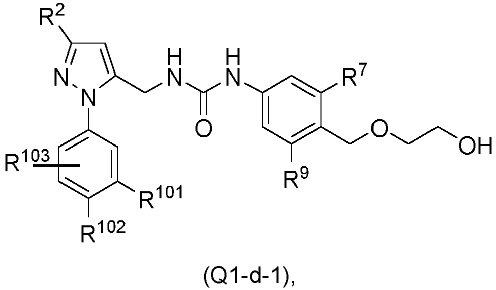 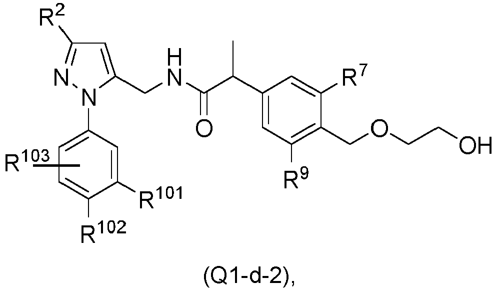 wherein the particular radicals, variables and indices have the meanings described herein in connection with the compounds according to the disclosure and preferred embodiments thereof.

Further preferred embodiments of the compound according to the disclosure of general formula (Q) have general formulae (Q2-a), (Q2-a-1) and/or (Q2-a-2): 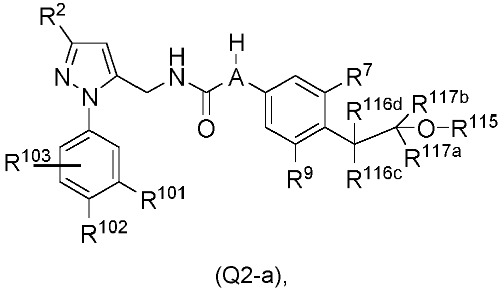 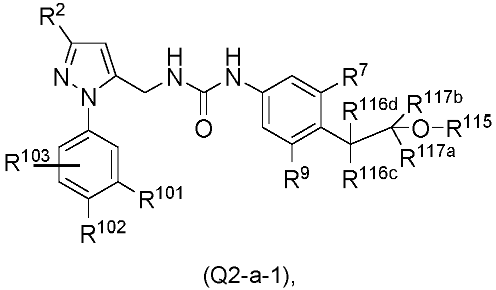 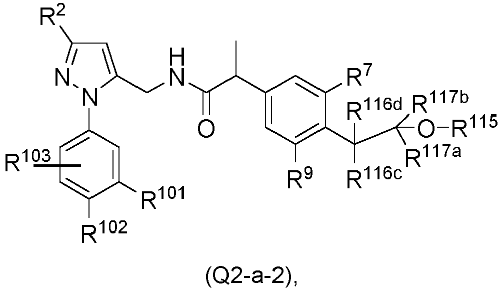 wherein the particular radicals, variables and indices have the meanings described herein in connection with the compounds according to the disclosure and preferred embodiments thereof.

Moreover, preferred embodiments of the compound according to the disclosure of general formula (Q) have general formulae (Q2-b), (Q2-b-1) and/or (Q2-b-2): 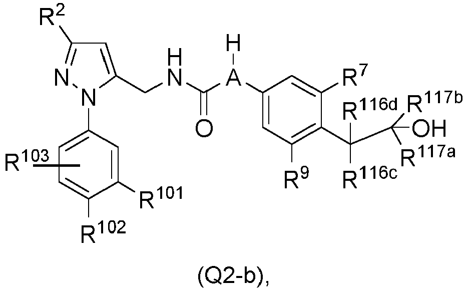 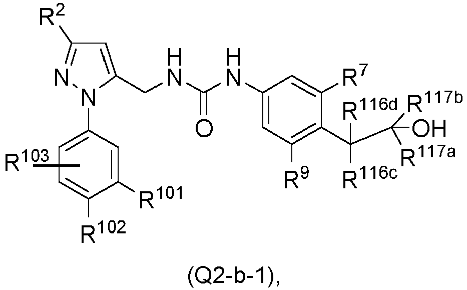 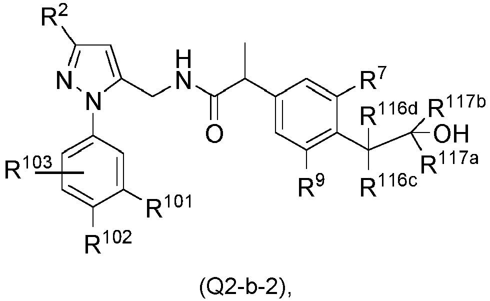 wherein the particular radicals, variables and indices have the meanings described herein in connection with the compounds according to the disclosure and preferred embodiments thereof.

Yet further preferred embodiments of the compound according to the disclosure of general formula (Q) have general formulae (Q2-c), (Q2-c-1) and/or (Q2-c-2): 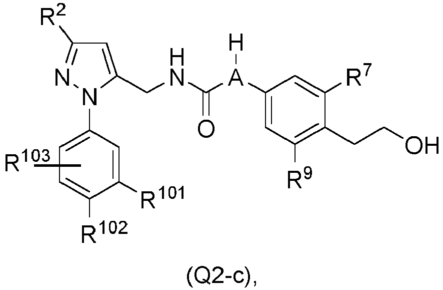 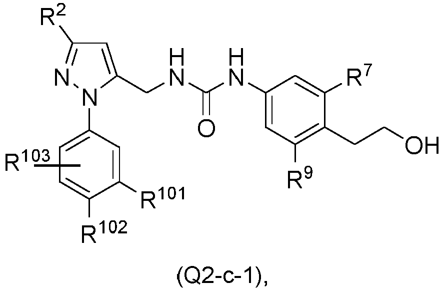 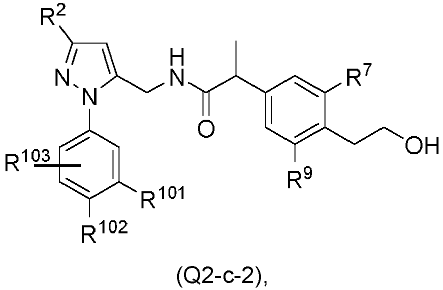 wherein the particular radicals, variables and indices have the meanings described herein in connection with the compounds according to the disclosure and preferred embodiments thereof.

Further preferred embodiments of the compound according to the disclosure of general formula (Q) have general formulae (Q3-a), (Q3-a-1) and/or (Q3-a-2): 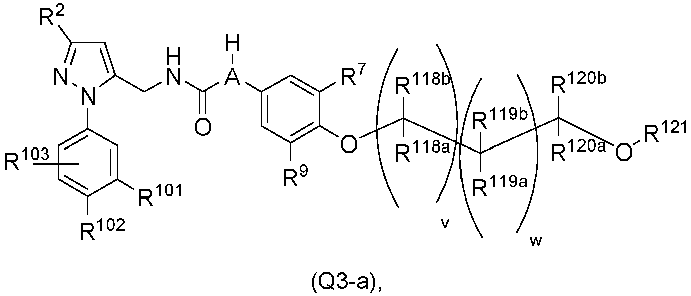 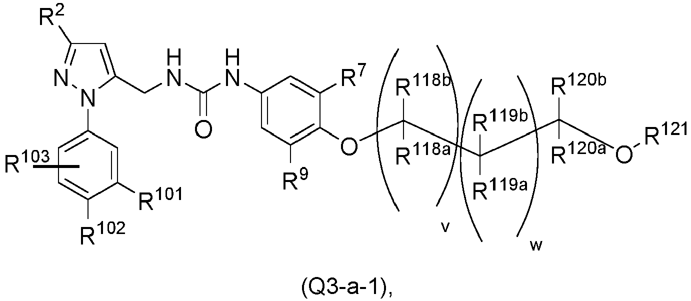 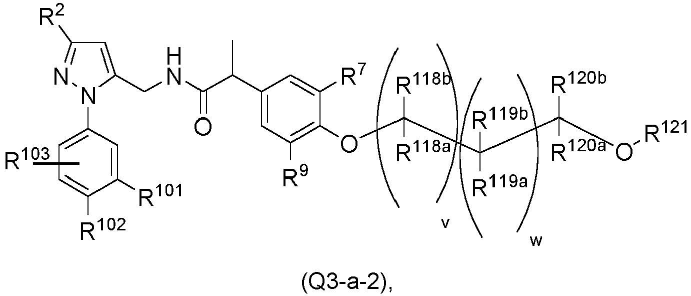 wherein the particular radicals, variables and indices have the meanings described herein in connection with the compounds according to the disclosure and preferred embodiments thereof.

Moreover, preferred embodiments of the compound according to the disclosure of general formula (Q) have general formulae (Q3-b), (Q3-b-1) and/or (Q3-b-2): 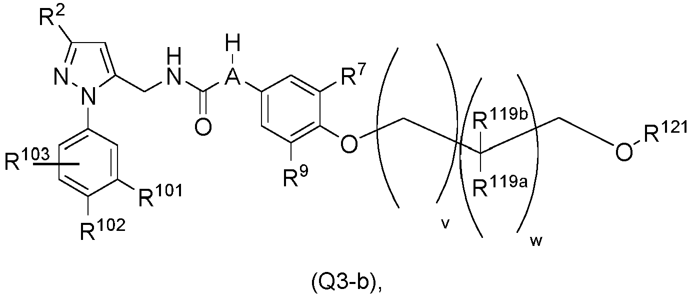 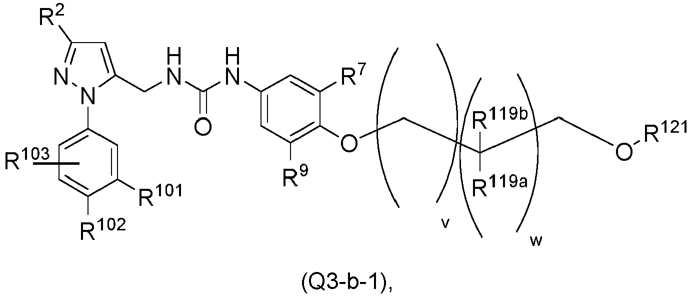 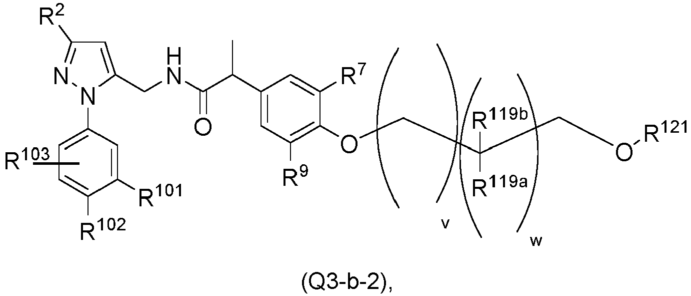 wherein the particular radicals, variables and indices have the meanings described herein in connection with the compounds according to the disclosure and preferred embodiments thereof.

In addition, preferred embodiments of the compound according to the disclosure of general formula (Q) have general formulae (Q3-c), (Q3-c-1) and/or (Q3-c-2): 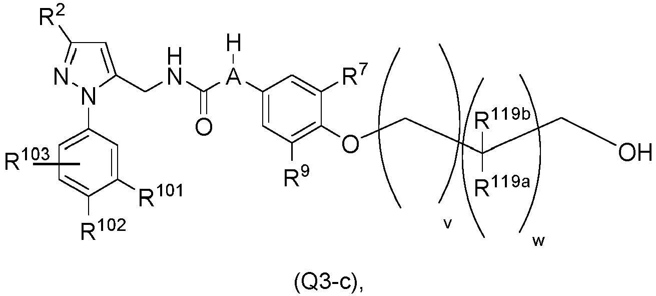 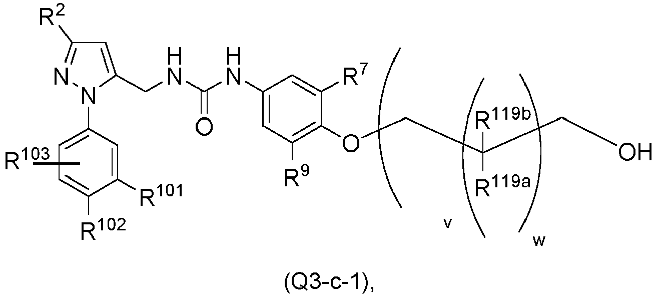 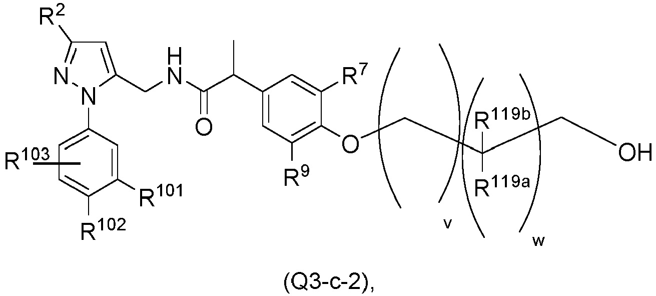 wherein the particular radicals, variables and indices have the meanings described herein in connection with the compounds according to the disclosure and preferred embodiments thereof.

Yet further preferred embodiments of the compound according to the disclosure of general formula (Q) have general formulae (Q3-d), (Q3-d-1) and/or (Q3-d-2): 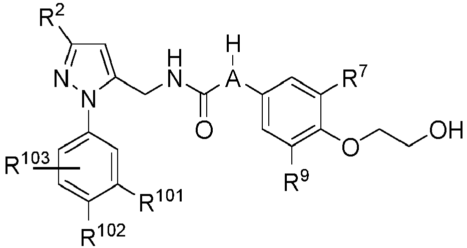 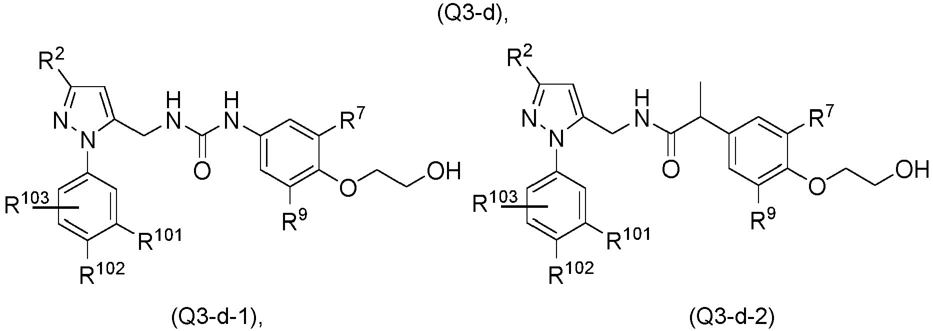 wherein the particular radicals, variables and indices have the meanings described herein in connection with the compounds according to the disclosure and preferred embodiments thereof.

Still further preferred embodiments of the compound according to the disclosure of general formula (Q) have general formulae (Q3-e), (Q3-e-1) and/or (Q3-e-2): 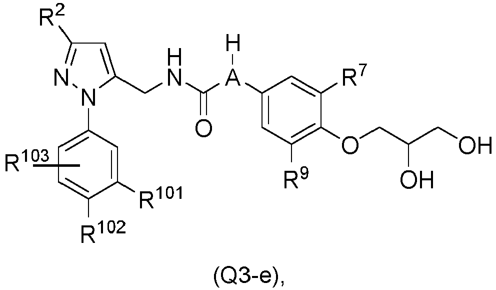 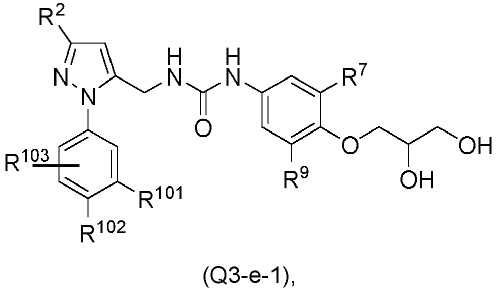 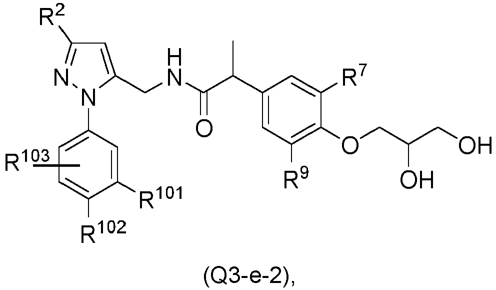 wherein the particular radicals, variables and indices have the meanings described herein in connection with the compounds according to the disclosure and preferred embodiments thereof.

Especially preferred embodiments of the compound according to the disclosure of general formula (Q) have general formulae (Q1-c), (Q1-c-1), (Q1-c-2), (Q1-d), (Q1-d-1), (Q1-d-2), (Q3-d), (Q3-d-1), (Q3-d-2) (Q3-e), (Q3-e-1) and/or (Q3-e-2), wherein the particular radicals, variables and indices have the meanings described herein in connection with the compounds according to the disclosure and preferred embodiments thereof.

In particularly preferred embodiments of the present disclosure radical R¹⁰¹ in the compound of general formula (Q), (Q0-a), (Q0-b), (Q1-a), (Q1-a-1), (Q1-a-2), (Q1-b), (Q1-b-1), (Q1-b-2), (Q1-c), (Q1-c-1), (Q1-c-2), (Q1-d), (Q1-d-1), (Q1-d-2), (Q2-a), (Q2-a-1), (Q2-a-2), (Q2-b), (Q2-b-1), (Q2-b-2), (Q2-c), (Q2-c-1), (Q2-c-2), (Q3-a), (Q3-a-1), (Q3-a-2), (Q3-b), (Q3-b-1), (Q3-b-2), (Q3-c), (Q3-c-1), (Q3-c-2), (Q3-d), (Q3-d-1), (Q3-d-2), (Q3-e), (Q3-e-1) and/or (Q3-e-2) represents F, Cl, CF₃ or O-CH₃, preferably F or Cl, most preferably Cl - preferably when R¹⁰³ is H and R¹⁰² represents H, F, Cl, CF₃ or OCH₃, more preferably when R¹⁰³ is H and R¹⁰² represents H, F or Cl, even more preferably when both R¹⁰² and R¹⁰³ denote H -, and the remaining particular radicals, variables and indices have the meanings described herein in connection with the compounds according to the disclosure and preferred embodiments thereof.

In further particularly preferred embodiments of the present disclosure in the compound of general formula (Q1-b), (Q1-b-1) and/or (Q1-b-2),
- A: denotes N or C(CH₃),
- R¹⁰¹: denotes F, Cl or OCH₃,
- R¹⁰²: denotes H or F,
- R¹⁰³: denotes H,
- R²: represents CF₃, tert.-butyl, or cyclopropyl,
- R⁷: denotes H or F,
- R⁹: represents H, and
- R¹¹⁵: denotes H or CH₂CH₂OH.

In further particularly preferred embodiments of the present disclosure in the compound of general formula (Q3-b), (Q3-b-1) and/or (Q3-b-2),
- A: denotes N or C(CH₃),
- R¹⁰¹: denotes F, Cl or OCH₃,
- R¹⁰²: denotes H or F,
- R¹⁰³: denotes H,
- R²: represents CF₃, tert.-butyl, or cyclopropyl,
- R⁷: denotes H or F,
- R⁹: represents H,
- R^{119a}: denotes H or OH
- R^{119b}: denotes H,
- v: denotes 0 or 1,
- w: denotes 0 or 1, and
- R¹²¹: denotes H.

Particularly preferred are compounds according to the disclosure from the group
- **A1**: N-[[5-tert-Butyl-2-(3-chlorophenyl)-2H-pyrazol-3-yl]-methyl]-2-(3,5-difluoro-4-methoxy-phenyl)-acetamide;
- **A2**: N-[[2-(3-Chlorophenyl)-5-(trifluoromethyl)-2H-pyrazol-3-yl]-methyl]-2-(3,5-difluoro-4-methoxy-phenyl)-acetamide;
- **A3**: N-[[2-(3-Chlorophenyl)-5-cyclopropyl-2H-pyrazol-3-yl]-methyl]-2-(3,5-difluoro-4-methoxy-phenyl)-propionamide;
- **A4**: N-[[5-tert-Butyl-2-(3-chlorophenyl)-2H-pyrazol-3-yl]-methyl]-2-(3,5-difluoro-4-methoxy-phenyl)-propionamide;
- **A5**: N-[[5-tert-Butyl-2-(3-chloro-4-fluoro-phenyl)-2H-pyrazol-3-yl]-methyl]-2-(3,5-difluoro-4-methoxy-phenyl)-propionamide;
- **A6**: N-[[5-tert-Butyl-2-(3-chloro-4-fluoro-phenyl)-2H-pyrazol-3-yl]-methyl]-2-(3,5-difluoro-4-methoxy-phenyl)-acetamide;
- **A7**: N-[[2-(3-Chloro-4-fluoro-phenyl)-5-(trifluoromethyl)-2H-pyrazol-3-yl]-methyl]-2-(3,5-difluoro-4-methoxy-phenyl)-acetamide;
- **A8**: 1-[[2-(3-Chlorophenyl)-5-(trifluoromethyl)-2H-pyrazol-3-yl]-methyl]-3-(3,5-difluoro-4-methoxy-phenyl)-urea;
- **A9**: N-[[2-(3-Chloro-4-fluoro-phenyl)-5-(trifluoromethyl)-2H-pyrazol-3-yl]-methyl]-2-(3,5-difluoro-4-methoxy-phenyl)-propionamide;
- **A10**: N-[[2-(3-Chlorophenyl)-5-(trifluoromethyl)-2H-pyrazol-3-yl]-methyl]-2-(3,5-difluoro-4-hydroxy-phenyl)-propionamide;
- **A11**: 1-[[5-tert-Butyl-2-(3-chlorophenyl)-2H-pyrazol-3-yl]-methyl]-3-(3,5-difluoro-4-hydroxy-phenyl)-urea;
- **A12**: 1-[[2-(3-Chlorophenyl)-5-(trifluoromethyl)-2H-pyrazol-3-yl]-methyl]-3-(3,5-difluoro-4-hydroxy-phenyl)-urea;
- **A13**: 1-[[5-tert-Butyl-2-(3-chlorophenyl)-2H-pyrazol-3-yl]-methyl]-3-(3,4-dimethoxyphenyl)-urea;
- **A14**: 1-[[2-(3-Chlorophenyl)-5-(trifluoromethyl)-2H-pyrazol-3-yl]-methyl]-3-(3,4-dimethoxyphenyl)-urea;
- **A15**: 1-[[2-(3-Chlorophenyl)-5-(trifluoromethyl)-2H-pyrazol-3-yl]-methyl]-3-(4-hydroxy-3-methoxy-phenyl)-urea;
- **A16**: 1-[[2-(3-Chlorophenyl)-5-(trifluoromethyl)-2H-pyrazol-3-yl]-methyl]-3-(3-hydroxy-4-methoxy-phenyl)-urea;
- **A17**: 1-[[5-tert-Butyl-2-(3-chlorophenyl)-2H-pyrazol-3-yl]-methyl]-3-[3-fluoro-4-(2-methoxy-ethoxy)-phenyl]-urea;
- **A18**: 1-[[2-(3-Chlorophenyl)-5-(trifluoromethyl)-2H-pyrazol-3-yl]-methyl]-3-[3-fluoro-4-(2-methoxy-ethoxy)-phenyl]-urea;
- **A19**: 1-[[5-tert-Butyl-2-(3-chlorophenyl)-2H-pyrazol-3-yl]-methyl]-3-[3-fluoro-4-(2-hydroxy-ethoxy)-phenyl]-urea;
- **A20**: 1-[[2-(3-Chlorophenyl)-5-(trifluoromethyl)-2H-pyrazol-3-yl]-methyl]-3-[3-fluoro-4-(2-hydroxy-ethoxy)-phenyl]-urea;
- **A21**: 1-[3-Fluoro-4-(2-hydroxy-ethoxy)-phenyl]-3-[[2-(3-fluorophenyl)-5-(trifluoromethyl)-2H-pyrazol-3-yl]-methyl]-urea;
- **A22**: 1-[[2-(3-Chloro-4-fluoro-phenyl)-5-(trifluoromethyl)-2H-pyrazol-3-yl]-methyl]-3-[3-fluoro-4-(2-hydroxy-ethoxy)-phenyl]-urea;
- **A23**: 1-[[2-(3-Chlorophenyl)-5-(trifluoromethyl)-2H-pyrazol-3-yl]-methyl]-3-[4-(2,3-dihydroxy-propoxy)-phenyl]-urea;
- **A24**: 1-[[5-tert-Butyl-2-(3-chlorophenyl)-2H-pyrazol-3-yl]-methyl]-3-[4-(2,3-dihydroxy-propoxy)-3-fluoro-phenyl]-urea;
- **A25**: 1-[[2-(3-Chlorophenyl)-5-(trifluoromethyl)-2H-pyrazol-3-yl]-methyl]-3-[4-(2,3-dihydroxy-propoxy)-3-fluoro-phenyl]-urea;
- **A26**: 1-[4-(2,3-Dihydroxy-propoxy)-phenyl]-3-[[2-(3-fluorophenyl)-5-(trifluoromethyl)-2H-pyrazol-3-yl]-methyl]-urea;
- **A27**: 1-[[5-tert-Butyl-2-(3-chlorophenyl)-2H-pyrazol-3-yl]-methyl]-3-[4-(hydroxymethyl)-phenyl]-urea;
- **A28**: 1-[[2-(3-Chlorophenyl)-5-(trifluoromethyl)-2H-pyrazol-3-yl]-methyl]-3-[4-(hydroxymethyl)-phenyl]-urea;
- **A29**: N-[[5-tert-Butyl-2-(3-chlorophenyl)-2H-pyrazol-3-yl]-methyl]-2-[3-fluoro-4-(hydroxymethyl)-phenyl]-acetamide;
- **A30**: N-[[2-(3-Chlorophenyl)-5-(trifluoromethyl)-2H-pyrazol-3-yl]-methyl]-2-[3-fluoro-4-(hydroxymethyl)-phenyl]-acetamide
- **A31**: N-[[2-(3-Chlorophenyl)-5-cyclopropyl-2H-pyrazol-3-yl]-methyl]-2-[3-fluoro-4-(hydroxymethyl)-phenyl]-propionamide;
- **A32**: N-[[5-tert-Butyl-2-(3-chlorophenyl)-2H-pyrazol-3-yl]-methyl]-2-[3-fluoro-4-(hydroxymethyl)-phenyl]-propionamide;
- **A33**: N-[[2-(3-Chlorophenyl)-5-(trifluoromethyl)-2H-pyrazol-3-yl]-methyl]-2-[3-fluoro-4-(hydroxymethyl)-phenyl]-propionamide;
- **A34**: (2S)-N-[[2-(3-Chlorophenyl)-5-(trifluoromethyl)-2H-pyrazol-3-yl]-methyl]-2-[3-fluoro-4-(hydroxymethyl)-phenyl]-propionamide;
- **A35**: (2R)-N-[[2-(3-Chlorophenyl)-5-(trifluoromethyl)-2H-pyrazol-3-yl]-methyl]-2-[3-fluoro-4-(hydroxymethyl)-phenyl]-propionamide;
- **A36**: 2-[3-Chloro-4-(hydroxymethyl)-phenyl]-N-[[2-(3-chlorophenyl)-5-(trifluoromethyl)-2H-pyrazol-3-yl]-methyl]-propionamide;
- **A37**: N-[[5-tert-Butyl-2-(3-fluorophenyl)-2H-pyrazol-3-yl]-methyl]-2-[3-fluoro-4-(hydroxymethyl)-phenyl]-propionamide;
- **A38**: 2-[3-Fluoro-4-(hydroxymethyl)-phenyl]-N-[[2-(3-fluorophenyl)-5-(trifluoromethyl)-2H-pyrazol-3-yl]-methyl]-propionamide;
- **A39**: N-[[5-tert-Butyl-2-(3,4-difluoro-phenyl)-2H-pyrazol-3-yl]-methyl]-2-[3-fluoro-4-(hydroxymethyl)-phenyl]-propionamide;
- **A40**: N-[[2-(3,4-Difluoro-phenyl)-5-(trifluoromethyl)-2H-pyrazol-3-yl]-methyl]-2-[3-fluoro-4-(hydroxymethyl)-phenyl]-propionamide;
- **A41**: N-[[5-tert-Butyl-2-(3-chloro-4-fluoro-phenyl)-2H-pyrazol-3-yl]-methyl]-2-[3-fluoro-4-(hydroxymethyl)-phenyl]-propionamide;
- **A42**: N-[[2-(3-Chloro-4-fluoro-phenyl)-5-(trifluoromethyl)-2H-pyrazol-3-yl]-methyl]-2-[3-fluoro-4-(hydroxymethyl)-phenyl]-propionamide;
- **A43**: 2-[3-Fluoro-4-(hydroxymethyl)-phenyl]-N-[[2-(3-methoxyphenyl)-5-(trifluoromethyl)-2H-pyrazol-3-yl]-methyl]-propionamide;
- **A44**: 2-[3-Fluoro-4-(hydroxymethyl)-phenyl]-N-[[2-(3-isopropyl-phenyl)-5-(trifluoromethyl)-2H-pyrazol-3-yl]-methyl]-propionamide;
- **A45**: N-[[5-tert-Butyl-2-(4-fluorophenyl)-2H-pyrazol-3-yl]-methyl]-2-[3-fluoro-4-(hydroxymethyl)-phenyl]-propionamide;
- **A46**: 2-[3-Fluoro-4-(hydroxymethyl)-phenyl]-N-[[2-(4-fluorophenyl)-5-(trifluoromethyl)-2H-pyrazol-3-yl]-methyl]-propionamide;
- **A47**: N-[[5-tert-Butyl-2-[3-(trifluoromethyl)phenyl]-2H-pyrazol-3-yl]-methyl]-2-[3-fluoro-4-(hydroxymethyl)-phenyl]-propionamide;
- **A48**: 2-[3-Fluoro-4-(hydroxymethyl)-phenyl]-N-[[5-(trifluoromethyl)-2-[3-(trifluoromethyl)phenyl]-2H-pyrazol-3-yl]-methyl]-propionamide;
- **A49**: N-[[5-tert-Butyl-2-(3-chlorophenyl)-2H-pyrazol-3-yl]-methyl]-2-[4-(hydroxymethyl)-3-methoxy-phenyl]-propionamide;
- **A50**: N-[[2-(3-Chlorophenyl)-5-(trifluoromethyl)-2H-pyrazol-3-yl]-methyl]-2-[4-(hydroxymethyl)-3-methoxy-phenyl]-propionamide;
- **A51**: N-[[5-tert-Butyl-2-(3-chlorophenyl)-2H-pyrazol-3-yl]-methyl]-2-[3-chloro-4-(hydroxymethyl)-phenyl]-propionamide;
- **A52**: N-[[2-(3-Chlorophenyl)-5-(trifluoromethyl)-2H-pyrazol-3-yl]-methyl]-2-[3,5-difluoro-4-(hydroxymethyl)-phenyl]-propionamide;
- **A53**: 1-[[2-(3-Chlorophenyl)-5-cyclopropyl-2H-pyrazol-3-yl]-methyl]-3-[3-fluoro-4-(hydroxymethyl)-phenyl]-urea;
- **A54**: 1-[[5-tert-Butyl-2-(3-fluorophenyl)-2H-pyrazol-3-yl]-methyl]-3-[3-fluoro-4-(hydroxymethyl)-phenyl]-urea;
- **A55**: 1-[[5-tert-Butyl-2-(3-chlorophenyl)-2H-pyrazol-3-yl]-methyl]-3-[3-fluoro-4-(hydroxymethyl)-phenyl]-urea;
- **A56**: 1-[[5-tert-Butyl-2-(m-tolyl)-2H-pyrazol-3-yl]-methyl]-3-[3-fluoro-4-(hydroxymethyl)-phenyl]-urea;
- **A57**: 1-[[5-tert-Butyl-2-(3,4-difluoro-phenyl)-2H-pyrazol-3-yl]-methyl]-3-[3-fluoro-4-(hydroxymethyl)-phenyl]-urea;
- **A58**: 1-[3-Fluoro-4-(hydroxymethyl)-phenyl]-3-[[2-phenyl-5-(trifluoromethyl)-2H-pyrazol-3-yl]-methyl]-urea;
- **A59**: 1-[[2-(3-Chlorophenyl)-5-(trifluoromethyl)-2H-pyrazol-3-yl]-methyl]-3-[3-fluoro-4-(hydroxymethyl)-phenyl]-urea;
- **A60**: 1-[[2-(4-Chlorophenyl)-5-(trifluoromethyl)-2H-pyrazol-3-yl]-methyl]-3-[3-fluoro-4-(hydroxymethyl)-phenyl]-urea;
- **A61**: 1-[3-Fluoro-4-(hydroxymethyl)-phenyl]-3-[[2-(2-fluorophenyl)-5-(trifluoromethyl)-2H-pyrazol-3-yl]-methyl]-urea;
- **A62**: 1-[3-Fluoro-4-(hydroxymethyl)-phenyl]-3-[[2-(3-fluorophenyl)-5-(trifluoromethyl)-2H-pyrazol-3-yl]-methyl]-urea;
- **A63**: 1-[3-Fluoro-4-(hydroxymethyl)-phenyl]-3-[[2-(4-fluorophenyl)-5-(trifluoromethyl)-2H-pyrazol-3-yl]-methyl]-urea;
- **A64**: 1-[[2-(3,4-Difluoro-phenyl)-5-(trifluoromethyl)-2H-pyrazol-3-yl]-methyl]-3-[3-fluoro-4-(hydroxymethyl)-phenyl]-urea;
- **A65**: 1-[[2-(3-Chloro-4-fluoro-phenyl)-5-(trifluoromethyl)-2H-pyrazol-3-yl]-methyl]-3-[3-fluoro-4-(hydroxymethyl)-phenyl]-urea;
- **A66**: 1-[[2-(3,5-Difluoro-phenyl)-5-(trifluoromethyl)-2H-pyrazol-3-yl]-methyl]-3-[3-fluoro-4-(hydroxymethyl)-phenyl]-urea;
- **A67**: 1-[3-Fluoro-4-(hydroxymethyl)-phenyl]-3-[[2-(3-methoxyphenyl)-5-(trifluoromethyl)-2H-pyrazol-3-yl]-methyl]-urea;
- **A68**: 1-[3-Fluoro-4-(hydroxymethyl)-phenyl]-3-[[2-(4-methoxyphenyl)-5-(trifluoromethyl)-2H-pyrazol-3-yl]-methyl]-urea;
- **A69**: 1-[3-Fluoro-4-(hydroxymethyl)-phenyl]-3-[[2-(o-tolyl)-5-(trifluoromethyl)-2H-pyrazol-3-yl]-methyl]-urea;
- **A70**: 1-[3-Fluoro-4-(hydroxymethyl)-phenyl]-3-[[2-(m-tolyl)-5-(trifluoromethyl)-2H-pyrazol-3-yl]-methyl]-urea;
- **A71**: 1-[3-Fluoro-4-(hydroxymethyl)-phenyl]-3-[[2-(p-tolyl)-5-(trifluoromethyl)-2H-pyrazol-3-yl]-methyl]-urea;
- **A72**: 1-[[2-(2,3-Dimethyl-phenyl)-5-(trifluoromethyl)-2H-pyrazol-3-yl]-methyl]-3-[3-fluoro-4-(hydroxymethyl)-phenyl]-urea;
- **A73**: 1-[[2-(3,5-Dimethyl-phenyl)-5-(trifluoromethyl)-2H-pyrazol-3-yl]-methyl]-3-[3-fluoro-4-(hydroxymethyl)-phenyl]-urea;
- **A74**: 1-[[2-(2,5-Dimethyl-phenyl)-5-(trifluoromethyl)-2H-pyrazol-3-yl]-methyl]-3-[3-fluoro-4-(hydroxymethyl)-phenyl]-urea;
- **A75**: 1-[[2-(3,4-Dimethyl-phenyl)-5-(trifluoromethyl)-2H-pyrazol-3-yl]-methyl]-3-[3-fluoro-4-(hydroxymethyl)-phenyl]-urea;
- **A76**: 1-[3-Fluoro-4-(hydroxymethyl)-phenyl]-3-[[2-(2-fluoro-3-methyl-phenyl)-5-(trifluoromethyl)-2H-pyrazol-3-yl]-methyl]-urea;
- **A77**: 1-[3-Fluoro-4-(hydroxymethyl)-phenyl]-3-[[2-(3-fluoro-5-methyl-phenyl)-5-(trifluoromethyl)-2H-pyrazol-3-yl]-methyl]-urea;
- **A78**: 1-[[2-(4-Chloro-3-methyl-phenyl)-5-(trifluoromethyl)-2H-pyrazol-3-yl]-methyl]-3-[3-fluoro-4-(hydroxymethyl)-phenyl]-urea;
- **A79**: 1-[[2-(4-Ethoxy-3-methyl-phenyl)-5-(trifluoromethyl)-2H-pyrazol-3-yl]-methyl]-3-[3-fluoro-4-(hydroxymethyl)-phenyl]-urea;
- **A80**: 1-[3-Fluoro-4-(hydroxymethyl)-phenyl]-3-[[2-(4-fluoro-3-methyl-phenyl)-5-(trifluoromethyl)-2H-pyrazol-3-yl]-methyl]-urea;
- **A81**: 1-[[2-(4-Cyano-3-methyl-phenyl)-5-(trifluoromethyl)-2H-pyrazol-3-yl]-methyl]-3-[3-fluoro-4-(hydroxymethyl)-phenyl]-urea;
- **A82**: 1-[3-Fluoro-4-(hydroxymethyl)-phenyl]-3-[[2-(3-isopropyl-phenyl)-5-(trifluoromethyl)-2H-pyrazol-3-yl]-methyl]-urea;
- **A83**: 1-[[2-[3-(Difluoro-methyl)-phenyl]-5-(trifluoromethyl)-2H-pyrazol-3-yl]-methyl]-3-[3-fluoro-4-(hydroxymethyl)-phenyl]-urea;
- **A84**: 1-[3-Fluoro-4-(hydroxymethyl)-phenyl]-3-[[2-[3-(methoxymethyl)-phenyl]-5-(trifluoromethyl)-2H-pyrazol-3-yl]-methyl]-urea;
- **A85**: 1-[[2-(3-Cyano-phenyl)-5-(trifluoromethyl)-2H-pyrazol-3-yl]-methyl]-3-[3-fluoro-4-(hydroxymethyl)-phenyl]-urea;
- **A86**: 1-[[2-(3-Dimethylamino-phenyl)-5-(trifluoromethyl)-2H-pyrazol-3-yl]-methyl]-3-[3-fluoro-4-(hydroxymethyl)-phenyl]-urea;
- **A87**: 1-[3-Fluoro-4-(hydroxymethyl)-phenyl]-3-[[2-(4-methoxy-3-methyl-phenyl)-5-(trifluoromethyl)-2H-pyrazol-3-yl]-methyl]-urea;
- **A88**: 1-[[2-(2-Ethoxy-5-methyl-phenyl)-5-(trifluoromethyl)-2H-pyrazol-3-yl]-methyl]-3-[3-fluoro-4-(hydroxymethyl)-phenyl]-urea;
- **A89**: 1-[[2-(2,3-Dichloro-phenyl)-5-(trifluoromethyl)-2H-pyrazol-3-yl]-methyl]-3-[3-fluoro-4-(hydroxymethyl)-phenyl]-urea;
- **A90**: 1-[[2-(3-Chloro-2-methoxy-phenyl)-5-(trifluoromethyl)-2H-pyrazol-3-yl]-methyl]-3-[3-fluoro-4-(hydroxymethyl)-phenyl]-urea;
- **A91**: 1-[[2-(5-Chloro-2-methoxy-phenyl)-5-(trifluoromethyl)-2H-pyrazol-3-yl]-methyl]-3-[3-fluoro-4-(hydroxymethyl)-phenyl]-urea;
- **A92**: 1-[3-Fluoro-4-(hydroxymethyl)-phenyl]-3-[[2-(3-methoxy-5-methyl-phenyl)-5-(trifluoromethyl)-2H-pyrazol-3-yl]-methyl]-urea;
- **A93**: 1-[[2-(3-Ethoxy-5-methyl-phenyl)-5-(trifluoromethyl)-2H-pyrazol-3-yl]-methyl]-3-[3-fluoro-4-(hydroxymethyl)-phenyl]-urea;
- **A94**: 1-[3-Fluoro-4-(hydroxymethyl)-phenyl]-3-[[2-(2-methoxy-3-methyl-phenyl)-5-(trifluoromethyl)-2H-pyrazol-3-yl]-methyl]-urea;
- **A95**: 1-[3-Fluoro-4-(hydroxymethyl)-phenyl]-3-[[2-(3-fluoro-4-methoxy-phenyl)-5-(trifluoromethyl)-2H-pyrazol-3-yl]-methyl]-urea;
- **A96**: 1-[[5-tert-Butyl-2-(3-chlorophenyl)-2H-pyrazol-3-yl]-methyl]-3-[3-chloro-4-(hydroxymethyl)-phenyl]-urea;
- **A97**: 1-[3-Chloro-4-(hydroxymethyl)-phenyl]-3-[[2-(3-chlorophenyl)-5-(trifluoromethyl)-2H-pyrazol-3-yl]-methyl]-urea;
- **A98**: N-[[5-tert-Butyl-2-(3-chlorophenyl)-2H-pyrazol-3-yl]-methyl]-2-[3-fluoro-4-(1-hydroxy-ethyl)-phenyl]-propionamide;
- **A99**: N-[[2-(3-Chlorophenyl)-5-(trifluoromethyl)-2H-pyrazol-3-yl]-methyl]-2-[3-fluoro-4-(1-hydroxy-ethyl)-phenyl]-propionamide;
- **A100**: N-[[5-tert-Butyl-2-(3-chlorophenyl)-2H-pyrazol-3-yl]-methyl]-2-[3-fluoro-4-(1-hydroxy-cyclopropyl)-phenyl]-propionamide;
- **A101**: N-[[2-(3-Chlorophenyl)-5-(trifluoromethyl)-2H-pyrazol-3-yl]-methyl]-2-[3-fluoro-4-(1-hydroxy-cyclopropyl)-phenyl]-propionamide;
- **A102**: 2-[3-Fluoro-4-(1-hydroxy-cyclopropyl)-phenyl]-N-[[2-(3-fluorophenyl)-5-(trifluoromethyl)-2H-pyrazol-3-yl]-methyl]-propionamide;
- **A103**: 2-[3-Fluoro-4-(1-hydroxy-cyclopropyl)-phenyl]-N-[[2-(3-methoxyphenyl)-5-(trifluoromethyl)-2H-pyrazol-3-yl]-methyl]-propionamide;
- **A104**: N-[[2-(3,4-Difluoro-phenyl)-5-(trifluoromethyl)-2H-pyrazol-3-yl]-methyl]-2-[3-fluoro-4-(1-hydroxy-cyclopropyl)-phenyl]-propionamide;
- **A105**: 1-[[5-tert-Butyl-2-(3-chlorophenyl)-2H-pyrazol-3-yl]-methyl]-3-[3-fluoro-4-(1-hydroxy-cyclopropyl)-phenyl]-urea;
- **A106**: 1-[[2-(3-Chlorophenyl)-5-(trifluoromethyl)-2H-pyrazol-3-yl]-methyl]-3-[3-fluoro-4-(1-hydroxy-cyclopropyl)-phenyl]-urea;
- **A107**: 1-[[5-tert-Butyl-2-(3-chlorophenyl)-2H-pyrazol-3-yl]-methyl]-3-[4-(3-hydroxy-oxetan-3-yl)-phenyl]-urea;
- **A108**: 1-[[2-(3-Chlorophenyl)-5-(trifluoromethyl)-2H-pyrazol-3-yl]-methyl]-3-[4-(3-hydroxy-oxetan-3-yl)-phenyl]-urea;
- **A109**: 1-[[5-tert-Butyl-2-(3-chlorophenyl)-2H-pyrazol-3-yl]-methyl]-3-[3-fluoro-4-(3-hydroxy-oxetan-3-yl)-phenyl]-urea;
- **A110**: 1-[[2-(3-Chlorophenyl)-5-(trifluoromethyl)-2H-pyrazol-3-yl]-methyl]-3-[3-fluoro-4-(3-hydroxy-oxetan-3-yl)-phenyl]-urea;
- **A111**: N-[[2-(3-Chlorophenyl)-5-(trifluoromethyl)-2H-pyrazol-3-yl]-methyl]-2-[3-fluoro-4-(2-methoxy-ethoxy-methyl)-phenyl]-propionamide;
- **A112**: N-[[2-(3-Chloro-4-fluoro-phenyl)-5-(trifluoromethyl)-2H-pyrazol-3-yl]-methyl]-2-[3-fluoro-4-(2-methoxy-ethoxy-methyl)-phenyl]-propionamide;
- **A113**: N-[[2-(3-Chlorophenyl)-5-(trifluoromethyl)-2H-pyrazol-3-yl]-methyl]-2-[3-fluoro-4-(2-hydroxy-ethoxy-methyl)-phenyl]-acetamide;
- **A114**: 2-[3-Fluoro-4-(2-hydroxy-ethoxy-methyl)-phenyl]-N-[[2-(3-methoxyphenyl)-5-(trifluoromethyl)-2H-pyrazol-3-yl]-methyl]-propionamide;
- **A115**: 2-[3-Fluoro-4-(2-hydroxy-ethoxy-methyl)-phenyl]-N-[[2-(3-isopropyl-phenyl)-5-(trifluoromethyl)-2H-pyrazol-3-yl]-methyl]-propionamide;
- **A116**: N-[[5-tert-Butyl-2-(3-fluorophenyl)-2H-pyrazol-3-yl]-methyl]-2-[3-fluoro-4-(2-hydroxy-ethoxy-methyl)-phenyl]-propionamide;
- **A117**: N-[[5-tert-Butyl-2-(3-chlorophenyl)-2H-pyrazol-3-yl]-methyl]-2-[3-fluoro-4-(2-hydroxy-ethoxy-methyl)-phenyl]-propionamide;
- **A118**: N-[[2-(3-Chlorophenyl)-5-(trifluoromethyl)-2H-pyrazol-3-yl]-methyl]-2-[3-fluoro-4-(2-hydroxy-ethoxy-methyl)-phenyl]-propionamide;
- **A119**: (2S)-N-[[2-(3-Chlorophenyl)-5-(trifluoromethyl)-2H-pyrazol-3-yl]-methyl]-2-[3-fluoro-4-(2-hydroxy-ethoxy-methyl)-phenyl]-propionamide;
- **A120**: (2R)-N-[[2-(3-Chlorophenyl)-5-(trifluoromethyl)-2H-pyrazol-3-yl]-methyl]-2-[3-fluoro-4-(2-hydroxy-ethoxy-methyl)-phenyl]-propionamide;
- **A121**: N-[[2-(3-Chloro-4-fluoro-phenyl)-5-(trifluoromethyl)-2H-pyrazol-3-yl]-methyl]-2-[3-fluoro-4-(2 -hydroxy-ethoxy-methyl)-phenyl]-propionamide;
- **A122**: 2-[3-Fluoro-4-(2-hydroxy-ethoxy-methyl)-phenyl]-N-[[2-(3-fluorophenyl)-5-(trifluoromethyl)-2H-pyrazol-3-yl]-methyl]-propionamide;
- **A123**: 2-[3-Fluoro-4-(2-hydroxy-ethoxy-methyl)-phenyl]-N-[[2-(m-tolyl)-5-(trifluoromethyl)-2H-pyrazol-3-yl]-methyl]-propionamide;
- **A124**: N-[[5-tert-Butyl-2-(4-fluorophenyl)-2H-pyrazol-3-yl]-methyl]-2-[3-fluoro-4-(2-hydroxy-ethoxy-methyl)-phenyl]-propionamide;
- **A125**: N-[[5-tert-Butyl-2-[3-(trifluoromethyloxy)-phenyl]-2H-pyrazol-3-yl]-methyl]-2-[3-fluoro-4-(2-hydroxy-ethoxy-methyl)-phenyl]-propionamide;
- **A126**: N-[[2-(3,5-Difluoro-phenyl)-5-(trifluoromethyl)-2H-pyrazol-3-yl]-methyl]-2-[3-fluoro-4-(2-hydroxy-ethoxy-methyl)-phenyl]-propionamide;
- **A127**: 1-[[2-(3-Chlorophenyl)-5-(trifluoromethyl)-2H-pyrazol-3-yl]-methyl]-3-[3-fluoro-4-(2-hydroxy-ethoxy-methyl)-phenyl]-urea;
- **A128**: 1-[[2-(3-Chlorophenyl)-5-(trifluoromethyl)-2H-pyrazol-3-yl]-methyl]-3-[3-fluoro-4-(2-methoxy-ethoxy-methyl)-phenyl]-urea;
- **A129**: 1-[[5-tert-Butyl-2-(3-chlorophenyl)-2H-pyrazol-3-yl]-methyl]-3-[3-fluoro-4-(2-hydroxy-ethoxy-methyl)-phenyl]-urea;
- **A130**: 1-[[2-(3-Chloro-4-fluoro-phenyl)-5-(trifluoromethyl)-2H-pyrazol-3-yl]-methyl]-3-[3-fluoro-4-(2-hydroxy-ethoxy-methyl)-phenyl]-urea;
- **A131**: 1-[3-Fluoro-4-(2-hydroxy-ethoxy-methyl)-phenyl]-3-[[2-(3-fluorophenyl)-5-(trifluoromethyl)-2H-pyrazol-3-yl]-methyl]-urea;
- **A132**: 1-[[2-(3,4-Difluoro-phenyl)-5-(trifluoromethyl)-2H-pyrazol-3-yl]-methyl]-3-[3-fluoro-4-(2-hydroxy-ethoxy-methyl)-phenyl]-urea;
- **A133**: 1-[[2-(2,3-Dichloro-phenyl)-5-(trifluoromethyl)-2H-pyrazol-3-yl]-methyl]-3-[3-fluoro-4-(2-hydroxy-ethoxy-methyl)-phenyl]-urea;
- **A134**: 1-[[5-tert-Butyl-2-(4-fluorophenyl)-2H-pyrazol-3-yl]-methyl]-3-[3-fluoro-4-(2-hydroxy-ethoxy-methyl)-phenyl]-urea;
- **A135**: 1-[[2-(3-Ethoxy-5-methyl-phenyl)-5-(trifluoromethyl)-2H-pyrazol-3-yl]-methyl]-3-[3-fluoro-4-(2-hydroxy-ethoxy-methyl)-phenyl]-urea;
- **A136**: 2-[3-Fluoro-4-(2-hydroxy-ethyl)-phenyl]-N-[[2-(3-fluorophenyl)-5-(trifluoromethyl)-2H-pyrazol-3-yl]-methyl]-propionamide;
- **A137**: 2-[3-Fluoro-4-(2-hydroxy-ethyl)-phenyl]-N-[[2-(3-isopropyl-phenyl)-5-(trifluoromethyl)-2H-pyrazol-3-yl]-methyl]-propionamide;
- **A138**: N-[[2-(3-Chlorophenyl)-5-(trifluoromethyl)-2H-pyrazol-3-yl]-methyl]-2-[3-fluoro-4-(2-hydroxy-ethyl)-phenyl]-propionamide;
- **A139**: N-[[5-tert-Butyl-2-(3-chlorophenyl)-2H-pyrazol-3-yl]-methyl]-2-[3-fluoro-4-(2-hydroxy-ethyl)-phenyl]-propionamide;
- **A140**: 1-[[5-tert-Butyl-2-(3-chlorophenyl)-2H-pyrazol-3-yl]-methyl]-3-[3-fluoro-4-(2-hydroxy-ethyl)-phenyl]-urea;
- **A141**: 1-[[2-(3-Chlorophenyl)-5-(trifluoromethyl)-2H-pyrazol-3-yl]-methyl]-3-[3-fluoro-4-(2-hydroxy-ethyl)-phenyl]-urea;
- **A142**: 1-[3-Fluoro-4-(2-hydroxy-ethyl)-phenyl]-3-[[2-(3-fluorophenyl)-5-(trifluoromethyl)-2H-pyrazol-3-yl]-methyl]-urea;
- **A143**: 1-[3-Fluoro-4-(2-hydroxy-ethyl)-phenyl]-3-[[2-(3-methoxyphenyl)-5-(trifluoromethyl)-2 H-pyrazol-3-yl]-methyl]-urea;
- **A144**: 1-[3-Fluoro-4-(2-hydroxy-ethyl)-phenyl]-3-[[2-(m-tolyl)-5-(trifluoromethyl)-2H-pyrazol-3-yl]-methyl]-urea;
- **A145**: N-[[5-tert-Butyl-2-(3-chlorophenyl)-2H-pyrazol-3-yl]-methyl]-2-[4-(1,2-dihydroxy-ethyl)-3-fluoro-phenyl]-propionamide;
- **A146**: N-[[2-(3-Chlorophenyl)-5-(trifluoromethyl)-2H-pyrazol-3-yl]-methyl]-2-[4-(1,2-dihydroxy-ethyl)-phenyl]-propionamide;
- **A147**: N-[[5-tert-Butyl-2-(3-chlorophenyl)-2H-pyrazol-3-yl]-methyl]-2-[4-(1,2-dihydroxy-ethyl)-phenyl]-propionamide;
- **A148**: N-[[2-(3-Chlorophenyl)-5-(trifluoromethyl)-2H-pyrazol-3-yl]-methyl]-2-[4-(1,2-dihydroxy-ethyl)-3-fluoro-phenyl]-propionamide;
- **A149**: 2-[4-(1,2-Dihydroxy-ethyl)-3-fluoro-phenyl]-N-[[2-(3-fluorophenyl)-5-(trifluoromethyl)-2H-pyrazol-3-yl]-methyl]-propionamide;
- **A150**: 1-[[5-tert-Butyl-2-(3-chlorophenyl)-2H-pyrazol-3-yl]-methyl]-3-[4-(1,2-dihydroxy-ethyl)-3-fluoro-phenyl]-urea;
- **A151**: 1-[[2-(3-Chlorophenyl)-5-(trifluoromethyl)-2H-pyrazol-3-yl]-methyl]-3-[4-(1,2-dihydroxy-ethyl)-3-fluoro-phenyl]-urea;
- **A152**: 1-[[5-tert-Butyl-2-(3-chlorophenyl)-2H-pyrazol-3-yl]-methyl]-3-[3-fluoro-4-[2-hydroxy-1-(hydroxymethyl)-ethyl]-phenyl]-urea;
- **A153**: 1-[[2-(3-Chlorophenyl)-5-(trifluoromethyl)-2H-pyrazol-3-yl]-methyl]-3-[3-fluoro-4-[2-hydroxy-1-(hydroxymethyl)-ethyl]-phenyl]-urea;
- **A154**: 1-[[2-(3-Chlorophenyl)-5-(trifluoromethyl)-2H-pyrazol-3-yl]-methyl]-3-[3-fluoro-4-[2-hydroxy-1-(hydroxymethyl)-1-methyl-ethyl]-phenyl]-urea;
- **A155**: 1-[[2-(3-Chlorophenyl)-5-(trifluoromethyl)-2H-pyrazol-3-yl]-methyl]-3-[4-[2-hydroxy-1-(hydroxymethyl)-1-methyl-ethyl]-phenyl]-urea;
- **A156**: 1-[[5-tert-Butyl-2-(3-chlorophenyl)-2H-pyrazol-3-yl]-methyl]-3-[3-fluoro-4-[2-hydroxy-1-(hydroxymethyl)-1-methyl-ethyl]-phenyl]-urea;
- **A157**: 1-[[5-tert-Butyl-2-(3-chlorophenyl)-2H-pyrazol-3-yl]-methyl]-3-[4-[2-hydroxy-1-(hydroxymethyl)-1-methyl-ethyl]-phenyl]-urea;
- **A158**: 1-[[2-(3-Chlorophenyl)-5-(trifluoromethyl)-2H-pyrazol-3-yl]-methyl]-3-[4-[1,2-dihydroxy-1-(hydroxymethyl)-ethyl]-phenyl]-urea; and
- **A159**: 1-[[5-tert-Butyl-2-(3-chlorophenyl)-2H-pyrazol-3-yl]-methyl]-3-[4-[1,2-dihydroxy-1-(hydroxymethyl)-ethyl]-phenyl]-urea;
optionally in the form of a single stereoisomer or a mixture of stereoisomers, in the form of the free compound and/or a physiologically acceptable salt thereof.
Compounds A27, A54, A55, A56, A57, A58, A59, A63, A65, A66, A70, A80, A82, and A96 are inventive compounds. Furthermore, preference may be given to compounds according to the invention that cause a 50% displacement of capsaicin, which is present at a concentration of 100 nM, in a FLIPR assay with CHO K1 cells which were transfected with the human VR1 gene at a concentration of less than 2 000 nM, preferably less than 1 000 nM, particularly preferably less than 300 nM, most particularly preferably less than 100 nM, even more preferably less than 75 nM, additionally preferably less than 50 nM, most preferably less than 10 nM.

In the process, the Ca²⁺ influx is quantified in the FLIPR assay with the aid of a Ca²⁺-sensitive dye (type Fluo-4, Molecular Probes Europe BV, Leiden, the Netherlands) in a fluorescent imaging plate reader (FLIPR, Molecular Devices, Sunnyvale, USA), as described hereinafter.

The substituted compounds according to the invention and corresponding stereoisomers and also the respective corresponding acids, bases, salts and solvates are toxicologically safe and are therefore suitable as pharmaceutical active ingredients in pharmaceutical compositions.

The present invention therefore further relates to a pharmaceutical composition containing at least one compound according to the invention, in each case if appropriate in the form of one of its pure stereoisomers, in particular enantiomers or diastereomers, its racemates or in the form of a mixture of stereoisomers, in particular the enantiomers and/or diastereomers, in any desired mixing ratio, or respectively in the form of a corresponding salt, or respectively in the form of a corresponding solvate, and also if appropriate one or more pharmaceutically compatible auxiliaries.

These pharmaceutical compositions according to the invention are suitable in particular for vanilloid receptor 1-(VR1/TRPV1) regulation, preferably for vanilloid receptor 1-(VR1/TRPV1) inhibition and/or for vanilloid receptor 1-(VR1/TRPV1) stimulation, i.e. they exert an agonistic or antagonistic effect.

Likewise, the pharmaceutical compositions according to the invention are preferably suitable for the prophylaxis and/or treatment of disorders or diseases which are mediated, at least in part, by vanilloid receptors 1.

The pharmaceutical composition according to the invention is suitable for administration to adults and children, including toddlers and babies.

The pharmaceutical composition according to the invention may be found as a liquid, semisolid or solid pharmaceutical form, for example in the form of injection solutions, drops, juices, syrups, sprays, suspensions, tablets, patches, capsules, plasters, suppositories, ointments, creams, lotions, gels, emulsions, aerosols or in multiparticulate form, for example in the form of pellets or granules, if appropriate pressed into tablets, decanted in capsules or suspended in a liquid, and also be administered as much.

In addition to at least one substituted compound according to the invention, if appropriate in the form of one of its pure stereoisomers, in particular enantiomers or diastereomers, its racemate or in the form of mixtures of the stereoisomers, in particular the enantiomers or diastereomers, in any desired mixing ratio, or if appropriate in the form of a corresponding salt or respectively in the form of a corresponding solvate, the pharmaceutical composition according to the invention conventionally contains further physiologically compatible pharmaceutical auxiliaries which can for example be selected from the group consisting of excipients, fillers, solvents, diluents, surface-active substances, dyes, preservatives, blasting agents, slip additives, lubricants, aromas and binders.

The selection of the physiologically compatible auxiliaries and also the amounts thereof to be used depend on whether the pharmaceutical composition is to be applied orally, subcutaneously, parenterally, intravenously, intraperitoneally, intradermally, intramuscularly, intranasally, buccally, rectally or locally, for example to infections of the skin, the mucous membranes and of the eyes. Preparations in the form of tablets, dragees, capsules, granules, pellets, drops, juices and syrups are preferably suitable for oral application; solutions, suspensions, easily reconstitutable dry preparations and also sprays are preferably suitable for parenteral, topical and inhalative application. The substituted compounds according to the invention used in the pharmaceutical composition according to the invention in a repository in dissolved form or in a plaster, agents promoting skin penetration being added if appropriate, are suitable percutaneous application preparations. Orally or percutaneously applicable preparation forms can release the respective substituted compound according to the invention also in a delayed manner.

The pharmaceutical compositions according to the invention are prepared with the aid of conventional means, devices, methods and process known in the art, such as are described for example in "Remington's Pharmaceutical Sciences", A.R. Gennaro (Editor), 17th edition, Mack Publishing Company, Easton, Pa, 1985, in particular in Part 8, Chapters 76 to 93. The corresponding description is introduced herewith by way of reference and forms part of the disclosure. The amount to be administered to the patient of the respective substituted compounds according to the invention of the above-indicated general formula I may vary and is for example dependent on the patient's weight or age and also on the type of application, the indication and the severity of the disorder. Conventionally 0.001 to 100 mg/kg, preferably 0.05 to 75 mg/kg, particularly preferably 0.05 to 50 mg of at least one such compound according to the invention are applied per kg of the patient's body weight.

The pharmaceutical composition according to the invention is preferably suitable for the treatment and/or prophylaxis of one or more disorders and/or diseases selected from the group consisting of pain, preferably pain selected from the group consisting of acute pain, chronic pain, neuropathic pain, visceral pain and joint pain; hyperalgesia; allodynia; causalgia; migraine; depression; nervous affection; axonal injuries; neurodegenerative diseases, preferably selected from the group consisting of multiple sclerosis, Alzheimer's disease, Parkinson's disease and Huntington's disease; cognitive dysfunctions, preferably cognitive deficiency states, particularly preferably memory disorders; epilepsy; respiratory diseases, preferably selected from the group consisting of asthma, bronchitis and pulmonary inflammation; coughs; urinary incontinence; overactive bladder (OAB); disorders and/or injuries of the gastrointestinal tract; duodenal ulcers; gastric ulcers; irritable bowel syndrome; strokes; eye irritations; skin irritations; neurotic skin diseases; allergic skin diseases; psoriasis; vitiligo; herpes simplex; inflammations, preferably inflammations of the intestine, the eyes, the bladder, the skin or the nasal mucous membrane; diarrhoea; pruritus; osteoporosis; arthritis; osteoarthritis; rheumatic diseases; eating disorders, preferably selected from the group consisting of bulimia, cachexia, anorexia and obesity; medication dependency; misuse of medication; withdrawal symptoms in medication dependency; development of tolerance to medication, preferably to natural or synthetic opioids; drug dependency; misuse of drugs; withdrawal symptoms in drug dependency; alcohol dependency; misuse of alcohol and withdrawal symptoms in alcohol dependency; for diuresis; for antinatriuresis; for influencing the cardiovascular system; for increasing vigilance; for the treatment of wounds and/or burns; for the treatment of severed nerves; for increasing libido; for modulating movement activity; for anxiolysis; for local anaesthesia and/or for inhibiting undesirable side effects, preferably selected from the group consisting of hyperthermia, hypertension and bronchoconstriction, triggered by the administration of vanilloid receptor 1 (VR1/TRPV1 receptor) agonists, preferably selected from the group consisting of capsaicin, resiniferatoxin, olvanil, arvanil, SDZ-249665, SDZ-249482, nuvanil and capsavanil.

Particularly preferably, the pharmaceutical composition according to the invention is suitable for the treatment and/or prophylaxis of one or more disorders and/or diseases selected from the group consisting of pain, preferably of pain selected from the group consisting of acute pain, chronic pain, neuropathic pain, visceral pain and joint pain; migraine; depression; neurodegenerative diseases, preferably selected from the group consisting of multiple sclerosis, Alzheimer's disease, Parkinson's disease and Huntington's disease; cognitive dysfunctions, preferably cognitive deficiency states, particularly preferably memory disorders; inflammations, preferably inflammations of the intestine, the eyes, the bladder, the skin or the nasal mucous membrane; urinary incontinence; overactive bladder (OAB); medication dependency; misuse of medication; withdrawal symptoms in medication dependency; development of tolerance to medication, preferably development of tolerance to natural or synthetic opioids; drug dependency; misuse of drugs; withdrawal symptoms in drug dependency; alcohol dependency; misuse of alcohol and withdrawal symptoms in alcohol dependency.

Most particularly preferably, the pharmaceutical composition according to the invention is suitable for the treatment and/or prophylaxis of pain, preferably of pain selected from the group consisting of acute pain, chronic pain, neuropathic pain and visceral pain.

The present invention further relates to a substituted compound according to the present invention and also if appropriate to a substituted compound according to the present invention and one or more pharmaceutically acceptable auxiliaries for use in vanilloid receptor 1-(VR1/TRPV1) regulation, preferably for use in vanilloid receptor 1-(VR1/TRPV1) inhibition and/or vanilloid receptor 1-(VR1/TRPV1) stimulation.

The present invention therefore further relates to a substituted compound according to the present invention and also if appropriate to a substituted compound according to the present invention and one or more pharmaceutically acceptable auxiliaries for use in the prophylaxis and/or treatment of disorders and/or diseases which are mediated, at least in part, by vanilloid receptors 1.

In particular, the present invention therefore further relates to a substituted compound according to the present invention and also if appropriate to a substituted compound according to the present invention and one or more pharmaceutically acceptable auxiliaries for use in the prophylaxis and/or treatment of disorders and/or diseases selected from the group consisting of pain, preferably pain selected from the group consisting of acute pain, chronic pain, neuropathic pain, visceral pain and joint pain; hyperalgesia; allodynia; causalgia; migraine; depression; nervous affection; axonal injuries; neurodegenerative diseases, preferably selected from the group consisting of multiple sclerosis, Alzheimer's disease, Parkinson's disease and Huntington's disease; cognitive dysfunctions, preferably cognitive deficiency states, particularly preferably memory disorders; epilepsy; respiratory diseases, preferably selected from the group consisting of asthma, bronchitis and pulmonary inflammation; coughs; urinary incontinence; overactive bladder (OAB); disorders and/or injuries of the gastrointestinal tract; duodenal ulcers; gastric ulcers; irritable bowel syndrome; strokes; eye irritations; skin irritations; neurotic skin diseases; allergic skin diseases; psoriasis; vitiligo; herpes simplex; inflammations, preferably inflammations of the intestine, the eyes, the bladder, the skin or the nasal mucous membrane; diarrhoea; pruritus; osteoporosis; arthritis; osteoarthritis; rheumatic diseases; eating disorders, preferably selected from the group consisting of bulimia, cachexia, anorexia and obesity; medication dependency; misuse of medication; withdrawal symptoms in medication dependency; development of tolerance to medication, preferably to natural or synthetic opioids; drug dependency; misuse of drugs; withdrawal symptoms in drug dependency; alcohol dependency; misuse of alcohol and withdrawal symptoms in alcohol dependency; for diuresis; for antinatriuresis; for influencing the cardiovascular system; for increasing vigilance; for the treatment of wounds and/or burns; for the treatment of severed nerves; for increasing libido; for modulating movement activity; for anxiolysis; for local anaesthesia and/or for inhibiting undesirable side effects, preferably selected from the group consisting of hyperthermia, hypertension and bronchoconstriction, triggered by the administration of vanilloid receptor 1 (VR1/TRPV1 receptor) agonists, preferably selected from the group consisting of capsaicin, resiniferatoxin, olvanil, arvanil, SDZ-249665, SDZ-249482, nuvanil and capsavanil.

Most particularly preferred is a substituted compound according to the present invention and also if appropriate to a substituted compound according to the present invention and one or more pharmaceutically acceptable auxiliaries for use in the prophylaxis and/or treatment of pain, preferably of pain selected from the group consisting of acute pain, chronic pain, neuropathic pain and visceral pain.

The present invention further relates to the use of at least one substituted compound according to the present invention and also if appropriate to the use of at least one substituted compound according to the present invention of one or more pharmaceutically acceptable auxiliaries for the preparation of a pharmaceutical composition for vanilloid receptor 1-(VR1/TRPV1) regulation, preferably for vanilloid receptor 1-(VR1/TRPV1) inhibition and/or for vanilloid receptor 1-(VR1/TRPV1) stimulation, and, further for the prophylaxis and/or treatment of disorders and/or diseases which are mediated, at least in part, by vanilloid receptors 1, such as e.g. disorders and/or diseases selected from the group consisting of pain, preferably pain selected from the group consisting of acute pain, chronic pain, neuropathic pain, visceral pain and joint pain; hyperalgesia; allodynia; causalgia; migraine; depression; nervous affection; axonal injuries; neurodegenerative diseases, preferably selected from the group consisting of multiple sclerosis, Alzheimer's disease, Parkinson's disease and Huntington's disease; cognitive dysfunctions, preferably cognitive deficiency states, particularly preferably memory disorders; epilepsy; respiratory diseases, preferably selected from the group consisting of asthma, bronchitis and pulmonary inflammation; coughs; urinary incontinence; overactive bladder (OAB); disorders and/or injuries of the gastrointestinal tract; duodenal ulcers; gastric ulcers; irritable bowel syndrome; strokes; eye irritations; skin irritations; neurotic skin diseases; allergic skin diseases; psoriasis; vitiligo; herpes simplex; inflammations, preferably inflammations of the intestine, the eyes, the bladder, the skin or the nasal mucous membrane; diarrhoea; pruritus; osteoporosis; arthritis; osteoarthritis; rheumatic diseases; eating disorders, preferably selected from the group consisting of bulimia, cachexia, anorexia and obesity; medication dependency; misuse of medication; withdrawal symptoms in medication dependency; development of tolerance to medication, preferably to natural or synthetic opioids; drug dependency; misuse of drugs; withdrawal symptoms in drug dependency; alcohol dependency; misuse of alcohol and withdrawal symptoms in alcohol dependency; for diuresis; for antinatriuresis; for influencing the cardiovascular system; for increasing vigilance; for the treatment of wounds and/or burns; for the treatment of severed nerves; for increasing libido; for modulating movement activity; for anxiolysis; for local anaesthesia and/or for inhibiting undesirable side effects, preferably selected from the group consisting of hyperthermia, hypertension and bronchoconstriction, triggered by the administration of vanilloid receptor 1 (VR1/TRPV1 receptor) agonists, preferably selected from the group consisting of capsaicin, resiniferatoxin, olvanil, arvanil, SDZ-249665, SDZ-249482, nuvanil and capsavanil.

Another aspect of the present invention is a method for vanilloid receptor 1-(VR1/TRPV1) regulation, preferably for vanilloid receptor 1-(VR1/TRPV1) inhibition and/or for vanilloid receptor 1-(VR1/TRPV1) stimulation, and, further, a method of treatment and/or prophylaxis of disorders and/or diseases, which are mediated, at least in part, by vanilloid receptors 1, in a mammal, preferably of disorders and/or diseases selected from the group consisting of pain, preferably pain selected from the group consisting of acute pain, chronic pain, neuropathic pain, visceral pain and joint pain; hyperalgesia; allodynia; causalgia; migraine; depression; nervous affection; axonal injuries; neurodegenerative diseases, preferably selected from the group consisting of multiple sclerosis, Alzheimer's disease, Parkinson's disease and Huntington's disease; cognitive dysfunctions, preferably cognitive deficiency states, particularly preferably memory disorders; epilepsy; respiratory diseases, preferably selected from the group consisting of asthma, bronchitis and pulmonary inflammation; coughs; urinary incontinence; overactive bladder (OAB); disorders and/or injuries of the gastrointestinal tract; duodenal ulcers; gastric ulcers; irritable bowel syndrome; strokes; eye irritations; skin irritations; neurotic skin diseases; allergic skin diseases; psoriasis; vitiligo; herpes simplex; inflammations, preferably inflammations of the intestine, the eyes, the bladder, the skin or the nasal mucous membrane; diarrhoea; pruritus; osteoporosis; arthritis; osteoarthritis; rheumatic diseases; eating disorders, preferably selected from the group consisting of bulimia, cachexia, anorexia and obesity; medication dependency; misuse of medication; withdrawal symptoms in medication dependency; development of tolerance to medication, preferably to natural or synthetic opioids; drug dependency; misuse of drugs; withdrawal symptoms in drug dependency; alcohol dependency; misuse of alcohol and withdrawal symptoms in alcohol dependency; for diuresis; for antinatriuresis; for influencing the cardiovascular system; for increasing vigilance; for the treatment of wounds and/or burns; for the treatment of severed nerves; for increasing libido; for modulating movement activity; for anxiolysis; for local anaesthesia and/or for inhibiting undesirable side effects, preferably selected from the group consisting of hyperthermia, hypertension and bronchoconstriction, triggered by the administration of vanilloid receptor 1 (VR1/TRPV1 receptor) agonists, preferably selected from the group consisting of capsaicin, resiniferatoxin, olvanil, arvanil, SDZ-249665, SDZ-249482, nuvanil and capsavanil, which comprises administering an effective amount of at least one substituted compound according to the invention to the mammal.

The effectiveness against pain can be shown, for example, in the *Bennett* or *Chung* model (Bennett, G.J. and Xie, Y.K., A peripheral mononeuropathy in rat that produces disorders of pain sensation like those seen in man, Pain 1988, 33(1), 87-107; Kim, S.H. and Chung, J.M., An experimental model for peripheral neuropathy produced by segmental spinal nerve ligation in the rat, Pain 1992, 50(3), 355-363), by tail flick experiments (e.g. according to D'Amour und Smith (J. Pharm. Exp. Ther. 72, 74 79 (1941)) or by the formalin test (e.g. according to D. Dubuisson et al., Pain 1977, 4, 161-174).

The present invention further relates to processes for preparing substituted compounds according to the invention.

In particular, the compounds according to the present disclosure of can be prepared by a process according to which at least one compound of general formula (Q-II), 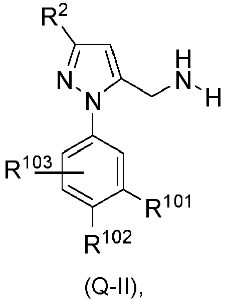 in which R¹⁰¹, R¹⁰², R¹⁰³ and R² have one of the foregoing meanings, is reacted in a reaction medium, if appropriate in the presence of at least one suitable coupling reagent, if appropriate in the presence of at least one base, with a compound of general formula (Q-III) with D = OH or Hal, 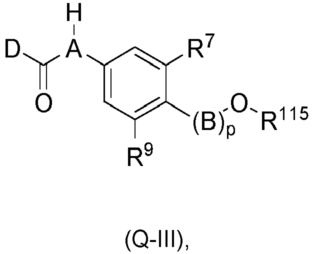 in which Hal represents a halogen, preferably Br or Cl, and R⁷, R⁹, B, R¹¹⁵ and p each have one of the foregoing meanings and A denotes CH or C(CH₃), in a reaction medium, if appropriate in the presence of at least one suitable coupling reagent, if appropriate in the presence of at least one base, to form a compound of general formula (Q), 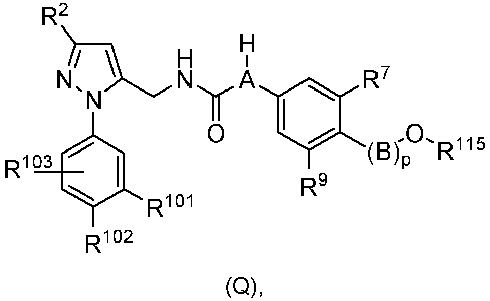 in which A represents CH or C(CH₃) and R¹⁰¹, R¹⁰², R¹⁰³ and R² as well as R⁷, R⁹, B, R¹¹⁵ and p have one of the foregoing meanings;
or in that at least one compound of general formula (Q-II), 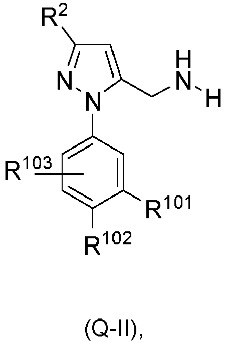 in which R¹⁰¹, R¹⁰², R¹⁰³ and R² have one of the foregoing meanings, is reacted to form a compound of general formula (Q-IV), 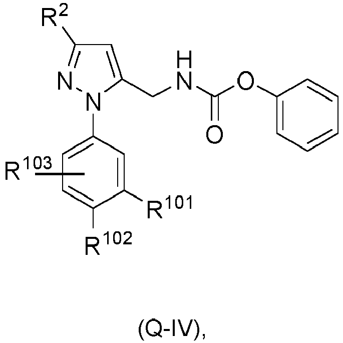 in which R¹⁰¹, R¹⁰², R¹⁰³ and R² have one of the foregoing meanings, in a reaction medium, in the presence of phenyl chloroformiate, if appropriate in the presence of at least one base and/or at least one coupling reagent, and said compound is if appropriate purified and/or isolated, and a compound of general formula (Q-IV) is reacted with a compound of general formula (Q-V), 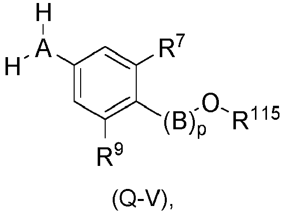 in which R⁷, R⁹, B, R¹¹⁵ and p have one of the foregoing meanings, and A denotes N, in a reaction medium, if appropriate in the presence of at least one suitable coupling reagent, if appropriate in the presence of at least one base, to form a compound of general formula (Q), 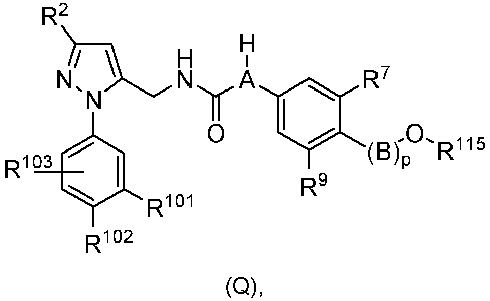 in which A represents N and R¹⁰¹, R¹⁰², R¹⁰³ and R² as well as R⁷, R⁹, B, R¹¹⁵ and p have one of the foregoing meanings.

The reaction of compounds of the above-indicated general formula (Q-II) with carboxylic acids of the above-indicated general formula (Q-III), particularly with D = OH, to form compounds of the above-indicated general formula (Q) is carried out preferably in a reaction medium selected from the group consisting of diethyl ether, tetrahydrofuran, acetonitrile, methanol, ethanol, (1,2)-dichloroethane, dimethylformamide, dichloromethane and corresponding mixtures, if appropriate in the presence of at least one coupling reagent, preferably selected from the group consisting of 1-benzotriazolyloxy-tris-(dimethylamino)-phosphonium hexafluorophosphate (BOP), dicyclohexylcarbodiimide (DCC), N'-(3-dimethylaminopropyl)-N-ethylcarbodiimide (EDCI), diisopropylcarbodiimide, 1,1'-carbonyldiimidazole (CDI), N-[(dimethylamino)-1H-1,2, 3-triazolo[4, 5-b]pyridino-1-yl-methylene]-N-methylmethanaminium hexafluorophosphate N-oxide (HATU), O-(benzotriazol-1-yl)-N,N,N,N-tetramethyluronium hexafluorophosphate (HBTU), O-(benzotriazol-1-yl)-N,N,N,N-tetramethyluronium tetrafluoroborate (TBTU), N-hydroxybenzotriazole (HOBt) and 1-hydroxy-7-azabenzotriazole (HOAt), if appropriate in the presence of at least one organic base, preferably selected from the group consisting of triethylamine, pyridine, dimethylaminopyridine, N-methylmorpholine and diisopropylethylamine, preferably at temperatures of from -70 °C to 100 °C.

Alternatively, the reaction of compounds of the above-indicated general formulae (Q-II) with carboxylic acid halides of the above-indicated general formula (Q-III) with D = Hal, in which Hal represents a halogen as the leaving group, preferably a chlorine or bromine atom, to form compounds of the above-indicated general formula (Q) is carried out in a reaction medium preferably selected from the group consisting of diethyl ether, tetrahydrofuran, acetonitrile, methanol, ethanol, dimethylformamide, dichloromethane and corresponding mixtures, if appropriate in the presence of an organic or inorganic base, preferably selected from the group consisting of triethylamine, dimethylaminopyridine, pyridine and diisopropylamine, at temperatures of from -70 °C to 100 °C.

The compounds of the above-indicated formulae (Q-II), (Q-III), (Q-IV), and (Q-V) are each commercially available and/or can be prepared using conventional processes known to the person skilled in the art. In particular, processes to prepare these compounds are e.g. disclosed in WO 2010/127855-A2, and WO 2010/127856-A2. The corresponding parts of these references are hereby deemed to be part of the disclosure.

All reactions which can be applied for synthesizing the compounds according to the present invention can each be carried out under the conventional conditions with which the person skilled in the art is familiar, for example with regard to pressure or the order in which the components are added. If appropriate, the person skilled in the art can determine the optimum procedure under the respective conditions by carrying out simple preliminary tests. The intermediate and end products obtained using the reactions described hereinbefore can each be purified and/or isolated, if desired and/or required, using conventional methods known to the person skilled in the art. Suitable purifying processes are for example extraction processes and chromatographic processes such as column chromatography or preparative chromatography. All of the process steps of the reaction sequences which can be applied for synthesizing the compounds according to the present invention as well as the respective purification and/or isolation of intermediate or end products, can be carried out partly or completely under an inert gas atmosphere, preferably under a nitrogen atmosphere.

The substituted compounds according to the invention can be isolated both in the form of their free bases, and also in the form of corresponding salts, in particular physiologically acceptable salts, and further in the form of a solvate such as hydrate.

The free bases of the respective substituted compounds according to the invention can be converted into the corresponding salts, preferably physiologically acceptable salts, for example by reaction with an inorganic or organic acid, preferably with hydrochloric acid, hydrobromic acid, sulphuric acid, methanesulphonic acid, p-toluenesulphonic acid, carbonic acid, formic acid, acetic acid, oxalic acid, succinic acid, tartaric acid, mandelic acid, fumaric acid, maleic acid, lactic acid, citric acid, glutamic acid, saccharic acid, monomethylsebacic acid, 5-oxoproline, hexane-1-sulphonic acid, nicotinic acid, 2, 3 or 4-aminobenzoic acid, 2,4,6-trimethylbenzoic acid, α-lipoic acid, acetyl glycine, hippuric acid, phosphoric acid and/or aspartic acid. The free bases of the respective inventive substituted compounds and of corresponding stereoisomers can likewise be converted into the corresponding physiologically acceptable salts using the free acid or a salt of a sugar additive, such as for example saccharin, cyclamate or acesulphame.

Accordingly, the substituted compounds according to the invention such as the free acids of the substituted compounds according to the invention can be converted into the corresponding physiologically acceptable salts by reaction with a suitable base. Examples include the alkali metal salts, alkaline earth metals salts or ammonium salts [NHₓR₄₋ₓ]⁺, in which x = 0, 1, 2, 3 or 4 and R represents a branched or unbranched C₁₋₄ alkyl residue.

The substituted compounds according to the invention and of corresponding stereoisomers can if appropriate, like the corresponding acids, the corresponding bases or salts of these compounds, also be obtained in the form of their solvates, preferably in the form of their hydrates, using conventional methods known to the person skilled in the art.

If the substituted compounds according to the invention are obtained, after preparation thereof, in the form of a mixture of their stereoisomers, preferably in the form of their racemates or other mixtures of their various enantiomers and/or diastereomers, they can be separated and if appropriate isolated using conventional processes known to the person skilled in the art. Examples include chromatographic separating processes, in particular liquid chromatography processes under normal pressure or under elevated pressure, preferably MPLC and HPLC processes, and also fractional crystallisation processes. These processes allow individual enantiomers, for example diastereomeric salts formed by means of chiral stationary phase HPLC or by means of crystallisation with chiral acids, for example (+)-tartaric acid, (-)-tartaric acid or (+)-10-camphorsulphonic acid, to be separated from one another.

The chemicals and reaction components used in the reactions and schemes described below are available commercially or in each case can be prepared by conventional methods known to the person skilled in the art. 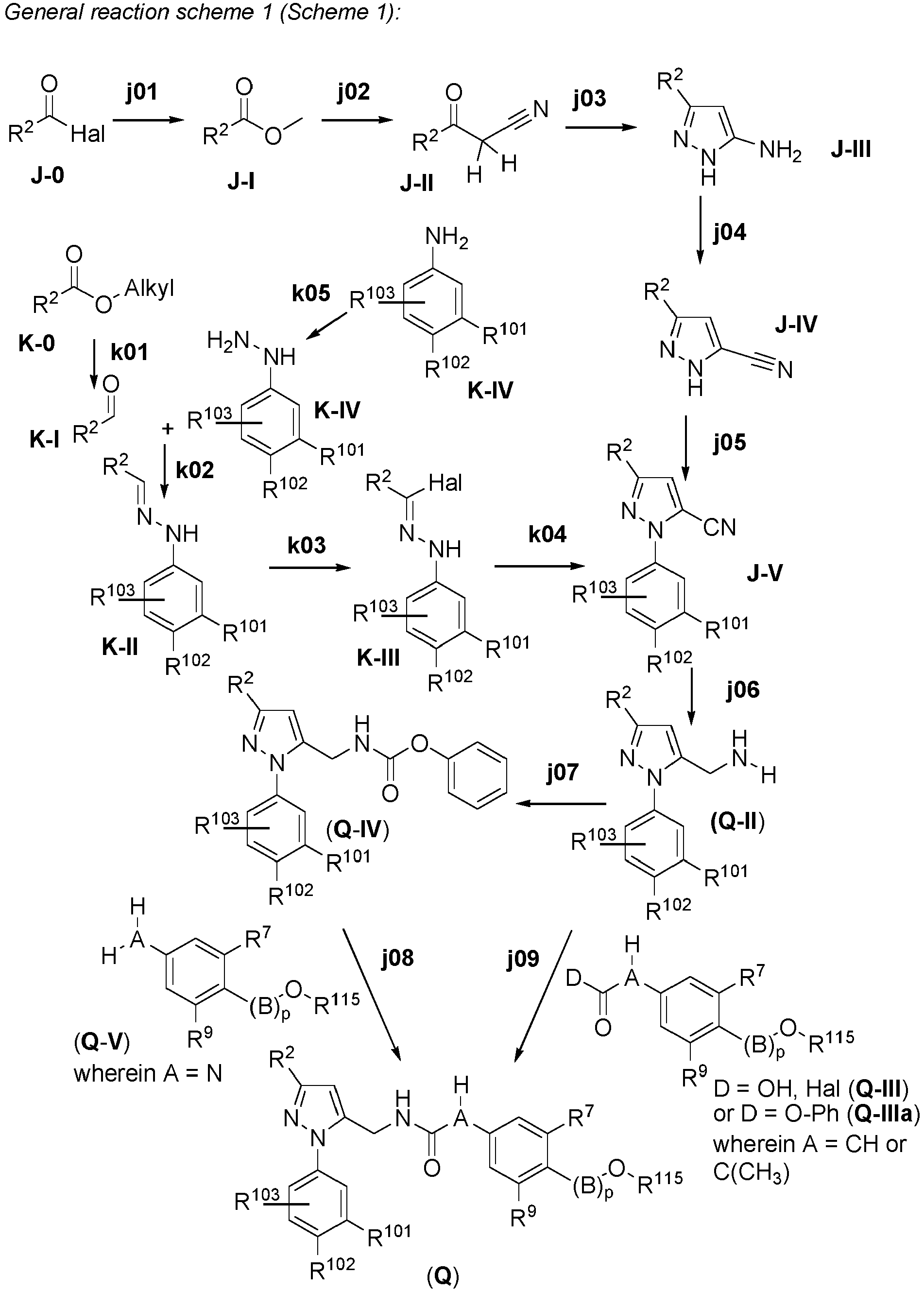 In step **j01** an acid halide **J-0**, in which Hal preferably represents Cl or Br, can be esterified using methanol to form the compound **J-I** by means of methods with which the person skilled in the art is familiar.

In step **j02** the methyl pivalate **J-I** can be converted into an oxoalkylnitrile **J-II** by means of methods known to the person skilled in the art, such as for example using acetonitrile CH₃-CN, if appropriate in the presence of a base.

In step **j03** the compound **J-II** can be converted into an amino-substituted pyrazolyl derivative **J-III** by means of methods known to the person skilled in the art, such as for example using hydrazine hydrate, with cyclization.

In step **j04** the amino compound **J-III** can first be converted into a diazonium salt by means of methods known to the person skilled in the art, such as for example using nitrite, and the diazonium salt can be converted into a cyano-substituted pyrazolyl derivative **J-IV** with elimination of nitrogen using a cyanide, if appropriate in the presence of a coupling reagent.

In step **j05** the compound J-IV can be substituted in the N position by means of methods known to the person skilled in the art, for example using a halide of part structure (QS2), i.e. Hal-(QS2), if appropriate in the presence of a base and/or a coupling reagent, wherein Hal is preferably Cl, Br or I, or using a boronic acid B(OH)₂(QS2) or a corresponding boronic acid ester, if appropriate in the presence of a coupling reagent and/or a base and the compound **J-V** can in this way be obtained.

Alternatively, a second synthesis pathway, in which in step **k01** an ester **K-0** is first reduced to form the aldehyde **K-I** by means of methods known to the person skilled in the art, for example using suitable hydrogenation reagents such as metal hydrides, is suitable for preparing the compound **J-V.**

In step **k02** the aldehyde **K-I** can then be reacted with a hydrazine **K-V**, which can be obtained in step **k05**, starting from the primary amine **K-IV**, by means of methods known to the person skilled in the art, to form the hydrazine **K-II** by means of methods known to the person skilled in the art with elimination of water.

In step **k03** the hydrazine **K-II** can be halogenated, preferably chlorinated, by means of methods known to the person skilled in the art with the double bond intact, such as for example using a chlorination reagent such as NCS, and the compound **K-III** can in this way be obtained. In step **k04** the hydrazonoyl halide **K-III** can be converted into a cyano-substituted compound **J-V** by means of methods known to the person skilled in the art, such as for example using a halogen-substituted nitrile, with cyclisation.

In step **j06** the compound **J-V** can be hydrogenated by means of methods known to the person skilled in the art, for example using a suitable catalyst such as palladium/activated carbon or using suitable hydrogenation reagents, and the compound (**Q-II**) can in this way be obtained.

In step **j07** the compound (**Q-II**) can be converted into the compound (**Q-IV**) by means of methods known to the person skilled in the art, such as for example using phenyl chloroformate, if appropriate in the presence of a coupling reagent and/or a base. In addition to the methods disclosed in the present document for preparing unsymmetrical ureas using phenyl chloroformate, there are further processes with which the person skilled in the art is familiar, based on the use of activated carbonic acid derivatives or isocyanates, if appropriate.

In step **j08** the amine (**Q-V**) can be converted into the urea compound (**Q**) (wherein A = N). This can be achieved by reaction with (**Q-IV**) by means of methods with which the person skilled in the art is familiar, if appropriate in the presence of a base.

In step **j09** the amine (**Q-II**) can be converted into the amide (Q) (wherein A = CH or C(CH₃)). This can for example be achieved by reaction with an acid halide, preferably a chloride of formula **(Q-III)** with D = Hal by means of methods with which the person skilled in the art is familiar, if appropriate in the presence of a base or by reaction with an acid of formula **(Q-III)** with D = OH, if appropriate in the presence of a suitable coupling reagent, for example HATU or CDI, if appropriate with the addition of a base. Further, the amine **(Q-II**) may be converted into the amide (**Q**) (wherein A = CH or C(CH₃)) by reaction of a compound **(Q-IIIa)** by means of methods with which the person skilled in the art is familiar, if appropriate in the presence of a base.

The compounds according to general formula (**Q**), wherein A = N, may be further prepared by a reaction sequence according to general reaction scheme 2.

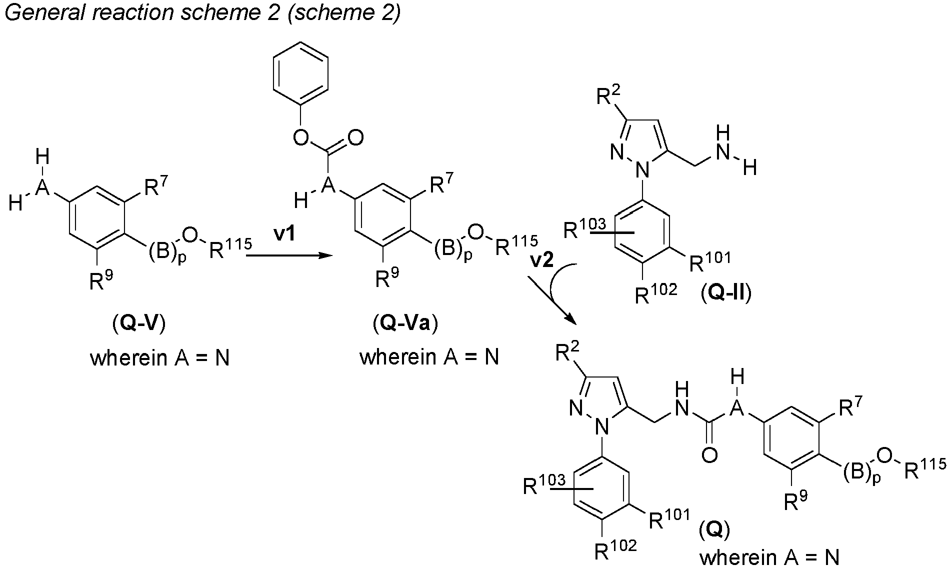

In step **v1** the compound (**Q-V**) can be converted into the compound (**Q-Va**) by means of methods known to the person skilled in the art, such as for example using phenyl chloroformate, if appropriate in the presence of a coupling reagent and/or a base. In addition to the methods disclosed in the present document for preparing unsymmetrical ureas using phenyl chloroformate, there are further processes with which the person skilled in the art is familiar, based on the use of activated carbonic acid derivatives or isocyanates, if appropriate.

In step **v2** the amine (**Q-II**) can be converted into the urea compound (**Q**) (wherein A = N). This can be achieved by reaction with (**Q-Va**) by means of methods with which the person skilled in the art is familiar, if appropriate in the presence of a base.

The methods with which the person skilled in the art is familiar for carrying out the reaction steps **j01** to **j09** and also **k01** to **k05** as well as **v1** and **v2** may be inferred from the standard works on organic chemistry such as, for example, J. March, Advanced Organic Chemistry, Wiley & Sons, 6th edition, 2007; F. A. Carey, R. J. Sundberg, Advanced Organic Chemistry, Parts A and B, Springer, 5th edition, 2007; team of authors, Compendium of Organic Synthetic Methods, Wiley & Sons. In addition, further methods and also literature references can be issued by the common databases such as, for example, the Reaxys® database of Elsevier, Amsterdam, NL or the SciFinder® database of the American Chemical Society, Washington, US.

### EXAMPLES

The following examples further illustrate the invention but are not to be construed as limiting its scope.

The indication "equivalents" ("eq." or "eq" or "equiv.") means molar equivalents, "RT" or "rt" means room temperature (23 ± 7 °C), "M" are indications of concentration in mol/l, "aq." means aqueous, "sat." means saturated, "sol." means solution, "conc." means concentrated.

Further abbreviations:
- d: days
- AcOH: acetic acid
- BH₃•S(CH₃)₂: borane-methyl sulfide complex (BH₃-DMS)
- BINAP: 2,2'-bis(diphenylphosphino)-1,1'-binaphthyl
- brine: saturated aqueous sodium chloride solution
- n-BuLi: n-butyllithium
- CC: column chromatography on silica gel
- CH₃COOK: potassium acetate
- DBU: 1,8-diazabicyclo[5.4.0]undec-7-en
- DCM: dichloromethane
- DIPEA: diisopropylethylamine
- DMA: dimethylamine
- DMAP: 4-dimethylaminopyridine
- DMF: N,N-dimethylformamide
- DMSO: dimethyl sulfoxide
- DPPF: 1,1'-bis(diphenylphosphino)ferrocene
- EDC: N-(3-dimethylaminopropyl)-N'-ethylcarbodiimide
- EDCI: N-ethyl-N'-(3-dimethylaminopropyl)carbodiimide hydrochloride
- ether: diethyl ether
- EtOAc: ethyl acetate
- EtOH: ethanol
- h: hour(s)
- GC: gas chromatography
- HBTU: O-(benzotriazol-1-yl)-N,N,N',N'-tetramethyluronium hexafluorophosphate
- HOBt: N-hydroxybenzotriazole
- H₂O: water
- m/z: mass-to-charge ratio
- MeOH: methanol
- MeCN: acetonitrile
- min or min.: minutes
- MS: mass spectrometry
- NBS: N-bromosuccinamide
- TEA: triethylamine
- NiBr₂ bipy: complex of nickel(II) bromide and 2,2'-bipyridine
- NMO: N-methylmorpholine-*N*-oxide
- NMP: N-methyl-2-pyrrolidon
- Pd / C: palladium on charcoal
- Pd₂(dba)₃: tris(dibenzylideneacetone)dipalladium(0)
- Pd(dppDCl₂: [1,1'-bis(diphenylphosphino)ferrocene]dichloropalladium(II)
- Pd(PPh₃)₄: tetrakis(triphenylphosphine)palladium(0)
- PE: petroleum ether
- TBAF: tetra-n-butylammonium fluoride
- TBTU: O-(benzotriazol-1-yl)-N,N,N',N'-tetramethyluronium tetrafluoroborate
- TBDMSCl: tert-butyldimethylsilyl chloride
- TLC: thin layer chromatography
- TFA: trifluoroacetic acid
- THF: tetrahydrofuran
- v/v: volume to volume
- w/w: weight in weight

The yields of the compounds prepared were not optimized.

All temperatures are uncorrected.

All starting materials which are not explicitly described were either commercially available (the details of suppliers such as for example Acros, Avocado, Aldrich, Apollo, Bachem, Fluka, FluoroChem, Lancaster, Manchester Organics, MatrixScientific, Maybridge, Merck, Rovathin, Sigma, TCI, Oakwood, etc. can be found in the Symyx® Available Chemicals Database of MDL, San Ramon, US or the SciFinder® Database of the ACS, Washington DC, US, respectively, for example) or the synthesis thereof has already been described precisely in the specialist literature (experimental guidelines can be found in the Reaxys® Database of Elsevier, Amsterdam, NL or the SciFinder® Database of the ACS, Washington DC, US, repspectively, for example) or can be prepared using the conventional methods known to the person skilled in the art.

The stationary phase used for the column chromatography was silica gel 60 (0.04 - 0.063 mm) from E. Merck, Darmstadt.

The mixing ratios of solvents or eluents for chromatography are specified in v/v.

All the intermediate products and exemplary compounds were analytically characterized by means of ¹H-NMR spectroscopy. In addition, mass spectrometry tests (MS, m/z for [M+H]⁺) were carried out for all the exemplary compounds and selected intermediate products.

### Synthesis of selected intermediate products:

### 1. Synthesis of (3-tert-butyl-1-(3-chlorophenyl)-1H-pyrazol-5-yl)methanamine (steps j01-j06)

Step **j01:** Pivaloyl chloride (J-0) (1 eq., 60 g) was added dropwise to a solution of methanol (120 mL) within 30 min at 0 °C and the mixture was stirred for 1 h at room temperature. After the addition of water (120 mL), the separated organic phase was washed with water (120 mL), dried over sodium sulphate and codistilled with dichloromethane (150 mL). The liquid product **J-I** was able to be obtained at 99 % purity (57 g).
Step **j02**: NaH (50 % in paraffin oil) (1.2 equivalents, 4.6 g) was dissolved in 1,4-dioxane (120 mL) and the mixture was stirred for a few minutes. Acetonitrile (1.2 equivalents, 4.2 g) was added dropwise within 15 min and the mixture was stirred for a further 30 min. The methyl pivalate (**J-I**) (1 equivalents, 10 g) was added dropwise within 15 min and the reaction mixture was refluxed for 3 h. After complete reaction, the reaction mixture was placed in iced water (200 g), acidified to pH 4.5 and extracted with dichloromethane (12 x 250 mL). The combined organic phases were dried over sodium sulphate, distilled and after recrystallisation from n-hexane (100 mL) 5 g of the product **(J-II**) (51 % yield) was able to be obtained as a solid brown substance.
Step **j03**: At room temperature 4,4-dimethyl-3-oxopentanenitrile (**J-II**) (1 equivalents, 5 g) was taken up in ethanol (100 mL), mixed with hydrazine hydrate (2 equivalents, 4.42 g) and refluxed for 3 h. The residue obtained after removal of the ethanol by distillation was taken up in water (100 mL) and extracted with ethyl acetate (300 mL). The combined organic phases were dried over sodium sulphate, the solvent was removed under vacuum and the product (**J-III**) (5 g, 89 % yield) was obtained as a light red solid after recrystallisation from n-hexane (200 mL).
Step **j04**: 3-Tert-butyl-1H-pyrazol-5-amine (**J-III**) (1 equivalents, 40 g) was dissolved in diluted HCI (120 mL of HCI in 120 mL of water) and mixed dropwise with NaNO₂(1.03 equivalents, 25 g in 100 mL) at 0 - 5 °C over a period of 30 min. After stirring for 30 minutes, the reaction mixture was neutralised with Na₂CO₃. A diazonium salt obtained by reaction of KCN (2.4 equivalents, 48 g), water (120 mL) and CuCN (1.12 equivalents, 31 g) was added dropwise to the reaction mixture within 30 min and the mixture was stirred for a further 30 min at 75 °C. After complete reaction, the reaction mixture was extracted with ethyl acetate (3 x 500 mL), the combined organic phases were dried over sodium sulphate and the solvent was removed under vacuum. The purification (silica gel: 100-200 mesh, eluent: 20 % ethyl acetate/n-hexane) of the residue by column chromatography produced a white solid (**J-IV**) (6.5 g, 15 %).
Step **j05** *(method 1):*
   3-tert.-butyl-1 H-pyrazol-5-carbonitrile (**J-IV**) (10 mmol) was added to a suspension of NaH (60 %) (12.5 mmol) in dimethylformamide (20 mL) at room temperature while stirring. After stirring for 15 minutes, 1-iodo-3-chlorobenzene (37.5 mmol) was added dropwise to this reaction mixture at room temperature. After stirring for 30 min at 100 °C, the reaction mixture was mixed with water (150 mL) and extracted with dichloromethane (3 x 75 mL). The combined organic extracts were washed with water (100 mL) and sat. NaCl solution (100 mL) and dried over magnesium sulphate. After removal of the solvent under vacuum, the residue was purified by column chromatography (silica gel: 100-200 mesh, eluent: various mixtures of ethyl acetate and cyclohexane as the mobile solvent) and the product **J-V** was obtained.
Step **j05** (*method 2):*
   A mixture of 3-tert-butyl-1 H-pyrazol-5-carbonitrile (**J-IV**) (10 mmol), a boronic acid B(OH)₂(3-chlorophenyl) or a corresponding boronic acid ester (20 mmol) and copper (II) acetate (15 mmol) is placed in dichloromethane (200 mL), mixed with pyridine (20 mmol) while stirring at room temperature and the mixture is stirred for 16 h. After removal of the solvent under vacuum, the residue obtained is purified by column chromatography (silica gel: 100-200 mesh, eluent: various mixtures of ethyl acetate and cyclohexane as the mobile solvent) and the product J-V is in this way obtained.
Step **j06:** (*method 1):*
   **J-V** was dissolved together with palladium on carbon (10 %, 500 mg) and concentrated HCI (3 mL) in methanol (30 mL) and exposed to a hydrogen atmosphere for 6 h at room temperature. The reaction mixture was filtered over celite and the filtrate was concentrated under vacuum. The residue was purified by means of flash chromatography (silica gel: 100-200 mesh, eluent: ethyl acetate) and the product (**U-II**) was in this way obtained.
Step j06: *(method 2):*
   **J-V** was dissolved in tetrahydrofuran (10 mL) and BH₃•S(CH₃)₂ (2.0 M in tetrahydrofuran, 3 mL, 3 equivalents) was added thereto. The reaction mixture was heated to reflux for 8 h, aq. 2 N HCI (2 N) was added thereto and the reaction mixture was refluxed for a further 30 minutes. The reaction mixture was mixed with aq. NaOH solution (2N) and washed with ethyl acetate. The combined organic phases were washed with sat. aq. NaCl solution and dried over magnesium sulphate. The solvent is removed under vacuum and the residue is purified by column chromatography (silica gel: 100-200 mesh, eluent: various mixtures of dichloromethane and methanol as the mobile solvent) and the product (**U-II**) is in this way obtained.

The following further intermediate products were/can be synthesized in a similar manner using the process described hereinbefore under **1**.:

| |
|---|
| (3-tert-butyl-1-(4-fluoro-3-chlorophenyl)-1 H-pyrazol-5-yl)methanamine |
| (3-tert-butyl-1-(3,4-difluoro-phenyl)-1 H-pyrazol-5-yl)methanamine |
| (3-tert-butyl-1-(3-fluorophenyl)-1 H-pyrazol-5-yl)methanamine |
| (3-tert-butyl-1-(4-fluorophenyl)-1 H-pyrazol-5-yl)methanamine |
| (3-tert-butyl-1-(3-trifluoromethylphenyl)-1 H-pyrazol-5-yl)methanamine |
| (3-tert-butyl-1-(3-trifluoromethoxyphenyl)-1 H-pyrazol-5-yl)methanamine |
| (3-tert-butyl-1-(3-methylphenyl)-1 H-pyrazol-5-yl)methanamine |

### 2. Synthesis of 1-(3-chlorophenyl)-3-(trifluoromethyl)-1H-pyrazol-5-yl-methanamine (steps k01-k05 and j06)

Step **k01:** LAIH (lithium aluminium hydride) (0.25 equivalents, 0.7g) was dissolved in dry diethyl ether (30 mL) under a protective gas atmosphere and stirred for 2 h at room temperature. The suspension obtained was taken up in diethyl ether (20 mL). Ethyl-2,2,2-trifluoroacetate (**K-0**) (1 equivalent, 10 g) was taken up in dry diethyl ether (20 mL) and added dropwise to the suspension at -78 °C over a period of 1 h. The mixture was then the stirred for a further 2 h at -78 °C. ethanol (95 %) (2.5 mL) was then added dropwise, the reaction mixture was heated to room temperature and placed on iced water (30 mL) with concentrated H₂SO₄ (7.5 mL). The organic phase was separated and concentrated under vacuum and the reaction product **K-I** was immediately introduced into the next reaction step **k02.**
Step **k05:** 3-chloroaniline (**K-IV**) (1 equivalent, 50 g) was dissolved at -5 to 0 °C in concentrated HCI (300 mL) and stirred for 10 min. A mixture of NaNO₂ (1.2 equivalents, 32.4 g), water (30 mL), SnCl₂•2H₂O (2.2 equivalents, 70.6 g) and concentrated HCI (100 mL) was added dropwise over a period of 3 h while maintaining the temperature. After stirring for a further 2 h at -5 to 0 °C, the reaction mixture was set to pH 9 using NaOH solution and extracted with ethyl acetate (250 mL). The combined organic phases were dried over magnesium sulphate and the solvent was removed under vacuum. The purification by column chromatography (silica gel: 100-200 mesh, eluent: 8 % ethyl acetate/n-hexane) produced 40 g (72 %) of (3-chlorophenyl)hydrazine (**K-IV**) as a brown oil.
Step **k02:** The aldehyde (**K-I**) (2 equivalents, 300 mL) obtained from **k01** and (3-chlorophenyl)hydrazine (**K-IV**) (1 equivalent, 20 g) were placed in ethanol (200 mL) and refluxed for 5 h. The solvent was removed under vacuum, the residue was purified by column chromatography (silica gel: 100-200 mesh, eluent: n-hexane) and the product (25 g, 72 %) **K-II** was obtained as a brown oil.
Step **k03:** The hydrazine **K-II** (1 equivalent, 25 g) was dissolved in dimethylformamide (125 mL). N-chlorosuccinimide (1.3 equivalents, 19.5 g) was added portionwise at room temperature within 15 min and the mixture was stirred for 3 h. The dimethylformamide was removed by distillation and the residue was taken up in ethyl acetate. The ethyl acetate was removed under vacuum, the residue obtained was purified by column chromatography (silica gel: 100-200 mesh, eluent: n-hexane) and the product **K-III** (26.5 g, 92 %) was obtained as a pink-coloured oil.
Step **k04:** At room temperature the hydrazonoyl chloride **K-III** (1 equivalent, 10 g) was taken up in toluene (150 mL) and mixed with 2-chloroacrylonitrile (2 equivalents, 6.1 mL) and triethylamine (2 equivalents, 10.7 mL). This reaction mixture was stirred for 20 h at 80 °C. The mixture was then diluted with water (200 mL) and the phases were separated. The organic phase was dried over magnesium sulphate and the solvent was removed under vacuum. The residue was purified by means of column chromatography (silica gel: 100-200 mesh, eluent: 5 % ethyl acetate/n-hexane) and the product (5.5 g, 52 %) was obtained as a white solid J-V.
Step **j06** *(method 3):*
   The carbonitrile **J-V** (1 equivalent, 1 g) was dissolved in methanolic ammonia solution (150 mL, 1:1) and hydrogenated in an H-cube (10 bar, 80 °C, 1 mL/min, 0.25 mol/L). After removal of the solvent under vacuum, (1-(3-chlorophenyl)-3-(trifluoromethyl)-1H-pyrazol-5-yl)methanamine (**II**) was able to be obtained as a white solid (0.92 g, 91 %).

The following further intermediate products were/can be synthesized in a similar manner using the process described hereinbefore under **2**.:

| |
|---|
| (1-(3-fluorophenyl)-3-(trifluoromethyl)-1 H-pyrazol-5-yl)methanamine |
| (1-(4-fluorophenyl)-3-(trifluoromethyl)-1 H-pyrazol-5-yl)methanamine |
| (1-(3-chloro-4-fluorophenyl)-3-(trifluoromethyl)-1 H-pyrazol-5-yl)methanamine |
| (1-(3,4-difluorophenyl)-3-(trifluoromethyl)-1 H-pyrazol-5-yl)methanamine |
| (1-(3-methoxyphenyl)-3-(trifluoromethyl)-1H-pyrazol-5-yl)methanamine |
| (1-(3-isopropylphenyl)-3-(trifluoromethyl)-1 H-pyrazol-5-yl)methanamine |
| (1-(3-trifluoromethylphenyl)-3-(trifluoromethyl)-1H-pyrazol-5-yl)methanamine |

### 3. Preparation of methyl phenyl (3-tert-butyl-1-(3-chlorophenyl)-1H-pyrazol-5-yl)methylcarbamate

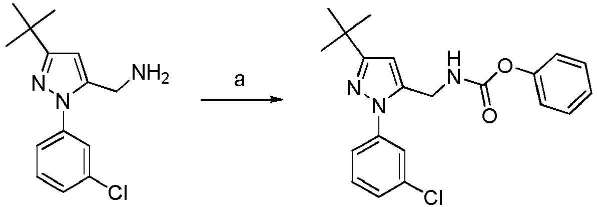

Step a: To a solution of (3-tert-butyl-1-(3-chlorophenyl)-1H-pyrazol-5-yl)methanamine (5 g, 18 mmol) in dimethylformamide (25 mL), potassium carbonate (9.16 g, 66 mmol, 3.5 eq) was added and cooled the contents to 0°C. Then phenyl chloroformate (3.28 g (2.65 mL), 20 mmol, 1.1 equivalents) was added dropwise for 15 minutes and the overall reaction mixture was stirred for another 15 minutes at 0 °C. Progress of the reaction was monitored by TLC (20 % ethyl acetate-n-hexane). On completion of the reaction, reaction contents were filtered, filtrate was diluted with cold water (100 mL) and the product extracted with ethyl acetate (3 × 25 mL). Combined organic layer was washed with brine solution (100 mL), dried over sodium sulphate and concentrated under reduced pressure. Crude obtained was purified by column chromatography (silica gel: 100-200 mesh, eluent: 10% ethyl acetate in n-hexane) to yield the required product as a white solid (3.2 g, 45 %).

### 4. Preparation of (1-(3-chlorophenyl)-3-cyclopropyl-1H-pyrazol-5-yl)methanamine hydrochloride

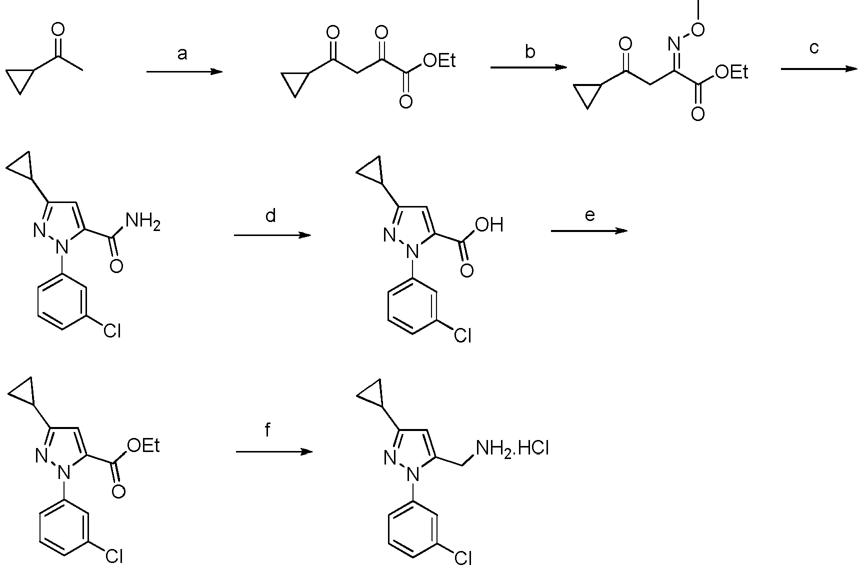

Step **a:** To a solution of sodium ethoxide (freshly prepared by dissolving sodium (1 g, 8.2 mmol, 1.2 equivalents) in ethanol (30 mL)), diethyl oxalate (0.92 mL, 6.85 mmol, 1 equivalent) was added at room temperature followed by addition of cyclopropyl methyl ketone (0.74 mL, 7.5 mmol, 1.1 equivalents) dropwise at 0 °C. The reaction mixture was slowly warmed to room temperature and stirred for 3 h. Ice cold water (10 mL) was added and ethanol was evaporated under reduced pressure. The residual aqueous layer was diluted with 2 N aq. HCI (15mL) and extracted with diethyl ether (2 × 25 mL). The organic layer was washed with brine solution and dried over sodium sulphate, filtered and concentrated to give a pale brown liquid (400 mg, 31 %).
Step **b:** To a solution of step-**a** product (200 mg, 0.543 mmol, 1 equivalent) in ethanol (8 mL), methoxylamine hydrochloride (30 % solution in water, 0.4 mL, 0.651 mmol, 1.2 equivalents) was added at room temperature and the reaction mixture stirred for 1 h. ethanol was evaporated under reduced pressure and the residual aqueous layer was extracted with ethyl acetate (15 mL). The organic layer was washed with water (10 mL), brine solution (10 mL), dried over sodium sulphate, filtered and concentrated under reduced pressure to give a pale yellow liquid (180 mg, 78 %).
Step **c:** A mixture of step-**b** product (1.1 g, 5.164 mmol, 1 equivalent) and 3-chlorophenyl hydrazine hydrochloride (1.84 g, 10.27 mmol, 2 equivalents) was taken in acetic acid (20 mL), 2-methoxy ethanol (10 mL) and the reaction mixture was heated at 105 °C for 3 h. Solvent was evaporated and the residue was extracted with ethyl acetate (60 mL). The organic layer washed with water (10 mL), brine solution (10 mL), dried over sodium sulphate, filtered and concentrated under reduced pressure to give a residue. Purification by column chromatography (silica gel: 100-200 mesh; eluent: ethyl acetate-petroleum ether (4:96)) afforded a pale brown semi solid (1.15g, 77 %).
Step **d:** To a solution of step-c product (2.5 g, 8.62 mmol, 1 eq) in tetrahydrofuran (15 mL) - methanol (9 mL) - water (3 mL), lithium hydroxide (1.08 g, 25.71 mmol, 3 equivalents) was added at 0 °C and the reaction mixture was stirred for 2 h at room temperature. Solvent was evaporated and pH of the residue was adjusted to ∼3 sing 2 N aqueous HCl (1.2 mL). The acidic aqueous layer was extracted with ethyl acetate (2 × 60 mL); the combined organic layer washed with water (10 mL), brine solution (10 mL), dried over sodium sulphate, filtered and concentrated under reduced pressure to give an off white solid (1.4 g, 62 %).
Step **e:** To a solution of step-**d** product (1.4 g, 5.34 mmol, 1 equivalent) in 1,4-dioxane (30 mL), pyridine (0.25 mL, 3.2 mmol, 0.6 equivalents) and di-tert-butyl dicarbonate (1.4 mL, 6.37 mmol, 1.2 equivalents) were added at 0 °C and the resulting mixture was stirred for 30 minutes at the same temperature. Ammonium bicarbonate (0.84 g, 10.63 mmol, 2 equivalents) was added at 0 °C and the reaction mixture was stirred at room temperature overnight. The reaction mixture was diluted with water (10 mL) and the aqueous layer was extracted with ethyl acetate (2 x 30 mL). The organic layer was washed with 2N HCl (20 mL), water (10 mL), brine solution (10 mL), dried over sodium sulphate, filtered and concentrated under reduced pressure to give a residue. Purification by column chromatography (silica gel: 100-200 mesh; eluent: ethyl acetate-petroleum ether (16:84)) gave a white solid (1 g, 72 %).
Step **f:** To a solution of step-**e** product (2 g, 7.66 mmol, 1 equivalent) in tetrahydrofuran (25 mL), BH₃.DMS (1.44 mL, 15.32 mmol, 2 equivalents) was added at 0 °C and the reaction mixture was heated at 70°C for 3 h. The reaction mixture was cooled to 0 °C and methanol (15 mL) was added and reaction mixture heated at reflux for 1 h. The reaction mixture was brought to room temperature and solvent was evaporated under reduced pressure. The residue was dissolved in ether (15 mL), cooled to 0 °C and a solution of HCl in 1,4-dioxane (3 mL) was added (pH of the reaction mixture ∼4). The precipitated solid was filtered and washed with diethyl ether (5 mL, thrice) to give the hydrochloride salt compound as a white solid (600 mg, 28 %).

### Synthesis of exemplary compounds:

### 1. Preparation of amides (A = CH or C(CH₃))

General directions for reacting amines of general formula (**Q-II**) with carboxylic acids of general formula or carboxylic acid derivatives of general formula (**Q-III**) to form compounds of general formula (Q), wherein A = CH or C(CH₃) (amides), as in scheme 1 (step **j09**).

### 1.1 Method A:

The acid of general formula (**Q-III**) (1 equivalent), the amine of general formula (**Q-II**) (1.2 equivalents) and EDCI (1.2 equivalents) are stirred in DMF (10 mmol of acid/20 mL) for 12 hours at RT and water is subsequently added thereto. The reaction mixture is repeatedly extracted with EtOAc, the aqueous phase is saturated with NaCl and subsequently reextracted with EtOAc. The combined organic phases are washed with 1 N HCI and brine, dried over magnesium sulphate and the solvent is removed under vacuum. The residue is purified by means of flash chromatography (SiO₂, EtOAc/hexane in different ratios such as 1:2) and the product (**Q**) is in this way obtained.

### 1.2 Method B:

The acid of general formula (**Q-III**) (1 equivalent) and the amine of general formulae (**Q-II**) (1.1 equivalents) are dissolved in dichloromethane (1 mmol of acid in 6 mL) and mixed with EDCI (1.5 equivalents), HOBt (1.4 equivalents) and triethylamine (3 equivalents) at 0 °C. The reaction mixture is stirred for 20 h at room temperature and the crude product is purified by means of column chromatography (SiO₂, n-hexane/EtOAc in different ratios such as 2:1) and (**Q**) is in this way obtained.

### 1.3 Method C:

The acid of general formula (**Q-III**) (1 equivalent) is first mixed with a chlorinating agent, preferably with thionyl chloride and the mixture obtained in this way is boiled under reflux and the acid (**Q-III**) is in this way converted into the corresponding acid chloride. The amine of general formulae **(Q-II**) (1.1 equivalents) is dissolved in dichloromethane (1 mmol of acid in 6 mL) and mixed with triethylamine (3 equivalents) at 0 °C. The reaction mixture is stirred for 20 h at room temperature and the crude product is purified by means of column chromatography (SiO₂, n-hexane/EtOAc in different ratios such as 2:1) and (**Q**) is in this way obtained.

### 1.4 Method D:

The phenyl ester (**Q-III***)* (1 equivalent) and the corresponding amine (**Q-II**) (1.1 equivalents) are dissolved in THF (10 mmol of the reaction mixture in 120 mL) and stirred for 16 h at room temperature after addition of DBU (1.5 equivalents). After removal of the solvent under vacuum, the residue obtained is purified by means of flash chromatography (SiO₂, EtOAc/hexane in different ratios such as 1:1) and (**Q**) is in this way obtained.

### 2. Preparation of ureas (A = N)

General directions for reacting amines of general formula (**Q-II**) or (**Q-V**) with phenyl chloroformate to form compounds of formula (**Q-IV**) or (**Q-Va**) (scheme 1, step **j07** and scheme 2, **step v1**) and subsequent reaction of compounds of formula (**Q-IV**) with amines of general formula (**Q-V**) (scheme 1, step **j08**) or of compounds of formula (**Q-Va**) with amines of general formula (**Q-II**) (scheme 2, step **v2**) to form compounds of general formula (**Q**), wherein A = N:
Step **j07**/step **v1**: The amine of general formula (**Q-II**) or (**Q-V**) (1 equivalent) is placed in dichloromethane (10 mmol of amine in 70 mL) and phenyl chloroformate (1.1 equivalents) is added thereto at room temperature and the mixture is stirred for 30 min. After removal of the solvent under vacuum, the residue is purified by means of flash chromatography (SiO₂, diethyl ether/hexane in different ratios such as 1:2) and (**Q-IV**) or (**Q-Va**) is in this way obtained.
Step **j08**/step **v2:** The carbamic acid phenyl ester (**Q-IV**) or (**Q-Va**) obtained (1 equivalent) and the corresponding amine (**Q-V**) or (**Q-II**) (1.1 equivalents) are dissolved in THF (10 mmol of the reaction mixture in 120 mL) and stirred for 16 h at room temperature after addition of DBU (1.5 equivalents). After removal of the solvent under vacuum, the residue obtained is purified by means of flash chromatography (SiO₂, EtOAc/hexane in different ratios such as 1:1) and (**Q**) is in this way obtained.

### Synthesis of the exemplary compounds:

The exemplary compounds **A1** to **A159** were obtained by one of the methods disclosed before and thereafter. The person skilled in the art is aware which method has to be employed to obtain a particular exemplary compound.

Compounds A27, A54, A55, A56, A57, A58, A59, A63, A65, A66, A70, A80, A82 and A96 are inventive compounds.

### Detailed synthesis of selected exemplary compounds

### Synthesis of example A17: 1-[[5-tert-Butyl-2-(3-chlorophenyl)-2H-pyrazol-3-yl]-methyl]-3-[3-fluoro-4-(2 -methoxy-ethoxy)-phenyl]-urea

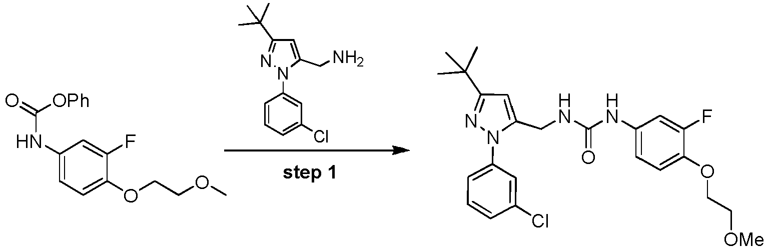

**Step 1:** To a stirred solution of (3-tert-butyl-1-(3-chlorophenyl)-1H-pyrazol-5-yl)methanamine (synthesis described for **example A18**) (81mg, 0.31 mmol, 1.0 eq.) in MeCN (7 mL) was added TEA (0.17 mL, 1.2 mmol, 4.0 eq.) followed by phenyl 3-fluoro-4-(2-methoxyethoxy)phenylcarbamate (95 mg, 0.31mmol, 1.0 eq.) at RT and stirred at reflux for 16 h. The solvent was evaporated under vacuum. The crude obtained was purified by column chromatography (eluent EtOAc/n-hexane 1:1) to yield **example A17** (121 mg; 83%).

### Synthesis of example A18: 1-[[2-(3-Chlorophenyl)-5-(trifluoromethyl)-2H-pyrazol-3-yl]-methyl]-3-[3-fluoro-4-(2-methoxy-ethoxy)-phenyl]-urea

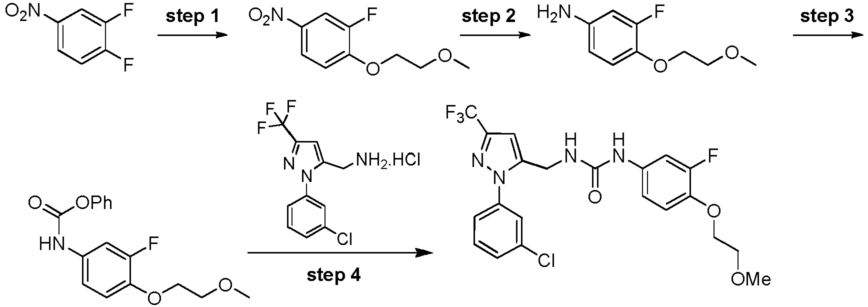

**Step 1:** To a stirred suspension of methyl glycol (2.2 mL, 28.28 mmol, 1.5 eq.) and K₂CO₃ (7.8 g, 56.60 mmol, 3.0 eq.) in DMF (20 mL) was added 1,2-difluoro-4-nitrobenzene (3.0 g, 18.85 mmol, 1.0 eq.) and the mixture was stirred for 16 h at 70°C. The reaction mixture was cooled and diluted with water (30 mL) and extracted with ethyl acetate (40 mL). The organic layer was separated and washed with water (50 mL), brine (50 mL), and dried over sodium sulfate and the solvent evaporated under vacuum. The crude was washed with n-pentane (30 mL) to obtain 2-fluoro-1-(2-methoxyethoxy)-4-nitrobenzene (3 g, 74%, as solid; TLC system: EtOAc/PE (1:9), R_{f}: 0.2).
**Step 2:** To a stirred solution of 2-fluoro-1-(2-methoxyethoxy)-4-nitrobenzene (2.5 g, 11.62 mmol, 1.0 eq.) in ethanol (20 mL) was added 10% Pd/C (500 mg) and stirred in hydrogen gas atmosphere for 16 h at RT. The catalyst was filtered over a celite pad and the filtrate was concentrated to get 3-fluoro-4-(2-methoxyethoxy)aniline (2.0 g, 93.9%; TLC system: EtOAc/PE (3:7), R_{f}: 0.3).
**Step 3:** To a stirred solution of 3-fluoro-4-(2-methoxyethoxy)aniline (2.0 g, 10.8 mmol, 1.0 eq) in acetone (50 mL) was added pyridine (2.5 mL, 32.4 mol, 3.0 eq.) and phenyl chloroformate (1.36 mL, 10.8 mmol, 1.0 eq.) at 0°C and stirred at RT for 1 h. The solvent was evaporated, the residue diluted with EtOAc (50 mL), washed with water (100 mL), brine (20 mL) and evaporated again. The resulting residue was purified by column chromatography using EtOAc/PE (1:4) as eluent to yield phenyl 3-fluoro-4-(2-methoxyethoxy)phenylcarbamate (2.7 g, 76.8%) as a white solid (TLC system: EtOAc/PE (3:7), R_{f}: 0.5).
**Step 4:** To a stirred solution of (1-(3-chlorophenyl)-3-(trifluoromethyl)-1H-pyrazol-5-yl)methanamine hydrochloride (102 mg, 0.327 mmol, 1.0 eq.) in DCM (5.0 mL) was added TEA (1.36 mL, 0.981 mmol, 3.0 eq) followed by phenyl 3-fluoro-4-(2-methoxyethoxy)phenylcarbamate (100 mg, 0.327 mmol, 1.0 eq.) at RT and stirred for 16 h. The reaction mixture was diluted with water (10 mL) and extracted with ethyl acetate (15 mL). The organic layer was washed with water (10 mL) and brine (5 mL), dried over anhydrous Na₂SO₄, and the solvent evaporated under vacuum. The crude obtained was purified by neutral alumina column chromatography using MeOH/CHCl₃ (1:9) as eluent to yield 1-((1-(3-chlorophenyl)-3-(trifluoromethyl)-1H-pyrazol-5-yl)methyl)-3-(3-fluoro-4-(2-methoxyethoxy)-phenyl)urea **(example A18)** (85 mg, 53%) as a white solid (TLC system: MeOH/CHCl₃ (1:9), R_{f}: 0.5).

### Synthesis of example A25: 1-[[2-(3-Chlorophenyl)-5-(trifluoromethyl)-2H-pyrazol-3-yl]-methyl]-3-[4-(2,3-dihydroxy-propoxy)-3-fluoro-phenyl]-urea

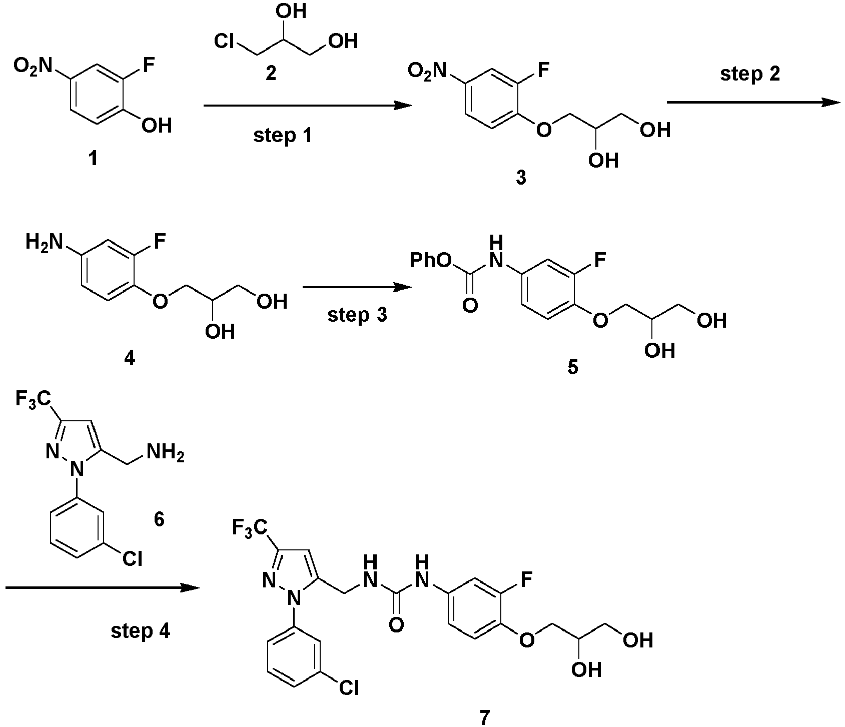

**Step 1:** To a stirred solution of 1 (300 mg, 1.91 mmol) in anhydrous DMF was added sodium hydride (99 mg, 2.48 mmol) and 3-chloro-1,2-propanediol 2 (0.21 mL, 2.48 mmol). The reaction mixture was stirred overnight under reflux. The reaction mixture was cooled to room temperature when the reaction was finished. The reaction mixture was extracted with EtOAc and washed with water and brine. The organic layer was dried over MgSO₄ and filtered. The solvent of the filtrate was removed under low pressure. The crude was purified by column chromatography to produce **3** (234 mg, 53 %).
**Step 2:** Starting material **3** (234 mg, 1.01 mmol) was dissolved in MeOH. Pd/C (23 mg) was added to it. The resulting mixture was stirred at room temperature for 2 h under H₂. TLC showed complete consumption of starting material. The mixture was filtered through celite bed and the filtrate was concentrated under reduced pressure to afford desired compound **4.** (202 mg, 99 %)
**Step 3:** Compound **4** (202 mg, 1.00 mmol) was dissolved in MeCN. To the reaction mixture were added to pyridine (0.09 mL, 1.10 mmol) and phenyl chloroformate (0.14 mL, 1.10 mmol) and stirred at room temperature for 3 h. TLC showed complete consumption of starting material. The reaction mixture was diluted with water and extracted with EtOAc. The organic part was washed with water and brine. The organic layer was dried over MgSO₄ and concentrated under reduced pressure. The crude was purified by column chromatography to give pure compound **5.** (81 mg, 25 %)
**Step 4:** To a solution of compound **5** (40 mg, 0.12 mmol) in DMF was added DMAP (15 mg, 0.12 mmol) and amine **6** (38 mg, 0.14 mmol) at room temperature. The reaction mixture was heated to 50 °C overnight (about 12 - 15 h). TLC showed complete consumption of starting material. The reaction mixture was diluted with water and extracted with EtOAc. The organic part was washed with water and brine. The organic layer was dried over MgSO₄ and concentrated under reduced pressure. The crude was purified by column chromatography to produce pure compound **7** (57 mg, 90 %).
   ¹H NMR (300 MHz, DMSO-d₆) δ 8.70 (s, 1 H, Ar-NH), 7.74 (s, 1 H, Ar-H), 7.61 (m, 3 H, Ar-H), 7.40 (dd, 1 H, J₁ = 13.92 Hz, J₂ = 2.19 Hz, Ar-H), 7.05 (t, 1 H, J = 9.15 Hz, Ar-H), 6.98 (m, 1 H, Ar-H), 6.81 (s, 1 H, Ar-H), 6.72 (t, 1 H, J = 5.49 Hz, Ar-H), 5.10 (m, 1 H, R-CH(OH)-R'), 4.60 (t, 1 H, J = 8.61 Hz, R-CH-O), 4.38 (m, 3 H, ArO-CH₂ and R-CH(OH)-R'), 4.28 ∼ 4.16 (m,2 H, Ar-CH₂)

### Synthesis of example A30: N-[[2-(3-Chlorophenyl)-5-(trifluoromethyl)-2H-pyrazol-3-yl]-methyl]-2-[3-fluoro-4-(hydroxymethyl)-phenyl]-acetamide

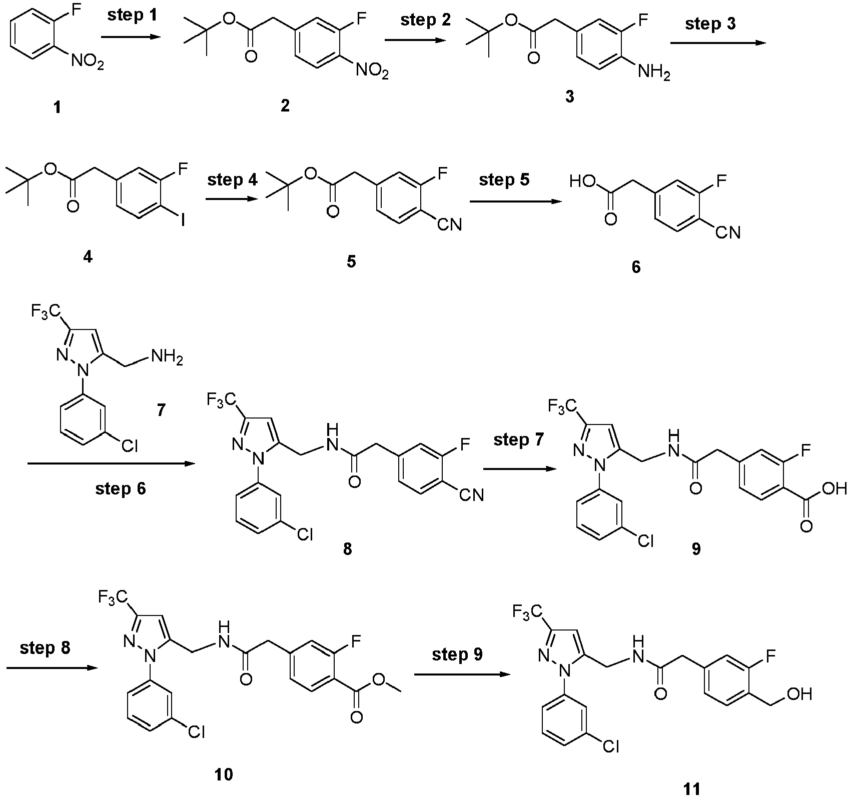

**Step 1:** A mixture of potassium tert-butoxide (5.58 g) in NMP (40 mL) was cooled under nitrogen to -20°C. A mixture of 2-fluoronitrobenzene (2 g) and tert-butylchloroacetate (3 mL) in NMP (40 mL) was added slowly at -10°C to -20°C. After 30 minutes, a further portion of potassium tert-butoxide (1.59 g) was added. The reaction mass was quenched into 30 mL of 2M HCI and 20 g crushed ice, then 40 mL n-hexane was added and the mixture stirred for 10 minutes. The layers were separated and the aqueous layer was extracted with n-hexane (2×30 mL). The combined n-hexane layer were washed with brine (2×20 mL), then dried over anhydrous sodium sulfate, then filtered and washed with 20 mL hexane. The solvent was then evaporated and purified by CC to give the desired compound **2** (2.17 g) as a yellow liquid by 60% yield.
**Step 2:** 10% Pd/C was added to a solution of 2 (2.17 g) in methanol and the mixture was charged with H₂ (gas). After stirring the reaction mixture for 2 h, the mixture was filtered using Celite and purified by column chromatography. 3 (959 mg) was obtained as yellow oil by 44% yield.
**Step 3:** To a solution of p-TsOH·H₂O (2.15 g, 11.28 mmol) in MeCN (20 mL) was added **3** (959 mg, 3.76 mmol). The resulting suspension was cooled to 10-15°C and to this was added, gradually, a solution of NaNO₂ (519 mg, 7.52 mmol) and Kl (1.56 g, 9.4 mmol) in H₂O. The reaction mixture was stirred for 10 min then allowed to come to 20°C and stirred until the starting material was consumed. To the reaction mixture was then added H₂O (50 mL), NaHCO₃ (1 M; until pH = 9-10) and Na₂S₂O₃ (2M, 10 mL). The precipitated aromatic iodide was filtered off and the mixture was extracted with EtOAc and purified by column chromatography. **4** (474 mg) was obtained as yellow oil by 38% yield.
**Step 4:** Compound **4** (474 mg), Pd₂(dba)₃ (2 mol%), dppf (4 mol%), Zn powder (12 mol%) and Zn(CN)₂ (1.5 equiv.) were placed in a flask which was flushed with N₂. DMA (0.02 equiv.) was added via syringe. The resulting mixture was heated at 120°C under N₂ with vigorous agitation until TLC showed the disappearance of **4** (15 h). The mixture was cooled to room temperature, diluted with ethyl acetate (50 mL), and then washed with 2 N NH₄OH solution and brine. After drying over Na₂SO₄, the ethyl acetate solution was concentrated by rotary evaporation. The residue was purified by CC using a mixed solvent of ethyl acetate and n-hexane to afford **5** (260 mg) as off white solid (78% yield).
**Step 5:** Compound **5** (260 mg) and 4M HCI in 1,4-dioxane (7 mL) was stirred for 18 h at about 25°C. Nitrogen was bubbled through the mixture to remove excess HCI over 7 h, then the mixture was concentrated. Toluene (2 mL) was distilled off then the residue was stirred with hexane (2 mL) for 10 minutes. The hexane was decanted off, and the residue was stirred with hexane (1 mL) for 10 minutes. Then the hexane was decanted off. The residue was stirred with toluene (1.5 mL) for 2 h at about 25°C. The solid was filtered and washed with 1:1 toluene/hexane (10 mL), then dried under vacuum to give the desired compound **6** (160 mg) as light brown solid by 80% yield.
**Step 6:** A solution of the carboxylic acid **6** (1.0 eq.) in DCM was cooled in an ice-bath and EDC (1.05 eq.), HOBt (1.05 eq.), TEA (3 equiv), and **7** (1.0 eq.) were added consecutively. The reaction mixture was stirred overnight at room temperature. Water was added to the reaction mixture and it was extracted with DCM. The combined organic extracts were washed successively with a saturated NaHCO₃ solution, 0.5 N HCI, and then water, and dried over MgSO₄. Evaporation of the solvent followed by CC using (EtOAc/n-hexane) afforded the **8** (230 mg) as off white solid (62% yield).
**Step 7:** Compound **8** (1.206 g) was suspended in 1:1 EtOH/4N NaOH (20 mL) and heated to 80°C for 4 h. Upon completion, the reaction was concentrated to remove EtOH and then was placed in an ice bath and neutralized to pH=7 by the addition of conc. HCI. The reaction volume was doubled by the addition of water, and allowed to cool in the ice bath before acidification by 1N HCl to pH=4-5, extracted with EtOAc. The extracted organic layer was dried over Na₂SO₄ and removed EtOAc by evaporation. The desired amide **9** was obtained as brown oil and was carried on the next step without further purification.
**Step 8:** To a stirred solution of **9** (1 equiv) in methanol, under ice-cooling, was added thionyl chloride (2.5 equiv) dropwise over 15 minutes. After stirring the reaction mixture for 15 minutes at 0°C, removed ice-bath and continue reaction at 40°C for 4 h, methanol is distilled out and water is added. The mixture is extracted with ethyl acetate and washed with saturated sodium bicarbonate solution, and brine. Drying (MgSO₄) and evaporation of the ethyl acetate and purification by column chromatography (EtOAc/n-hexane) gave the ester **10** (76 mg) in pure form as a brown solid (30% yield.
**Step 9:** A solution of compound **10** (76 mg, 0.16 mmol, 1 equiv) in anhydrous THF was drop-wise added to lithium aluminum hydride (1.5 equiv) in anhydrous THF at 0°C. The reaction mixture was stirred at 0°C for 2 h. After that, brine was added slowly at 0°C, then THF was removed by evaporation and the mixture extracted with ethyl acetate and water. The extracted organic layer was dried over Na₂SO₄ and ethyl acetate was removed by evaporation. The residue was purified by CC (EtOAc/ n-hexane) and product **11** (43 mg) was obtained as a white solid by 60% yield.
   ¹H NMR (300 MHz, CD₃OD): δ 7.58 (s, 1H), 7.49-7.52 (m, 2H), 7.42-7.46 (m, 1H), 7.36-7.38 (m, 1H), 6.94-7.01 (m, 2H), 6.66 (s, 1H), 4.63 (s, 2H), 4.45 (d, *J* = 2.19 Hz, 2H), 3.46 (d, *J* = 2.19 Hz, 2H).

### Synthesis of example A33: N-[[2-(3-Chlorophenyl)-5-(trifluoromethyl)-2H-pyrazol-3-yl]-methyl]-2-[3-fluoro-4-(hydroxymethyl)-phenyl]-propionamide

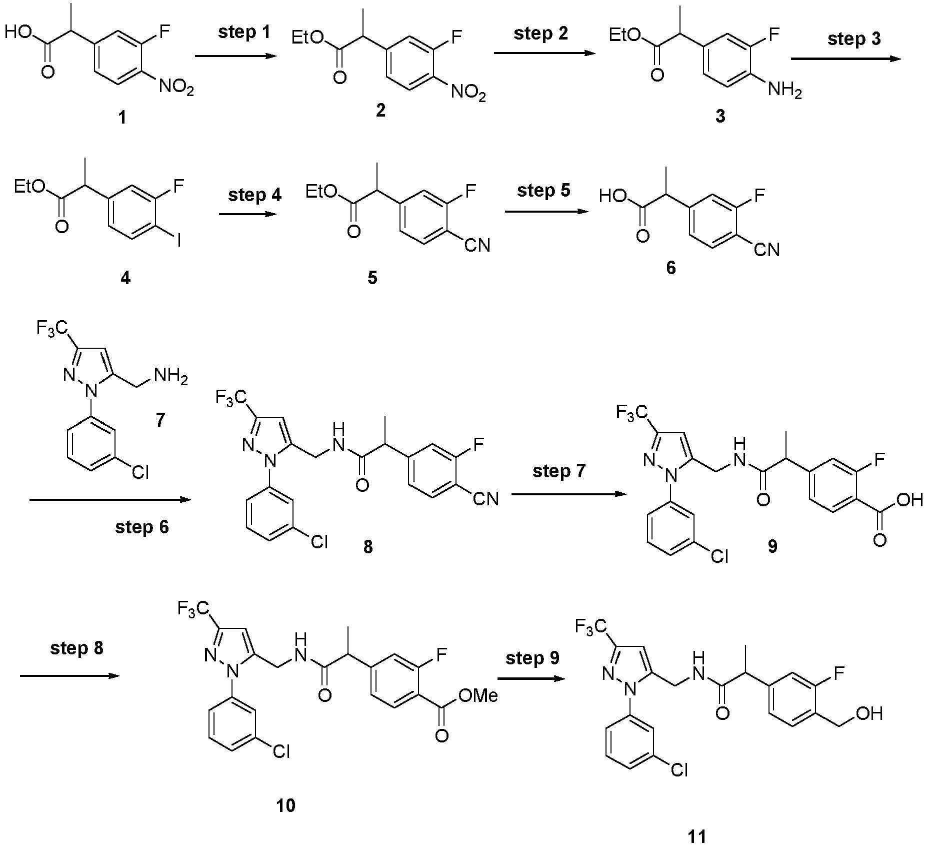

**Step 1:** To a stirred solution of **1** (6 g, 28.15mmol) in 60 mL of methanol, under ice-cooling, was added thionyl chloride (5.12 mL, 70.37 mmol) dropwise over 15 minutes. After stirring the reaction mixture for 2 h, methanol was distilled and 60 mL of water was added. The residue was extracted with ethyl acetate and washed with 50 mL of saturated sodium bicarbonate solution, brine. After drying (over MgSO₄) and evaporation of the ethyl acetate and purification of the residue by CC, **2** was obtained as a yellow oil in 98% yield.
**Step 2:** 10% Pd/C was added to a solution of **2** in methanol and the mixture was charged with H₂ (gas). After stirring the reaction mixture for 6 h, the mixture was filtered using Celite and the residue was purified by CC to obtain **3** obtained as a yellow oil by 90% yield.
**Step 3:** To a solution of p-TsOH.H₂O (6.45 g, 34 mmol) in Me CN (20 mL) was added **3** (11.3 mmol). The resulting suspension was cooled to 10-15°C and to this was added, gradually, a solution of NaNO₂ (1.56 g, 22.6 mmol) and Kl (4.69 g, 28.3 mmol) in H₂O. The reaction mixture was stirred for 10 min then allowed to come to 20°C and stirred until the starting material was consumed. To the reaction mixture was then added H₂O (50 mL), NaHCO₃ (1 M; until pH = 9-10) and Na₂S₂O₃ (2M, 10 mL). The precipitated solid was extracted with EtOAc. The organic layer was washed with water, dried (MgSO₄) and concentrated in vacuo. The residue was purified by CC using EtOAc/n-hexane as solvent system to give the desired product **4** as a yellow oil (2.19 g, 63% yield).
**Step 4:** Compound **4**, Pd₂(dba)₃ (2 mol%), dppf (4 mol%), Zn powder (12 mol%) and Zn(CN)₂ (1.5 equiv.) were placed in a flask which was flushed with N₂. DMA (0.02 equiv.) was added via syringe. The resulting mixture was heated at 120°C under N₂ with vigorous agitation until TLC showed the disappearance of **4** (15 h). The mixture was cooled to room temperature, diluted with ethyl acetate (50 mL), and then washed with 2 N NH₄OH solution and brine. After drying over Na₂SO₄, the ethyl acetate solution was concentrated by rotary evaporation. The residue was purified by CC using a mixed solvent of ethyl acetate and n-hexane to afford methyl 2-(4-cyano-3-fluorophenyl)propanoate **5** as yellow oil (80% yield).
**Step 5:** To a solution of **5** (2.15 g, 10.4 mmol) in THF (10 mL) was added a 20 mL mixture solvents of THF and H₂O (1:1), and LiOH·H₂O (1.09 g, 26 mmol). The reaction mixture was stirred at room temperature for 2 h. To the reaction mixture was then added H₂O (50 mL), it was cooled, and acidified by diluted HCI until a pH of 1-2. The mixture is extracted with ethyl acetate. The organic layer was washed with water, dried (Mg₂SO₄) and concentrated in vacuo to gain product **6** as off white solid (1.98 g, 99% yield). The product was carried on to the next step without further purification
**Step 6:** A solution of the carboxylic acid **6** (1.0 eq.) in DCM was cooled in an ice-bath and EDC (1.05 eq.), HOBt (1.05 eq.), TEA (3 eq.), and **7** (1.0 eq.) were added consecutively. The reaction mixture was stirred overnight at room temperature. Water was added to the reaction mixture and it was extracted with DCM. The combined organic extracts were washed successively with a saturated NaHCO₃ solution, 0.5 N HCI, and then water, and dried over MgSO₄. Evaporation of the solvent followed by CC (EtOAc/n-hexane) afforded the desired amide **8** as off white solid (60% yield).
**Step 7:** Compound **8** (1.206g) was suspended in 1:1 EtOH/4N NaOH (20 mL) and the mixture was heated to 80°C for 4 h. Upon completion, the mixture was concentrated to remove EtOH and then was placed in an ice bath and neutralized to pH=7 by the addition of conc. HCI. The reaction volume was doubled by the addition of water, and allowed to cool in the ice bath before acidification by 1N HCI to pH=4-5, then the mixture was extracted with EtOAc. The extracted organic layer was dried over Na₂SO₄ and EtOAc was removed by evaporation. The residue was purified by CC (MeOH/DCM =1:10) and the desired product **9** was obtained as off white solid (1.067 g, 85% yield).
**Step 8:** To a stirred solution of **9** (1 equiv.) in methanol, under ice-cooling, was added thionyl chloride (2.5 equiv) dropwise over 15 minutes. After stirring the reaction mixture for 15 minutes at 0°C, the ice-bath was removed and stirring was continued at 40°C for 4 h, then methanol is distilled and water is added. The mixture is extracted with ethyl acetate and washed with saturated sodium bicarbonate solution, and brine. Drying (over MgSO₄) and evaporation of the ethyl acetate and purification by CC (EtOAc/n-hexane) gave the ester **10** in pure form as a white solid (58% yield).
**Step 9:** A solution of compound **10** (1 equiv) in anhydrous THF was drop-wise added to lithium aluminum hydride (1.5 equiv) in anhydrous THF at 0°C. The reaction mixture was stirred at 0°C for 2 h. After that, brine was added slowly at 0°C, then the THF was removed by evaporation and the residue extracted with ethyl acetate and water. The extracted organic layer was dried over Na₂SO₄ and ethyl acetate was removed by evaporation. The residue was purified by CC (EtOAc/n-hexane) and the desired product **11** was obtained as a white solid (85% yield).
   ¹H NMR (300 MHz, CDCl₃): δ7.39-7.45 (m, 4H), 7.29 (t, *J* = 2.01 Hz, 1H), 6.92-7.02 (m, 2H), 6.42 (s, 1H), 5.58 (s, NH), 4,76 (d, *J* = 5,85 Hz, 2H), 4.39-4.53 (m, 2H), 3.52 (q, *J* = 6.96 Hz, 1H), 1.84 (t, *J* = 6.04 Hz, OH), 1.49 (d, *J* = 7.14 Hz, 3H).

### Synthesis of example A46: 2-(3-fluoro-4-(hydroxymethyl)phenyl)-N-((1-(4-fluorophenyl)-3-(trifluoromethyl)-1 H-pyrazol-5-yl)methyl)propanamide

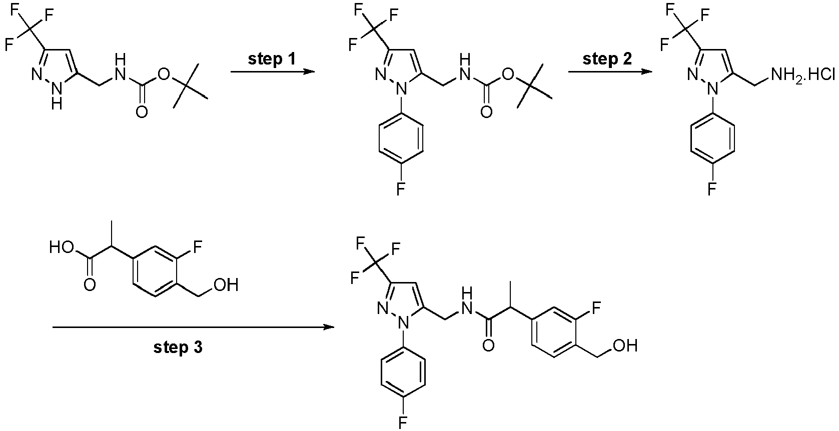

**Step** 1:To a mixture of tert-butyl (3-(trifluoromethyl)-1H-pyrazol-5-yl)methylcarbamate (501 mg, 1.89 mmol, 1 equiv.), 4-fluorophenylboronic acid (529 mg, 3.78 mmol, 2 equiv.) and copper acetate (517 mg, 2.83 mmol, 1.5 equiv.) in dichloromethane (28 mL) was added pyridine (301 mg, 0.301 mL, 3.78 mmol, 2 equiv) and the mixture was stirred in the presence of air for 2 d at room temperature. The reaction mixture was filtered over silica gel, the filter cake was washed with 250 mL of dichloromethane and the solvent of the filtrate was evaporated to give tert-butyl (1-(4-fluorophenyl)-3-(trifluoromethyl)-1H-pyrazol-5-yl)methylcarbamate (501 mg, 74%).
**Step 2:** In 9 mL of dioxane, tert-butyl (1-(4-fluorophenyl)-3-(trifluoromethyl)-1H-pyrazol-5-yl)methylcarbamate (501 mg, 1.39 mmol) was dissolved and hydrogen chloride in dioxane (2.27 mL, c = 4 mol/L, 9.06 mml, 6.5 equiv.) was added. The reaction mixture was stirred overnight and filtered, the filter cake was washed with ether (2 x 15 mL) and dried to give (1-(4-fluorophenyl)-3-(trifluoromethyl)-1H-pyrazol-5-yl)methanamine hydrochloride (188 mg, 46%).
**Step 3:** To a stirred solution of (1-(4-fluorophenyl)-3-(trifluoromethyl)-1H-pyrazol-5-yl)methanamine hydrochloride(101 mg, 0.342 mmol, 1.0 eq) in THF/DMF (1/20, v/v, 2.8 mL) was added 2-(3-fluoro-4-hydroxymethyl)phenyl)propionic acid (68 mg, 0.348 mmol, 1.02 equiv.), HOBt (46 mg. 0.342 mmol, 1 equiv.), TBTU (151 mg, 0.342 mmo, 1 equiv.) and DIPEA (0.232 mL, 176 mg, 1.37 mmol, 4 equiv.) and the mixture was stirred for 3 d at room temperature The reaction mixture was diluted with 20 mL of EtOAc and washed with 20 mL of water. The aqueous phase was extracted with EtOAc (3x 20 mL), the combined organic phases were dried over magnesium sulfate, evaporated and the residue was purified by column chromatography (EtOAc/cyclohexane (1:1) as eluent) to give 2-(3-fluoro-4-(hydroxymethyl)phenyl)-N-((1-(4-fluorophenyl)-3-(trifluoromethyl)-1H-pyrazol-5-yl)methyl)propanamide (119 mg, 79%).

### Synthesis of example A50: N-[[2-(3-Chlorophenyl)-5-(trifluoromethyl)-2H-pyrazol-3-yl]-methyl]-2-[4-(hydroxymethyl)-3-methoxy-phenyl]-propionamide

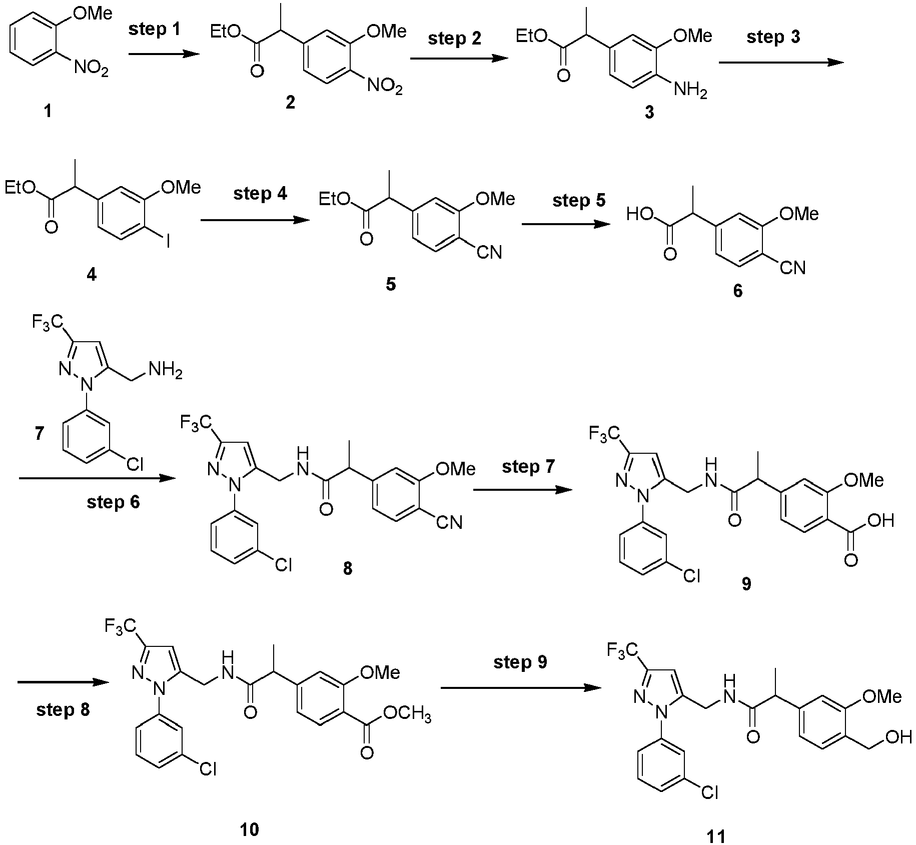

**Step 1:** To a stirred solution of 1 (3 g, 19.590 mmol) in DMF were added potassium tert-butoxide (8.792 g, 78.36 mmol) and ethyl 2-chloropropionate (2.5 mL, 19.59 mmol) while maintaining the temperature below -30 °C. The reaction mixture was stirred for 5 min at - 30 °C, then ethyl 2-chloropropionate (0.25 mL, 1.959 mmol) was added to mixture. The reaction mixture was stirred for 10 min at room temperature. The residue was dissolved in EtOAc and washed with water and brine. The organic layer was dried (MgSO₄) and filtered. The filtrate removed in vacuo. The crude was purified by column chromatography. **2** (683 mg) was obtained as 14 % yield.
**Step 2:** To a stirred solution of **2** (683 mg, 2.697 mmol) in tetrahydrofuran and ethanol as co-solvent was added 10 % palladium on carbon (70 mg). The mixture was charged with H₂ (gas) balloon. The resulting mixture was stirred for 15 h, then filtered using celite. The filtrate removed in vacuo. The crude was purified by column chromatography. **3** (447 mg) was obtained as 74 % yield.
**Step 3:** To a stirred solution of **3** (447 mg, 2.002 mmol) in MeCN and water were added p-TsOH·H₂O (1.142 g, 6.006 mmol), sodium nitrite (276 mg, 4.004 mmol) and potassium iodide (831 mg, 5.005 mmol). The reaction mixture was stirred for 4 h at room temperature. The mixture dissolved in EtOAc and washed with water and brine. The organic layer was dried (MgSO₄) and filtered. The filtrate removed in vacuo. The crude was purified by column chromatography. **4** (468 mg) was obtained as 70 % yield.
**Step 4:** To a stirred solution of **4** (626 mg, 1.873 mmol) in DMF were added zinc cyanide (227 mg, 1.929 mmol) and Pd(PPh₃)₄ (216 mg, 0.1873 mmol). The reaction mixture was stirred for 36 hat 120 °C, then cooled to room temperature, diluted with EtOAc. The mixture was filtered using celite pad. The filtrate was dissolved in EtOAc and extracted with NaHCO₃. The organic layer was dried (MgSO₄) and filtered. The filtrate removed in vacuo. The crude was purified by column chromatography. **5** (222 mg) was obtained as 51 % yield
**Step 5:** To a stirred solution of **5** (222 mg, 0.952 mmol) in co-solvent with THF and water (1:1) were added sodium hydroxide (95 mg, 2.38 mmol). The reaction mixture was stirred for 15 hours at room temperature, then acidified to a pH of 3-4 with AcOH. The residue was dissolved in EtOAc and washed with water and brine. The organic layer was dried (MgSO₄) and filtered. The filtrate removed in vacuo. The crude was purified by column chromatography. 6 (188 mg) was obtained in 96 % yield.
**Step 6:** To a stirred solution of **6** (108 mg, 0.526 mmol) and 7 (160 mg, 0.578 mmol) in acetonitrile were added EDC (151 mg, 0.789 mmol), HOBt (106 mg, 0.789 mmol) and triethylamine (0.18 mL, 1.315 mmol). The reaction mixture was stirred for 15 h at room temperature. The residue dissolved in EtOAc and washed with water and brine. The organic layer was dried (MgSO₄) and filtered. The filtrate removed in vacuo. The crude was purified by column chromatography. **8** (194 mg) was obtained as 80 % yield.
**Step 7:** To a stirred solution of **8** (194 mg, 0.419 mmol) in ethanol (5 mL) were added 4 N NaOH (5 ml). The reaction mixture was stirred for 6 h at 80 °C then cooled to room temperature. The mixture was acidified to a pH of 4-5 by 1 N HCI. The residue was dissolved in EtOAc and the solution washed with water and brine. The organic layer was dried (MgSO₄) and filtered. The filtrate removed in vacuo. The crude was purified by column chromatography. **9** (218 mg) was obtained as 99 % yield.
**Step 8:** To a stirred solution of **9** (218 mg, 0.452 mmol) in methanol were added thionyl chloride (0.08 mL, 1.131 mmol) while the temperature maintaining below 0°C. The reaction mixture was stirred for 24 hours at 35 °C, then cooled to room temperature. The solvent was evaporated then the residue was dissolved in EtOAc. The solution was washed with water and brine. The organic layer was dried (MgSO₄) and filtered. The solvent of the filtrate was removed in vacuo. The crude was purified by column chromatography. **10** (145 mg) was obtained as 65 % yield.
**Step 9:** To a stirred solution of **10** (145 mg, 0.292 mmol) in tetrahydrofurane, cooled to 0°C, were added lithium aluminum hydride (17 mg, 0.438 mmol). The resulting reaction mixture was stirred for 2 h at 0 °C. Water and 4 N NaOH were added to the mixture for quenching. The residue was dissolved in EtOAc then washed with water. The organic layer was dried (MgSO₄) and filtered. The solvent was removed in vacuo. The crude was purified by column chromatography. **11** (111 mg) was obtained as 81 % yield.
   ¹H NMR (300 MHz, CDCl₃) δ 7.42 (m, 3H), 7.27 (m, 2H), 6.78 (dd, 1H, J=7.5 Hz), 6.74 (s, 1H), 6.40 (s, 1H), 5.57 (t, 1H), 4.67 (d, 2H, J=6.21 Hz), 4.44 (m, 2H), 3.87 (s, 3H), 3.53 (q, 1H), 2.24 (t, 1H), 1.50 (d, 3H).

### Synthesis of example A59: 1-[[2-(3-Chlorophenyl)-5-(trifluoromethyl)-2H-pyrazol-3-yl]-methyl]-3-[3-fluoro-4-(hydroxymethyl)-phenyl]-urea

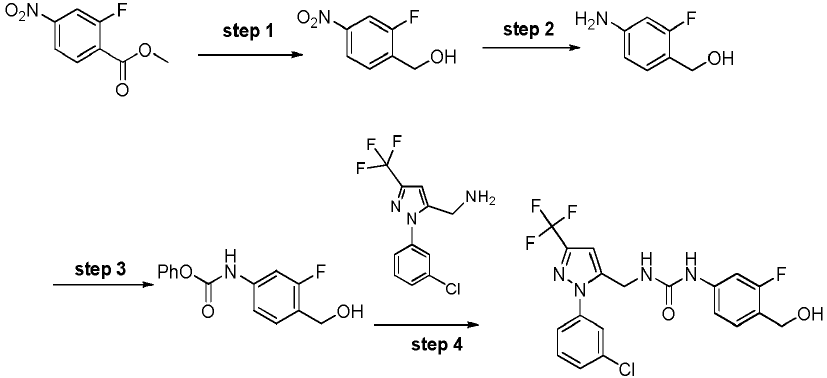

**Step 1:** To a stirred solution of methyl 2-fluoro-4-nitrobenzoate (10.0 g, 49.7 mmol, 1 eq.) in methanol (100 mL) was added sodium borohydride (9.40 g, 248.7 mmol, 5 eq.) at RT and stirred for 4h. The methanol was evaporated and the residue was diluted with ethyl acetate (50 mL x 2) washed with water (50 mL) and brine (50 mL). The ethyl acetate layer was dried over Na₂SO₄, evaporated under vacuum to get (2-fluoro-4-nitrophenyl)methanol (8 g, 94%, off-white solid; TLC system: EtOAc/PE (3:7), R_{f}: 0.30).
**Step 2:** To a stirred solution of (2-fluoro-4-nitrophenyl)methanol (3.0 g, 1.0 eq.) in EtOAc (30 mL) was added 10% Pd-C and the reaction mixture was stirred under H₂ gas balloon at RT for 6 h. The reaction mixture was passed through a celite pad and the solvent evaporated. The residue was purified by neutral alumina column using PE/EtOAc (3:2) as eluent to get (4-amino-2-fluorophenyl)methanol (1.1 g, 48%) as a solid; TLC system: EtOAc/PE (1:1), R_{f}: 0.3).
**Step 3:** To a stirred solution of (4-amino-2-fluorophenyl)methanol (100 mg, 0.709 mmol, 1 eq.) in acetone (1.0 mL) was added pyridine (0.17 mL, 2.12 mmol, 3 eq.) followed by phenyl chloroformate (0.092 mL, 0.709 mmol, 1 eq.) at 0°C and stirred at RT for 1 h. The solvent was evaporated and the residue obtained was purified by CC using ethyl acetate/PE (7:13) as eluent to get phenyl 3-fluoro-4-(hydroxymethyl)phenylcarbamate (110 mg, 60%, off-white solid; TLC system: EtOAc/PE (1:1), R_{f}: 0.4).
**Step 4:** To a stirred solution of (1-(3-chlorophenyl)-3-(trifluoromethyl)-1H-pyrazol-5-yl)methanamine (100 mg, 0.316 mmol, 1.0 eq.) in DCM (2.0 mL) was added TEA (0.07 mL, 0.632 mmol, 3.0 eq) followed by phenyl 3-fluoro-4-(hydroxymethyl)phenylcarbamate (82.4 mg, 0.316 mmol, 1.0 eq.) at RT and stirred for 16 h. After completion of the reaction, a solid precipitate was filtered and washed with DCM (2 mL) followed by n-pentane (5 mL) and dried to get 1-((1-(3-chlorophenyl)-3-(trifluoromethyl)-1H-pyrazol-5-yl)methyl)-3-(3-fluoro-4-(hydroxymethyl)phenyl)urea (compound A59) (80 mg; 47%, white solid; TLC system: EtOAc/PE (3:2); R_{f}: 0.2).

### Synthesis of example A60: 1-[[2-(4-Chlorophenyl)-5-(trifluoromethyl)-2H-pyrazol-3-yl]-methyl]-3-[3-fluoro-4-(hydroxymethyl)-phenyl]-urea

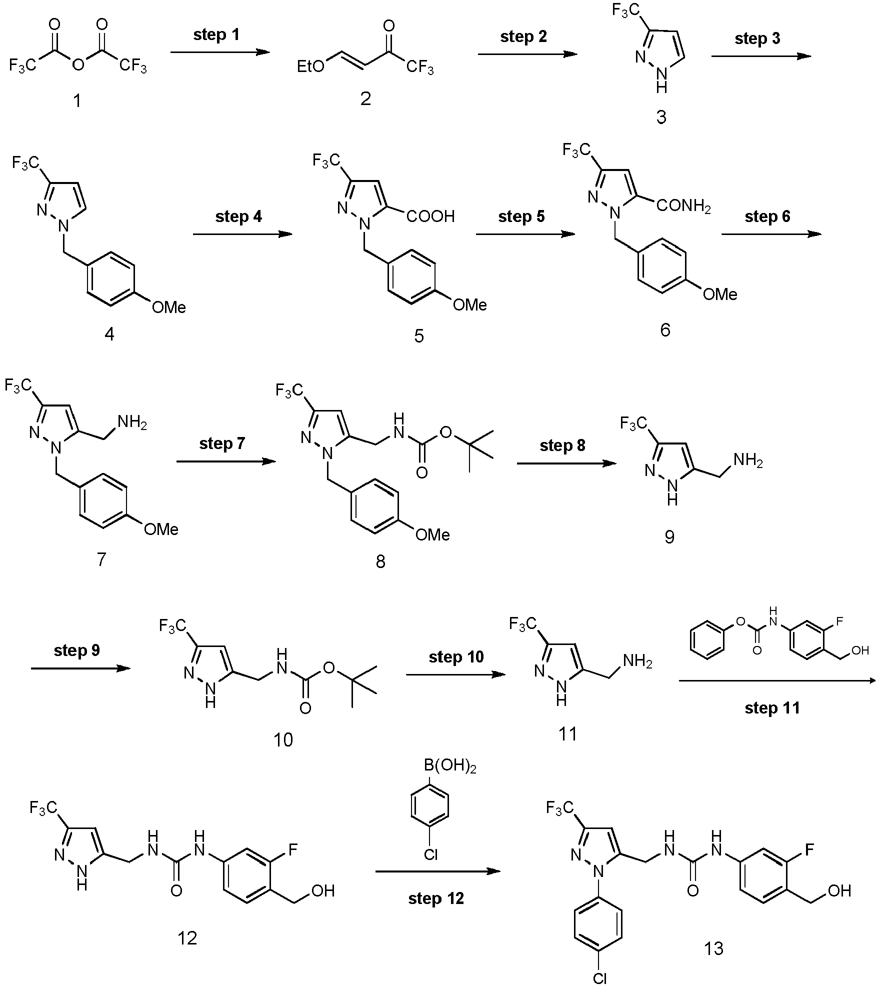

**Step 1:** DMAP (4.25 g, 34 mmol, 0.01 eq) was added to DCM (3 L) and the contents were cooled to -10°C. Trifluoroacetic (triflic) anhydride (765 g, 3.2 mol, 1.05 eq) was added followed by ethyl vinyl ether (250 g, 3.04 mol) drop wise for 45 min at -10°C. Then the overall reaction mixture was initially stirred for 8 h at 0°C and later for overnight at room temperature. Progress of the reaction was monitored by TLC (10% ethyl acetate/hexane, R_{f}∼0.7). On completion of the reaction, reaction contents were treated with saturated NaHCO₃ solution (600 mL) and the organic layer was separated. The aqueous layer was extracted with DCM (2 × 500 mL). The combined organic layer was washed with water (2 × 1 ltr), dried over sodium sulfate and concentrated under reduced pressure to give the crude product as a brown colored liquid (450 g, crude).
**Step 2:** Hydrazine dihydrochloride (225 g, 2.14 mol, 1.6 eq) in ethanol (1400 mL) was stirred well. TEA (135.4 g (185.4 mL), 1.34 mol, 1 eq) was added drop wise for 45 min at ambient temperature. Then 2 (225 g, crude) was added drop wise at room temperature and the overall reaction mixture was refluxed overnight. Progress of the reaction was monitored by TLC (20% ethyl acetate/hexane, R_{f}∼0.4). On completion of the reaction, ethanol was distilled off completely, residue was taken in ice water (500 mL) and the product extracted with ethyl acetate (2 × 400 mL). The combined extract was washed with ice water (300 mL), dried over sodium sulfate and concentrated under reduced pressure to yield the required product as a off white solid (175 g,crude).
**Step 3:** NaH (33.08 g (19.85 mol, 60%), 1.5 eq) was washed with hexane, dry DMF (500 mL) was added drop wise under N₂ atmosphere and stirred well. A solution of **3** (75 g, 0.55 mol) in DMF (125 mL) was added drop wise under N₂ atmosphere. Then a solution of 4-methoxylbenzyl chloride (86.3 g, 0.55 mol, 1 eq) in DMF (125 mL) was added drop wise and the overall reaction mixture was allowed to stir for 12 h at room temperature. Progress of the reaction was monitored by TLC (10% ethyl acetate/hexane, R_{f}∼0.4). On completion of the reaction, reaction contents were poured into ice water (500 mL) and the product was extracted with ethyl acetate (2 × 400 mL). The ethyl acetate layer was washed with 2N HCI (2x200ml). Then the contents were dried over sodium sulfate and concentrated under reduced pressure. Obtained crude was purified by silica gel by column chromatography with 10% ethyl acetate/Hexane to yield the required product as a brown colored liquid (98 g, 70% yield).
**Step 4:** Diisopropyl amine (28.4 mol, 39.4 mL), 1.2 eq) was taken in THF (500 mL), stirred well and cooled the contents to 0°C. n-BuLi (234.4 mL, 1.5 eq) was added drop wise at 0°C and the contents were stitrred for 1 h at 0°C. Then the mixture was cooled -78°C, a solution of **4** (62 g, 0.24 mol) in THF (200 mL) was added drop wise for 30 min and the contents for were stirred for another h at-78°C. Then dry CO₂ gas was bubbled through the reaction mixture for 1.5 h. Progress of the reaction was monitored by TLC (10% ethyl acetate/hexane, R_{f}∼0.1). On completion of the reaction, reaction contents were poured into ice water (300 mL) and the aqueous layer was extracted with ethyl acetate (2 × 200 mL) in basic condition. The aqueous layer was acidified with 20% HCl solution and extracted with ethyl acetate (2 × 200 mL). The combined organic layer was dried over sodium sulfate and concentrated under reduced pressure to yield the required product as an off white solid (40 g, 55% yield).
**Step 5:** To a solution of **5** (50 g, 0.16 mol) in DCM (750 mL, 15 times), a catalytic amount of DMF was added and the mixture was cooled to 0°C. Thionyl chloride (99.3 g (61 mL), 0.83 mol, 5 eq) was added drop wise for 30 min at 0°C. The overall reaction mixture was heated to reflux and maintained at this temperature for 2 h. Progress of the reaction was monitored by TLC (10% ethyl acetate/hexane, R_{f}∼0.4). On disappearance of the starting material, DCM was distilled off completely. Above prepared acid chloride was dissolved in DCM (500 mL) and added drop wise to aqueous ammonia solution (700 mL) at 0°C. The overall reaction mixture was allowed to stirr for 1 h and the progress of the reaction was monitored by TLC (10% ethyl acetate/hexane, R_{f}∼0.7). On completion of the reaction, ice cold water (200 mL) was added and the product extracted with ethyl acetate (2 × 200 mL). The combined organic layer was dried over sodium sulfate and concentrated under reduced pressure to yield the required product as an off white solid (37 g, crude). Crude obtained was directly used for the next step.
**Step 6:** Lithium aluminium hydride (LAH) (4.7 g, 0.12 mol, 1 eq) was charged into a flask. THF (250 mL) was added at 0°C. Then a solution of **6** (37 g, 0.12 mol) in THF (120 mL) was added drop wise for 30 min at 0°C and the reaction mixture was heated to reflux for 5 h. Progress of the reaction was monitored by TLC (50% ethyl acetate/hexane, R_{f}∼0.2). As the reaction was not moved completely, LAH (2.3 g) was added again and the mixture was refluxed for another 4 h. After completion of the reaction the reaction contents were slowly added to saturated sodium sulfate solution (1 L) and filtered over celite and the product extracted with ethyl acetate (2 × 500 mL). Combined extract was dried over sodium sulfate and concentrated under reduced pressure to obtain the crude product as an off white solid (32.5 g, crude). Crude obtained was directly used for the next step.
**Step 7:** To a solution of **7** ((80 g, 0.28 mol) in DCM (600 mL) cooled at 0°C, TEA (22.7 g (30.2 mL), 0.026 mol, 0.8 eq) was added drop wise for 10 min. Then Boc anhydride (Boc₂O) (61.2 g (62.5 mL), 0.28 mol, 1 eq) was added drop wise for 20-30 min at 0°C. Overall reaction mixture initially stirred for 30 min at 0°C and stirred for 1 h at room temperature. Progress of the reaction was monitored by TLC (20% ethyl acetate/hexane, R_{f}∼0.6). On completion of the reaction, DCM was distilled off completely, and the residue was taken in ice water (500 mL) and the product extracted with ethyl acetate (2x 300 mL). Combined extract was dried over sodium sulfate and concentrated under reduced pressure. Crude obtained was recrystalised from n-hexane (200 mL) to yield the required product as an off white solid (80 g, 74% yield).
**Step 8:** To a stirred solution of **8** (20 g, 0.052 mol) in toluene (300 mL, 15 times), cooled to 0°C, was added aluminum chloride (17.34 g, 0.129 mol, 2.5 eq) portion wise for 30 min. The reaction mixture was slowly heated to 50-60°C and alloweded to stir for 2 h at the same temperature. Progress of the reaction was monitored by TLC (20% ethyl acetate/hexane, R_{f}∼0.1). On completion of the reaction, reaction contents were treated with diluted HCI, then ice cold water (300 mL) was added and the mixture extracted with ethyl acetate (2 × 100 mL). The aqueous layer was basified with sodium hydroxide solution and extracted with ethyl acetate and dried over sodium sulfate and concentrated under reduced pressure to give the crude product as a brown colored solid (4.6 g, crude). The crude obtained was directly used for the next step.
**Step 9: 9** (7 g, 42.4 mmol, 1 eq) was given in DCM (7 mL, 10 times) at room temperature, then to that mixture TEA (5.86 mL, 72.4 mmol, 1eq) was added at room temperature and the mixture stirred for 10 min and cooled to 0-5°C. Boc₂O (9.24 g, 42.4 mmol, 1 eq) was added drop wise to reaction mixture for 30 min and the temperature was maintained for 3 h at 0-5°C. Progress of the reaction was monitored by the TLC (30 % ethyl acetate/Hexane). On completion of the reaction, the reaction mixture was brought to ambient temperature for 2 h and the DCM was distilled off, the residue obtained was treated with water (50 mL) and extracted with ethyl acetate (100 mL). The combined organic layer was dried over sodium sulphate, then the solvent was evaporated under vacuum. The obtained crude was purified with column chromatography to yield the required product as a white colored solid (5 g, Yield 44.48 %).
**Step 10:** To a stirred solution of compound **10** (5 g, 18.8 mmol) in MeOH (36 mL) was added HCI in 2-propanol (5,8 mL, 29.2 mmol) and the mixture was stirred over 48 h at room temperature. The reaction mixture was concentrated in vacuo, diethylether (20 mL) was added and the obtained precipitate filtered off and washed with diethylether (5 mL). After drying the desired product was obtained in 97 % yield (3.67 g).
**Step 11:**To a stirred solution of compound **11** (2.8 g, 13.9 mmol) in DCM (76 mL) was added TEA (7 mL, 41.7mmol, 3.0 eq) followed by phenyl 3-fluoro-4-(hydroxymethyl)phenylcarbamate (3.63 g, 31.9mmol, 1.0 eq.) (synthesis described for in **example A58**) at room temperature and the mixture was stirred for 16 h. After completion of the reaction, a solid precipitate was filtered and washed with DCM (2 mL) followed by n-pentane (5 mL) and dried to get **12** (3.38 g, 73 %).
**Step 12:** 4-Chloro-phenylboronic acid (93 mg, 0.06 mmol), compound **12** (99 mg, 0.3 mmol) and copper(II)-acetate (0.044 mL, 0.45 mmol) were added to DCM (4.5 mL). At room temperature was added pyridine (0.48 mL, 6 mmol) and the mixture was stirred for 48 h. The reaction mixture was concentrated in vacuo, the solid obtained was purified by column chromatography (eluent: cyclohexane/ethyl acetate (1:2)) to afford 1-((1-(4-chlorophenyl)-3-(trifluoromethyl)-1H-pyrazol-5-yl)methyl)-3-(3-fluoro-4-(hydroxymethyl)phenyl)urea (compound A60) (76 mg, 57 %).

### Exemples 58, 61 - 88 and 92 - 95 were prepared in a similar manner or may be prepared analogously according to example A60.

### Synthesis of example A89: 1-[[2-(2,3-Dichloro-phenyl)-5-(trifluoromethyl)-2H-pyrazol-3-yl]-methyl]-3-[3-fluoro-4-(hydroxymethyl)-phenyl]-urea

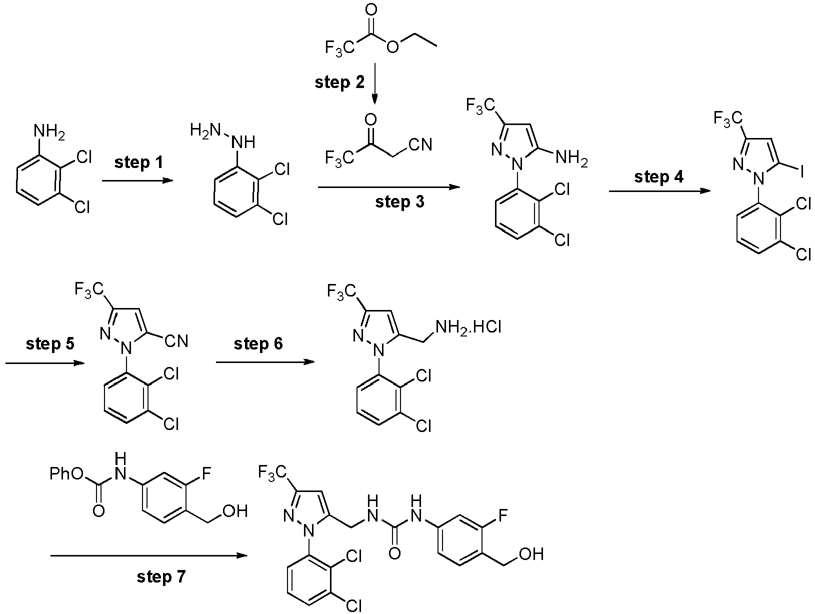

**Step 1:** To a stirred suspension of 2,3-dichloroaniline (35 g, 216.02 mmol, 1.0 eq.) in conc. HCI (150 mL) was added a solution of NaNO₂ (17.9 g, 259.22 mmol, 1.2 eq.) in water (50 mL) dropwise at -5 to 0°C until the reaction mixture formed a clear solution. The reaction mixture was added dropwise to a stirred solution of SnCl₂ (90.1 g, 475.24 mmol, 2.2 eq.) in conc. HCI (300 mL) at -5°C for 30 min. A solid precipitate obtained was filtered off and washed with excess of ice cold water to get (2,3-dichlorophenyl)hydrazine (45.8 g, 98 %, pale yellow solid; TLC system: EtOAc/PE (3:7) R_{f}: 0.55).
**Step 2:** To a stirred suspension of 60% NaH (33.80 g, 1.408 mol, 2 eq.) in dioxane (700mL) was added acetonitrile (44.3 mL, 0.845 mol, 1.2 eq.) at 0°C over a period of 30 min followed by ethyl trifluoroacetate (83.70 mL, 0.704 mol, 1.0 eq.). The reaction mixture was slowly and carefully heated to 100°C for 3 h. The dioxane was evaporated under reduced pressure and the pH was adjusted to ∼6 with 1N HCl and the mixture extracted with ether (200 mL x 4), dried (MgSO₄) and the solvent evaporated under vacuum to get 4,4,4-trifluoro-3-oxobutanenitrile (95g, crude, brown oil).
**Step 3:** To a stirred suspension of 4,4,4-trifluoro-3-oxobutanenitrile (20 g, 145.98 mmol, 1.0 eq.) in ethanol (200 mL) was added (2,3-dichlorophenyl)hydrazine (47.5 g, 145.98 mmol, 1.0 eq) and refluxed for 3 h. Ethanol was evaporated, the residue diluted with water and extracted with ethyl acetate (300 mL x 2), dried (Na₂SO₄), and the solvent evaporated under vacuum. The crude was purified by CC using EtOAc/PE (3:7) as eluent to get 1-(2,3-dichlorophenyl)-3-(trifluoromethyl)-1H-pyrazol-5-amine (17.02 g, 39.65%, yellow solid; TLC system: EtOAc/PE (1:1) R_{f}: 0.6).
**Step 4:** To a stirred suspension of KI (30.38 g, 186.03 mmol, 3.0 eq.) and isoamylnitrile (26.2 mL, 186.03 mmol, 3.0eq) in acetonitrile (150 mL) was added 1-(2,3-dichlorophenyl)-3-(trifluoromethyl)-1H-pyrazol-5-amine (18 g, 61.01 mmol, 1.0 eq.) and refluxed for 12 h. Acetonitrile was evaporated, the residue diluted with water and extracted with ethyl acetate (250 mL x 2), dried (Na₂SO₄), the solvent evaporated under vacuum. The crude was purified by CC using EtOAc/PE (1:9) to get 1-(2,3-dichlorophenyl)-5-iodo-3-(trifluoromethyl)-1H-pyrazole (11.2 g, crude, yellow oil; TLC system: EtOAc/PE (1:9) R_{f}: 0.6).
**Step 5:** To a stirred solution of 1-(2,3-dichlorophenyl)-5-iodo-3-(trifluoromethyl)-1H-pyrazole (11.1 g, 27.58 mmol, 1.0 eq) in NMP (50 mL) was added CuCN (2.39 g, 27.58 mmol, 1.0 eq.) and heated to 200 °C for 2h. The reaction mixture was passed through a celite pad and washed with excess of ethyl acetate. The filtrate was washed with water and the ethyl acetate layer was separated, dried (Na₂SO₄), and the solvent evaporated. The residue obtained was purified by CC using ethyl acetate/PE (1:9) to get 1-(2,3-dichlorophenyl)-3-(trifluoromethyl)-1H-pyrazole-5-carbonitrile (5.02 g, crude, yellow solid) .
**Step 6:** To a stirred solution of 1-(2,3-dichlorophenyl)-3-(trifluoromethyl)-1H-pyrazole-5-carbonitrile (5.02 g, 16.46 mmol, 1.0 eq.) in THF (50 mL) was added BH₃•S(CH₃)₂ (3.74 g, 49.38 mmol, 3.0 eq.) at 0°C and heated to reflux for 1 h. The reaction mixture was cooled to 0°C and quenched with 1N HCl and basified with 1N solution of NaOH to a pH of ∼10 and extracted with ethyl acetate (100mL x 2), dried (Na₂SO₄), the solvent evaporated to get a pale yellow oil. The oil was treated with ether HCI to get (1-(2,3-dichlorophenyl)-3-(trifluoromethyl)-1H-pyrazol-5-yl)methanamine hydrochloride (1.15, 27% yield over 3 steps) as a white solid (TLC system: CHCl₃/MeOH (9:1), R_{f} 0.55).
**Step 7:** To a stirred solution of phenyl 3-fluoro-4-(hydroxymethyl)phenylcarbamate (0.175 g, 0.63 mmol, 1.0 eq) in DCM (5 mL) was added TEA (0.25 mL, 1.9 mmol, 3.0 eq.) followed by compound (1-(2,3-dichlorophenyl)-3-(trifluoromethyl)-1H-pyrazol-5-yl)methanamine hydrochloride (0.218 mg, 0.63 mmol, 1.0 eq.) at RT and stirred for 16 h at RT. The reaction mixture was diluted with water (10 mL) and extracted into DCM (25 mL).The organic layer was washed with brine (10 mL), dried over (Na₂SO₄) and the solvent evaporated under vacuum. The residue obtained was purified by CC using EtOAc/PE (1:1) as eluent to get 1-((1-(2,3-dichlorophenyl)-3-(trifluoromethyl)-1H-pyrazol-5-yl)methyl)-3-(3-fluoro-4-(hydroxymethyl)phenyl)urea(70 mg; 23%) as white solid (TLC system: EtOAc, R_{f}: 0.55).

### Synthesis of example A90: 1-[[2-(3-Chloro-2-methoxy-phenyl)-5-(trifluoromethyl)-2H-pyrazol-3-yl]-methyl]-3-[3-fluoro-4-(hydroxymethyl)-phenyl]-urea

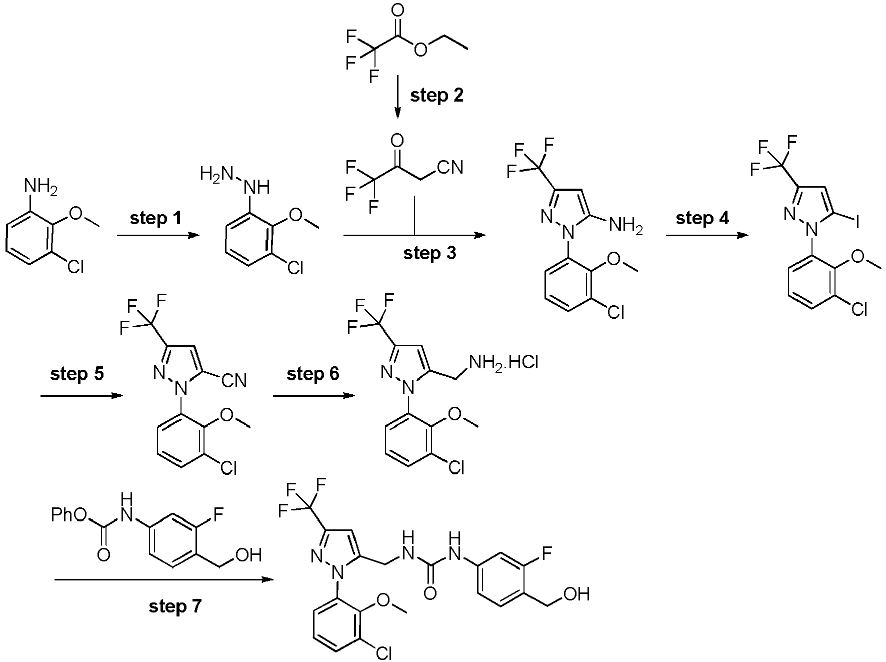

**Step** 1: To a stirred suspension of 3-chloro-2-methoxyaniline (5 g, 31.7 mmol, 1.0 eq) in conc. HCI (25 mL) was added a solution of NaNO₂ (3.28 g, 47.55 mmol, 1.5 eq.) in water (5 mL) dropwise at -5 to 0°C until the reaction mixture formed a clear solution. The reaction mixture was added drop wise to a stirred solution of SnCl₂ (13.24 g, 69.74 mmol, 2.2eq) in conc. HCI (50 mL) at -5°C for 30 min. A solid precipitate obtained was filtered off and washed with excess of ice cold water to get (3-chloro-2-methoxyphenyl)hydrazine (6.049 g, 94 %, brown solid; TLC system: EtOAc/PE (3:7) R_{f}: 0.4).
**Step 2:** To a stirred suspension of 60% NaH (33.80 g, 1.408 mol, 2 eq) in dioxane (700 mL) was added acetonitrile (44.3 mL, 0.845 mol, 1.2 eq.) at 0°C over a period of 30 min followed ethyl trifluoroacetate (83.70 mL, 0.704 mol, 1.0 eq). The reaction mixture was slowly and carefully heated to 100°C for 3 h. The dioxane was evaporated under reduced pressure and pH was adjusted to ∼6 with 1N HCl and extracted with ether (200mL x 4), dried (MgSO₄) and evaporated under vacuum to get 4,4,4-trifluoro-3-oxobutanenitrile (95 g, crude, brown oil). The compound was confirmed by matching with the TLC of reference compound.
**Step 3:** To a stirred solution of 4,4,4-trifluoro-3-oxobutanenitrile (1.64 g, 11.96 mmol, 1.0 eq) in ethanol (15 mL) was added (3-chloro-2-methoxyphenyl)hydrazine (2.5 g, 11.96 mmol, 1.0 eq) and the mixture was refluxed for 3 h. Ethanol was evaporated, the mixture diluted with water and extracted with ethyl acetate (300 mL x 2), dried (Na₂SO₄), evaporated again under vacuum. The crude was purified by CC using EtOAc/PE (1:4) as eluent to get 1-(3-chloro-2-methoxyphenyl)-3-(trifluoromethyl)-1H-pyrazol-5-amine (0.84 g, 24 %, yellow oil; TLC system: EtOAc/PE (2:3),R_{f}: 0.5).
**Step 4:** To a stirred suspension of KI (1.36 g, 8.25 mmol, 3.0 eq) and isoamylnitrile (1.1 mL, 8.25 mmol, 3.0 eq) in acetonitrile (20 mL) was added 1-(3-chloro-2-methoxyphenyl)-3-(trifluoromethyl)-1H-pyrazol-5-amine (0.8 g, 2.75 mmol, 1.0 eq) and refluxed for 12 h. Acetonitrile was evaporated, the mixture diluted with water and extracted with ethyl acetate (250 mL x 2), dried (Na₂SO₄), the solvent evaporated under vacuum. The crude was purified by CC using EtOAc/PE (1:19) to get 1-(3-chloro-2-methoxyphenyl)-5-iodo-3-(trifluoromethyl)-1H-pyrazole (0.2 g, crude, yellow oil; TLC system: EtOAc/PE (2:3), R_{f}: 0.75).
**Step 5:** To a stirred solution of 1-(3-chloro-2-methoxyphenyl)-5-iodo-3-(trifluoromethyl)-1H-pyrazole (0.2 g, 0.49 mmol, 1.0 eq) in NMP (5 mL) was added CuCN (0.044 g, 0.49 mmol, 1.0 eq) and heated to 200 °C for 2h. The reaction mixture was passed through celite pad and washed with excess of ethyl acetate. The filtrate was washed with water and ethyl acetate layer was separated, dried (Na₂SO₄), and the solvent evaporated. The residue obtained was purified by CC using ethyl acetate/PE (1:19) to get 1-(3-chloro-2-methoxyphenyl)-3-(trifluoromethyl)-1H-pyrazole-5-carbonitrile (0.05 g, crude, yellow solid; TLC system: EtOAc/PE (1:9) R_{f}: 0.5).
**Step 6:** To a stirred solution of 1-(3-chloro-2-methoxyphenyl)-3-(trifluoromethyl)-1H-pyrazole-5-carbonitrile (2.03 g, 6.74 mmol, 1.0 eq) in THF (20 mL) was added BH₃-DMS (0.512 g, 6.74 mmol, 1.0 eq) at 0°C and heated to reflux for 3 h. The reaction mixture was cooled to 0°C and quenched with 1N HCl and basified with 1N solution of NaOH to a pH of -10 and extracted with ethyl acetate (100mL x 2), dried (Na₂SO₄), the solvent evaporated to get a pale yellow oil. The oil was treated with ether and HCI to get (1-(3-chloro-2-methoxyphenyl)-3-(trifluoromethyl)-1H-pyrazol-5-yl)methanamine hydrochloride (0.20, 6% yield over 3 steps) as an off white solid (TLC system: CHCl₃/MeOH (9:1), R_{f} 0.5).
**Step 7:** To a stirred solution of (1-(3-chloro-2-methoxyphenyl)-3-(trifluoromethyl)-1H-pyrazol-5-yl)methanamine hydrochloride (100 mg, 0.38 mmol, 1.0 eq) in DCM (5 mL) was added TEA (0.16 mL, 1.14 mmol, 3.0 eq) followed by phenyl 3-fluoro-4-(hydroxymethyl)phenylcarbamate (131 mg, 0.38 mmol, 1.0 eq) at RT and stirred for 16h. The reaction mixture was diluted with water (15 mL), extracted with DCM (2x15 mL). The combined organic layer was washed with brine (15 mL), dried over anhydrous Na₂SO₄ and evaporated under vacuum. Crude was purified by CC to get 1-((1-(3-chloro-2-methoxyphenyl)-3-(trifluoromethyl)-1H-pyrazol-5-yl)methyl)-3-(3-fluoro-4-(hydroxymethyl)phenyl)urea (110 mg; 61%) as off white solid (TLC system: MeOH/CHCl₃ (1:9); R_{f}: 0.55).

### Synthesis of example A91: 1-[[2-(5-Chloro-2-methoxy-phenyl)-5-(trifluoromethyl)-2H-pyrazol-3-yl]-methyl]-3-[3-fluoro-4-(hydroxymethyl)-phenyl]-urea

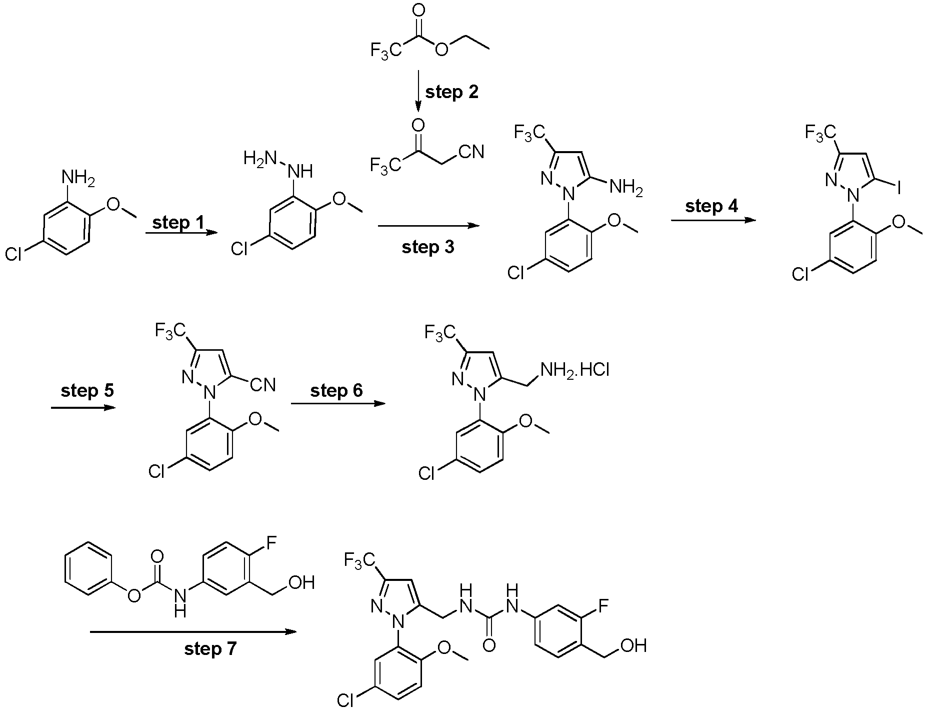

**Step 1:** To a stirred suspension of 5-chloro-2-methoxyaniline (20.0 g, 126.98 mmol, 1.0 eq) in conc. HCI (250 mL) was added a solution NaNO₂ (10.5 g, 152.38 mmol, 1.2 eq) in water (40 mL) dropwise at -5-0°C until reaction mixture formed a clear solution. The reaction mixture was added dropwise to a stirred solution of SnCl₂ (52.90 g, 279.36 mmol, 2.2 eq) in conc. HCl (250 mL) at -5°C for 30 min. The solid precipitate obtained was filtered off and washed with excess of ice cold water to get (5-chloro-2-methoxyphenyl)hydrazine (25.3 g, 92%, off-white solid; TLC system: EtOAc/PE (3:7) R_{f}: 0.6).
**Step 2:** As described for example **A89, step 2.**
**Step 3:** To a stirred suspension of 4,4,4-trifluoro-3-oxobutanenitrile (25.0 g, 182.44 mmol, 1.0 eq) in ethanol (500 mL) was added get (5-chloro-2-methoxyphenyl)hydrazine (38.13 g, 182.44 mmol, 1.0 eq) and the mixture refluxed for 3 h. Ethanol was evaporated, the residue diluted with water and extracted with ethyl acetate (300 mL x 2), dried (Na₂SO₄), the solvent evaporated under vacuum. The crude was purified by CC using EtOAC/PE (2:3) as eluent to get 1-(5-chloro-2-methoxyphenyl)-3-(trifluoromethyl)-1H-pyrazol-5-amine (13.09 g, 25%, yellow solid; TLC system: EtOAc/PE (1:1) R_{f}: 0.6).
**Step 4:** To a stirred suspension of KI (17.11 g, 103.08mmol, 3.0 eq) and isoamylnitrile (13.9 mL, 103.8 mmol, 3.0 eq) in acetonitrile (150 mL) was added 1-(5-chloro-2-methoxyphenyl)-3-(trifluoromethyl)-1H-pyrazol-5-amine (10 g, 34.36 mmol, 1.0 eq) and the mixture refluxed for 12 h. Acetonitrile was evaporated, the mixture diluted with water and extracted with ethyl acetate (200 mL x 2), dried (Na₂SO₄), the solvent evaporated under vacuum. The crude was purified by CC using EtOAc/PE (1:3) to get 1-(5-chloro-2-methoxyphenyl)-5-iodo-3-(trifluoromethyl)-1H-pyrazole (4.84 g, crude, yellow solid; TLC system: EtOAc/PE (1:4) R_{f}: 0.65).
**Step 5:** To a stirred solution of 1-(5-chloro-2-methoxyphenyl)-5-iodo-3-(trifluoromethyl)-1H-pyrazole (4.83 g, 12.01 mmol, 1.0 eq) in NMP (20 mL) was added CuCN (1.03g, 12.01 mmol, 1.0 eq) and the mixture heated to 200 °C for 2 h. The reaction mixture was passed through a celite pad and washed with excess of ethyl acetate. The filtrate was washed with water and the ethyl acetate layer was separated, dried (Na₂SO₄), and the solvent evaporated. The residue obtained was purified by CC using ethyl acetate/PE (1:19) to get 1-(5-chloro-2-methoxyphenyl)-3-(trifluoromethyl)-1H-pyrazole-5-carbonitrile (3.85 g, crude, yellow solid; TLC system: EtOAc/PE (1:4) R_{f}: 0.65).
**Step 6:** To a stirred solution of 1-(5-chloro-2-methoxyphenyl)-3-(trifluoromethyl)-1H-pyrazole-5-carbonitrile (3.09 g, 10.27 mmol, 1.0 eq) in THF (40 mL) was added BH₃-DMS (2.30 g, 30.81 mmol, 3.0 eq) at 0 °C and heated to reflux for 1h. The reaction mixture was cooled to 0°C and quenched with 1N HCl and basified with 1N solution of NaOH to a pH of ∼10 and the mixture extracted with ethyl acetate (100mL x 2), dried (Na₂SO₄), the solvent evaporated to get a pale yellow oil. The oil was treated with ether and HCl to get (1-(5-chloro-2-methoxyphenyl)-3-(trifluoromethyl)-1H-pyrazol-5-yl)methanamine hydrochloride (1.3 g, 20% yield over 3 steps) as white solid (TLC system: CHCl₃/MeOH (9:1), R_{f} 0.5).
**Step 7:** To a stirred solution of phenyl 4-fluoro-3-(hydroxymethyl)phenylcarbamate(0.2 g, 0.76 mmol, 1.0 eq) in DCM (5 mL) was added TEA (0.2306 g, 2.28 mmol, 3.0 eq) followed by (1-(5-chloro-2-methoxyphenyl)-3-(trifluoromethyl)-1H-pyrazol-5-yl)methanamine hydrochloride (0.2306 mg, 0.76 mmol, 1.0 eq) at RT and stirred for 16 h at RT. The solvent mixture was evaporated to get a residue, diluted with EtOAC (25 mL), washed with water (20 mL), brine (15 mL), dried over Na₂SO₄ and evaporated under vacuum. The residue obtained was purified by preparative HPLC to get 1-((1-(5-chloro-2-methoxyphenyl)-3-(trifluoromethyl)-1H-pyrazol-5-yl)methyl)-3-(3-fluoro-4-(hydroxymethyl)phenyl)urea (0.120 g; 33%) as white solid (TLC system: EtOAc, R_{f}: 0.6).

### Synthesis of example A100: N-[[5-tert-Butyl-2-(3-chlorophenyl)-2H-pyrazol-3-yl]-methyl]-2-[3-fluoro-4-(1-hydroxy-cyclopropyl)-phenyl]-propionamide

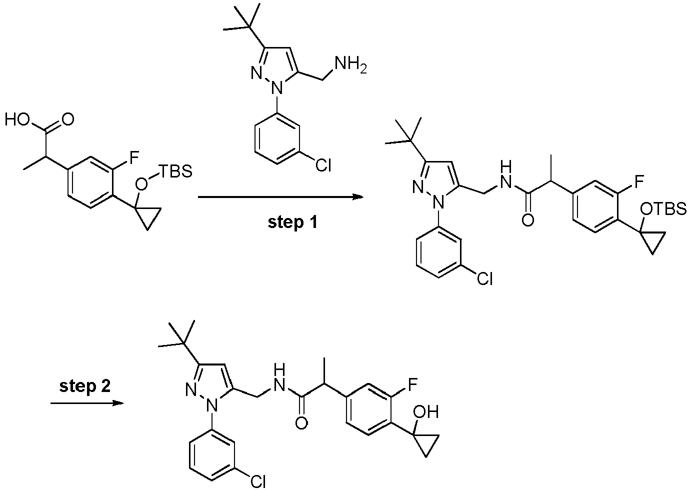

**Step 1:** To a stirred solution of 2-(4-(1-(tert-butyldimethylsilyloxy)cyclopropyl)-3-fluorophenyl)propanoic acid (135 mg, 0.400 mmol, 1 eq) in THF/DMF (3 mL/0.15 mL) was added DIPEA (0.271 mL, 1.6 mmol, 4 eq) followed by TBTU (131 mg, 0.400 mmol, 1eq) and HOBt (131 mg, 0.400 mmol, 1eq) at RT and the mixture was stirred for 3 h, then (3-tert-butyl-1-(3-chlorophenyl)-1H-pyrazol-5-yl)methanamine (175 mg, 0.56 mmol) was added and the mixture stirred for another 36 h. After completion of the reaction the reaction EtOAc was added and the mixture was washed with H₂O (15 mL), dried (Na₂SO₄) and the solvent evaporated. The crude product was purified by CC using EtOAc/cyclohexane (1:4) to get N-((3-tert-butyl-1-(3-chlorophenyl)-1H-pyrazol-5-yl)methyl)-2-(4-(1-(tert-butyldimethylsilyloxy)cyclopropyl)-3-fluorophenyl)propanamide (143 mg; 61%).
**Step 2:** To a stirred solution of N-((3-tert-butyl-1-(3-chlorophenyl)-1H-pyrazol-5-yl)methyl)-2-(4-(1-(tert-butyldimethylsilyloxy)cyclopropyl)-3-fluorophenyl)propanamide (141 mg, 0.240 mmol, 1 eq) in THF (2 mL) was added TBAF (c = 1 mol/L in THF, 0.61 mL, 0.61 mmol, 2.6 eq) at room temperature and the mixture was stirred for 5 h after which the solvent was evaporated. The residue was purified by CC using EtOAc/cyclohexane 1:3 as eluent to yield N-((3-tert-butyl-1-(3-chlorophenyl)-1H-pyrazol-5-yl)methyl)-2-(3-fluoro-4-(1-hydroxycyclopropyl)phenyl)propanamide (55 mg, 49%; TLC system: methanol/CHCl₃ (1: 9) R_{f}: 0.5).

### Synthesis of example A101: N-[[2-(3-Chlorophenyl)-5-(trifluoromethyl)-2H-pyrazol-3-yl]-methyl]-2-[3-fluoro-4-(1-hydroxy-cyclopropyl)-phenyl]-propionamide

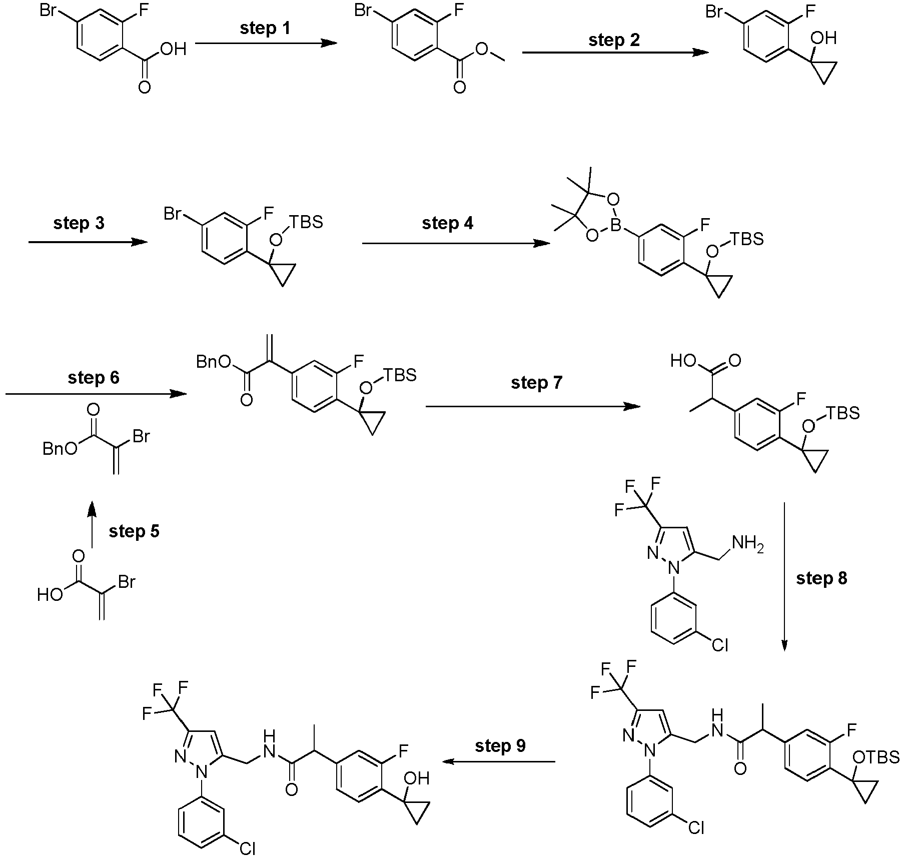

**Step 1:** To a stirred solution of 4-bromo-2-fluorobenzoic acid (15.0 g, 68.49 mmol, 1 eq) in MeOH (150 mL), SOCl₂ (23.09 mL, 136.9 mmol, 2 eq) was added at 0°C for 15 min and the mixture was stirred at RT for 1 2h. The MeOH was evaporated and the residue was diluted with ethyl acetate (250 mL), washed with a saturated aqueous solution of NaHCO₃, brine (150 mL) and water (150 mL). The ethyl acetate layer was dried over Na₂SO₄, evaporated under vacuum to get methyl 4-bromo-2-fluorobenzoate (15 g, 93%) as an off white solid (LC-MS purity, 99%; TLC system: EtOAc/PE (3:7), R_{f}: 0.8).
**Step 2:** To a stirred solution of Ti(OiPr)₄ (44.68 mL, 141.63 mmol, 1 eq) in diethyl ether (250 mL) was added freshly prepared ethyl magnesium iodide (212.44 mL, 424.89 mmol, 3 eq) in diethyl ether (220 mL) at -78 °C for 45 min. The reaction mixture was stirred for 90 min at -78 °C, then methyl 4-bromo-2-fluorobenzoate (33.0 g, 141.63 mmol, 1.0 eq) was added and the mixture allowed to stir at RT for 16 h. Saturated aqueous NH₄Cl (30 mL) was added and the mixture passed through celite pad. The ether layer was separated, washed with 1 N HCI (250 mL), brine (500 mL), water (750 mL), dried over MgSO₄ and the solvent evaporated. The residue obtained was purified by CC using PE/EtOAc (19:1) as eluent to get 1-(4-bromo-2-fluorophenyl)cyclopropanol (9.01 g, 28%; TLC system: PE/EtOAc (9:1), R_{f}: 0.3).
**Step 3:** To a stirred solution of 1-(4-bromo-2-fluorophenyl)cyclopropanol (9.01 g, 39.0 mmol, 1 eq) in DCM (100 mL) imidazole (5.33 g, 78 mmol, 2eq) was added at 0 °C followed by TBDMSCI (7.08 g, 46.8 mmol, 1.2 eq) and the reaction mixture was stirred for 3 h. The reaction mixture was washed with water (100 mL), brine (100 mL), dried over Na₂SO₄ and the solvent was evaporated to get (1-(4-bromo-2-fluorophenyl)cyclopropoxy)(tert-butyl)dimethylsilane which was purified by CC using PE as eluent to yield pure (1-(4-bromo-2-fluorophenyl)cyclopropoxy)(tert-butyl)dimethylsilane (9.01 g, 67%; TLC system: PE, R_{f}: 0.8) as a colourless oil.
**Step 4:** To a stirred and degassed (degassed with Argon) solution of (1-(4-bromo-2-fluorophenyl)cyclopropoxy)(tert-butyl)dimethylsilane (9.87g, 28.6 mmol, 1 eq), KOAc (8.45 g, 85.8 mmol, 3eq) and bis-pinacolatodiborane (7.99 g, 31.46 mmol, 1.1 eq) in dioxane (100 mL) was added Pd(PPh₃)₂Cl₂ (2.01 g, 2.86 mmol, 0.1 eq) and the mixture heated to 90 °C for 3 h. The dioxane was evaporated and the residue obtained was diluted with PE (30 mL) to filter it through flourosil pad and then evaporated to get crude tert-butyl(1-(2-fluoro-4-(4,4,5,5-tetramethyl-1,3,2-dioxaborolan-2-yl)phenyl)cyclopropoxy)dimethylsilane (10.23 g) as a semi solid (TLC system: PE, R_{f}: 0.3).
**Step 5:** As described for example **A118, step 6.**
**Step 6:** To a stirred and degassed (degassed with Argon) suspension of tert-butyl(1-(2-fluoro-4-(4,4,5,5-tetramethyl-1,3,2-dioxaborolan-2-yl)phenyl)cyclopropoxy)-dimethylsilane (10.23 g, 26.09 mmol, 1eq), Cs₂CO₃ (30.69 g, 78.27 mmol, 3 eq), and benzyl 2-bromoacrylate (9.59g, 39.14 mmol, 1.5 eq) in DMF (100 mL) was added Pd(dppf)Cl₂ (1.06 g, 1.305 mmol, 0.05 eq) and the mixture heated to 90-100 °C for 1 h. The reaction mixture was filtered through celite pad, diluted with water (100 mL) and extracted with ethyl acetate (250 mL). The ethyl acetate layer was washed with water (150 mL), brine (200 mL), dried (Na₂SO₄) and the solvent evaporated to get crude benzyl 2-(4-(1-(tert-butyldimethylsilyloxy)-cyclopropyl)-3-fluorophenyl)acrylate (4.14g) as a yellow oil which was used for the next stage without purification (TLC system: EtOAc/PE (1:49), R_{f}: 0.5).
**Step 7:** To a stirred solution of crude benzyl 2-(4-(1-(tert-butyldimethylsilyloxy)cyclopropyl)-3-fluorophenyl)acrylate (4.14g) in EtOAc (30 mL) was added 20% Pd(OH)₂ (207 mg, 5% moleq.) and stirred under hydrogen gas balloon at RT for 1 h. The Pd(OH)₂ was filtered off, filtrate evaporated and residue obtained was purified by silica gel (neutral, 100-200) column using ethyl acetate/PE (3:17) as eluent to get crude 2-(4-(1-(tert-butyldimethylsilyloxy)cyclopropyl)-3-fluorophenyl)propanoic acid (1.8g, 64% LC-MS, HPLC purity) as off white solid. The crude product on prep HPLC purification provided (1.23 g, 13% yield over 3 steps). [TLC system: EtOAc/PE (1:5), R_{f}: 0.6].
**Step 8:** To a stirred solution of 2-(4-(1-(tert-butyldimethylsilyloxy)cyclopropyl)-3-fluorophenyl)propanoic acid (190 mg, 0.56 mmol, 1 eq) in DCM (10 mL) was added DIPEA (0.29 mL, 1.68 mmol, 3 eq) followed by EDC.HCl (128 mg, 0.67 mmol, 1.2 eq) and HOBt (103 mg, 0.67 mmol, 1.2 eq) at RT and stirred for 10 min when (1-(3-chlorophenyl)-3-(trifluoromethyl)-1H-pyrazol-5-yl)methanamine(175 mg, 0.56 mmol) was added and the mixture stirred for 3 h. After completion of the reaction the reaction mixture was washed with H₂O (15 mL), brine (10 mL), dried (Na₂SO₄) and the solvents evaporated. The crude product was purified by CC using EtOAc/PE (3:7) to get 2-(4-(1-(tert-butyldimethylsilyloxy)-cyclopropyl)-3-fluorophenyl)-N-((1-(3-chlorophenyl)-3-(trifluoromethyl)-1H-pyrazol-5-yl)methyl)propanamide (103 mg; 31%) as off white solid (TLC system: EtOAc/PE (3:7) R_{f}: 0.5).
**Step 9:** To a stirred solution of 2-(4-(1-(tert-butyldimethylsilyloxy)cyclopropyl)-3-fluorophenyl)-N-((1-(3-chlorophenyl)-3-(trifluoromethyl)-1H-pyrazol-5-yl)methyl)-propanamide (103 mg, 0.173 mmol, 1 eq) in THF (5 mL) was added TBAF (90 mg, 0.346 mmol, 2 eq) at room temperature and the mixture was stirred for 2 h after which THF was evaporated and the residue was dissolved in ethyl acetate (15 mL), washed with water (10 mL x 2), brine (10 mL) and the solvent evaporated. The residue was purified by CC using methanol/CHCl₃ (1:9) as eluent to give N-((1-(3-chlorophenyl)-3-(trifluoromethyl)-1H-pyrazol-5-yl)methyl)-2-(3-fluoro-4-(1-hydroxycyclopropyl)phenyl)propanamide (41 mg, 49%) as off white solid (TLC system: methanol/CHCl₃ (1:9) R_{f}: 0.5).

### Synthesis of example A105: 1-[[5-tert-Butyl-2-(3-chlorophenyl)-2H-pyrazol-3-yl]-methyl]-3-[3-fluoro-4-(1-hydroxy-cyclopropyl)-phenyl]-urea

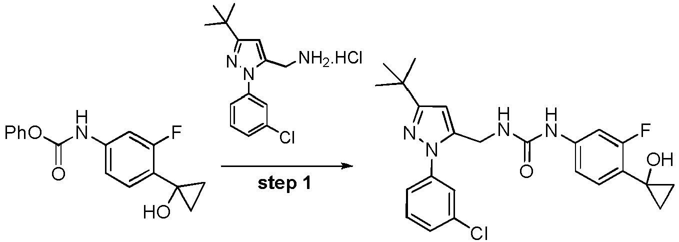

Step 1: To a stirred solution of (3-tert-butyl-1-(3-chlorophenyl)-1H-pyrazol-5-yl)methanamine hydrochloride (synthesis described for **example A106**) (0.104 mg, 0.348 mmol, 1.0 eq) in MeCN (8 mL) was added TEA (0.193 mL, 1.39 mmol, 4.0 eq) followed by addition of phenyl 3-fluoro-4-(1-hydroxycyclopropyl)phenylcarbamate (0.10 mg, 0.355 mmol, 1.02 eq) at RT and the mixture was stirred at reflux overnight. The solvents were evaporated and the crude product was purified by CC (eluent EtOAc/cyclohexane 1:2) to get 1-((3-tert-butyl-1-(3-chlorophenyl)-1H-pyrazol-5-yl)methyl)-3-(3-fluoro-4-(1-hydroxycyclopropyl)phenyl)urea (104 mg; 65%).

### Synthesis of example A106: 1-[[2-(3-Chlorophenyl)-5-(trifluoromethyl)-2H-pyrazol-3-yl]-methyl]-3-[3-fluoro-4-(1-hydroxy-cyclopropyl)-phenyl]-urea

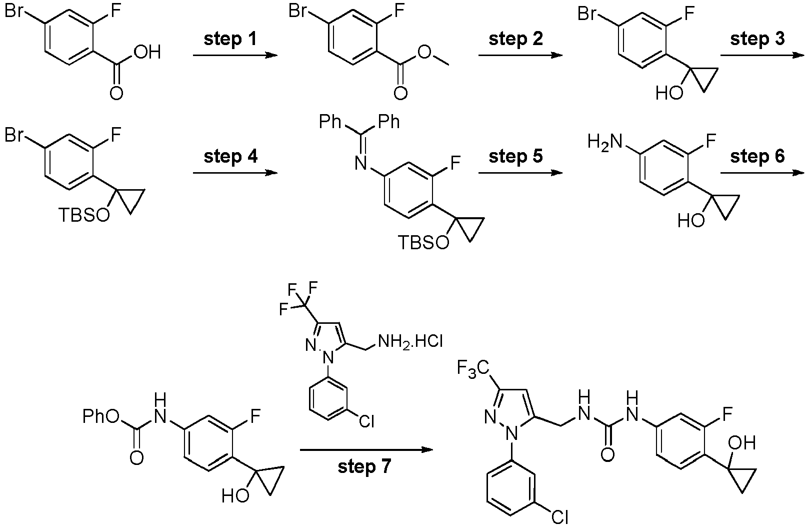

**Steps 1-3:** as described for the synthesis of **example A101**
**Step 4:** To a degassed suspension of (1-(4-bromo-2-fluorophenyl)cyclopropoxy)(tert-butyl)dimethylsilane (4.0 g, 11.59 mmol, 1.0 eq) in toluene (50 mL), was added BINAP (0.261 g, 0.347 mmol, 0.03 eq), benzophenone imine (2.1 g, 11.59 mmol, 1.0 eq) followed by Cs₂CO₃ (5.64g, 17.38mmol, 1.5 eq) in a sealed tube, then was added Pd₂(dba)₃ (0.138 g, 0.15 mmol, 0.013 eq) and the mixture heated to 100°C for 8 h. The reaction mixture was diluted with EtOAc (120 mL), washed with water (25mL x 2), brine (25 mL) and dried over Na₂SO₄ and the solvent was evaporated under vacuum to get 4-(1-(tert-butyldimethylsilyloxy)cyclopropyl)-N-(diphenylmethylene)-3-fluoroaniline (4.8 g) as a crude as yellow oil (TLC system: EtOAc/PE (1:49), R_{f}: 0.75).
**Step 5:** To a stirred solution of 4-(1-(tert-butyldimethylsilyloxy)cyclopropyl)-N-(diphenylmethylene)-3-fluoroaniline (4.5 mg, 10.11 mmol, 1.0 eq) in THF (10 mL) was added 1N HCl (20 mL) and stirred vigorously for 1 h. The mixture was extracted with EtOAc (20mL). The aqueous layer was basified with 1N aq. NaOH (30 mL) and extracted with EtOAc (50 mL). The combined organic layers were extracted with brine (20 mL) and dried over Na₂SO₄ and the solvent evaporated under vaccum; the resulting residue was purified by CC using EtOAc/PE (1:1) as eluent to get 1-(4-amino-2-fluorophenyl)cyclopropanol (0.7g, 49%, yellow solid; TLC system: EtOAc/PE (1:1), R_{f}: 0.25).
**Step 6:** To a stirred solution of 1-(4-amino-2-fluorophenyl)cyclopropanol (0.7mg, 4.19 mmol, 1.0 eq) in acetone (10 mL) was added pyridine (0.33mL, 4.19 mmol, 1.0 eq) and phenyl chloroformate (0.5 mL, 4.19 mmol, 1.0 eq) and stirred at 0°C to RT for 1 h. The solvent was evaporated, the residue diluted with water (10 mL x 2), extracted with EtOAc (25mL), brine (10 mL) and dried over Na₂SO₄ and evaporated under vacuum; the resulting residue was purified by CC using EtOAc/PE (1:4 as eluent to get phenyl 3-fluoro-4-(1-hydroxycyclopropyl)phenylcarbamate (0.9 g, 75%, off white solid; TLC system: EtOAc/PE (1:1), R_{f}: 0.6).
**Step 7:** To a stirred solution of (1-(3-chlorophenyl)-3-(trifluoromethyl)-1H-pyrazol-5-yl)methanamine hydrochloride (0.141 mg, 0.45 mmol, 1.0 eq) in DCM (5 mL) was added TEA (0.137 mg, 1.35 mmol, 3.0 eq) followed by phenyl 3-fluoro-4-(1-hydroxycyclopropyl)-phenylcarbamate (0.130 mg, 0.45 mmol, 1.0 eq) at RT and stirred overnight. The reaction mixture was diluted with DCM (20 mL), washed with water (10 mL), brine (5 mL), dried over anhydrous Na₂SO₄ and the solvents evaporated under vacuum. Crude was purified by CC by using EtOAc/PE (1:1) to get 1-((1-(3-chlorophenyl)-3-(trifluoromethyl)-1H-pyrazol-5-yl)methyl)-3-(3-fluoro-4-(1-hydroxycyclopropyl)-phenyl)urea (0.0912 g; 43%) as pale yellow solid (TLC system: EtOAc/PE (3:1); R_{f}: 0.45).

### Synthesis of example A107: 1-[[5-tert-Butyl-2-(3-chlorophenyl)-2H-pyrazol-3-yl]-methyl]-3-[4-(3-hydroxy-oxetan-3-yl)-phenyl]-urea

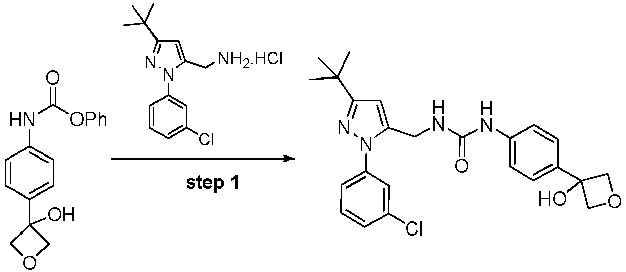

**Step 1:** To a stirred solution of phenyl 4-(3-hydroxyoxetan-3-yl)phenylcarbamate (synthesis described for **example A108**) (97 mg, 0.34 mmol, 1.02 eq) and (3-tert-butyl-1-(3-chlorophenyl)-1H-pyrazol-5-yl)methanamine hydrochloride (100 mg, 0.33 mmol, 1.0 eq) in MeCN (8 mL) was added TEA (0.185 mL, 1.33 mmol, 4.0 eq) at RT and the mixture stirred at reflux overnight. The solvents were evaporated and the crude product was purified by CC (EtOAc/cyclohexane 3:2 as eluent) to get 1-((3-tert-butyl-1-(3-chlorophenyl)-1H-pyrazol-5-yl)methyl)-3-(4-(3-hydroxyoxetan-3-yl)phenyl)urea (92 mg; 61 %).

### Synthesis of example A108: 1-[[2-(3-Chlorophenyl)-5-(trifluoromethyl)-2H-pyrazol-3-yl]-methyl]-3-[4-(3-hydroxy-oxetan-3-yl)-phenyl]-urea

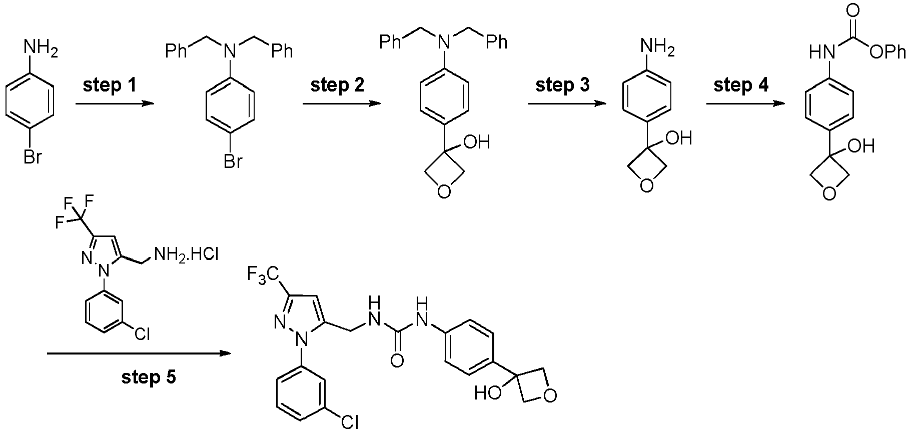

**Step 1:** To a stirred solution of 4-bromoaniline (500 mg, 2.92 mmol, 1.0 eq) and K₂CO₃ (1.21 g, 8.76 mmol, 3.0 eq) in CH₃CN (10 mL) was added benzyl bromide (1.24 g, 7.30 mmol, 2.5 eq) and the mixture stirred for 16 h at 80°C and cooled to RT. K₂CO₃ was filtered off, the filtrate concentrated, the resulting crude was purified by CC using EtOAc/PE (1:19) to get N,N-dibenzyl-4-bromoaniline (900 mg, 87 %; TLC system: EtOAc/PE (3:7), R_{f}: 0.7).
**Step 2:** To a stirred solution of N,N-dibenzyl-4-bromoaniline (500 mg, 1.42 mmol, 1.0 eq) in dry THF (20mL), cooled to -78°C, was added n-BuLi (118 g, 1.84 mmol, 1.3 eq) slowly and the mixture stirred at -78°C for 15 min. Then 3-oxatanone was added (103 g, 1.43 mmol, 1.0 eq) and the temperature was raised to -20°C. The mixture was quenched with sat. NH₄Cl (50 mL), extracted with EtOAC (50mL x 2).The organic layer was washed with brine (50 mL), dried over anhydrous Na₂SO₄, concentrated and the resulting crude was purified by CC using EtOAc/PE (1:4) to get 3-(4-(dibenzylamino)phenyl)oxetan-3-ol (320 mg 65 %; TLC system: EtOAc/PE (2:3), R_{f}: 0.3).
**Step 3:** To a stirred solution of 3-(4-(dibenzylamino)phenyl)oxetan-3-ol (1.5 g, 5.217 mmol, 1.0 eq) in THF (50 mL) and EtOH (50 mL) was added 10%Pd/C (300 mg) and the mixture was stirred in hydrogen atmosphere at 40 psi H₂ for 5h. The mixture was passed through celite, the filtrate concentrated under reduced pressure to get 3-(4-aminophenyl)oxetan-3-ol (1.1 g, 77%; TLC system: EtOAc/PE (7:3), R_{f}: 0.55).
**Step 4:** To a stirred solution of 3-(4-aminophenyl)oxetan-3-ol (1.19g, 6.66 mmol, 1.0 eq) in acetone (20 mL) was added pyridine (1.05 g, 13.29 mmol, 2.0 eq) and phenyl chloroformate (1.05g, 6.68 mmol, 1.1 eq) at 0 °C and the mixture was stirred at 0°C for 30 min. The solvent was evaporated. The resulting residue was purified by CC using EtOAc/PE (3:7) as eluent to get phenyl 4-(3-hydroxyoxetan-3-yl)phenylcarbamate(1.2 g, 63%) as a white solid (TLC system: EtOAc/PE (7:3), R_{f}: 0.7).
**Step 5:** To a stirred solution of phenyl 4-(3-hydroxyoxetan-3-yl)phenylcarbamate (100 mg, 0.35 mmol, 1.0 eq) and (1-(3-chlorophenyl)-3-(trifluoromethyl)-1H-pyrazol-5-yl)methanamine hydrochloride (119.3 mg, 0.35 mmol, 1.0 eq) in DCM (10 mL) was added TEA (106 mg, 1.049 mmol, 3.0 eq) at RT and stirred at 40°C overnight. The mixture was diluted with DCM (30 mL) and washed with water (10 mL), brine (10 mL) and dried over anhydrous Na₂SO₄ and concentrated. Crude was purified by CC using MeOH/CHCl₃ (1:9) to get 1-((1-(3-chlorophenyl)-3-(trifluoromethyl)-1H-pyrazol-5-yl)methyl)-3-(4-(3-hydroxyoxetan-3-yl)phenyl)urea (100 mg; 61 %) as a white solid (TLC system: MeOH/CHCl₃ (1:9); R_{f}: 0.45).

### Synthesis of example A109: 1-[[5-tert-Butyl-2-(3-chlorophenyl)-2H-pyrazol-3-yl]-methyl]-3-[3-fluoro-4-(3-hydroxy-oxetan-3-yl)-phenyl]-urea

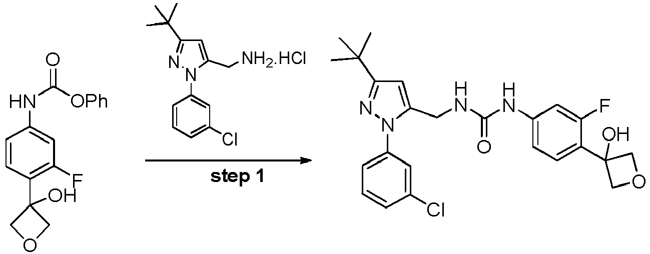

Step 1: To a stirred solution of (3-tert-butyl-1-(3-chlorophenyl)-1H-pyrazol-5-yl)methanamine hydrochloride (96 mg, 0.32mmol, 1.0 eq) in MeCN (8 mL) was added TEA (0.178 mL, 1.28 mmol, 4.0 eq) followed by phenyl 3-fluoro-4-(3-hydroxyoxetan-3-yl)phenylcarbamate (99 mg, 0.3 3mmol, 1.02 eq) at RT and stirred at reflux for 16 h. The solvent was evaporated and the crude product was purified by CC (EtOAc/cyclohexane 1:1 as eluent) to get 1-((3-tert-butyl-1-(3-chlorophenyl)-1H-pyrazol-5-yl)methyl)-3-(3-fluoro-4-(3-hydroxyoxetan-3-yl)phenyl)urea (116mg; 77%).

### Synthesis of example A110: 1-[[5-tert-Butyl-2-(3-chlorophenyl)-2H-pyrazol-3-yl]-methyl]-3-[3-fluoro-4-(3-hydroxy-oxetan-3-yl)-phenyl]-urea

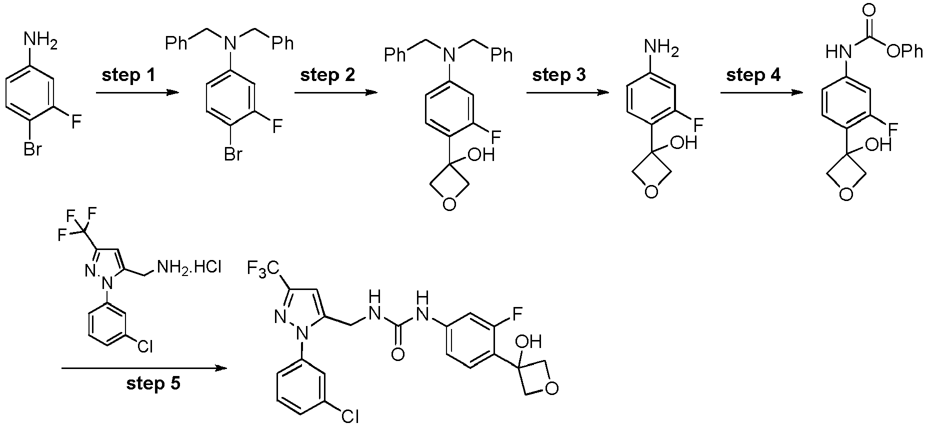

**Step 1:** To a suspension of 4-bromo-3-fluoroaniline (10.0 g, 52.6 mmol, 1.0 eq) and K₂CO₃ (21.7 g, 157.8 mmol, 3.0eq) in MeCN (10mL) was added benzyl bromide (19.8 g, 115.8 mmol, 2.2 eq) and the mixture was stirred for 16 h at 80 °C and then filtered through celite pad, and the solvents was evaporated under vacuum and washed with PE (100 mL) to get N,N-dibenzyl-4-bromo-3-fluoroaniline (13.8 g, 55 %; TLC system: EtOAc/PE (1:9), R_{f}: 0.6).
**Step 2:** To a stirred solution of N,N-dibenzyl-4-bromo-3-fluoroaniline (6.8 g, 18.38 mmol, 1.0 eq) in dry THF (150 mL) was cooled to -78°C was added n-BuLi (1.4 g, 22.6 mmol, 1.2 eq) slowly and the mixture was stirred at -78°C for 30 min., followed by addition of 3-oxatanone (1.32 g, 18.38 mmol, 1.0 eq) and the temperature was raised to RT. The mixture was quenched with sat. NH₄Cl (20 mL), diluted with water (50 mL) extracted with EtOAC (50mL x 2).The organic layer was washed with brine (25 mL), dried over anhydrous Na₂SO₄ and evaporated under vacuum. The resulting crude was purified by CC using EtOAc/PE (2:3) to get 3-(4-(dibenzylamino)-2-fluorophenyl)oxetan-3-ol (3.0 g, 45 %; TLC system: EtOAc/PE (1:1), R_{f}: 0.45).
**Step 3:** To a stirred solution of 3-(4-(dibenzylamino)-2-fluorophenyl)oxetan-3-ol (2.15 g, 5.92 mmol, 1.0 eq) in THF (125 mL) was added 10% Pd/C (0.230 g) under argon and stirred under hydrogen balloon pressure for 16 h. The mixture was passed through celite, and the filtrate concentrated under vacuum. The resulting crude product was purified by CC using EtOAc/PE (7:3) to get 3-(4-amino-2-fluorophenyl)oxetan-3-ol (0.9 g, 73 %; TLC system: EtOAc 100%, R_{f}: 0.5).
**Step 4:** To a stirred solution of 3-(4-amino-2-fluorophenyl)oxetan-3-ol (0.93 g, 5.08 mmol, 1.0 eq) in acetone (10 mL) was added pyridine (0.82 mL, 10.16 mmol, 2.0 eq) and phenyl chloroformate (0.7 mL, 5.59 mmol, 1.1 eq) at 0 °C and the mixture was stirred at RT for 1 h. The solvents was evaporated and the crude was extracted with EtOAC (25 mL), diluted with water (15 mL), washed with brine (10 mL), dried over anhydrous Na₂SO₄ and evaporated under vacuum. The resulting residue was washed with diethyl ether (25 mL) to get phenyl 3-fluoro-4-(3-hydroxyoxetan-3-yl)phenylcarbamate (0.892 g, 59%) as a off white solid (TLC system: 100 % EtOAc, R_{f}: 0.55).
**Step 5:** To a stirred solution of (1-(3-chlorophenyl)-3-(trifluoromethyl)-1H-pyrazol-5-yl)methanamine hydrochloride (0.206 mg, 0.66 mmol, 1.0 eq) in DCM (5 mL) was added TEA (0.13 g, 1.32 mmol, 2.0 eq) followed by phenyl 3-fluoro-4-(3-hydroxyoxetan-3-yl)phenylcarbamate (0.2 g, 0.66 mmol, 1.0 eq) at RT and stirred for 16 h. The reaction mixture was extracted with DCM (20mL), washed with water (15 mL), brine (15mL), dried over anhydrous Na₂SO₄ and evaporated under vacuum. Crude was purified by CC using EtOAc/PE (7:3) to get 1-((1-(3-chlorophenyl)-3-(trifluoromethyl)-1H-pyrazol-5-yl)methyl)-3-(3-fluoro-4-(3-hydroxyoxetan-3-yl)phenyl)urea (0.075 g; 23%) as off white solid (TLC system: EtOAc/PE (1:1); R_{f}: 0.55).

### Synthesis of example A111: N-[[2-(3-Chlorophenyl)-5-(trifluoromethyl)-2H-pyrazol-3-yl]-methyl]-2-[3-fluoro-4-(2-methoxy-ethoxy-methyl)-phenyl]-propionamide

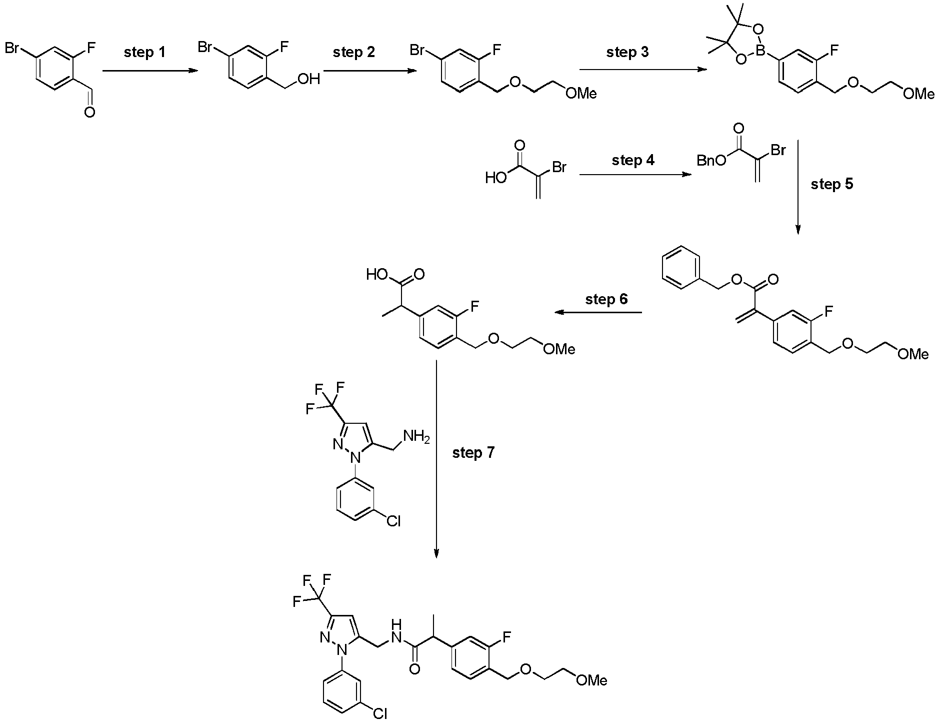

**Step 1:** as described for **example A118, step 3**
**Step 2:** To a stirred solution of (4-bromo-2-fluorophenyl)methanol (10 g, 49.02 mmol) in THF (250 mL) at 0°C, was added 60% NaH (2.93 g, 73.53 mmol) slowly in portions. After addition, the suspension was heated to 50°C for 30 min, cooled to RT, then was added 1-bromo-2-methoxy ethane (5 mL, 53.92 mmol) and the mixture was stirred at RT for 20 h until complete consumption of (4-bromo-2-fluorophenyl)methanol, as evidenced by TLC analysis. The reaction mixture was diluted with ice cold water (100 mL) and concentrated under reduced pressure. The obtained aqueous residue was extracted with EtOAc (2 × 150mL); the combined EtOAc layers were washed with brine solution (50 mL), dried over anhydrous NaSO₄, filtered and concentrated. The obtained crude compound was purified by CC using 5% EtOAc in PE as eluent to afford 4-bromo-2-fluoro-1-((2-methoxyethoxy)methyl)benzene (6 g, 47%) as yellow liquid (TLC solvent system: 30% EtOAc-PE; R_{f}: 0.4).
**Step** 3: A stirred suspension of 4-bromo-2-fluoro-1-((2-methoxyethoxy)methyl)benzene (6.0 g, 22.8 mmol), bispinacolatodiboron (5.8 g, 22.8 mmol), CH₃COOK (6.7 g, 68.4mmol) in THF (50 mL) was deoxygenated by purging with a stream of Argon for 30 min, and Pd (PPh₃)₂Cl₂ (36.5 mg, 0.228 mmol) was added, and purging was continued for further 10 min. The reaction mixture was stirred at 100°C for 1h until complete consumption of 4-bromo-2-fluoro-1-((2-methoxyethoxy)methyl)benzene, as evidenced by TLC analysis. The reaction mixture was concentrated and the obtained crude compound was purified by CC using 10% EtOAc in PE as eluent to afford 2-(3-fluoro-4-((2-methoxyethoxy)methyl)phenyl)-4,4,5,5-tetramethyl-1,3,2-dioxaborolane (5 g, 62%) as a pale yellow oil. (TLC solvent system: 30% EtOAc-PE; R_{f}: 0.4).
**Step 4**: as described for **example A118, step 6**
**Step 5:** A suspension of 2-(3-fluoro-4-((2-methoxyethoxy)methyl)phenyl)-4,4,5,5-tetramethyl-1,3,2-dioxaborolane (5 g, 16.129 mmol), Cs₂CO₃ (15.7 g, 48.38 mmol) in DMF (50 mL) was deoxygenated by purging Argon for 30 min at RT. Pd(dppf)Cl₂ (657 mg, 0.806 mmol) was added and purging was continued. After 10min, benzyl 2-bromoacrylate (4.6 g, 19.35 mmol) was added and stirred at 100°C for 1 h until complete consumption of 2-(3-fluoro-4-((2-methoxyethoxy)methyl)phenyl)-4,4,5,5-tetramethyl-1,3,2-dioxaborolane, as evidenced by TLC analysis. The reaction mixture was diluted with ethyl acetate (200 mL), filtered through a celite pad, washed with ethyl acetate (2x25 mL). The filtrate was washed with water (2x100mL), brine (50 mL), dried over anhydrous NaSO₄, filtered and concentrated. The obtained crude compound was purified by CC using 10% ethyl acetate in PE as eluent to affordbenzyl 2-(3-fluoro-4-((2-methoxyethoxy)methyl)phenyl)acrylate (1.4 g, 25%) as pale brown oil (TLC solvent system: 30% EtOAc-PE; R_{f}: 0.4).
**Step 6:** A suspension of benzyl 2-(3-fluoro-4-((2-methoxyethoxy)methyl)phenyl)acrylate (2.8 g, 8.139 mmol), 10% Pd/C (300 mg) in MeOH (20 mL) was hydrogenated (balloon pressure) at RT for 1 h until complete consumption of benzyl 2-(3-fluoro-4-((2-methoxyethoxy)methyl)phenyl)acrylate, as evidenced by TLC analysis. The reaction mixture was filtered through celite pad, washed with MeOH (2×15 mL). The combined filtrate was concentrated and the obtained crude compound was purified by CC using 30% EtOAc in PE as eluent to afford 2-(3-fluoro-4-((2-methoxyethoxy)methyl)phenyl)propanoic acid (1.2 g, 58%) as a colorless oil (TLC solvent system: 30% EtOAc-PE; R_{f}: 0.15).
**Step 7:** To a stirred DCM (5.0 mL) solution of2-(3-fluoro-4-((2-methoxyethoxy)methyl)-phenyl)propanoic acid (82.0 mg, 0.321 mmol, 1.0 eq), DIPEA (0.168 mL, 0.961 mmol, 3.0 eq), EDC.HCl (74.0 mg, 0.387 mmol, 1.2 eq) and HOBt (59.0 mg, 0.387 mmol, 1.2 eq) were sequentially added at RT and the mixture stirred for 15 min. (1-(3-chlorophenyl)-3-(trifluoromethyl)-1H-pyrazol-5-yl)methanamine (100 mg, 0.321 mmol, 1 eq) was then added and the mixture stirred for 16 h. On completion of the reaction the mixture was washed with H₂O (20 mL), brine (10 mL), the layers were separated, dried (Na₂SO₄) and the solvent evaporated. The crude product was purified by CC using EtOAc/CHCl₃ (1:9) to get N-((3-tert-butyl-1-(3-chlorophenyl)-1H-pyrazol-5-yl)methyl)-2-(3-fluoro-4-((2-methoxyethoxy)methyl)phenyl)propanamide (115 mg; 70%, white solid; TLC system: EtOAc/CHCl₃ (1:9), R_{f}: 0.5).

### Synthesis of example A117: N-[[5-tert-Butyl-2-(3-chlorophenyl)-2H-pyrazol-3-yl]-methyl]-2-[3-fluoro-4-(2-hydroxy-ethoxy-methyl)-phenyl]-propionamide

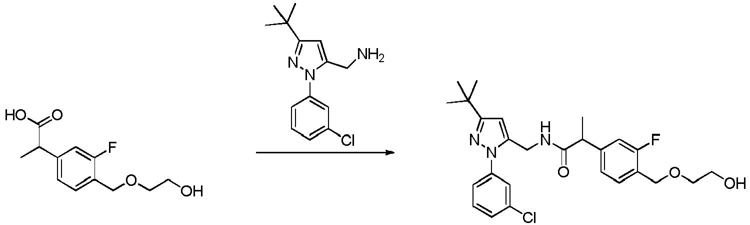

To a stirred THF/DMF (2.0 mL/0.1 mL) solution of 2-(3-fluoro-4-((2-hydroxyethoxy)methyl)phenyl)propanoic acid (synthesis described for **example A118**) (60.0 mg, 0.25 mmol, 1.0 eq) DIPEA (0.169 mL, 0.13 mmol, 4.0 eq), TBTU (81 mg, 0.25 mmol, 1eq) and HOBt (33 mg, 0.25 mmol, 1eq) were sequentially added at RT and the mixture stirred for 15 min. (3-tert-butyl-1-(3-chlorophenyl)-1H-pyrazol-5-yl)methanamine (65 mg, 0.25mmol, 1 eq) was then added and the mixture stirred for 36 h. After completion of the reaction the solvent was evaporated. The crude product was purified by CC using EtOAc/cyclohexane (1:1) to get N-((3-tert-butyl-1-(3-chlorophenyl)-1H-pyrazol-5-yl)methyl)-2-(3-fluoro-4-((2-hydroxyethoxy)methyl)phenyl)-propanamide (106 mg; 87%).

### Synthesis of example A118: N-[[2-(3-Chlorophenyl)-5-(trifluoromethyl)-2H-pyrazol-3-yl]-methyl]-2-[3-fluoro-4-(2-hydroxy-ethoxy-methyl)-phenyl]-propionamide

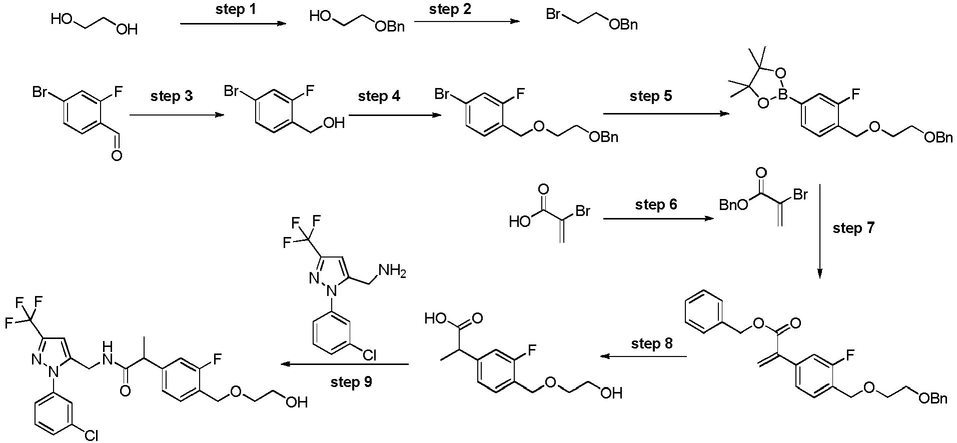

**Step 1:** To a solution of diethylengylcole (30 g, 483.87 mmol) in THF (300 mL) at 0 °C, a 60% suspension of NaH (9.67 g, 241.93 mmol) was slowly added in portions which was followed by the addition of benzyl bromide (28.9 mL, 241.93 mmol) after which the suspension was stirred at RT for 16h until complete consumption of starting material, as evidenced by TLC analysis. The reaction mixture was then diluted with ice cold water (100 mL), extracted with EtOAc (3x250 mL) and the combined EtOAc layers were washed with brine (50 mL), dried over anhydrous NaSO₄, filtered and concentrated. The obtained crude compound was purified by CC using 25% EtOAc in PE as eluent to afford 2-(benzyloxy)ethanol (25 g, 34%) as pale yellow liquid (TLC solvent system: 40% EtOAc in PE; R_{f}: 0.3).
**Step 2:** To a stirred solution of 2-(benzyloxy)ethanol (17.0 g, 111.84 mmol) and PPh₃ (35.0 g, 134.21 mmol) in DCM (170 mL) at -10 °C to -5 °C, NBS (23.88 g, 134.21 mmol) was added slowly in portions maintaining the temperature at -10 °C to -5 °C. After addition, the reaction mixture was allowed to stir at RT for 1 h until complete consumption starting material, as evidenced by TLC analysis. The reaction mixture was concentrated, the obtained crude compound was purified by CC using 5% EtOAc in PE as eluent to afford ((2-bromoethoxy)methyl)benzene (13.5 g, 56.2%) as pale yellow liquid (TLC solvent system: 30% EtOAc in PE; R_{f}: 0.7).
**Step 3:** To a stirred solution of 4-Bromo-2-fluoro benzaldehyde (15 g, 79.36 mmol) in MeOH (100 mL) at -5 °C to 0 °C was added NaBH₄ (6.0 g, 158.73 mmol) in equal portions and stirred at RT for 1h until the starting material was completely consumed, as evidenced by TLC analysis. The reaction mixture was then diluted with ice cold water (100 mL) and concentrated under reduced pressure. The residue obtained on concentration was extracted with EtOAc (2x200 mL) and separated. The combined EtOAc layers were washed with brine solution (50 mL), dried over anhydrous NaSO₄, filtered and concentrated to afford (4-bromo-2-fluorophenyl)methanol (30 g, 99 % (from 2 batches))] as colorless oil (TLC solvent system: 30% EtOAc in PE; R_{f}: 0.3).
**Step 4:** To a stirred solution of (4-bromo-2-fluorophenyl)methanol(5 g, 24.509 mmol) in DMF (50 mL) at 0 °C NaH (60% suspension in mineral oil, 1.96 g, 49.018 mmol) was added in portions. To the resulting suspension ((2-bromoethoxy)methyl)benzene (6.32 g, 29.41 mmol) was added at 0 °C, and the reaction mixture was allowed to stir at RT for 16 h until complete consumption of (4-bromo-2-fluorophenyl)methanol, as evidenced by TLC analysis. The reaction mixture was quenched with MeOH (5 mL), diluted with ice cold water (50 mL) and was extracted with EtOAc (3×100 mL). The combined EtOAc layer was washed with water (50 mL), brine (50 mL), dried over anhydrous NaSO₄, filtered and concentrated. The obtained crude compound was purified by CC using 5% EtOAc in PE as eluent to afford 1-((2-(benzyloxy)ethoxy)methyl)-4-bromo-2-fluorobenzene (4.2 g, 51%) as yellow liquid (TLC solvent system: 30% EtOAc in PE; R_{f}: 0.6).
**Step 5:** A suspension of 1-((2-(benzyloxy)ethoxy)methyl)-4-bromo-2-fluorobenzene (14.0 g, 41.297 mmol), bis-pinacolatodiborone (20.9 g, 82.59 mmol), CH₃COOK (8.09 g, 82.59 mmol) in 1, 4-dioxane (50 mL) was deoxygenated by purging with a stream of argon for 30 min to which Pd(PPh₃)₂Cl₂ (2.89 g, 4.129 mmol) was added and purging was continued for further 10 min. The reaction mixture was stirred at 100 °C for 30h until complete consumption of 1-((2-(benzyloxy)ethoxy)methyl)-4-bromo-2-fluorobenzene, as evidenced by TLC analysis. The reaction mixture was concentrated and the obtained crude compound was purified by column chromatography (60-120 mesh florosil) using 5% EtOAc in PE as eluent to afford 2-(4-((2-(benzyloxy)ethoxy)methyl)-3-fluorophenyl)-4,4,5,5-tetramethyl-1,3,2-dioxaborolane (12.5 g, 79%) as a pale yellow oil (TLC solvent system: 30% EtOAc in PE; R_{f}: 0.45).
**Step 6:** A suspension of 2-bromoacrylic acid (25.0 g, 166.66 mmol), benzyl bromide (21.8.0 mL, 183.32 mmol) and K₂CO₃ (46 g, 333.32 mmol) in acetonitrile (250 mL) was stirred at 80 °C for 3h until complete consumption of 2-bromoacrylic acid, as evidenced by TLC analysis. The reaction mixture was filtered and concentrated. The obtained crude compound was purified by CC using 5% EtOAc in PE as eluent to afford benzyl 2-bromoacrylate (22 g, 53%) as a yellow liquid (TLC solvent system: 5% EtOAc in PE; R_{f}: 0.7).
**Step 7:** A suspension of 2-(4-((2-(benzyloxy)ethoxy)methyl)-3-fluorophenyl)-4,4,5,5-tetramethyl-1,3,2-dioxaborolane (16 g, 41.45 mmol), Cs₂CO₃ (40.5 g, 124.35 mmol) in DMF (150ml) was deoxygenated by purging argon for 30min at RT and Pd(dppf)Cl₂ (1.69 g, 2.072 mmol) was added and purging continued. After 10 min, benzyl 2-bromoacrylate (15.7 g, 62.17 mmol) was added and stirred at 100 °C for 1h until complete consumption of 2-(4-((2-(benzyloxy)ethoxy)methyl)-3-fluorophenyl)-4,4,5,5-tetramethyl-1,3,2-dioxaborolane, as evidenced by TLC analysis. The reaction mixture was diluted with ethyl acetate (300 mL), filtered through a Celite and washed with ethyl acetate (2x50 mL). The combined filtrate was washed with water (3x200 mL), brine (50 mL), dried over anhydrous NaSO₄, filtered and concentrated. The obtained crude compound was purified by CC using 10% ethyl acetate in PE as eluent to afford benzyl 2-(4-((2-(benzyloxy)ethoxy)methyl)-3-fluorophenyl)acrylate (2.8 g, 16%) as pale brown oil (TLC solvent system: 10% EtOAc in PE; R_{f}: 0.3).
**Step 8:** A suspension of benzyl 2-(4-((2-(benzyloxy)ethoxy)methyl)-3-fluorophenyl)acrylate (2.2 g, 5.23 mmol), 10% Pd(OH)₂ (300 mg) in EtOAc (20 mL) was hydrogenated (balloon pressure) at RT for 2h until complete consumption of benzyl 2-(4-((2-(benzyloxy)ethoxy)methyl)-3-fluorophenyl)acrylate, as evidenced by TLC analysis. The reaction mixture was filtered through Celite, washed with MeOH (2×15 mL). The combined filtrate was concentrated and the obtained crude compound was purified by dissolving in EtOAc (30 mL) and shaken with aq 10% NaHCO₃ solution (12 mL). The EtOAc layer was separated; the aq layer was acidified with aq. citric acid solution (pH∼5) and extracted with EtOAc (2x 30 mL). The combined EtOAc layer was washed with water (10 mL), brine (10 mL) dried over anhydrous NaSO₄, filtered and concentrated to afford 2-(3-fluoro-4-((2-hydroxyethoxy)methyl)phenyl)propanoic acid (450 mg, 36%) as colorless oil (TLC solvent system: 100% EtOAc; R_{f}: 0.15).
**Step 9:** To a stirred DCM (5.0 mL) solution of 2-(3-fluoro-4-((2-hydroxyethoxy)methyl)-phenyl)propanoic (116.0 mg, 0.481 mmol, 1.0 eq) DIPEA (0.34 mL, 1.924 mmol, 4.0 eq), EDC.HCl (76.5 mg, 0.4 mmol, 1.2 eq) and HOBt (61.0 mg, 0.4 mmol, 1.2 eq) were sequentially added at RT and stirred for 15 min. (1-(3-chlorophenyl)-3-(trifluoromethyl)-1H-pyrazol-5-yl)methanamine (150 mg, 0.481 mmol, 1 eq) was then added and the RM stirred for 3h. After completion of the reaction the RM was washed with H₂O (20 mL), brine (10 mL), separated, dried (Na₂SO₄) and evaporated. The crude product was purified CC using EtOAc/PE (55:45) to get N-((1-(3-chlorophenyl)-3-(trifluoromethyl)-1H-pyrazol-5-yl)methyl)-2-(3-fluoro-4-((2-hydroxyethoxy)methyl)phenyl)propanamide (85 mg; 35%, pale yellow semi solid; TLC system: EtOAc/PE (4:1) R_{f}: 0.3).

### Synthesis of example A124: N-((3-tert-butyl-1-(4-fluorophenyl)-1 H-pyrazol-5-yl)methyl)-2-(3-fluoro-4-((2-hydroxyethoxy)methyl)phenyl)propanamide

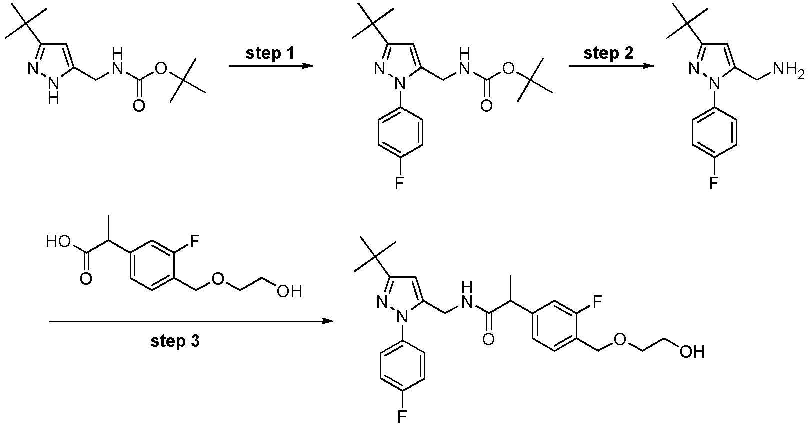

**Step 1:** To a mixture of tert-butyl (3-tert-butyl-1H-pyrazol-5-yl)methylcarbamate (501 mg, 1.98 mmol, 1 equiv.), 4-fluorophenylboronic acid (554 mg, 3.96 mmol, 2 equiv.) and copper acetate (541 mg, 2.97 mmol, 1.5 equiv.) in dichloromethane (30 mL) was added pyridine (315 mg, 0.315 mL, 3.96 mmol, 2 equiv) and the mixture was stirred in the presence of air for 2 d at room temperature. The reaction mixture was filtered over silica gel, the filter cake was washed with dichloromethane (250 mL) and the solvent of the filtrate was evaporated to give tert-butyl (3-tert-butyl-1-(4-fluorophenyl)-1H-pyrazol-5-yl)methylcarbamate (232 mg, 34 %).
**Step 2:** In 3 mL of dichloromethane, tert-butyl (3-tert-butyl-1-(4-fluorophenyl)-1H-pyrazol-5-yl)methylcarbamate (232 mg, 0.668 mmol, 1 equiv.) was dissolved and trifluoroacetic acid (0.496 mg, 0.331 ml, 6.5 equiv.) was added. The reaction mixture was stirred overnight at room temperature, extracted with aqueous sodium carbonate (c = 1 mol/L), dried over magnesium sulfate and evaporated to give (3-tert-butyl-1-(4-fluorophenyl)-1H-pyrazol-5-yl)methanamine (127 mg) which was used without further purification.
**Step 3:** To a stirred solution of (3-tert-butyl-1-(4-fluorophenyl)-1H-pyrazol-5-yl)methanamine (60 mg, 0.25 mmol, 1.0 eq) in THF/DMF (1/20, v/v, 2 mL) was added 2-(3-fluoro-4-((2-hydroxyethoxy)methyl)phenyl)propanoic acid (61 mg, 0.25 mmol, 1 equiv.), HOBt (35 mg. 0.25 mmol, 1 equiv.), TBTU (80 mg, 0.25 mmo, 1 equiv.) and DIPEA (0.168 mL, 127 mg, 1.01 mmol, 4 equiv.) and the mixture was stirred for 3 d at room temperature. The reaction mixture was evaporated and the residue was purified by column chromatography (EtOAc/cyclohexane (1:2) as eluent) to give N-((3-tert-butyl-1-(4-fluorophenyl)-1H-pyrazol-5-yl)methyl)-2-(3-fluoro-4-((2-hydroxyethoxy)methyl)phenyl)propanamide (93 mg, 78 %).

### Synthesis of example A125: N-((3-tert-butyl-1-(3-(trifluoromethoxy)phenyl)-1H-pyrazol-5-yl)methyl)-2-(3-fluoro-4-((2-hydroxyethoxy)methyl)phenyl)propanamide

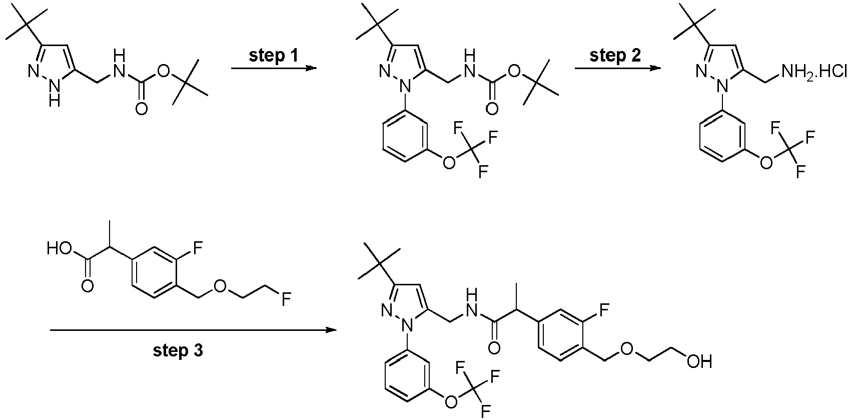

**Step** 1: To a mixture tert-butyl (3-tert-butyl-1H-pyrazol-5-yl)methylcarbamate (501 mg, 1.98 mmol, 1 equiv.), 3-(trifluoromethoxy)-phenylboronic acid (814 mg, 3.96 mmol, 2 equiv.) and copper acetate (541 mg, 2.97 mmol, 1.5 equiv.) in dichloromethane (30 mL) was added pyridine (315 mg, 0.315 mL, 3.96 mmol, 2 equiv.) and the mixture was stirred in the presence of air for 2 d at room temperature. The reaction mixture was filtered over silica gel, the filter cake was washed with dichloromethane (250 mL) and the solvent of the filtrate was evaporated to give tert-butyl (3-tert-butyl-1-(3-(trifluoromethoxy)phenyl)-1H-pyrazol-5-yl)methylcarbamate (294 mg, 36 %).
**Step 2:** In 4.7 mL of dioxane, tert-butyl (3-tert-butyl-1-(3-(trifluoromethoxy)phenyl)-1H-pyrazol-5-yl)methylcarbamate (294 mg, 0.711 mmol, 1 equiv.) was dissolved and hydrogen chloride in dioxane (1.16 mL, c = 4 mol/L, 4.62 mmo, 6.5 equiv.) was added. The reaction mixture was stirred overnight at room temperature: The suspension was evaporated and the product was precipitated in ether/pentane (7 mL, 1/2.5, v/v). The precipitate was filtered off, washed with n-pentane (2 x 5 mL) and dried to (3-tert-butyl-1-(3-(trifluoromethoxy)phenyl)-1H-pyrazol-5-yl)methanamine hydrochloride (119 mg, 48 %).
**Step 3:** To a stirred solution of (3-tert-butyl-1-(3-(trifluoromethoxy)phenyl)-1H-pyrazol-5-yl)methanamine hydrochloride (119 mg, 0.351 mmol, 1 eq) in THF/DMF (1/20, v/v, 3 mL) was added 2-(3-fluoro-4-((2-hydroxyethoxy)methyl)phenyl)propanoic acid (84 mg, 0.35 mmol, 1 equiv.), HOBt (49 mg. 0.35 mmol, 1 equiv.), TBTU (112 mg, 0.351 mmo, 1 equiv.) and DIPEA (0.234 mL, 178 mg, 1.40 mmol, 4 equiv.) and the mixture was stirred for at room temperature overnight. The reaction mixture was evaporated and the residue was purified by column chromatography (EtOAc/cyclohexane (1:2) as eluent) to give N-((3-tert-butyl-1-(3-(trifluoromethoxy)phenyl)-1H-pyrazol-5-yl)methyl)-2-(3-fluoro-4-((2-hydroxyethoxy)methyl)phenyl)propanamide (137 mg, 73 %).

### Synthesis of example A126: N-((1-(3,5-difluorophenyl)-3-(trifluoromethyl)-1H-pyrazol-5-yl)methyl)-2-(3-fluoro-4-((2-hydroxyethoxy)methyl)phenyl)propanamide

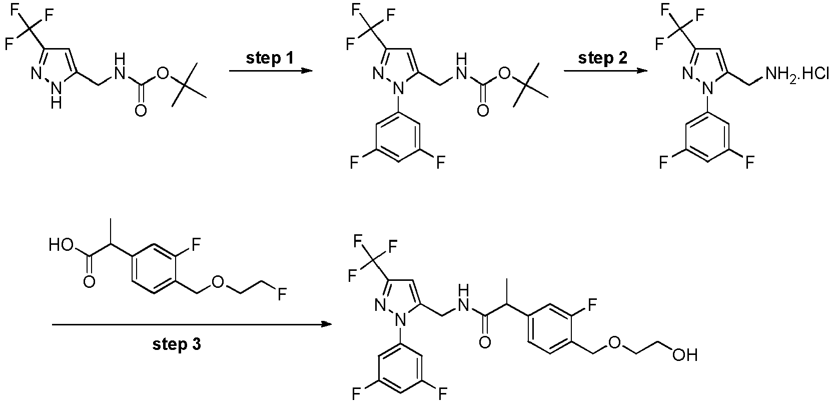

**Step 1:** To a mixture tert-butyl (3-(trifluoromethyl)-1H-pyrazol-5-yl)methylcarbamate (251 mg, 0.946 mmol, 1 equiv.), 3,5-difluorophenylboronic acid (299 mg, 1.89 mmol, 2 equiv.) and copper acetate (258 mg, 1.49 mmol, 1.5 equiv.) in dichloromethane (14 mL) was added pyridine (151 mg, 0.151 mL, 1.89 mmol, 2 equiv) and the mixture was stirred in the presence of air for 2 d at room temperature. The reaction mixture was filtered over silica gel, the filter cake was washed with dichloromethane (250 mL) and the solvent of the filtrate was evaporated to give tert-butyl (1-(3,5-difluorophenyl)-3-(trifluoromethyl)-1H-pyrazol-5-yl)methylcarbamate (214 mg, 60 %).
**Step 2:** In 4 mL of dioxane, tert-butyl (1-(3,5-difluorophenyl)-3-(trifluoromethyl)-1H-pyrazol-5-yl)methylcarbamate(214 mg, 0.567 mmol, 1 equiv.) was dissolved and hydrogen chloride in dioxane (0.923 mL, c = 4 mol/L, 3.69 mmo, 6.5 equiv.) was added. The reaction mixture was stirred overnight at room temperature, the precipitate was filtered off, washed with dioxane (2 x 15 mL) and dried to give (1-(3,5-difluorophenyl)-3-(trifluoromethyl)-1H-pyrazol-5-yl)methanamine hydrochloride (139 mg, 78 %).
**Step 3:** To a stirred solution of (1-(3,5-difluorophenyl)-3-(trifluoromethyl)-1H-pyrazol-5-yl)methanamine hydrochloride (16 mg, 0.050 mmol, 1.0 eq) in THF/DMF (1/20, v/v, 0.5 mL) was added 2-(3-fluoro-4-((2-hydroxyethoxy)methyl)phenyl)propanoic acid (11 mg, 0.050 mmol, 1 equiv.), HOBt (7 mg. 0.05 mmol, 1 equiv.), TBTU (16 mg, 0.050 mmo, 1 equiv.) and DIPEA (0.033 mL, 25 mg, 0.20 mmol, 4 equiv.) and the mixture was stirred for at room temperature overnight. The solvent of the reaction mixture was evaporated and the residue was purified by column chromatography (EtOAc/cyclohexane (1:1) as eluent) to give N-((1-(3,5-difluorophenyl)-3-(trifluoromethyl)-1H-pyrazol-5-yl)methyl)-2-(3-fluoro-4-((2-hydroxyethoxy)methyl)phenyl)propanamide (23 mg, 92 %).

### Synthesis of example A127: 1-[[2-(3-Chlorophenyl)-5-(trifluoromethyl)-2H-pyrazol-3-yl]-methyl]-3-[3-fluoro-4-(2-hydroxy-ethoxy-methyl)-phenyl]-urea

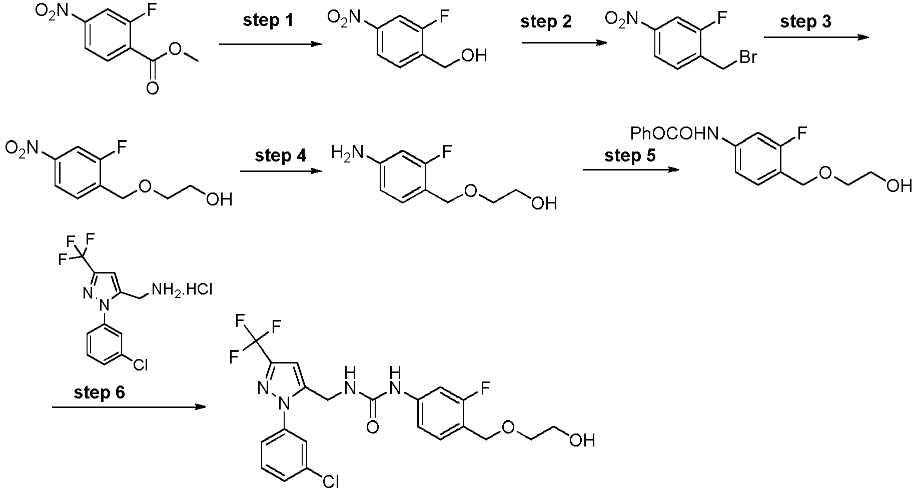

**Step 1: as described for example A59, step 1**
**Step 2:** To a cooled (0 °C) and stirred solution of DCM (100 mL) containing (2-fluoro-4-nitrophenyl)methanol (9 g, 52.6 mmol, 1 eq), triphenylphosphine (16.5 g, 63.1 mmol, 1.2 eq) was added followed by addition of NBS (11.24 g, 63.1 mmol, 1.2 eq), the mixture was allowed to warm to RT and stirred for 2 h. The DCM was evaporated under reduced pressure and the residue was purified by CC using PE/EtOAc (9:1) as eluent to get 1-(bromomethyl)-2-fluoro-4-nitrobenzene (10.50 g, 85%; TLC system: PE/EtOAc (7:3), R_{f}: 0.6).
**Step 3:** To a cooled (0 °C) suspension of 60% NaH (1.39 g, 57.8 mmol, 1.5 eq) in ethylene glycol (90 mL) 1-(bromomethyl)-2-fluoro-4-nitrobenzene (9.0 g, 38.6 mmol, 1 eq) was added and the mixture was stirred at RT for 16 h. The reaction mixture was diluted with water (100 mL) and extracted with EtOAc (30 mL X 2). The ethyl acetate layers were collected and dried (Na₂SO₄) and evaporated under vacuum. The residue was purified by CC using PE/EtOAc (7:3) as eluent to get 2-(2-fluoro-4-nitrobenzyloxy)ethanol (5 g, 67%, oil; TLC system: PE/ EtOAc (3:2), R_{f}: 0.3).
**Step 4:** To a stirred THF (50 mL) solution of 2-(2-fluoro-4-nitrobenzyloxy)ethanol (5.0 g, 1.0 eq) 10% Pd/C was added and reaction mixture stirred under H₂ gas balloon at for 16 h. The reaction mixture was passed through celite pad and the solvent evaporated. The residue was purified by CC using PE/EtOAc (3:2) as eluent to get 2-(4-amino-2-fluorobenzyloxy)ethanol (3.0g, 60%, solid; TLC system: EtOAc/PE (3:2), R_{f}: 0.3.).
**Step 5:** To a stirred solution of 2-(4-amino-2-fluorobenzyloxy)ethanol (2.5 g, 13.5 mmol, 1eq) in acetone (25 mL) pyridine (3.26 mL,40.5 mmol, 3 eq) was added followed by phenyl chloroformate (1.7 mL, 13.5 mmol, 1 eq) at 0 °C and the mixture was stirred at RT for 1 h. The solvent was evaporated and residue obtained was purified by CC using ethyl acetate/PE (7:13) as eluent to get 2-(4-(benzoyloxyamino)-2-fluorobenzyloxy)ethanol (2.8 g, 70%, white solid; TLC system: EtOAc/PE (1:1), R_{f}: 0.4).
**Step 6:** To a stirred solution of (1-(3-chlorophenyl)-3-(trifluoromethyl)-1H-pyrazol-5-yl)methanamine hydrochloride (102 mg, 0.327 mmol, 1.0 eq) in DCM (2.0 mL) was added Et₃N (0.09 mL, 0.654 mmol, 2.0 eq) followed by 2-(4-(benzoyloxyamino)-2-fluorobenzyloxy)ethanol (100 mg, 0.327 mmol, 1.0 eq) at RT and stirred for 16 h. The reaction mixture washed with water (2 mL) and the solvent evaporated to get 1-((1-(3-chlorophenyl)-3-(trifluoromethyl)-1H-pyrazol-5-yl)methyl)-3-(3-fluoro-4-((2-hydroxyethoxy)-methyl)phenyl)urea (80 mg; 50%, off-white solid; TLC system: EtOAc/ PE (3:2), R_{f}: 0.2).

### Synthesis of example A128: 1-[[2-(3-Chlorophenyl)-5-(trifluoromethyl)-2H-pyrazol-3-yl]-methyl]-3-[3-fluoro-4-(2-methoxy-ethoxy-methyl)-phenyl]-urea

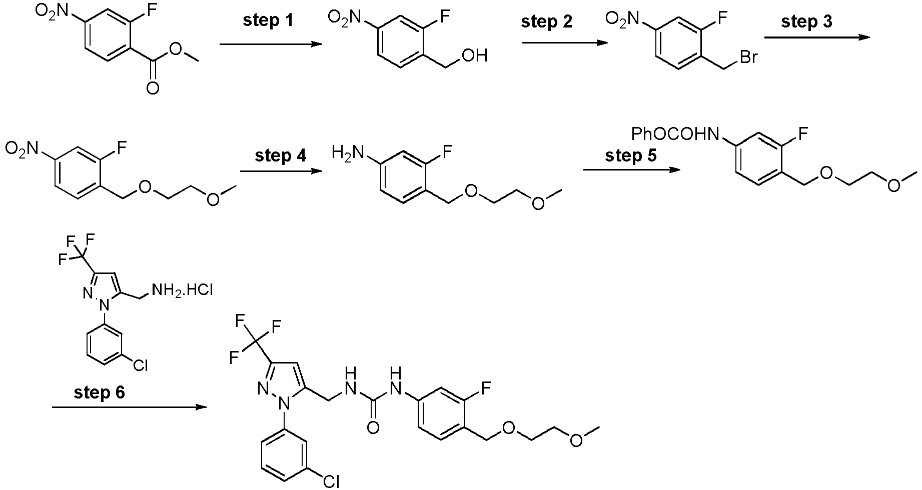

**Step 1:** as described for **example A59, step 1**
**Step 2:** as described for **example A127, step 2**
**Step 3:** To a suspension of K₂CO₃ (8.8g, 64.0 mmol, 1.5 eq) in methoxyethanol (100 mL) was added 1-(bromomethyl)-2-fluoro-4-nitrobenzene (10.0 g, 42.0 mmol, 1 eq) and the mixture was stirred at RT for 5 h. The reaction mixture was diluted with water (100 mL) and extracted with EtOAc (30 mL x 2). The ethyl acetate layers were collected and dried (Na₂SO₄) and evaporated under vacuum. The residue was purified by CC using PE/EtOAc (17:3) as eluant to get 2-fluoro-1-((2-methoxyethoxy)methyl)-4-nitrobenzene(4 g, 40%) as oil (TLC system: PE/ EtOAc (7:3), R_{f}: 0.5).
**Step 4:** To a stirred solution of 2-fluoro-1-((2-methoxyethoxy)methyl)-4-nitrobenzene (4.0 g, 1.0 eq) in THF (50 mL) was added 10% Pd-C and the reaction mixture was stirred under H₂ gas balloon at for 16 h. The reaction mixture was passed through celite pad and the solvent evaporated to get 3-fluoro-4-((2-methoxyethoxy)methyl)aniline (2.50 g, 72 %) as solid (TLC system: EtOAc/PE (1:1), R_{f}: 0.3).
**Step 5:** To a stirred solution of 3-fluoro-4-((2-methoxyethoxy)methyl)aniline (2.5 g, 13.0 mmol, 1eq) in acetone (25 mL) was added pyridine (3.15 mL, 39.0 mmol, 3 eq) followed by phenyl chloroformate (1.64 mL, 13.0 mmol, 1 eq) at 0 °C and the mixture was stirred at RT for 1 h. The solvent was evaporated and residue obtained was purified by CC using ethyl acetate/PE (3:7) as eluent to get O-benzoyl-N-(3-fluoro-4-((2-methoxyethoxy)methyl)phenyl)hydroxylamine as white solid (TLC system: EtOAc/PE (1:1), R_{f}: 0.4).
**Step 6:** To a stirred solution of (1-(3-chlorophenyl)-3-(trifluoromethyl)-1H-pyrazol-5-yl)methanamine hydrochloride (80 mg, 0.256 mmol, 1.0 eq) in DCM (2.0 mL) was added TEA (0.07 mL, 0.512 mmol, 2.0 eq) followed by O-benzoyl-N-(3-fluoro-4-((2-methoxyethoxy)methyl)phenyl)hydroxylamine (80.1 mg, 0.256 mmol, 1.0 eq) at RT and the mixture was stirred for 16h. The separated solid was filtered, washed with DCM (2 mL) to get 1-((1-(3-chlorophenyl)-3-(trifluoromethyl)-1H-pyrazol-5-yl)methyl)-3-(3-fluoro-4-((2-methoxy-ethoxy)methyl)phenyl)urea (70 mg; 55%, white solid) (TLC system: EtOAc/ PE (3:2); R_{f}: 0.2)

### Synthesis of example A129: 1-[[5-tert-Butyl-2-(3-chlorophenyl)-2H-pyrazol-3-yl]-methyl]-3-[3-fluoro-4-(2-hydroxy-ethoxy-methyl)-phenyl]-urea

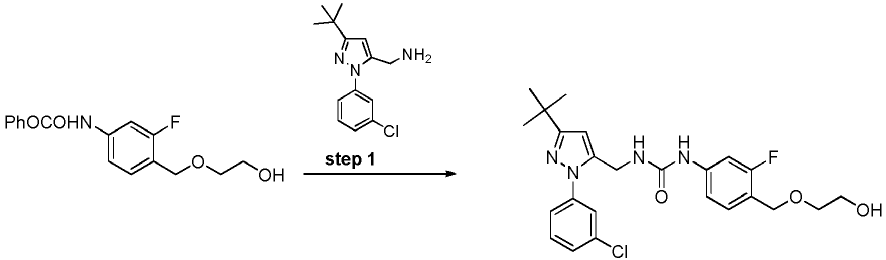

**Step 1:** To a stirred solution of ((3-tert-butyl-1-(3-chlorophenyl)-1H-pyrazol-5-yl)methanamine (108 mg, 0.409 mmol, 1.0 eq) in MeCN (7 mL) was added TEA (0.266 mL, 0.1:64 mmol, 4.0 eq) followed by 2-(4-(benzoyloxyamino)-2-fluorobenzyloxy)ethanol (133 mg, 0.438 mmol, 1.07 eq) at reflux and stirred for 16h. The solvent was evaporated and the residue was purified by CC (EtOAc/hexane 2:1) to get 1-((3-tert-butyl-1-(3-chlorophenyl)-1H-pyrazol-5-yl)methyl)-3-(3-fluoro-4-((2-hydroxyethoxy)methyl)phenyl)urea (96 mg; 49%).

### Synthesis of example A133: 1-[[2-(2,3-Dichloro-phenyl)-5-(trifluoromethyl)-2H-pyrazol-3-yl]-methyl]-3-[3-fluoro-4-(2-hydroxy-ethoxy-methyl)-phenyl]-urea

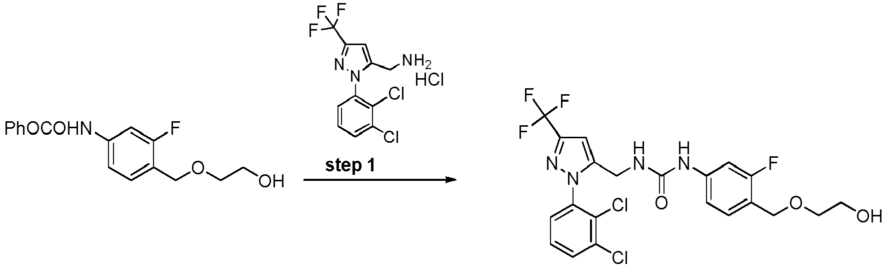

**Step 1:** To a stirred solution (1-(2,3-dichlorophenyl)-3-(trifluoromethyl)-1H-pyrazol-5-yl)methanamine hydrochloride (101 mg, 0.291mmol, 1.0 eq) in MeCN (7 mL) was added TEA (0.266 mL, 0.164 mmol, 4.0 eq) followed by 2-(4-(benzoyloxyamino)-2-fluorobenzyloxy)ethanol (90 mg, 0.297 mmol, 1.07 eq) at reflux and the mixture was stirred for 16 h. The solvent was evaporated and the residue was purified by CC (EtOAc/cyclohexane 2:1) to get 1-((1-(2,3-dichlorophenyl)-3-(trifluoromethyl)-1H-pyrazol-5-yl)methyl)-3-(3-fluoro-4-((2-hydroxyethoxy)methyl)phenyl)urea (101 mg; 67%).

### Synthesis of example A134: 1-((3-tert-butyl-1-(4-fluorophenyl)-1H-pyrazol-5-yl)methyl)-3-(3-fluoro-4-((2-hydroxyethoxy)methyl)phenyl)urea

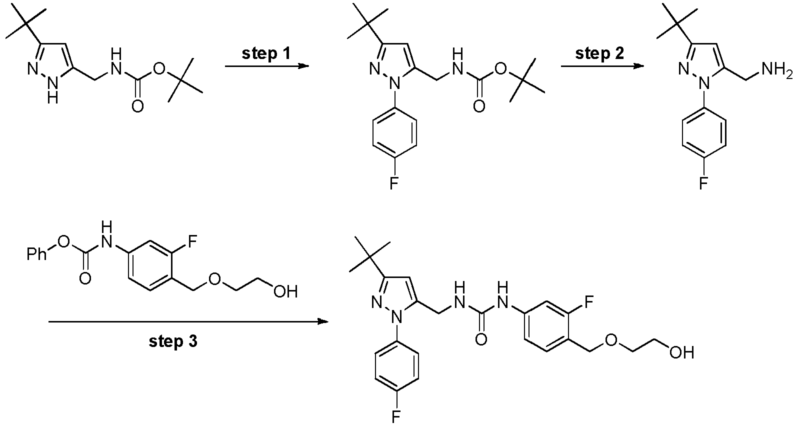

**Step 1:**To a mixture of tert-butyl (3-tert-butyl-1H-pyrazol-5-yl)methylcarbamate (501 mg, 1.98 mmol, 1 equiv.), 4-fluorophenylboronic acid (554 mg, 3.96 mmol, 2 equiv.) and copper acetate (541 mg, 2.97 mmol, 1.5 equiv.) in dichloromethane (30 mL) was added pyridine (315 mg, 0.315 mL, 3.96 mmol, 2 equiv) and the mixture was stirred in the presence of air for 2 d at room temprerature. The reaction mixture was filtered over silica gel, the filter cake was washed with dichloromethane (250 mL) and the solvent of the filtrate was evaporated to give tert-butyl (3-tert-butyl-1-(4-fluorophenyl)-1H-pyrazol-5-yl)methylcarbamate (232 mg, 34 %).
**Step 2:** In 3 mL of dichloromethane, tert-butyl (3-tert-butyl-1-(4-fluorophenyl)-1H-pyrazol-5-yl)methylcarbamate (232 mg, 0.668 mmol, 1 equiv.) was dissolved and trifluoroacetic acid (0.496 mg, 0.331 mml, 6.5 equiv.) was added. The reaction mixture was stirred overnight at room temperature, extracted with aqueous sodium carbonate (c = 1 mol/L), dried over magnesium sulfate and evaporated to give (3-tert-butyl-1-(4-fluorophenyl)-1H-pyrazol-5-yl)methanamine (127 mg) which was used without further purification.
**Step 3:** To a stirred solution of (3-tert-butyl-1-(4-fluorophenyl)-1H-pyrazol-5-yl)methanamine(60 mg, 0.247 mmol, 1.0 eq) in acetonitrile (6 mL) was added TEA (0.136 mL, 99 mg, 0.986 mmol, 4.0 eq) followed by phenyl 3-fluoro-4-((2-hydroxyethoxy)methyl)-phenylcarbamate (76 mg, 0.252 mmol, 1.02 eq.) and stirred at reflux for 16 h. The solvent of the reaction mixture was evaporated and the residue was purified by column chromatography (EtOAc/cyclohexane (1:2)) to get 1-((3-tert-butyl-1-(4-fluorophenyl)-1H-pyrazol-5-yl)methyl)-3-(3-fluoro-4-((2-hydroxyethoxy)methyl)phenyl)urea (83 mg, 73 %).

### Synthesis of example A135: 1-((1-(3-ethoxy-5-methylphenyl)-3-(trifluoromethyl)-1H-pyrazol-5-yl)methyl)-3-(3-fluoro-4-((2-hydroxyethoxy)methyl)phenyl)urea

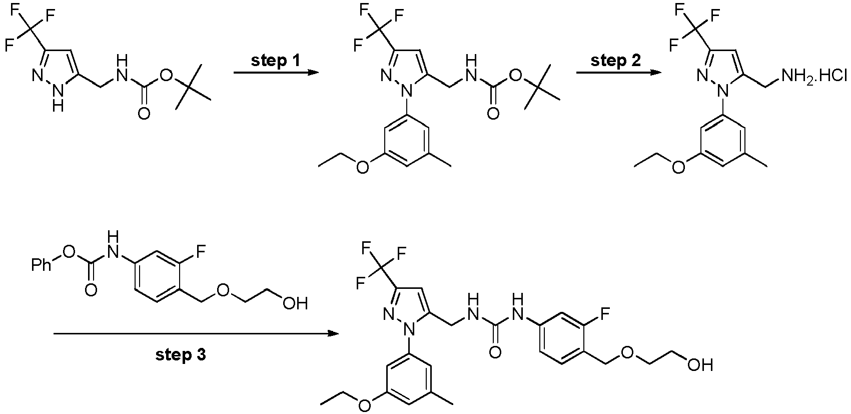

**Step 1:To** a mixture of tert-butyl (3-(trifluoromethyl)-1H-pyrazol-5-yl)methylcarbamate (251 mg, 0.947 mmol, 1 equiv.), 3-ethoxy-5-methylphenylboronic acid (341 mg, 1.89 mmol, 2 equiv.) and copper acetate (259 mg, 1.42 mmol, 1.5 equiv.) in dichloromethane was added pyridine (151 mg, 0.151 mL, 1.89 mmol, 2 equiv) and the mixture was stirred for 2 d at room temperature. The reaction mixture was filtered over silica gel, the filter cake was washed with 250 mL of dichloromethane and the filtrate was evaporated to give tert-butyl (1-(3-ethoxy-5-methylphenyl)-3-(trifluoromethyl)-1H-pyrazol-5-yl)methylcarbamate (354 mg, 94 %).
**Step 2:** In 6 mL of dioxane, tert-butyl (1-(3-ethoxy-5-methylphenyl)-3-(trifluoromethyl)-1H-pyrazol-5-yl)methylcarbamate (354 mg, 0.886 mmol) was dissolved and hydrogen chloride in dioxane (1.44 mL, c = 4 mol/L, 5.76 mml, 6.5 equiv.) was added. The reaction mixture was stirred overnight and filtered, the filtercake was washed with dioxane (2 x 15 mL) and dried to give (1-(3-ethoxy-5-methylphenyl)-3-(trifluoromethyl)-1H-pyrazol-5-yl)methanamine hydrochloride (211 mg, 71 %).
**Step 3:** To a stirred solution of (1-(3-ethoxy-5-methylphenyl)-3-(trifluoromethyl)-1H-pyrazol-5-yl)methanamine hydrochloride(100 mg, 0.300 mmol, 1.0 eq) in acetonitrile (7 mL) was added TEA (0.166 mL, 1.20 mmol, 4.0 eq) followed by phenyl 3-fluoro-4-((2-hydroxyethoxy)methyl)-phenylcarbamate(93 mg, 0.306 mmol, 1.02 eq) and stirred at reflux for 16 h. The solvent of the reaction mixture was evaporated and the residue was purified by column chromatography (EtOAc/cyclohexane (1:1)) to get 1-((1-(3-ethoxy-5-methylphenyl)-3-(trifluoromethyl)-1H-pyrazol-5-yl)methyl)-3-(3-fluoro-4-((2-hydroxyethoxy)methyl)phenyl)urea (60 mg, 39%).

### Synthesis of example A140: 1-[[5-tert-Butyl-2-(3-chlorophenyl)-2H-pyrazol-3-yl]-methyl]-3-[3-fluoro-4-(2-hydroxy-ethyl)-phenyl]-urea

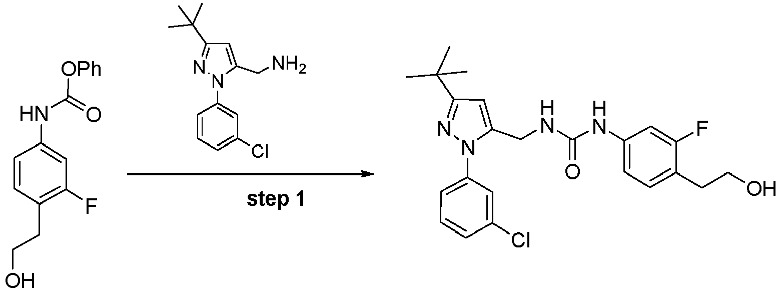

**Step 1:** To a stirred solution of (3-tert-butyl-1-(3-chlorophenyl)-1H-pyrazol-5-yl)methanamine (synthesis described for **example A141)** (102 mg, 0.387mmol, 1.0 eq) in MeCN (9 mL) was added TEA (0.214 mL, 1.55 mmol, 4.0 eq) followed by phenyl 3-fluoro-4-(2-hydroxyethyl)phenylcarbamate (108 mg, 0.395mmol, 1.02 eq) and the mixture was stirred for 16 h at reflux. The reaction mixture was concentrated under vacuum and the residue purified by CC using EtOAc/hexane (2:1) as eluent to get 1-((3-tert-butyl-1-(3-chlorophenyl)-1H-pyrazol-5-yl)methyl)-3-(3-fluoro-4-(2-hydroxyethyl)phenyl)urea (159 mg; 92%, white solid).

### Synthesis of example A138: N-[[2-(3-Chlorophenyl)-5-(trifluoromethyl)-2H-pyrazol-3-yl]-methyl]-2-[3-fluoro-4-(2-hydroxy-ethyl)-phenyl]-propionamide

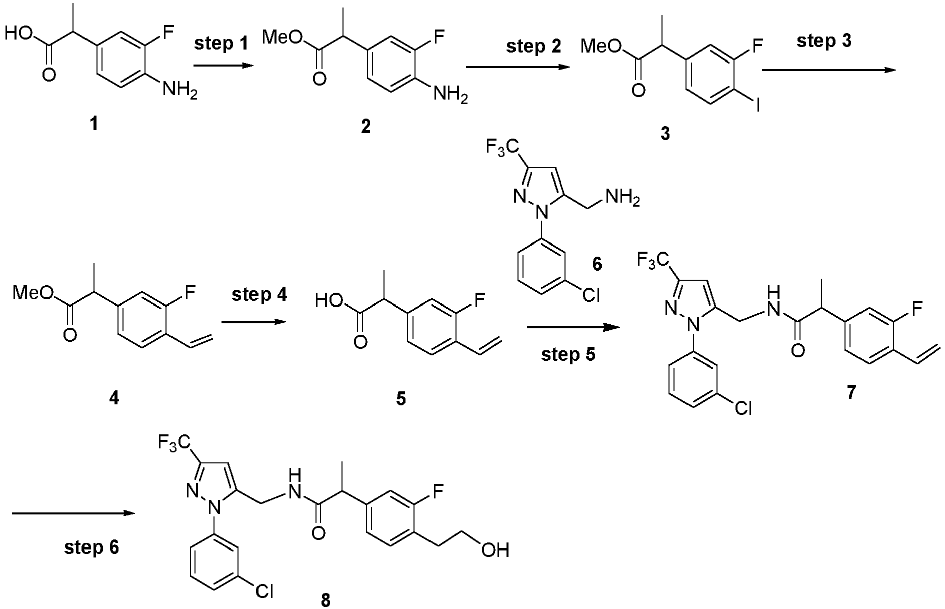

**Step 1:** To a stirred solution of 1 (2.5 g, 13.7 mmol) in 25 mL of methanol, under ice-cooling, was added thionyl chloride (1.5 mL, 20.5 mmol) dropwise over 10 minutes. After stirring the reaction mixture for 2 h, methanol is distilled out and 30 mL of water is added. The separated ester is extracted with ethyl acetate and washed with 15 mL of saturated sodium bicarbonate solution, and brine. Drying (Mg₂SO₄) and evaporation of the ethyl acetate and purification by CC yielded (2.23 g) as yellow oil by 83% yield.
**Step 2:** To a solution of p-TsOH·H₂O (6.45 g, 34 mmol) in MeCN (20 mL) was added **2** (11.3 mmol). The resulting suspension was cooled to 10-15°C and to this was added, gradually, a solution of NaNO₂ (1.56 g, 22.6 mmol) and KI (4.69 g, 28.3 mmol) in H₂O. The reaction mixture was stirred for 10 min then allowed to come to 20°C and stirred until the starting material was consumed. To the reaction mixture was then added H₂O (50 mL), NaHCO₃ (1 M; until pH = 9-10) and Na₂S₂O₃ (2M, 10 mL). The precipitated aromatic iodide as filtered off and the mixture extracted with EtOAc and purified by column chromatography to yield **3** (1.43 g) as yellow oil by 42% yield.
**Step 3:** A 100-mL round-bottom flask containing Pd(PPh₃)₄ (0.231 mmol), LiCI (5.56 mmol) and DMF (15.00 mL) was purged with nitrogen gas several times. To the flask were then added **3** (4.63 mmol) and tributyl(vinyl)tin (5.56 mmol). The reaction mixture was stirred at room temperature for 15 h. The reaction mixture was then treated with 10 mL of saturated CsF₂ solution and allowed to stir for 30 min at ambient temperature, filtered through Celite and silica gel, and diluted with 50 mL (3 times) of EtOAc. The organic layer was washed with water, dried (Mg₂SO₄) and concentrated in vacuo. The residue was purified by CC using EtOAc/n-hexane as solvent system to give the desired product **4** as yellow oil by 83% yield (800 mg).
**Step 4:** To a solution of **4** (800 mg, 3.84 mmol) in THF (5 mL) was added a 10 mL mixture solvents of THF and H₂O (1:1), and LiOH·H₂O (403 mg, 9.61 mmol). The reaction mixture was stirred at room temperature for 2 h. To the reaction mixture was then added H₂O (50 mL), the mixture was cooled, and acidified by diluted HCI to a pH of 1-2. The mixture is extracted with ethyl acetate. The organic layer was washed with water, dried (Mg₂SO₄) and concentrated in vacuo. **5** (748mg) was obtained as yellow oil by 99% yield. The product was carried on to next step without further purification
**Step 5:** A solution of the carboxylic acid **5** (748 mg, 3.85 mmol) in DCM was cooled in an icebath and EDC (1.05 equiv), HOBt (1.05 equiv), TEA (3 equiv), and **6** (1 equiv) were added consecutively. The reaction mixture was stirred overnight at room temperature. Water was added to the reaction mixture and it was extracted with DCM. The combined organic extracts were washed successively with saturated NaHCO₃ solution (30 mL), 0.5 N HCI (30 mL), and then water (30 mL), and dried over Mg₂SO₄. Evaporation of the solvent followed by CC purification afforded 7 (1.04 g) as off white solid by 60% yield.
**Step 6:** Compound **7** (259 mg, 0.59 mmol) in 2M BH₃.SMe₂ in THF (0.53 mL) was stirred for 1h at 0°C, then 1h at ambient temperature. A solution of 1N NaOH (1.6 mL) was added to the reaction mixture at 0°C, then a solution of 30% H₂O₂ (1.2 mL) was added. The mixture was stirred 30 min at 0°C, then 30 min at ambient temperature. Ethyl acetate (30mL) was added, the organic layer was separated, washed with water (30mL), saturated NaCO₃ (30mL), saturated NaCl (30mL) and dried over Na₂SO₄. After evaporation of the solvent in vacuo, the crude product was purified by CC to afford 8 (131 mg) as a white solid by 47% yield.
   ¹H NMR (300 MHz, CDCl₃): δ 7.37-7.45 (m, 3H), 7.29 (t, *J* = 2.01 Hz, 1H), 7.21 (d, *J* = 7.68 Hz, 1H), 6.91-6.95 (m, 2H), 6.41 (s, 1H), 5.57 (s, NH), 4.45-4.48 (dd, *J₁=* 5.67 Hz, *J₂*= 2.67 Hz, 2H), 3.50 (t, *J* = 7.32 Hz, 1H), 2.90 (t, *J* = 6.96 Hz, 1H), 1.84 (t, *J* = 6.78 Hz, 2H), 1.48 (d, *J* = 7.14 Hz, 3H).

### Synthesis of example A141: 1-[[2-(3-Chlorophenyl)-5-(trifluoromethyl)-2H-pyrazol-3-yl]-methyl]-3-[3-fluoro-4-(2-hydroxy-ethyl)-phenyl]-urea

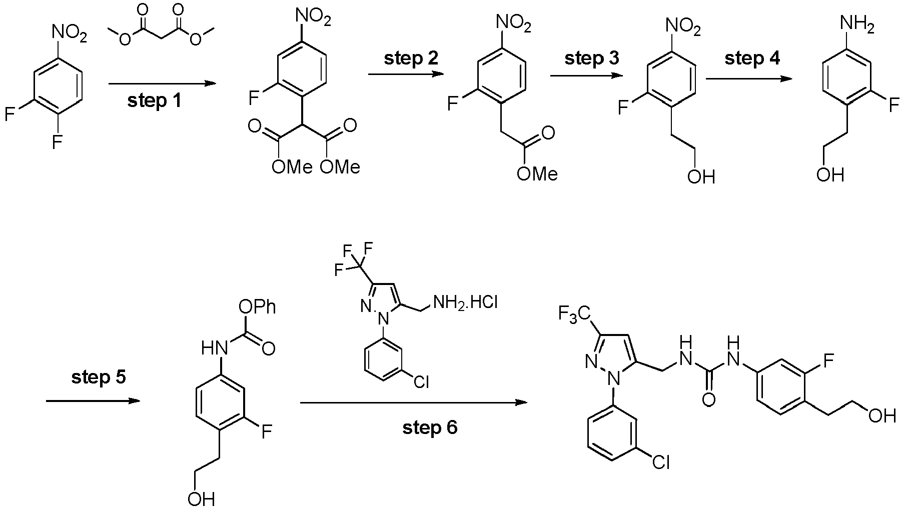

**Step 1:** To a stirred solution of dimethyl malonate (6.22g, 47.1 mmol, 1.5 eq) in DMF (30 mL) was added K₂CO₃ (8.70 g, 63 mmol, 2.0 eq) and heated to 50 °C for 30 min when 1,2-difluoro-4-nitrobenzene(5.0g, 31.4 mmol, 1.0 eq) was added and the reaction mixture was stirred for 5 h at 50 °C. The reaction mixture was cooled to RT, filtered to remove K₂CO₃, and the DMF was evaporated under vacuum. The residue was diluted with EtOAc (100 mL), washed with water, brine, dried over Na₂SO₄ and evaporated to get dimethyl 2-(2-fluoro-4-nitrophenyl)malonate (6.5 g, 76%) as a solid (TLC system: EtOAc/PE (3:7), R_{f}: 0.50).
**Step 2:** To a stirred solution of dimethyl 2-(2-fluoro-4-nitrophenyl)malonate (5.0 g, 18.5 mmol, 1.0 eq) in DMSO (30 mL) was added NaCl (1.07 g, 18.5 mmol, 1.0 eq) and water (0.5 mL) and stirred at 120 °C for 3 h. The reaction mixture was diluted with water (100 mL), extracted with EtOAc (50 mL x 2), washed with brine, dried over Na₂SO₄ and the solvent evaporated. The crude residue was purified by CC using PE/EtOAc (19:1) to get methyl 2-(2-fluoro-4-nitrophenyl)acetate (2.5g, 64%) as a viscous oil (TLC system: EtOAc/PE (1:4), R_{f}: 0.55).
**Step 3:** To a stirred solution of methyl 2-(2-fluoro-4-nitrophenyl)acetate (3.0g, 14.0 mmol, 1.0 eq) in methanol (30 mL) was added NaBH₄ (2.08g, 36.3mmol, 4.0eg) at 0°C and stirred at 70 °C for 16h, then the methanol was evaporated and the residue was diluted with water (30 mL), the pH adjusted to being neutral with 2N HCl and the mixture extracted with EtOAc (50 mL x 3). The organic layer was separated, washed with brine, dried over Na₂SO₄ and the solvent evaporated to yield 2-(2-fluoro-4-nitrophenyl)ethanol (2.0 g, 77%) as a viscous oil (TLC system: EtOAc/PE (3:7), R_{f}: 0.3).
**Step 4:** To a stirred solution of 2-(2-fluoro-4-nitrophenyl)ethanol (200 mg, 0.94 mmol, 1.0 eq) in ethanol (10 mL) was added 10% Pd/C (50 mg) and the mixture was stirred under a H₂ gas balloon at RT for 5 h. The reaction mixture was passed through a celite bed and the sovent of the filtrate evaporated to get 2-(4-amino-2-fluorophenyl)ethanol (130 mg, 75%) as a viscous oil (TLC system: EtOAc/PE (1:1), R_{f}: 0.2).
**Step 5:** To a stirred solution of 2-(4-amino-2-fluorophenyl)ethanol (1.3 g, 8.38 mmol, 1.0 eq) in acetone (50 mL) was added pyridine (1.32 g, 16.7 mmol, 2.0 eq) and phenyl chloroformate (1.45 g, 9.23 mmol, 1.1 eq) at 0 °C and the mixture was stirred at RT for 2h. The solvent was evaporated, the residue diluted with EtOAc (50 mL), washed with water (100 mL) followed by brine and the solvent evaporated. The residue obtained was purified by CC using EtOAc/PE (1:4) to get phenyl 3-fluoro-4-(2-hydroxyethyl)phenylcarbamate (1.5 g, 65%, white solid). (TLC system: EtOAc/PE (1:1), R_{f}: 0.3).
**Step 6:** To a stirred solution of(1-(3-chlorophenyl)-3-(trifluoromethyl)-1H-pyrazol-5-yl)methanamine hydrochloride (227 mg, 0.73 mmol, 1.0 eq) in THF (10 mL) was added TEA (147 mg, 1.45 mmol, 2.0 eq) followed by phenyl 3-fluoro-4-(2-hydroxyethyl)phenylcarbamate (200 mg, 0.73mmol, 1.0 eq) at RT and the mixture was stirred for 16 h. The reaction mixture was concentrated under vacuum and the residue purified by CC using neutral alumina and EtOAc/PE (3:2) as eluent), followed by preparative HPLC to get 1-((1-(3-chlorophenyl)-3-(trifluoromethyl)-1H-pyrazol-5-yl)methyl)-3-(3-fluoro-4-(2-hydroxyethyl)phenyl)urea (150 mg; 45%, white solid; TLC system: EtOAc/PE (3:2); R_{f}: 0.2).

### Synthesis of example A151: 1-[[2-(3-Chlorophenyl)-5-(trifluoromethyl)-2H-pyrazol-3-yl]-methyl]-3-[4-(1,2-dihydroxy-ethyl)-3-fluoro-phenyl]-urea

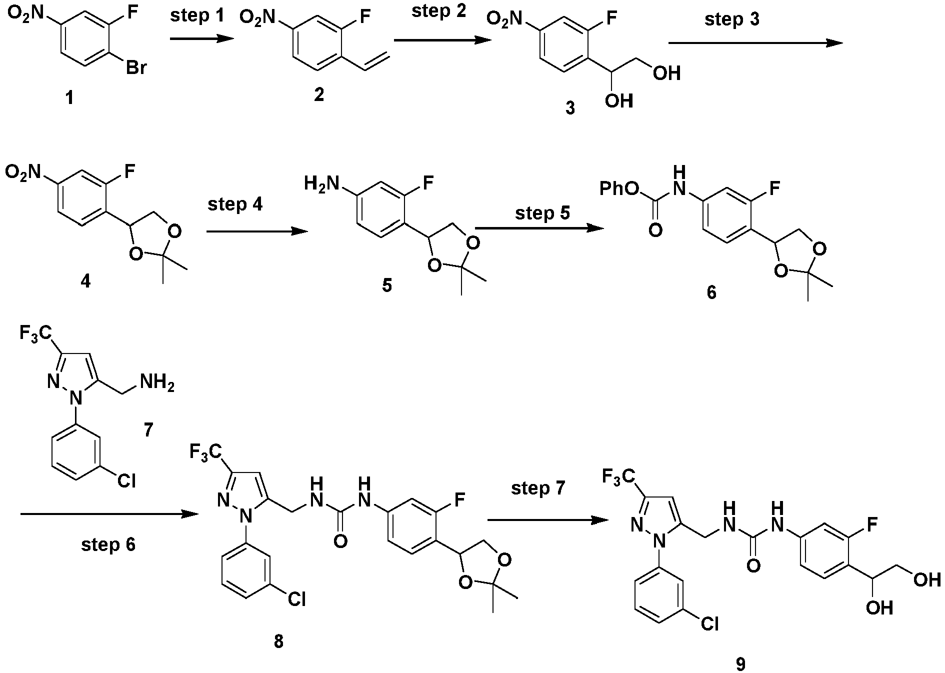

**Step 1:** To a stirred solution of **1** (1 g, 4.54 mmol) in anhydrous THF was added lithium chloride (962 mg, 22.7 mmol), Pd(PPh₃)₄ (524 mg, 0.454 mmol) and tributylvinyl tin (1.59 mL, 5.45 mmol) at room temperature. The reaction mixture was stirred overnight under reflux. The reaction mixture was cooled to room temperature until the reaction was finished. The reaction mixture was filtered on Celite bed, and then the solvent of the filtrate was reduced in vacco. The residue was extracted with EtOAc and washed with water and brine. The organic layer was dried over MgSO₄ and filtered. The solvent of the filtrate was removed under low pressure. The crude was purified by column chromatography to produce **2** (285 mg) with 38 % yield.
**Step 2:** To a stirred solution of **2** (285 mg, 1.70 mmol) in water and acetone as co-solvent was added 0.5 % OsO₄ in H₂O and 50 % NMO in H₂O. The reaction mixture was stirred overnight at room temperature. The reaction mixture was diluted with DCM and washed with water and brine. The organic layer was dried over MgSO₄ and filtered. The solvent of the filtrate was removed under low pressure. The crude was purified by column chromatography to afford **3** (262 mg, 76 %).
**Step 3:** To a stirred solution of 3 (262 mg, 1.30 mmol) in DCM was added p-TsOH·H₂O (247 mg, 1.30 mmol) and 2,2-dimethoxypropane (0.32 mL, 2.60 mmol). The reaction mixture was stirred for 2 h at room temperature. The mixture dissolved in DCM and washed with water and brine. The organic layer was dried over MgSO₄ and filtered. The solvent of the filtrate was removed under low pressure. The crude was purified by column chromatography to produce 4 (262 mg, 83 %).
**Step 4:** Starting material **4** (262 mg, 1.09 mmol) was dissolved in MeOH. Pd/C (26 mg) was added to it. The resulting mixture was stirred at room temperature for 2 h under H₂. TLC showed complete consumption of starting material. The mixture was filtered through celite bed and the filtrate was concentrated under reduced pressure to afford desired compound **5** (227 mg, 99 %).
**Step 5:** Compound **5** (227 mg, 1.07 mmol) was dissolved in MeCN. The reaction mixture was added to pyridine (0.09 mL, 1.18 mmol) and phenyl chloroformate (0.15 mL, 1.18 mmol) and stirred at room temperature for 3 h. TLC showed complete consumption of starting material. The reaction mixture was diluted with water and extracted with EtOAc. The organic part was washed with water and brine. The organic layer was dried over MgSO₄ and concentrated under reduced pressure. The crude was purified by column chromatography to give pure compound **6** (403 mg, 99 %).
**Step 6:** To a solution of compound **6** (150 mg, 0.45 mmol) in DMF was added DMAP (55 mg, 0.45 mmol) and amine **7** (125 mg, 0.45 mmol) at room temperature. The reaction mixture was heated to 50°C overnight (about 12 - 15 h). TLC showed complete consumption of starting material. The reaction mixture was diluted with water and extracted with EtOAc. The organic part was washed with water and brine. The organic layer was dried over MgSO₄ and concentrated under reduced pressure. The crude was purified by CC to produce pure compound **8** (213 mg, 92 %).
**Step 7:** To a stirred solution of **8** (78 mg, 0.20 mmol) in water was added conc. HCI (0.1 mL). The resulting reaction mixture was stirred at ambient temperature for 3 h. TLC showed complete consumption of starting material. The reaction mixture was diluted with water and extracted with EtOAc. The organic part was washed with water and brine. The organic layer was dried over MgSO₄ and concentrated under reduced pressure. The crude was purified by column chromatography to afford desired compound **9** (55 mg, 76 %).
   ¹H NMR (300 MHz, DMSO-d₆): δ 8.86(s, 1 H, Ar-NH), 7.76 (s, 1 H, Ar-H), 7.63 (m, 3 H, Ar-H), 7.36 ∼ 7.27 (m, 2 H, Ar-H), 6.99 (dd, 1 H, J₁ = 8.40 Hz, J₂ = 2.40 Hz, Ar-H), 6.80 (m, 2 H, Ar-H and ArCH₂-NH), 5.25 (d, 1 H, J = 4.20 Hz, Ar-CH), 4.74 (m, 2 H, R-CH₂-O), 4.41 (d, 2 H, J = 5.40 Hz, Ar-CH₂).

### Synthesis of example A152: 1-[[5-tert-Butyl-2-(3-chlorophenyl)-2H-pyrazol-3-yl]-methyl]-3-[3-fluoro-4-[2-hydroxy-1-(hydroxymethyl)-ethyl]-phenyl]-urea

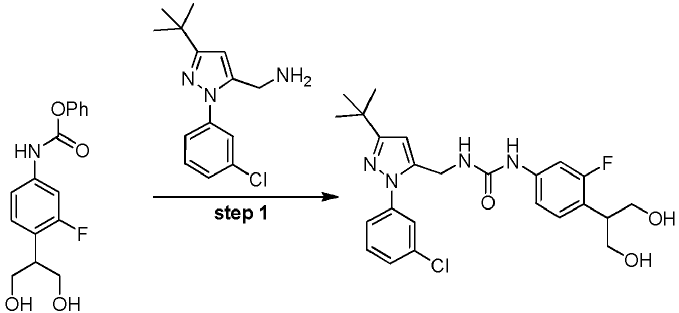

**Step 1:** To a stirred solution of (3-tert-butyl-1-(3-chlorophenyl)-1H-pyrazol-5-yl)methanamine (synthesis described for **example A153**) (102 mg, 0.387 mmol, 1.0 eq) in MeCN (9 mL) was added TEA (0.2145 mL, 1.55 mmol, 4.0 eq) followed by phenyl 4-(1,3-dihydroxypropan-2-yl)-3-fluorophenylcarbamate (120 mg, 0.395 mmol, 1.02 eq) at RT and the mixture was stirred at reflux for 16 h. The solvent was evaporated and the crude product was purified by CC using EtOAc as eluent to get 1-((3-tert-butyl-1-(3-chlorophenyl)-1H-pyrazol-5-yl)methyl)-3-(4-(1,3-dihydroxypropan-2-yl)-3-fluorophenyl)urea (162 mg; 88%).

### Synthesis of example A153: 1-[[2-(3-Chlorophenyl)-5-(trifluoromethyl)-2H-pyrazol-3-yl]-methyl]-3-[3-fluoro-4-[2-hydroxy-1-(hydroxymethyl)-ethyl]-phenyl]-urea

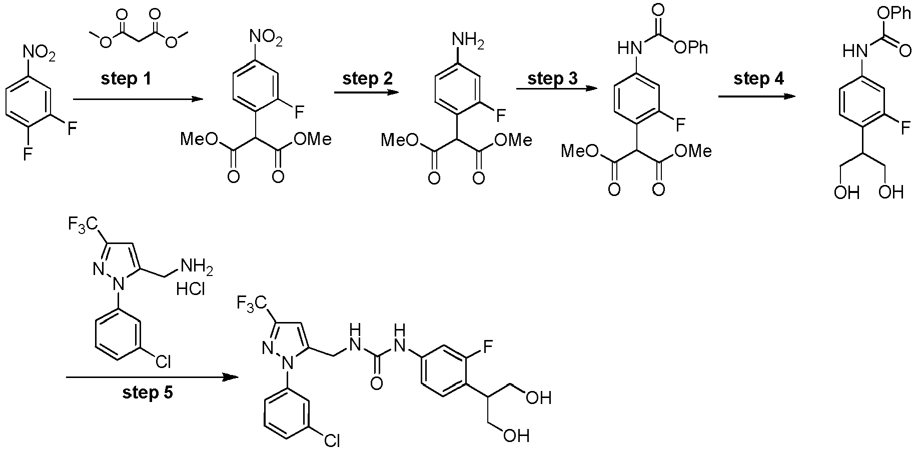

**Step 1:** as described for **example A141, step 1**
**Step 2:** To a stirred ethanol solution (40 mL) of dimethyl 2-(2-fluoro-4-nitrophenyl)malonate (4.0 g, 14.76 mmol, 1.0 eq) 10% Pd-C (400 mg) was added and the mixture was stirred under a H₂ gas balloon at RT for 2h, then the mixture was passed through a celite bed and the solvent of the filtrate was evaporated to get dimethyl 2-(4-amino-2-fluorophenyl)malonate (3.2g, 87%, solid; TLC system: EtOAc/PE (3:7), R_{f}: 0.3).
**Step 3:** To a stirred solution of dimethyl 2-(4-amino-2-fluorophenyl)malonate (3.0 g, 12.448 mmol, 1.0 eq) in acetone (30 mL) was added pyridine (3.0 mL, 37.344 mmol, 3.0 eq) and phenyl chloroformate (1.89 mL, 14.937 mmol, 1.2 eq) at 0 °C and the mixture was stirred at RT for 1h. The solvent was evaporated, the residue diluted with EtOAc (50 mL), washed with water (100 mL), brine (20 mL) and the solvent evaporated. The resulting residue was purified by CC using EtOAc/PE (1:4) as eluent to get dimethyl 2-(2-fluoro-4-(phenoxycarbonylamino)phenyl)malonate (3.5 g, 78%, white solid; TLC system: EtOAc/PE (3:7), R_{f}: 0.5).
**Step 4:** To a stirred solution of dimethyl 2-(2-fluoro-4-(phenoxycarbonylamino)-phenyl)malonate (3.0 g, 8.31 mmol, 1.0 eq) in ethanol (15 mL) and THF (15 mL) was added NaBH₄ (621 mg, 16.62 mmol, 2.0 eq) followed by lithium chloride (705 g, 16.62 mmol, 2.0 eq) at 0°C and the mixture was stirred at 0 °C for 5 h. The solvent was evaporated and the residue diluted with water (30 mL) and extracted with EtOAc (2 x 50 mL). The organic layer was separated, washed with brine, dried over Na₂SO₄ and evaporated. The residue was purified by CC using MeOH/DCM (1:19) as eluent to get phenyl 4-(1,3-dihydroxypropan-2-yl)-3-fluorophenylcarbamate (482 mg, 19%, white solid; TLC system: MeOH/DCM (1:9), R_{f}: 0.6)
**Step 5:** To a stirred solution of (1-(3-chlorophenyl)-3-(trifluoromethyl)-1H-pyrazol-5-yl)methanamine hydrochloride (153 mg, 0.4918 mmol, 1.0 eq) in DMF (5 mL) was added TEA (0.205 mL, 1.4754 mmol, 3.0 eq) followed by phenyl 4-(1,3-dihydroxypropan-2-yl)-3-fluorophenylcarbamate (150 mg, 0.4918 mmol, 1.0 eq) at RT and the mixture stirred for 16 h. The reaction mixture was diluted with water (10 mL) and extracted with ethyl acetate (15 mL). The organic layer was washed with water (10 mL) and brine (5 mL), dried over anhydrous Na₂SO₄, and evaporated under vacuum. The crude was purified by neutral alumina column chromatography using MeOH/CHCl₃ (1:19) as eluent to get 1-((1-(3-chlorophenyl)-3-(trifluoromethyl)-1H-pyrazol-5-yl)methyl)-3-(4-(1,3-dihydroxypropan-2-yl)-3-fluorophenyl)urea (85.5mg; 36%) as off white solid; TLC system: MeOH/CHCl₃ (1:9); R_{f}: 0.5).

### Synthesis of example A154: 1-[[2-(3-Chlorophenyl)-5-(trifluoromethyl)-2H-pyrazol-3-yl]-methyl]-3-[3-fluoro-4-[2-hydroxy-1-(hydroxymethyl)-1-methyl-ethyl]-phenyl]-urea

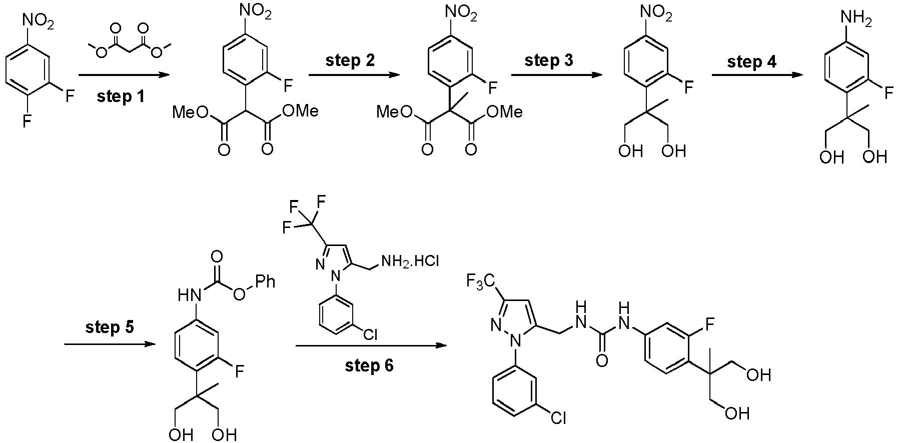

**Step 1:** as described for **example A141, step 1**
**Step 2:** To a stirred solution of dimethyl 2-(2-fluoro-4-nitrophenyl)malonate (200 mg, 0.73 mmol, 1.0 eq) and K₂CO₃ (204 mg 1.47 mmol 2.0 eq) in DMF (5 mL), was added methyl iodide (210 mg, 1.47 mmol 2.0 eq) at RT and the mixture was stirred at RT for 6 h. K₂CO₃ was filtered off and the filtrate concentrated under vacuum. The residue was diluted with water (50 mL) and extracted with EtOAc (20 mL). The organic layer was separated washed with brine solution, dried over anhydrous Na₂SO₄ and the solvent evaporated to get dimethyl 2-(2-fluoro-4-nitrophenyl)-2-methylmalonate (150 mg, 71%; TLC system: EtOAc/PE (1:9), R_{f}: 0.4).
**Step 3:** To a stirred solution of dimethyl 2-(2-fluoro-4-nitrophenyl)-2-methylmalonate (1.0g, 3.50 mmol, 1.0 eq) in methanol (20 mL) was added NaBH₄ (0.67gm, 17.63 mmol, 5.0 eq) at 0°C and stirred at RT for 16h. The mixture was diluted with water (20 mL), extracted with ethyl acetate (20 mL). The organic layer was washed with brine, dried over anhydrous Na₂SO₄, and the solvent was vaporated under reduced pressure. The crude obtained was purified by CC using EtOAc/PE (2:3) to get 2-(2-fluoro-4-nitrophenyl)-2-methylpropane-1,3-diol (400 mg, 50%; TLC system: EtOAc/PE (1:1), R_{f}: 0.3).
**Step 4:** To a stirred solution of 2-(2-fluoro-4-nitrophenyl)-2-methylpropane-1,3-diol (100mg, 0.34mmol, 1.0 eq) in ethanol was added 10% Pd/C (30 mg) and the mixture wasstirred under hydrogen gas balloon atmosphere for 3h at RT. The reaction mixture was filtered through celite pad and filtrate concentrated under vacuum to get 2-(4-amino-2-fluorophenyl)-2-methylpropane-1,3-diol (65mg, 76%; TLC system: EtOAc/PE (1:1), R_{f}: 0.4).
**Step 5:** To a stirred solution of 2-(4-amino-2-fluorophenyl)-2-methylpropane-1,3-diol (1.5 g, 7.53 mmol, 1.0 eq) in acetone (50 mL) was added pyridine (1.2 g, 15.18 mmol, 2.0 eq) and phenyl chloroformate (1.3 g, 8.28 mmol, 1.1 eq) at 0 °C and the mixture was stirred at RT for 1h. The acetone was evaporated, and the residue obtained was diluted with EtOAc (50 mL), washed with water (100 mL), brine (20 mL) and the solvent evaporated. The crude product was purified by silica CC using EtOAc/PE (3:2) as eluent to get phenyl 4-(1,3-dihydroxy-2-methylpropan-2-yl)-3-fluorophenylcarbamate (1.7g, 71%) as a white solid (TLC system: EtOAc/PE (7:3), R_{f}: 0.55).
**Step 6:** To a stirred solution of (1-(3-chlorophenyl)-3-(trifluoromethyl)-1H-pyrazol-5-yl)methanamine hydrochloride (195 mg, 0.62 mmol, 1.0 eq) in DMF (10 mL) was added TEA (190 mg, 1.24 mmol, 2.0 eq) followed by phenyl 4-(1,3-dihydroxy-2-methylpropan-2-yl)-3-fluorophenylcarbamate (200 mg, 0.62 mmol, 1.0 eq) at RT and the mixture was stirred for 16 h at 50°C. The reaction mixture was concentrated under vacuum and the residue purified by neutral alumina column chromatography using MeOH/CHCl₃ (0.5: 9.5) as eluent to get 1-((1-(3-chlorophenyl)-3-(trifluoromethyl)-1H-pyrazol-5-yl)methyl)-3-(4-(1,3-dihydroxy-2-methylpropan-2-yl)-3-fluorophenyl)urea (170 mg; 54%) as a white solid (TLC system: MeOH/CHCl₃ (1:9); R_{f}: 0.45).

### Synthesis of example A155: 1-[[2-(3-Chlorophenyl)-5-(trifluoromethyl)-2H-pyrazol-3-yl]-methyl]-3-[4-[2-hydroxy-1-(hydroxymethyl)-1-methyl-ethyl]-phenyl]-urea

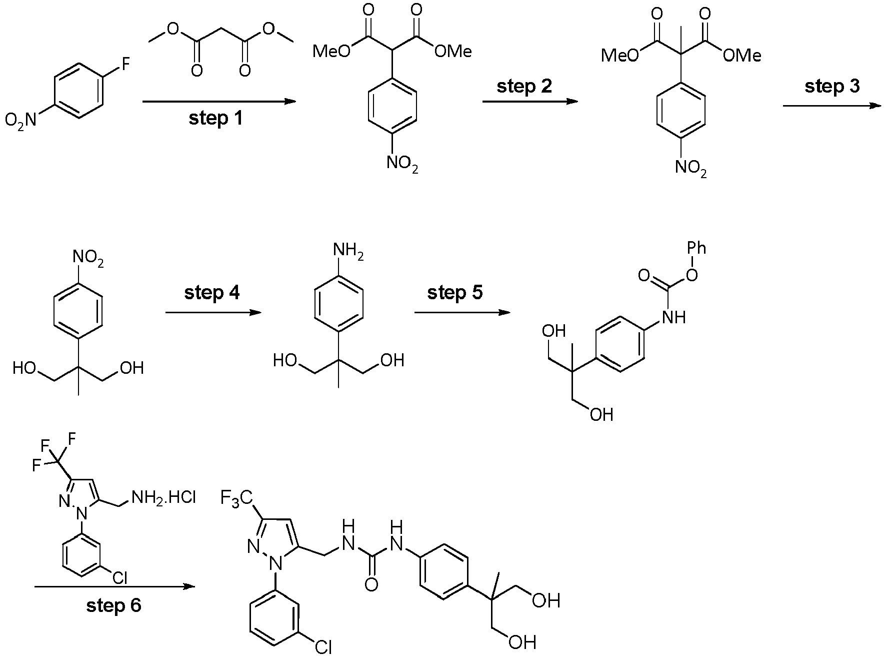

**Step 1:** To a suspension of dimethyl malonate (9.77 mL, 85.07 mmol, 1.2 eq) in DMF (100 mL) and K₂CO₃ (29.36 g, 212.76 mmol, 3.0 eq) was added 1-fluoro-4-nitrobenzene (7.51 mL, 70.91 mmol, 1.0 eq) at RT and the mixture was stirred for 16h at 70 °C. The reaction mixture was cooled to RT and diluted with water (200 mL) and extracted with ethyl acetate (2 x 100 mL). The ethyl acetate layers were collected, washed with cold water (2 x 50 mL), brine (50 mL), dried over sodium sulfate and evaporated under vacuum. The crude product was washed with n-pentane (50 mL) to get dimethyl 2-(4-nitrophenyl)malonate 14 g, 78%) as pale yellow solid (TLC system: EtOAc /PE (3:7), R_{f}: 0.50).
**Step 2:** To a stirred solution of dimethyl 2-(4-nitrophenyl)malonate (10 g, 39.52 mmol, 1.0 eq) and K₂CO₃ (10.9 g 79.05 mmol 2.0 eq) in DMF (40 mL), was added methyl iodide (4.94 mL, 79.05 mmol 2.0 eq) at 0 °C and the mixture stirred at RT for 18h. K₂CO₃ was filtered off and the filtrate concentrated under vacuum. The residue was diluted with water (80 mL) and extracted with EtOAc (2 x 80 mL). The organic layer was separated washed with brine solution, dried over anhydrous Na₂SO₄ and the solvent evaporated to get dimethyl 2-methyl-2-(4-nitrophenyl)malonate(7 g, 66%; TLC system: EtOAc/PE (3:7), R*_{f}*: 0.5.)
**Step** 3: To a stirred solution of dimethyl 2-methyl-2-(4-nitrophenyl)malonate (5 g, 18.72 mmol, 1.0 eq) in methanol (50 mL) was added NaBH₄ (2.12 g, 56.17 mmol, 3.0 eq) at 0 °C and stirred at RT for 16h. The solvent was evaporated and the residue was diluted with water (50 mL). The product was extracted with EtOAc (2 x 60 mL). The combined organic layer was washed with brine (20 mL), dried over anhydrous Na₂SO₄, evaporated under reduced pressure. The crude obtained was purified by CC using EtOAc/PE (1:1) to get 2-methyl-2-(4-nitrophenyl)propane-1,3-diol (1.6 g 40%; TLC system: EtOAc/PE (1:1), R*_{f}*: 0.2).
**Step 4:** To a stirred solution of 2-methyl-2-(4-nitrophenyl)propane-1,3-diol (1.2 g, 5.68 mmol, 1.0 eq) in ethanol (30 mL) was added 10% Pd/C (300 mg) and the mixture was stirred under hydrogen gas balloon atmosphere for 5h at RT. The reaction mixture was filtered through celite pad and filtrate concentrated under vacuum to get 2-(4-aminophenyl)-2-methylpropane-1,3-diol (1 g, 97%; TLC system: chloroform /MeOH (9:1); R*_{f}*: 0.3).
**Step 5:** To a stirred solution of 2-(4-aminophenyl)-2-methylpropane-1,3-diol(1 g, 5.52 mmol, 1.0 eq) in sat. aq. NaHCO₃ (4 mL), water (2 mL) and THF (4 mL) was added phenyl chloroformate (0.76 mL, 6.08 mmol, 1.1 eq) at 0 °C and the mixture was stirred at 0 °C for 0.5 h. The mixture was diluted with water (10 mL) and extracted with EtOAc (2 x 30 mL). The combined organic layer was washed with brine (10 mL) and the solvent evaporated. The crude product was purified by CC using chloroform/MeOH (95:5) as eluent to get phenyl 4-(1,3-dihydroxy-2-methylpropan-2-yl)phenylcarbamate (1.1 g, 60%) as a white solid (TLC system: chloroform - MeOH; 9:1; R*_{f}*: 0.4).
**Step 6:** To a stirred solution of (1-(3-chlorophenyl)-3-(trifluoromethyl)-1H-pyrazol-5-yl)methanamine hydrochloride (103 mg, 0.33 mmol, 1.0 eq) in DMF (5 mL) was added TEA (0.092 mL, 0.66 mmol, 2.0 eq) followed by phenyl 4-(1,3-dihydroxy-2-methylpropan-2-yl)phenylcarbamate (100 mg, 0.33 mmol, 1.0 eq) at 0°C and the mixture was stirred for 16h at RT. The reaction mixture was concentrated under vacuum and the residue purified by neutral alumina column chromatography using MeOH/CHCl₃ (0.5: 9.5) as eluent to get 1-((1-(3-chlorophenyl)-3-(trifluoromethyl)-1H-pyrazol-5-yl)methyl)-3-(4-(1,3-dihydroxy-2-methylpropan-2-yl)phenyl)urea (105 mg; 65%) as a white solid (TLC system: MeOH/CHCl₃ (1:9), R_{f}: 0.3).

### Synthesis of example A156: 1-[[5-tert-Butyl-2-(3-chlorophenyl)-2H-pyrazol-3-yl]-methyl]-3-[3-fluoro-4-[2-hydroxy-1-(hydroxymethyl)-1-methyl-ethyl]-phenyl]-urea

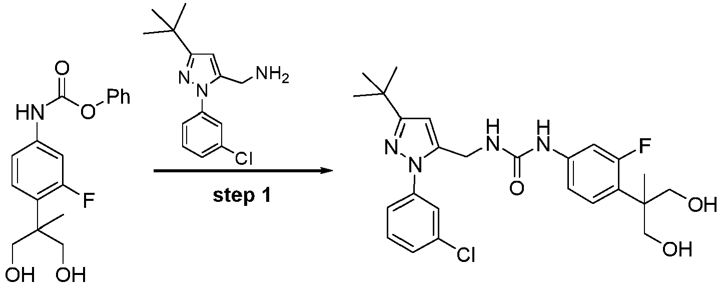

**Step 1:** To a stirred solution of (1-(3-chlorophenyl)-3-(trifluoromethyl)-1H-pyrazol-5-yl)methanamine (synthesis described for **example A154**) (80 mg, 0.31 mmol, 1.0 eq) in MeCN (7 mL) was added TEA (0.17 mL, 1.2 mmol, 4 eq) followed by phenyl 4-(1,3-dihydroxy-2-methylpropan-2-yl)-3-fluorophenylcarbamate (99 mg, 0.31 mmol, 1.0 eq) at RT and the mixture stirred for 16h at reflux. The reaction mixture was concentrated under vacuum and residue purified by CC using EtOAc/n-hexane (4:1) as eluent to get1-((3-tert-butyl-1-(3-chlorophenyl)-1H-pyrazol-5-yl)methyl)-3-(4-(1,3-dihydroxy-2-methylpropan-2-yl)-3-fluorophenyl)urea (112 mg; 75%).

### Synthesis of example A157: 1-[[5-tert-Butyl-2-(3-chlorophenyl)-2H-pyrazol-3-yl]-methyl]-3-[4-[2-hydroxy-1-(hydroxymethyl)-1-methyl-ethyl]-phenyl]-urea

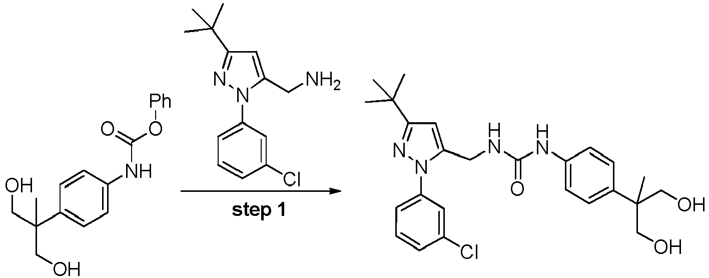

**Step 1:** To a stirred solution of (3-tert-butyl-1-(3-chlorophenyl)-1H-pyrazol-5-yl)methanamine (80 mg, 0.31 mmol, 1.0 eq) in MeCN (7 mL) was added TEA (0.17 mL, 1.2 mmol, 4 eq) followed by phenyl 4-(1,3-dihydroxy-2-methylpropan-2-yl)phenylcarbamate (94 mg, 0.31 mmol, 1 eq) and the mixture was stirred for 16 h at reflux. The reaction mixture was concentrated under vacuum and the residue was purified by CC using EtOAc/n-hexane 1:1 as eluent to get 1-((3-tert-butyl-1-(3-chlorophenyl)-1H-pyrazol-5-yl)methyl)-3-(4-(1,3-dihydroxy-2-methylpropan-2-yl)phenyl)urea (106 mg; 73%).

### Synthesis of example A158: 1-[[2-(3-Chlorophenyl)-5-(trifluoromethyl)-2H-pyrazol-3-yl]-methyl]-3-[4-[1,2-dihydroxy-1-(hydroxymethyl)-ethyl]-phenyl]-urea

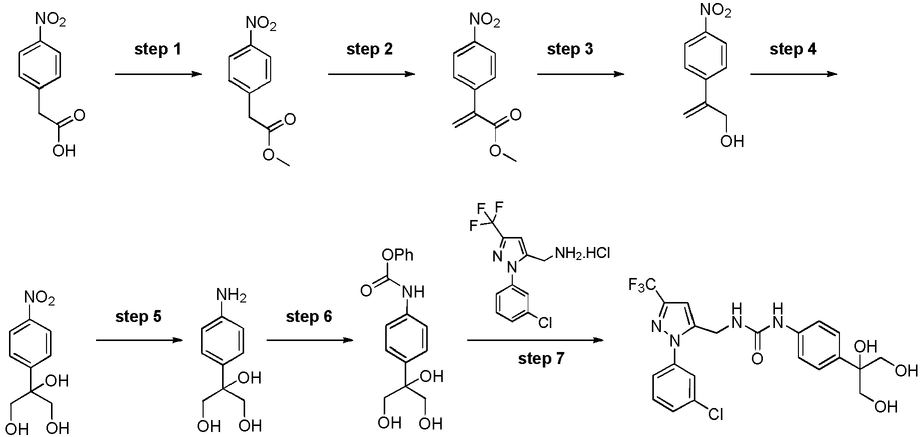

**Step** 1: To a stirred MeOH (100 mL) solution of 2-(4-nitrophenyl)acetic acid (10.0 g, 55.202 mmol, 1.0 eq) SOCl₂ (6.7 mL, 110.045 mmol, 2.0 eq) was added at 0°C for 10 min and the mixture was stirred at RT for 2 h, then excess of SOCl₂ was removed under vacuum and the residue was dissolved in EtOAC (50 mL), washed with water, sat. (aq) NaHCO₃, dried (Na₂SO₄) and the solvent was evaporated to get methyl 2-(4-nitrophenyl)acetate (10.5g, 98%, solid; TLC system: EtOAc/PE (3:7), R_{f}: 0.60).
**Step 2:** To a stirred solution of methyl 2-(4-nitrophenyl)acetate (8.0g, 40.994 mmol, 1.0 eq) in DMSO (40 mL) was added tetramethyldiaminomethane (8.38mL, 61.491 mmol, 1.5 eq) followed by acetic anhydride (12.78 mL, 135.17 mmol, 3.3eq) at RT and the mixture was stirred for 2h. The reaction mixture was diluted with water (40 mL), extracted with ether (80mL x 2) and the solvent evaporated. The crude product was purified by CC using EtOAc/PE (1:9) as eluent) to get methyl 2-(4-nitrophenyl)acrylate (4.0 g, 47%, solid; TLC system: EtOAc/PE (3:7), R_{f}: 0.60).
**Step 3:** To a stirred solution of methyl 2-(4-nitrophenyl)acrylate (4.0 g, 19.323 mmol, 1.0 eq) in ether (40 mL) was added DIBALH (20% solution in toluene) (40 mL, 57.97 mmol, 3.0 eq) at -78 °C, then the mixture was allowed to warm to 0°C over a period of 2 h, then the mixture was quenched with water (1.0 mL), NaOH solution (15%) (3 mL), extracted with ether (20mL x 2), washed with water (20 mL), brine (20 mL), dried (MgSO₄) and the solvent evaporated. The resulting residue was purified by CC using EtOAc/PE (3:7) as eluent to get 2-(4-nitrophenyl)prop-2-en-1-ol (1.5 g, 43%, oil); TLC system: EtOAc/PE (1:1), R_{f}: 0.4).
**Step 4:** To a stirred acetone (8.0 mL) solution containing water (2.0 mL) 2-(4-nitrophenyl)prop-2-en-1-ol (1.5 g, 8.379 mmol, 1.0 eq), osmium tetraoxide (2.5% solution in 2-methyl-2-propanol) (1.4 mL, 0.141 mmol, 0.017 eq) and NMO (50% aq. Solution) (4 mL, 16.73 mmol, 2 eq) were added at 0 °C and the mixture was stirred for 1 h at RT, then saturated NaHCO₃ solution in water (3 mL) was added and the mixture was extracted with ethyl acetate (30 mL), washed with water (5 mL), brine (5 mL), dried (Na₂SO₄) and the solvent evaporated. The resulting residue was purified by CC using MeOH/CHCl₃ (1:19) as eluent to get 2-(4-nitrophenyl)propane-1,2,3-triol (1.1 g, 61%, solid; TLC system: MeOH/CHCl₃ (1: 9), R_{f}: 0.5).
**Step 5:** To a stirred solution of 2-(4-nitrophenyl)propane-1,2,3-triol (1.1 g, 5.164 mmol, 1.0 eq) in ethanol (25 mL) was added 10% Pd/C (200mg) and the mixture was stirred under a H₂ gas balloon at RT for 1 h. The reaction mixture was passed through a celite bed and the solvent of the filtrate was evaporated to get 2-(4-aminophenyl)propane-1,2,3-triol (900mg, 95%, solid; TLC system: MeOH/CHCl₃ (1:9), R_{f}: 0.3).
**Step 6:** To a stirred solution of 2-(4-aminophenyl)propane-1,2,3-triol (900 mg, 4.918 mmol, 1.0 eq) in THF/H₂O/sat. NaHCO₃ (4 mL: 2 mL: 4 mL) was added phenyl chloroformate (0.68mL, 5.367 mmol, 1.1 eq) at 0 °C and the mixture was stirred at RT for 0.5 h, then the THF was evaporated, the residue was diluted with EtOAc (20 mL), washed with water (20 mL) followed by brine and the solvent evaporated. The resulting residue was purified by CC using MeOH/CHCl₃ (1:19) as eluent to get phenyl 4-(1,2,3-trihydroxypropan-2-yl)phenylcarbamate (900 mg, 60%, white solid; TLC system: MeOH/CHCl₃ (1:9), R_{f}: 0.4).
**Step 7:** To a stirred THF (10 mL) solution of (1-(3-chlorophenyl)-3-(trifluoromethyl)-1 H-pyrazol-5-yl)methanamine hydrochloride (102 mg, 0.33 mmol, 1.0 eq), TEA (0.1mL, 0.66 mmol, 2.0 eq) followed by compound phenyl 4-(1,2,3-trihydroxypropan-2-yl)phenylcarbamate (100 mg, 0.33 mmol, 1.0 eq) was added at RT and the mixture stirred for 16 h, then it was concentrated under vacuum and the residue was purified by CC using MeOH/CHCl₃ (3:17) as eluent to get 1-((1-(3-chlorophenyl)-3-(trifluoromethyl)-1H-pyrazol-5-yl)methyl)-3-(4-(1,2,3-trihydroxypropan-2-yl)phenyl)urea (120 mg; 75%, white solid; TLC system: MeOH/CHCl₃ (1:4); R_{f}: 0.3).

### Synthesis of example A159: 1-[[5-tert-Butyl-2-(3-chlorophenyl)-2H-pyrazol-3-yl]-methyl]-3-[4-[1,2-dihydroxy-1-(hydroxymethyl)-ethyl]-phenyl]-urea

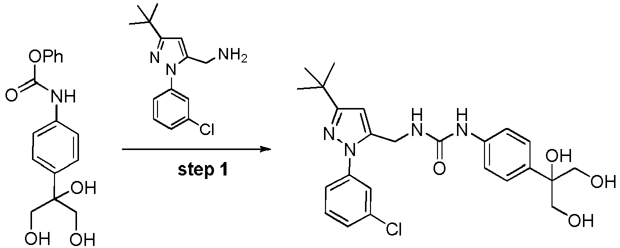

**Step** 1: To a stirred MeCN (9 mL) solution of (3-tert-butyl-1-(3-chlorophenyl)-1H-pyrazol-5-yl)methanamine (102 mg, 0.387 mmol, 1.0 eq), TEA (0.214 mL, 1.55 mmol, 4.0 eq) followed by phenyl 4-(1,2,3-trihydroxypropan-2-yl)phenylcarbamate (119 mg, 0.395 mmol, 1.02 eq) was added at RT and the mixture was stirred for 16 h at reflux, then it was concentrated under vacuum and the residue purified by CC using EtOAc as eluent to get 1-((3-tert-butyl-1-(3-chlorophenyl)-1H-pyrazol-5-yl)methyl)-3-(4-(1,2,3-trihydroxypropan-2-yl)phenyl)urea (122 mg; 67%).

Mass spectrometric data are cited hereinafter by way of example for the following exemplary compounds (Table 1):

**Table 1**

| **Exemplary compound** | **[M+H]** | **Exemplary compound** | **[M+H]** | **Exemplary compound** | **[M+H]** | **Exemplary compound** | **[M+H]** |
|---|---|---|---|---|---|---|---|
| **A1** | 448.3 | **A49** | 456.4 | **A95** | 457.1 | **A150** | 461.4 |
| **A2** | 460.2 | **A50** | 468.4 | **A96** | 447.9 | **A151** | 473.5 |
| **A3** | 446.0 | **A51** | 461.3 | **A97** | 459.8 | **A152** | 475.0 |
| **A4** | 462.4 | **A52** | 474.1 | **A98** | 458.4 | **A153** | 487.2 |
| **A5** | 480.0 | **A53** | 414.9 | **A99** | 470.4 | **A154** | 501.0 |
| **A6** | 465.9 | **A54** | 414.9 | **A100** | 470.2 | **A155** | 483.0 |
| **A7** | 477.9 | **A55** | 461.5 | **A101** | 482.0 | **A156** | 489.2 |
| **A8** | 461.2 | **A56** | 411.1 | **A102** | 466.0 | **A157** | 471.2 |
| **A9** | 491.9 | **A57** | 433.2 | **A103** | 478.0 | **A158** | 484.9 |
| **A10** | 459.9 | **A58** | 409.1 | **A104** | 483.9 | **A159** | 473.0 |
| **A11** | 435.2 | **A59** | 443.1 | **A105** | 457.2 | **A26** | 469.1 |
| **A12** | 447.1 | **A60** | 443.1 | **A106** | 469.1 | **A46** | 440.1 |
| **A13** | 443.3 | **A61** | 427.1 | **A107** | 455.2 | **A47** | 478.0 |
| **A14** | 455.3 | **A62** | 426.9 | **A108** | 467.1 | **A48** | 489.9 |
| **A15** | 441.0 | **A63** | 427.1 | **A109** | 473.2 | **A124** | 472.2 |
| **A16** | 441.0 | **A64** | 444.9 | **A110** | 485.1 | **A125** | 538.2 |
| **A17** | 475.2 | **A65** | 461.1 | **A111** | 513.9 | **A126** | 502.1 |
| **A18** | 486.9 | **A66** | 445.1 | **A112** | 532.1 | **A134** | 459.2 |
| **A19** | 461.1 | **A67** | 438.9 | **A113** | 486.1 | **A135** | 511.2 |
| **A20** | 473.0 | **A68** | 439.1 | **A114** | 496.2 | **A142** | 440.9 |
| **A21** | 457.1 | **A69** | 423.1 | **A115** | 508.2 | **A143** | 452.8 |
| **A22** | 490.9 | **A70** | 423.1 | **A116** | 472.2 | **A144** | 437.1 |
| **A23** | 484.9 | **A71** | 423.1 | **A117** | 488.2 | **A149** | 470.1 |
| **A24** | 491.4 | **A72** | 437.2 | **A118** | 499.9 | | |
| **A25** | 503.2 | **A73** | 437.2 | **A119** | 500.1 | | |
| **A27** | 413.3 | **A74** | 437.2 | **A120** | 500.1 | | |
| **A28** | 425.1 | **A75** | 437.2 | **A121** | 518.2 | | |
| **A29** | 430.3 | **A76** | 441.1 | **A122** | 484.2 | | |
| **A30** | 442.3 | **A77** | 441.1 | **A123** | 480.2 | | |
| **A31** | 428.4 | **A78** | 457.1 | **A127** | 487.1 | | |
| **A32** | 444.5 | **A79** | 467.2 | **A128** | 501.1 | | |
| **A33** | 456.2 | **A80** | 441.1 | **A129** | 475.1 | | |
| **A34** | 456.0 | **A81** | 448.1 | **A130** | 505.1 | | |
| **A35** | 456.0 | **A82** | 451.2 | **A131** | 470.5 | | |
| **A36** | 472.8 | **A83** | 459.1 | **A132** | 456.0 | | |
| **A37** | 427.9 | **A84** | 453.2 | **A133** | 459.0 | | |
| **A38** | 439.8 | **A85** | 434.1 | **A136** | 454.1 | | |
| **A39** | 446.0 | **A86** | 452.2 | **A137** | 477.9 | | |
| **A40** | 457.9 | **A87** | 453.2 | **A138** | 470.3 | | |
| **A41** | 462.3 | **A88** | 467.2 | **A139** | 458.5 | | |
| **A42** | 474.2 | **A89** | 477.1 | **A140** | 445.1 | | |
| **A43** | 451.9 | **A90** | 473.1 | **A141** | 457.0 | | |
| **A44** | 463.9 | **A91** | 473.1 | **A145** | 474.5 | | |
| **A45** | 428.0 | **A92** | 453.2 | **A146** | 468.3 | | |
| **A47** | 478.0 | **A93** | 467.2 | **A147** | 456.4 | | |
| **A48** | 489.9 | **A94** | 453.2 | **A148** | 486.3 | | |

### Pharmacological methods

### I. Functional testing carried out on the vanilloid receptor 1 (VRI/TRPV1 receptor)

The agonistic or antagonistic effect of the substances to be tested on the rat-species vanilloid receptor 1 (VR1/TRPV1) can be determined using the following assay. In this assay, the influx of Ca²⁺ through the receptor channel is quantified with the aid of a Ca²⁺-sensitive dye (type Fluo-4, Molecular Probes Europe BV, Leiden, the Netherlands) in a fluorescent imaging plate reader (FLIPR, Molecular Devices, Sunnyvale, USA).

### Method:

Complete medium: 50 mL HAMS F12 nutrient mixture (Gibco Invitrogen GmbH, Karlsruhe, Germany) with 10 % by volume of FCS (foetal calf serum, Gibco Invitrogen GmbH, Karlsruhe, Germany, heat-inactivated); 2mM L-glutamine (Sigma, Munich, Germany); 1 % by weight of AA solution (antibiotic/antimyotic solution, PAA, Pasching, Austria) and 25 ng/mL NGF medium (2.5 S, Gibco Invitrogen GmbH, Karlsruhe, Germany)

Cell culture plate: Poly-D-lysine-coated, black 96-well plates having a clear base (96-well black/clear plate, BD Biosciences, Heidelberg, Germany) are additionally coated with laminin (Gibco Invitrogen GmbH, Karlsruhe, Germany), the laminin being diluted with PBS (Ca-Mg-free PBS, Gibco Invitrogen GmbH, Karlsruhe, Germany) to a concentration of 100 µg/mL. Aliquots having a laminin concentration of 100 µg/mL are removed and stored at -20 °C. The aliquots are diluted with PBS in a ratio of 1:10 to 10 µg/mL of laminin and respectively 50 µL of the solution are pipetted into a recess in the cell culture plate. The cell culture plates are incubated for at least two h at 37 °C, the excess solution is removed by suction and the recesses are each washed twice with PBS. The coated cell culture plates are stored with excess PBS which is not removed until just before the feeding of the cells.

### Preparation of the cells:

The vertebral column is removed from decapitated rats and placed immediately into cold HBSS buffer (Hank's buffered saline solution, Gibco Invitrogen GmbH, Karlsruhe, Germany), i.e. buffer located in an ice bath, mixed with 1 % by volume (per cent by volume) of an AA solution (antibiotic/antimyotic solution, PAA, Pasching, Austria). The vertebral column is cut longitudinally and removed together with fasciae from the vertebral canal. Subsequently, the dorsal root ganglia (DRG) are removed and again stored in cold HBSS buffer mixed with 1 % by volume of an AA solution. The DRG, from which all blood remnants and spinal nerves have been removed, are transferred in each case to 500 µL of cold type 2 collagenase (PAA, Pasching, Austria) and incubated for 35 minutes at 37 °C. After the addition of 2.5 % by volume of trypsin (PAA, Pasching, Austria), incubation is continued for 10 minutes at 37 °C. After complete incubation, the enzyme solution is carefully pipetted off and 500 µL of complete medium are added to each of the remaining DRG. The DRG are respectively suspended several times, drawn through cannulae No. 1, No. 12 and No. 16 using a syringe and transferred to a 50 mL Falcon tube which is filled up to 15 mL with complete medium. The contents of each Falcon tube are respectively filtered through a 70 µm Falcon filter element and centrifuged for 10 minutes at 1,200 rpm and room temperature. The resulting pellet is respectively taken up in 250 µL of complete medium and the cell count is determined.

The number of cells in the suspension is set to 3 x 10⁵ per mL and 150 µL of this suspension are in each case introduced into a recess in the cell culture plates coated as described hereinbefore. In the incubator the plates are left for two to three days at 37 °C, 5 % by volume of CO₂ and 95 % relative humidity. Subsequently, the cells are loaded with 2 µM of Fluo-4 and 0.01 % by volume of Pluronic F127 (Molecular Probes Europe BV, Leiden, the Netherlands) in HBSS buffer (Hank's buffered saline solution, Gibco Invitrogen GmbH, Karlsruhe, Germany) for 30 min at 37 °C, washed 3 times with HBSS buffer and after further incubation for 15 minutes at room temperature used for Ca²⁺ measurement in a FLIPR assay. The Ca²⁺-dependent fluorescence is in this case measured before and after the addition of substances (λex = 488 nm, λem = 540 nm). Quantification is carried out by measuring the highest fluorescence intensity (FC, fluorescence counts) over time.

### FLIPR assay:

The FLIPR protocol consists of 2 substance additions. First the compounds to be tested (10 µM) are pipetted onto the cells and the Ca²⁺ influx is compared with the control (capsaicin 10 µM). This provides the result in % activation based on the Ca²⁺ signal after the addition of 10 µM of capsaicin (CP). After 5 minutes' incubation, 100 nM of capsaicin are applied and the Ca²⁺ influx is also determined.

Desensitising agonists and antagonists lead to suppression of the Ca²⁺ influx. The % inhibition is calculated compared to the maximum achievable inhibition with 10 µM of capsazepine.

Triple analyses (n=3) are carried out and repeated in at least 3 independent experiments (N=4).

Starting from the percentage displacement caused by different concentrations of the compounds to be tested of general formula I, IC₅₀ inhibitory concentrations which cause a 50-% displacement of capsaicin were calculated. Kᵢ values for the test substances were obtained by conversion by means of the Cheng-Prusoff equation (Cheng, Prusoff; Biochem. Pharmacol. 22, 3099-3108, 1973).

### II. Functional testing carried out on the vanilloid receptor 1 (VRI/TRPV1 receptor)

The agonistic or antagonistic effect of the substances to be tested on the vanilloid receptor 1 (VR1) can also be determined using the following assay. In this assay, the influx of Ca²⁺ through the channel is quantified with the aid of a Ca²⁺-sensitive dye (type Fluo-4, Molecular Probes Europe BV, Leiden, the Netherlands) in a fluorescent imaging plate reader (FLIPR, Molecular Devices, Sunnyvale, USA).

### Method:

Chinese hamster ovary cells (CHO K1 cells, European Collection of Cell Cultures (ECACC) United Kingdom) are stably transfected with the VR1 gene. For functional testing, these cells are plated out on poly-D-lysine-coated black 96-well plates having a clear base (BD Biosciences, Heidelberg, Germany) at a density of 25,000 cells/well. The cells are incubated overnight at 37 °C and 5 % CO₂ in a culture medium (Ham's F12 nutrient mixture, 10 % by volume of FCS (foetal calf serum), 18 µg/mL of L-proline). The next day the cells are incubated with Fluo-4 (Fluo-4 2 µM, 0.01 % by volume of Pluronic F127, Molecular Probes in HBSS (Hank's buffered saline solution), Gibco Invitrogen GmbH, Karlsruhe, Germany) for 30 minutes at 37 °C. Subsequently, the plates are washed three times with HBSS buffer and after further incubation for 15 min. at RT used for Ca²⁺ measurement in a FLIPR assay. The Ca²⁺-dependent fluorescence is measured before and after the addition of the substances to be tested (λex wavelength = 488 nm, λem = 540 nm). Quantification is carried out by measuring the highest fluorescence intensity (FC, fluorescence counts) over time.

### FLIPR assay:

The FLIPR protocol consists of 2 substance additions. First the compounds to be tested (10 µM) are pipetted onto the cells and the Ca²⁺ influx is compared with the control (capsaicin 10 µM) (% activation based on the Ca²⁺ signal after the addition of 10 µM of capsaicin). After 5 minutes' incubation, 100 nM of capsaicin are applied and the Ca²⁺ influx is also determined.

Desensitising agonists and antagonists led to suppression of the Ca²⁺ influx. The % inhibition is calculated compared to the maximum achievable inhibition with 10 µM of capsazepine.

Starting from the percentage displacement caused by different concentrations of the compounds to be tested of general formula I, IC₅₀ inhibitory concentrations which cause a 50-per cent displacement of capsaicin were calculated. Kᵢ values for the test substances were obtained by conversion by means of the Cheng-Prusoff equation (Cheng, Prusoff; Biochem. Pharmacol. 22, 3099-3108, 1973).

### Pharmacological data

The affinity of the compounds according to the invention for the vanilloid receptor 1 (VR1/TRPV1 receptor) was determined as described hereinbefore (pharmacological method I or II).

The compounds according to the invention display outstanding affinity to the VR1/TRPV1 receptor (Tables 2 and 3).

In Tables 2 and 3 the abbreviations below have the following meanings:
Cap = capsaicin
AG = agonist

The value after the "@"symbol indicates the concentration at which the inhibition (as a percentage) was respectively determined.

**Table 2**

| **Exemplary compound** | ***(f)* Ki (human being) [nM] Cap** | **Exemplary compound** | ***(f)* Ki (human being) [nM] Cap** |
|---|---|---|---|
| **A1** | 12 | **A53** | 18 |
| **A2** | 33 | **A54** | 6 |
| **A3** | 6 | **A55** | 3 |
| **A4** | 2 | **A56** | 2 |
| **A5** | AG | **A57** | 2 |
| **A6** | 14 | **A58** | 6 |
| **A7** | 26 | **A59** | 7 |
| **A8** | 55 | **A60** | 10 |
| **A9** | 7 | **A61** | 26 |
| **A10** | 1 | **A62** | 30 |
| **A11** | AG | **A63** | 9 |
| **A12** | 8 | **A64** | 26 |
| **A13** | AG | **A65** | 6 |
| **A14** | 25 | **A66** | 9 |
| **A15** | AG | **A67** | 43 |
| **A16** | AG | **A68** | 13 |
| **A17** | AG | **A69** | 45 |
| **A18** | 68 | **A70** | 9 |
| **A19** | 13 | **A71** | 42 |
| **A20** | 37 | **A72** | 14 |
| **A21** | 7 | **A73** | 27 |
| **A22** | 40 | **A74** | 44 |
| **A23** | 35 | **A75** | 19 |
| **A24** | AG | **A76** | 41 |
| **A25** | 65 | **A77** | 19 |
| **A29** | 1 | **A78** | 11 |
| **A30** | 21 | **A79** | 20 |
| **A31** | 3 | **A80** | 7 |
| **A32** | AG | **A81** | 21 |
| **A33** | 2 | **A82** | 2 |
| **A34** | 37 | **A83** | 12 |
| **A35** | 1 | **A84** | 53 |
| **A36** | 0.3 | **A85** | 53% @ 5µM |
| **A37** | AG | **A86** | 25 |
| **A38** | 11 | **A87** | 63 |
| **A39** | AG | **A88** | 86 |
| **A40** | 27 | **A89** | 45 |
| **A41** | AG | **A90** | 27 |
| **A42** | 3 | **A91** | 51 |
| **A43** | 21 | **A92** | 19 |
| **A44** | 0.2 | **A93** | 18 |
| **A45** | 1 | **A94** | 30 |
| **A47** | AG | **A95** | 13 |
| **A48** | AG | **A96** | 2 |
| **A49** | AG | **A97** | 11 |
| **A50** | 1 | **A98** | AG |
| **A51** | AG | **A99** | 6 |
| **A52** | 140 | **A100** | 0.7 |

**Table 3**

| **Exemplary compound** | ***(f)* Ki (human being) [nM] Cap** | **Exemplary compound** | ***(f)* Ki (human being) [nM] Cap** |
|---|---|---|---|
| **A101** | 4 | **A158** | 13% @ 5µM |
| **A102** | 15 | **A159** | 24% @ 5µM |
| **A103** | 22 | **A27** | 1 |
| **A104** | 12 | **A28** | 22 |
| **A105** | 19 | **A26** | 34% @ 1µM |
| **A106** | 31 | **A46** | 46 |
| **A107** | 45 | **A124** | 33% @ 1µM |
| **A108** | 48 | **A125** | 15 |
| **A109** | 43 | **A126** | 79 |
| **A110** | 48 | **A134** | 23% @ 1µM |
| **A111** | 38 | **A135** | 15% @ 1µM |
| **A112** | 79 | | |
| **A113** | 63 | | |
| **A114** | 46 | | |
| **A115** | 3 | | |
| **A116** | 18 | | |
| A**117** | 9 | | |
| **A118** | 27 | | |
| **A119** | 57 | | |
| **A120** | 5 | | |
| **A121** | 20 | | |
| **A122** | 45 | | |
| **A123** | 24 | | |
| **A127** | 40 | | |
| **A128** | 67 | | |
| **A129** | 43 | | |
| **A130** | 47 | | |
| **A131** | 56 | | |
| **A132** | 48% @ 5µM | | |
| **A133** | 12 | | |
| **A136** | 40 | | |
| **A137** | 0.6 | | |
| **A138** | 8 | | |
| **A139** | AG | | |
| **A140** | AG | | |
| **A141** | 26 | | |
| **A145** | AG | | |
| **A146** | 13 | | |
| **A147** | AG | | |
| **A148** | 4 | | |
| **A150** | 19 | | |
| **A151** | 62 | | |
| **A152** | 39% @ 5µM | | |
| **A153** | 50% @ 5µM | | |
| **A154** | 117 | | |
| **A155** | 26% @ 5µM | | |
| **A156** | 84 | | |
| **A157** | 35% @ 5µM | | |

## Claims

1. A compound selected from the group consisting of:
**A27** 1-[[5-tert-Butyl-2-(3-chlorophenyl)-2H-pyrazol-3-yl]-methyl]-3-[4-(hydroxymethyl)-phenyl]-urea,
**A54** 1-[[5-tert-Butyl-2-(3-fluorophenyl)-2H-pyrazol-3-yl]-methyl]-3-[3-fluoro-4-(hydroxymethyl)-phenyl]-urea,
**A55** 1-[[5-tert-Butyl-2-(3-chlorophenyl)-2H-pyrazol-3-yl]-methyl]-3-[3-fluoro-4-(hydroxymethyl)-phenyl]-urea,
**A56** 1-[[5-tert-Butyl-2-(m-tolyl)-2H-pyrazol-3-yl]-methyl]-3-[3-fluoro-4-(hydroxymethyl)-phenyl]-urea,
**A57** 1-[[5-tert-Butyl-2-(3,4-difluoro-phenyl)-2H-pyrazol-3-yl]-methyl]-3-[3-fluoro-4-(hydroxymethyl)-phenyl]-urea,
**A58** 1-[3-Fluoro-4-(hydroxymethyl)-phenyl]-3-[[2-phenyl-5-(trifluoromethyl)-2H-pyrazol-3-yl]-methyl]-urea,
**A59** 1-[[2-(3-Chlorophenyl)-5-(trifluoromethyl)-2H-pyrazol-3-yl]-methyl]-3-[3-fluoro-4-(hydroxymethyl)-phenyl]-urea,
**A63** 1-[3-Fluoro-4-(hydroxymethyl)-phenyl]-3-[[2-(4-fluorophenyl)-5-(trifluoromethyl)-2H-pyrazol-3-yl]-methyl]-urea,
**A65** 1-[[2-(3-Chloro-4-fluoro-phenyl)-5-(trifluoromethyl)-2H-pyrazol-3-yl]-methyl]-3-[3-fluoro-4-(hydroxymethyl)-phenyl]-urea,
**A66** 1-[[2-(3,5-Difluoro-phenyl)-5-(trifluoromethyl)-2H-pyrazol-3-yl]-methyl]-3-[3-fluoro-4-(hydroxymethyl)-phenyl]-urea,
**A70** 1-[3-Fluoro-4-(hydroxymethyl)-phenyl]-3-[[2-(m-tolyl)-5-(trifluoromethyl)-2H-pyrazol-3-yl]-methyl]-urea,
**A80** 1-[3-Fluoro-4-(hydroxymethyl)-phenyl]-3-[[2-(4-fluoro-3-methyl-phenyl)-5-(trifluoromethyl)-2H-pyrazol-3-yl]-methyl]-urea,
**A82** 1-[3-Fluoro-4-(hydroxymethyl)-phenyl]-3-[[2-(3-isopropyl-phenyl)-5-(trifluoromethyl)-2H-pyrazol-3-yl]-methyl]-urea, and
**A96** 1-[[5-tert-Butyl-2-(3-chloroph enyl)-2H-pyrazol-3-yl]-methyl]-3-[3-chloro-4-(hydroxymethyl)-phenyl]-urea,
optionally in the form of a single stereoisomer or a mixture of stereoisomers, in the form of the free compound and/or a physiologically acceptable salt thereof.

2. A pharmaceutical composition comprising at least one compound according to claim 1.

3. A compound according to claim 1 for use in the treatment and/or prophylaxis of one or more diseases and/or disorders selected from the group consisting of:
pain, preferably of pain selected from the group consisting of acute pain, chronic pain, neuropathic pain, visceral pain and joint pain; hyperalgesia; allodynia; causalgia; migraine; depression; nervous affection; axonal injuries; neurodegenerative diseases, preferably selected from the group consisting of multiple sclerosis, Alzheimer's disease, Parkinson's disease and Huntington's disease; cognitive dysfunctions, preferably cognitive deficiency states, particularly preferably memory disorders; epilepsy; respiratory diseases, preferably selected from the group consisting of asthma, bronchitis and pulmonary inflammation; coughs; urinary incontinence; overactive bladder (OAB); disorders and/or injuries of the gastrointestinal tract; duodenal ulcers; gastric ulcers; irritable bowel syndrome; strokes; eye irritations; skin irritations; neurotic skin diseases; allergic skin diseases; psoriasis; vitiligo; herpes simplex; inflammations, preferably inflammations of the intestine, the eyes, the bladder, the skin or the nasal mucous membrane; diarrhoea; pruritus; osteoporosis; arthritis; osteoarthritis; rheumatic diseases; eating disorders, preferably selected from the group consisting of bulimia, cachexia, anorexia and obesity; medication dependency; misuse of medication; withdrawal symptoms in medication dependency; development of tolerance to medication, preferably to natural or synthetic opioids; drug dependency; misuse of drugs; withdrawal symptoms in drug dependency; alcohol dependency; misuse of alcohol and withdrawal symptoms in alcohol dependency; for diuresis; for antinatriuresis; for influencing the cardiovascular system; for increasing vigilance; for the treatment of wounds and/or burns; for the treatment of severed nerves; for increasing libido; for modulating movement activity; for anxiolysis; for local anaesthesia and/or for inhibiting undesirable side effects, preferably selected from the group consisting of hyperthermia, hypertension and bronchoconstriction, triggered by the administration of vanilloid receptor 1 (VR1/TRPV1 receptor) agonists, preferably selected from the group consisting of capsaicin, resiniferatoxin, olvanil, arvanil, SDZ-249665, SDZ-249482, nuvanil and capsavanil.

## Patentansprüche

1. Eine Verbindung, ausgewählt aus der Gruppe bestehend aus:
**A27** 1-[[5-tert-Butyl-2-(3-chlorphenyl)-2H-pyrazol-3-yl]-methyl]-3-[4-(hydroxymethyl)-phenyl]-harnstoff,
**A54** 1-[[5-tert-Butyl-2-(3-fluorphenyl)-2H-pyrazol-3-yl]-methyl]-3-[3-fluor-4-(hydroxymethyl)-phenyl]-harnstoff,
**A55** 1-[[5-tert-Butyl-2-(3-chlorphenyl)-2H-pyrazol-3-yl]-methyl]-3-[3-fluor-4-(hydroxymethyl)-phenyl]-harnstoff,
**A56** 1-[[5-tert-Butyl-2-(m-tolyl)-2H-pyrazol-3-yl]-methyl]-3-[3-fluor-4-(hydroxymethyl)-phenyl]-harnstoff,
**A57** 1-[[5-tert-Butyl-2-(3,4-difluorphenyl)-2H-pyrazol-3-yl]-methyl]-3-[3-fluor-4-(hydroxymethyl)-phenyl]-harnstoff,
**A58** 1-[3-Fluor-4-(hydroxymethyl)-phenyl]-3-[[2-phenyl-5-(trifluormethyl)-2H-pyrazol-3-yl]-methyl]-harnstoff,
**A59** 1-[[2-(3-Chlorphenyl)-5-(trifluormethyl)-2H-pyrazol-3-yl]-methyl]-3-[3-fluor-4-(hydroxymethyl)-phenyl]-harnstoff,
**A63** 1-[3-Fluor-4-(hydroxymethyl)-phenyl]-3-[[2-(4-fluorphenyl)-5-(trifluormethyl)-2H-pyrazol-3-yl]-methyl]-harnstoff,
**A65** 1-[[2-(3-Chlor-4-fluorphenyl)-5-(trifluormethyl)-2H-pyrazol-3-yl]-methyl]-3-[3-fluor-4-(hydroxymethyl)-phenyl]-harnstoff,
**A66** 1-[[2-(3,5-Difluorphenyl)-5-(trifluormethyl)-2H-pyrazol-3-yl]-methyl]-3-[3-fluor-4-(hydroxymethyl)-phenyl]-harnstoff,
**A70** 1-[3-Fluor-4-(hydroxymethyl)-phenyl]-3-[[2-(m-tolyl)-5-(trifluormethyl)-2H-pyrazol-3-yl]-methyl]-harnstoff,
**A80** 1-[3-Fluor-4-(hydroxymethyl)-phenyl]-3-[[2-(4-fluor-3-methyl-phenyl)-5-(trifluormethyl)2H-pyrazol-3-yl]-methyl]-harnstoff,
**A82** 1-[3-Fluor-4-(hydroxymethyl)-phenyl]-3-[[2-(3-isopropyl-phenyl)-5-(trifluormethyl)-2H-pyrazol-3-yl]-methyl]-harnstoff, und
**A96** 1-[[5-tert-Butyl-2-(3-chlorphenyl)-2H-pyrazol-3-yl]-methyl]-3-[3-chlor-4-(hydroxymethyl)-phenyl]-harnstoff,
gegebenenfalls in Form eines einzelnen Stereoisomers oder einer Mischung von Stereoisomeren, in Form der freien Verbindung und/oder eines physiologisch verträglichen Salzes davon.

2. Pharmazeutische Zusammensetzung, die mindestens eine Verbindung nach Anspruch 1 enthält.

3. Verbindung gemäß Anspruch 1 zur Verwendung bei der Behandlung und/oder Prophylaxe von einer oder mehreren Krankheiten und/oder Störungen ausgewählt aus der Gruppe bestehend aus:
Schmerzen, bevorzugt von Schmerzen, die aus der Gruppe ausgewählt werden, die aus akuten Schmerzen, chronischen Schmerzen, neuropathischen Schmerzen, viszeralen Schmerzen und Gelenkschmerzen besteht; Hyperalgesie; Allodynie; Kausalgie; Migräne; Depression; Nervenschädigungen; axonale Verletzungen; neurodegenerative Erkrankungen, bevorzugt ausgewählt aus der Gruppe bestehend aus Multipler Sklerose, Alzheimer, Parkinson und Huntington; kognitive Dysfunktionen, bevorzugt kognitive Mangelzustände, besonders bevorzugt Gedächtnisstörungen; Epilepsie; Erkrankungen der Atemwege, bevorzugt ausgewählt aus der Gruppe bestehend aus Asthma, Bronchitis und Lungenentzündung; Husten; Harninkontinenz; überaktive Blase (OAB); Störungen und/oder Verletzungen des Magen-Darm-Traktes; Zwölffingerdarmgeschwüre; Magengeschwüre; Reizdarmsyndrom; Schlaganfälle; Augenreizungen; Hautreizungen; neurotische Hauterkrankungen; allergische Hauterkrankungen; Psoriasis; Vitiligo; Herpes simplex; Entzündungen, bevorzugt Entzündungen des Darms, der Augen, der Blase, der Haut oder der Nasenschleimhaut; Durchfall; Juckreiz; Osteoporose; Arthritis; Osteoarthritis; rheumatische Erkrankungen; Essstörungen, bevorzugt ausgewählt aus der Gruppe bestehend aus Bulimie, Kachexie, Anorexie und Adipositas; Medikamentenabhängigkeit; Medikamentenmissbrauch; Entzugserscheinungen bei Medikamentenabhängigkeit; Entwicklung einer Toleranz gegenüber Medikamenten, bevorzugt gegenüber natürlichen oder synthetischen Opioiden; Drogenabhängigkeit; Drogenmissbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des Herz-Kreislauf-Systems; zur Erhöhung der Wachsamkeit; zur Behandlung von Wunden und/oder Verbrennungen; zur Behandlung durchtrennter Nerven; zur Steigerung der Libido; zur Modulation der Bewegungsaktivität; zur Anxiolyse; zur Lokalanästhesie und/oder zur Hemmung unerwünschter Nebenwirkungen, bevorzugt ausgewählt aus der Gruppe bestehend aus Hyperthermie, Bluthochdruck und Bronchokonstriktion, ausgelöst durch die Verabreichung von Agonisten des Vanilloidrezeptors 1 (VR1/TRPV1-Rezeptor), bevorzugt ausgewählt aus der Gruppe bestehend aus Capsaicin, Resiniferatoxin, Olvanil, Arvanil, SDZ-249665, SDZ-249482, Nuvanil und Capsavanil.

## Revendications

1. Composé choisi dans le groupe constitué par :
**A27** 1-[[5-tert-Butyl-2-(3-chlorophényl)-2H-pyrazol-3-yl]-méthyl]-3-[4-(hydroxyméthyl)-phényl]-urée,
**A54** 1-[[5-tert-Butyl-2-(3-fluorophényl)-2H-pyrazol-3-yl]-méthyl]-3-[3-fluoro-4-(hydroxyméthyl)-phényl]-urée,
**A55** 1-[[5-tert-Butyl-2-(3-chlorophényl)-2H-pyrazol-3-yl]-méthyl]-3-[3-fluoro-4-(hydroxyméthyl)-phényl]-urée,
**A56** 1-[[5-tert-Butyl-2-(m-tolyl)-2H-pyrazol-3-yl]-méthyl]-3-[3-fluoro-4-(hydroxyméthyl)-phényl]-urée,
**A57** 1-[[5-tert-Butyl-2-(3,4-difluoro-phényl)-2H-pyrazol-3-yl]-méthyl]-3-[3-fluoro-4-(hydroxyméthyl)-phényl]-urée,
**A58** 1-[3-Fluoro-4-(hydroxyméthyl)-phényl]-3-[[2-phényl-5-(trifluorométhyl)-2H-pyrazol-3-yl]-méthyl]-urée,
**A59** 1-[[2-(3-Chlorophényl)-5-(trifluorométhyl)-2H-pyrazol-3-yl]-méthyl]-3-[3-fluoro-4-(hydroxyméthyl)-phényl]-urée,
**A63** 1-[3-Fluoro-4-(hydroxyméthyl)-phényl]-3-[[2-(4-fluorophényl)-5-(trifluorométhyl)-2H-pyrazol-3-yl]-méthyl]-urée,
**A65** 1-[[2-(3-Chloro-4-fluoro-phényl)-5-(trifluorométhyl)-2H-pyrazol-3-yl]-méthyl]-3-[3-fluoro-4-(hydroxyméthyl)-phényl]-urée,
**A66** 1-[[2-(3,5-Difluoro-phényl)-5-(trifluorométhyl)-2H-pyrazol-3-yl]-méthyl]-3-[3-fluoro-4-(hydroxyméthyl)-phényl]-urée,
**A70** 1-[3-Fluoro-4-(hydroxyméthyl)-phényl]-3-[[2-(m-tolyl)-5-(trifluorométhyl)-2H-pyrazol-3-yl]-méthyl]-urée,
**A80** 1-[3-Fluoro-4-(hydroxyméthyl)-phényl]-3-[[2-(4-fluoro-3-méthyl-phényl)-5-(trifluorométhyl)-2H-pyrazol-3-yl]-méthyl]-urée,
**A82** 1-[3-Fluoro-4-(hydroxyméthyl)-phényl]-3-[[2-(3-isopropyl-phényl)-5-(trifluorométhyl)-2H-pyrazol-3-yl]-méthyl]-urée, et
**A96** 1-[[5-tert-Butyl-2-(3-chlorophényl)-2H-pyrazol-3-yl]-méthyl]-3-[3-chloro-4-(hydroxyméthyl)-phényl]-urée, éventuellement sous la forme d'un stéréoisomère unique ou d'un mélange de stéréoisomères, sous la forme du composé libre et/ou d'un sel physiologiquement acceptable de celui-ci.

2. Composition pharmaceutique comprenant au moins un composé selon la revendication 1.

3. Composé selon la revendication 1 pour utilisation dans le traitement prophylactique et/ou thérapeutique d'un ou de plusieurs troubles et/ou maladies choisis dans le groupe constitué par les suivants :
douleur, préférentiellement douleur choisie dans le groupe constitué par la douleur aiguë, la douleur chronique, la douleur neuropathique, la douleur viscérale et la douleur articulaire ; hyperalgésie ; allodynie ; causalgie ; migraine ; dépression ; affection nerveuse ; lésions axonales ; maladies neurodégénératives, préférentiellement choisies dans le groupe constitué par la sclérose en plaques, la maladie d'Alzheimer, la maladie de Parkinson et la chorée de Huntington ; dysfonctionnements cognitifs, préférentiellement les états de déficience cognitive, particulièrement préférentiellement les troubles de la mémoire ; épilepsie ; maladies respiratoires, préférentiellement choisies dans le groupe constitué par l'asthme, la bronchite et l'inflammation pulmonaire ; toux ; incontinence urinaire ; vessie hyperactive ; troubles et/ou lésions du tractus gastrointestinal ; ulcères duodénaux ; ulcères gastriques ; syndrome du côlon irritable ; accidents vasculaires cérébraux ; irritations oculaires ; irritations cutanées ; maladies névrotiques cutanées ; maladies allergiques cutanées ; psoriasis ; vitiligo ; herpès simplex ; inflammations, préférentiellement inflammations de l'intestin, des yeux, de la vessie, de la peau ou de la muqueuse nasale ; diarrhée ; prurit ; ostéoporose ; arthrite ; arthrose ; maladies rhumatoïdes ; troubles de l'alimentation, préférentiellement choisis dans le groupe constitué par la boulimie, la cachexie, l'anorexie et l'obésité ; dépendance aux médicaments ; utilisation abusive de médicaments ; symptômes de manque dans la dépendance aux médicaments ; développement d'une tolérance aux médicaments, préférentiellement aux opioïdes naturels ou synthétiques ; dépendance aux drogues ; utilisation abusive de drogues ; symptômes de manque dans la dépendance aux drogues ; dépendance à l'alcool ; utilisation abusive d'alcool et symptômes de manque dans la dépendance à l'alcool ; pour la diurèse ; pour l'anti-natriurèse ; pour l'influence sur le système cardiovasculaire ; pour l'augmentation de la vigilance ; pour le traitement des blessures et/ou des brûlures ; pour le traitement des nerfs amputés ; pour l'augmentation de la libido ; pour la modulation de l'activité de mouvement ; pour l'anxiolyse ; pour l'anesthésie locale et/ou pour l'inhibition des effets secondaires indésirables, préférentiellement choisis dans le groupe constitué par l'hyperthermie, l'hypertension et la bronchoconstriction, déclenchés par l'administration d'agonistes du récepteur vanilloïde 1 (récepteur VR1/TRPV1), préférentiellement choisis dans le groupe constitué par la capsaïcine, la résinifératoxine, l'olvanil, l'arvanil, SDZ-249665, SDZ-249482, le nuvanil et le capsavanil.
